(19) **Europäisches Patentamt / European Patent Office / Office européen des brevets**

(11) **EP 1 576 169 B1**

(12) **EUROPEAN PATENT SPECIFICATION**

(45) Date of publication and mention of the grant of the patent:
**10.08.2016 Bulletin 2016/32**

(21) Application number: **03726083.3**

(22) Date of filing: **19.03.2003**

(51) Int Cl.:
**A61K 39/395** (2006.01)     **A61P 35/00** (2006.01)

(86) International application number:
**PCT/US2003/008490**

(87) International publication number:
**WO 2003/080856 (02.10.2003 Gazette 2003/40)**

(54) **THERAPEUTIC POLYPEPTIDES AND METHODS OF USE**

THERAPEUTISCHE POLYPEPTIDE UND VERWENDUNGSVERFAHREN

POLYPEPTIDES THERAPEUTIQUES ET METHODES D'UTILISATION ASSOCIEES

(84) Designated Contracting States:
**AT BE BG CH CY CZ DE DK EE ES FI FR GB GR HU IE IT LI LU MC NL PT RO SE SI SK TR**

(30) Priority: **19.03.2002 US 365491 P**
**13.09.2002 US 410618 P**

(43) Date of publication of application:
**21.09.2005 Bulletin 2005/38**

(73) Proprietor: **Celldex Therapeutics, Inc.**
**Hampton, NJ 08827 (US)**

(72) Inventors:
- **LEPLEY, Denise, M.**
  **Branford, CT 06405 (US)**
- **RIEGER, Daniel K.**
  **Branford, CT 06405 (US)**
- **TSE, Kam-Fai**
  **Clinton, CT 06413 (US)**
- **RASTELLI, Luca**
  **Guilford, CT 06437 (US)**
- **SMITHSON, Glennda**
  **Guilford, CT 06435 (US)**
- **MESRI, Mehdi**
  **Brandford, CT 06405 (US)**
- **OOI, Chean, Eng**
  **Branford, CT 06405 (US)**

- **ANDERSON, David, W.**
  **Branford, CT 06405 (US)**
- **GUO, Xiaojia**
  **Branford, CT 06405 (US)**
- **GIOT, Loic**
  **Madison, CT 06443 (US)**
- **STARLING, Gary**
  **Middletown, CT 06457 (US)**

(74) Representative: **Finnie, Isobel Lara et al**
**Haseltine Lake LLP**
**Lincoln House, 5th Floor**
**300 High Holborn**
**London WC1V 7JH (GB)**

(56) References cited:
WO-A-02/081517     WO-A-02/098920
WO-A-03/002722     WO-A-03/003906
WO-A-03/025138     WO-A-03/042661
US-B2- 7 041 284

- FEIGELSTOCK D ET AL: "The human homolog of HAVcr-1 codes for a hepatitis A virus cellular receptor" JOURNAL OF VIROLOGY, THE AMERICAN SOCIETY FOR MICROBIOLOGY, US, vol. 72, no. 8, 1 August 1998 (1998-08-01), pages 6621-6628, XP002959127 ISSN: 0022-538X

**Description**

**FIELD OF THE INVENTION**

The present invention relates to novel polypeptides, and the nucleic acids encoding them, having properties related to stimulation of biochemical or physiological responses in a cell, a tissue, an organ or an organism. More particularly, the novel polypeptides are gene products of novel genes, or are specified biologically active fragments or derivatives thereof. Methods of use encompass diagnostic and prognostic assay procedures as well as methods of treating diverse pathological conditions.

**BACKGROUND OF THE INVENTION**

**Kidney Cancer**

Renal cell carcinoma (RCC or kidney cancer) is the most common primary cancer that occurs in the kidney and accounts for more than 85% of all primary renal neoplasms in adults. Transitional cell carcinomas of the renal pelvis are the next most common and account for approximately 8% of all primary renal neoplasms. Malignant renal neoplasms can be primary or metastatic tumors. Renal cell carcinoma usually occurs in adults, between the ages of 40 and 60 years, with the incidence in men being double of that in women. Renal cell carcinoma represents 2% to 3% of all cancers and 2% of all cancer deaths. In 2002, renal cell cancer was diagnosed in approximately 31,000 individuals in the United States, almost 40% of whom will eventually die of the disease.

Renal cell carcinoma occurs predominantly at the age of 60 to 90, with a 1.27% lifetime risk of developing renal cell carcinoma for a 40-year-old man and 0.51% risk of death from this disease. The incidence of renal cell cancer increased steadily from 1975 to 1995 in both females (3.1% in white women; 4.3% in black women) and males (2.3% in white men; 3.9% in black men), as did the mortality rates. Since 1950, there has been a 126% increase in the incidence of RCC, accompanied by a 37% increase in annual mortality caused by RCC.

Kidney cancer often goes undiagnosed or is mis-diagnosed until it has spread (metastasized). As a result, 15-25% of kidney cancer patients have metastatic disease at time of diagnosis. One-third of patients with renal cell carcinoma have distant metastases at the time the primary tumor is diagnosed. Surgery has been the primary therapy for renal cell carcinoma for more than 100 years. Historically, kidney cancer has proven to be resistant to many chemotherapy agents, and response rates for traditional chemotherapy used for kidney cancer are often below 10%. Satisfactory treatment of renal cancer is an unmet medical need, as existing therapeutics have not been successful in curtailing the disease and increasing mortality. Consequently, a therapeutic that can successfully treat renal cancer has the beneficial effects of decreasing morbidity and mortality, while potentially saving the healthcare system millions of dollars in costs associated with invasive surgical procedures, immuno- and chemotherapy, and ancillary support services.

**Inflammation**

Asthma is a chronic inflammatory disorder of the airways in which many cells, including mast cells, eosinophils, and T lymphocytes, play a role. In affected individuals this inflammation results in recurrent episodes of wheezing, coughing, breathlessness, and chest tightness. These symptoms are associated with variable airflow limitation that can be reversed either spontaneously or with treatment. The Centers for Disease Control have shown that the prevalence of asthma has increased approximately 42% in the United States from 1982 to 1992, from 34.7 to 49.4 cases per 1000. For ages 5-34 years, the rate increased 52% from 34.6 to 52.6 cases per 1000. During this same period, hospitalization rates and the annual death rate from asthma also increased. From 1980 to 1993, asthma accounted for 3850 deaths among persons aged 0 to 24 years, with the annual age-specific asthma death rate increasing 118%. In this same period, the annual hospitalization rate for asthma among persons aged 0 to 24 years increased 28%.

Changes in the risk factors thought to cause and worsen asthma are responsible for much of this recent increase in asthma prevalence. Children and adults with severe asthma symptoms have lower lung function than those with less severe symptoms. Satisfactory treatment of asthma is an unmet medical need, as existing therapeutics have not been successful in curtailing the incidence or the severity of the disease. Consequently, a therapeutic that can successfully treat asthma has the beneficial effects of decreasing morbidity.

Eukaryotic cells are characterized by biochemical and physiological processes which under normal conditions are exquisitely balanced to achieve the preservation and propagation of the cells. When such cells are components of multicellular organisms such as vertebrates, or more particularly organisms such as mammals, the regulation of the biochemical and physiological processes involves intricate signaling pathways. Frequently, such signaling pathways involve extracellular signaling proteins, cellular receptors that bind the signaling proteins, and signal transducing components located within the cells.

Signaling proteins can be classified as endocrine effectors, paracrine effectors or autocrine effectors. Endocrine effectors are signaling molecules secreted by a given organ into the circulatory system, which are then transported to a distant target organ or tissue. The target cells include the receptors for the endocrine effector, and when the endocrine effector binds, a signaling cascade is induced. Paracrine effectors involve secreting cells and receptor cells in close proximity to each other, for example two different classes of cells in the same tissue or organ. One class of cells secretes the paracrine effector, which then reaches the second class of cells, for example by diffusion through the extracellular fluid. The second class of cells contains the receptors for the paracrine effector; binding of the effector results in induction of the signaling cascade that elicits the corresponding biochemical or physiological effect. Autocrine effectors are highly analogous to paracrine effectors, except that the same cell type that secretes the autocrine effector also contains the receptor. Thus the autocrine effector binds to receptors on the same cell, or on identical neighboring cells. The binding process then elicits the characteristic biochemical or physiological effect.

Signaling processes can elicit a variety of effects on cells and tissues including by way of nonlimiting example induction of cell or tissue proliferation, suppression of growth or proliferation, induction of differentiation or maturation of a cell or tissue, and suppression of differentiation or maturation of a cell or tissue.

Many pathological conditions involve dysregulation of expression of important effector proteins. In certain classes of pathologies the dysregulation is manifested as diminished or suppressed level of synthesis and secretion of protein effectors. In other classes of pathologies the dysregulation is manifested as increased or up-regulated level of synthesis and secretion of protein effectors. In a clinical setting a subject may be suspected of suffering from a condition brought on by altered or mis-regulated levels of a protein effector of interest. Therefore there is a need to assay for the level of the protein effector of interest in a biological sample from such a subject, and to compare the level with that characteristic of a nonpathological condition. There also is a need to provide the protein effector as a product of manufacture. Administration of the effector to a subject in need thereof is useful in treatment of the pathological condition. Accordingly, there is a need for a method of treatment of a pathological condition brought on by a diminished or suppressed levels of the protein effector of interest. In addition, there is a need for a method of treatment of a pathological condition brought on by a increased or up-regulated levels of the protein effector of interest.

Therefore there is a need to assay for the level of a protein effector of interest in a biological sample from such a subject, and to compare this level with that characteristic of a nonpathological condition. In particular, there is a need for such an assay based on the use of an antibody that binds immunospecifically to the antigen. There further is a need to inhibit the activity of the protein effector in cases where a pathological condition arises from elevated or excessive levels of the effector based on the use of an antibody that binds immunospecifically to the effector. Thus, there is a need for the antibody as a product of manufacture. There further is a need for a method of treatment of a pathological condition brought on by an elevated or excessive level of the protein effector of interest based on administering the antibody to the subject.

## SUMMARY OF THE INVENTION

The invention is based in part upon the discovery of isolated polypeptides including amino acid sequences selected from mature forms of the amino acid sequences selected from the group consisting of SEQ ID NO:2n, wherein n is an integer between 1 and 12. The novel nucleic acids and polypeptides are referred to herein as NOVX, or NOV1, NOV2, NOV3, *etc.,* nucleic acids and polypeptides. These nucleic acids and polypeptides, as well as derivatives, homologs, analogs and fragments thereof, are hereinafter collectively designated as "NOVX" nucleic acid or polypeptide sequences.

This disclosure is based in part upon variants of a mature form of the amino acid sequence selected from the group consisting of SEQ ID NO:2n, wherein n is an integer between 1 and 12, wherein any amino acid in the mature form is changed to a different amino acid, provided that no more than 15% of the amino acid residues in the sequence of the mature form are so changed. The disclosure includes the amino acid sequences selected from the group consisting of SEQ ID NO:2n, wherein n is an integer between 1 and 12. The disclosure comprises variants of the amino acid sequence selected from the group consisting of SEQ ID NO:2n, wherein n is an integer between 1 and 12 wherein any amino acid specified in the chosen sequence is changed to a different amino acid, provided that no more than 15% of the amino acid residues in the sequence are so changed. The disclosure also involves fragments of any of the mature forms of the amino acid sequences selected from the group consisting of SEQ ID NO:2n, wherein n is an integer between 1 and 12, or any other amino acid sequence selected from this group. The disclosure also comprises fragments from these groups in which up to 15% of the residues are changed.

In another aspect, the disclosure encompasses polypeptides that are naturally occurring allelic variants of the sequence selected from the group consisting of SEQ ID NO:2n, wherein n is an integer between 1 and 12. These allelic variants include amino acid sequences that are the translations of nucleic acid sequences differing by a single nucleotide from nucleic acid sequences selected from the group consisting of SEQ ID NOS: 2n-1, wherein n is an integer between 1 and 12. The variant polypeptide where any amino acid changed in the chosen sequence is changed to provide a conservative substitution.

This disclosure comprises a pharmaceutical composition involving a polypeptide with an amino acid sequence selected from the group consisting of SEQ ID NO:2n, wherein n is an integer between 1 and 12 and a pharmaceutically acceptable carrier. This disclosure involves a kit, including, in one or more containers, this pharmaceutical composition.

In another embodiment, the invention includes the use of a therapeutic in the manufacture of a medicament for treating a syndrome associated with a human disease, the disease being selected from a pathology associated with a polypeptide with an amino acid sequence selected from the group consisting of SEQ ID NO:2n, wherein n is an integer between 1 and 12 wherein said therapeutic is the polypeptide selected from this group.

In another aspect, the disclosure comprises a method for determining the presence or amount of a polypeptide with an amino acid sequence selected from the group consisting of SEQ ID NO:2n, wherein n is an integer between 1 and 12 in a sample, the method involving providing the sample; introducing the sample to an antibody that binds immunospecifically to the polypeptide; and determining the presence or amount of antibody bound to the polypeptide, thereby determining the presence or amount of polypeptide in the sample.

In another aspect, the disclosure includes a method for determining the presence of or predisposition to a disease associated with altered levels of a polypeptide with an amino acid sequence selected from the group consisting of SEQ ID NO:2n, wherein n is an integer between 1 and 12 in a first mammalian subject, the method involving measuring the level of expression of the polypeptide in a sample from the first mammalian subject; and comparing the amount of the polypeptide in this sample to the amount of the polypeptide present in a control sample from a second mammalian subject known not to have, or not to be predisposed to, the disease, wherein an alteration in the expression level of the polypeptide in the first subject as compared to the control sample indicates the presence of or predisposition to the disease.

In another aspect, the disclosure involves a method of identifying an agent that binds to a polypeptide with an amino acid sequence selected from the group consisting of SEQ ID NO:2n, wherein n is an integer between 1 and 12, the method including introducing the polypeptide to the agent; and determining whether the agent binds to the polypeptide. The agent could be a cellular receptor or a downstream effector.

In another aspect, the disclosure involves a method for identifying a potential therapeutic agent for use in treatment of a pathology, wherein the pathology is related to aberrant expression or aberrant physiological interactions of a polypeptide with an amino acid sequence selected from the group consisting of SEQ ID NO:2n, wherein n is an integer between 1 and 12, the method including providing a cell expressing the polypeptide and having a property or function ascribable to the polypeptide; contacting the cell with a composition comprising a candidate substance; and determining whether the substance alters the property or function ascribable to the polypeptide; whereby, if an alteration observed in the presence of the substance is not observed when the cell is contacted with a composition devoid of the substance, the substance is identified as a potential therapeutic agent.

In another aspect, the disclosure involves a method for screening for a modulator of activity or of latency or predisposition to a pathology associated with a polypeptide having an amino acid sequence selected from the group consisting of SEQ ID NO:2n, wherein n is an integer between 1 and 12, the method including administering a test compound to a test animal at increased risk for a pathology associated with the polypeptide of the invention, wherein the test animal recombinantly expresses the polypeptide of the invention; measuring the activity of the polypeptide in the test animal after administering the test compound; and comparing the activity of the protein in the test animal with the activity of the polypeptide in a control animal not administered the polypeptide, wherein a change in the activity of the polypeptide in the test animal relative to the control animal indicates the test compound is a modulator of latency of, or predisposition to, a pathology associated with the polypeptide of the invention. The recombinant test animal could express a test protein transgene or express the transgene under the control of a promoter at an increased level relative to a wild-type test animal The promoter may or may not be the native gene promoter of the transgene.

In another aspect, the disclosure involves a method for modulating the activity of a polypeptide with an amino acid sequence selected from the group consisting of SEQ ID NO:2n, wherein n is an integer between 1 and 12, the method including introducing a cell sample expressing the polypeptide with a compound that binds to the polypeptide in an amount sufficient to modulate the activity of the polypeptide.

In another aspect, the disclosure involves a method of treating or preventing a pathology associated with a polypeptide with an amino acid sequence selected from the group consisting of SEQ ID NO:2n, wherein n is an integer between 1 and 12, the method including administering the polypeptide to a subject in which such treatment or prevention is desired in an amount sufficient to treat or prevent the pathology in the subject. The subject could be human.

In another aspect, the disclosure involves a method of treating a pathological state in a mammal, the method including administering to the mammal a polypeptide in an amount that is sufficient to alleviate the pathological state, wherein the polypeptide is a polypeptide having an amino acid sequence at least 95% identical to a polypeptide having the amino acid sequence selected from the group consisting of SEQ ID NO:2n, wherein n is an integer between 1 and 12 or a biologically active fragment thereof.

In another aspect, the disclosure involves an isolated nucleic acid molecule comprising a nucleic acid sequence encoding a polypeptide having an amino acid sequence selected from the group consisting of a mature form of the amino acid sequence given SEQ ID NO:2n, wherein n is an integer between 1 and 12; a variant of a mature form of the amino acid sequence selected from the group consisting of SEQ ID NO:2n, wherein n is an integer between 1 and 12 wherein any amino acid in the mature form of the chosen sequence is changed to a different amino acid, provided that no more than 15% of the amino acid residues in the sequence of the mature form are so changed; the amino acid sequence selected from the group consisting of SEQ ID NO:2n, wherein n is an integer between 1 and 12; a variant of the amino acid sequence selected from the group consisting of SEQ ID NO:2n, wherein n is an integer between 1 and 12, in which any amino acid specified in the chosen sequence is changed to a different amino acid, provided that no more than 15% of the amino acid residues in the sequence are so changed; a nucleic acid fragment encoding at least a portion of a polypeptide comprising the amino acid sequence selected from the group consisting of SEQ ID NO:2n, wherein n is an integer between 1 and 12 or any variant of the polypeptide wherein any amino acid of the chosen sequence is changed to a different amino acid, provided that no more than 10% of the amino acid residues in the sequence are so changed; and the complement of any of the nucleic acid molecules.

In another aspect, the disclosure comprises an isolated nucleic acid molecule having a nucleic acid sequence encoding a polypeptide comprising an amino acid sequence selected from the group consisting of a mature form of the amino acid sequence given SEQ ID NO:2n, wherein n is an integer between 1 and 12, wherein the nucleic acid molecule comprises the nucleotide sequence of a naturally occurring allelic nucleic acid variant.

In another aspect, the disclosure involves an isolated nucleic acid molecule including a nucleic acid sequence encoding a polypeptide having an amino acid sequence selected from the group consisting of a mature form of the amino acid sequence given SEQ ID NO:2n, wherein n is an integer between 1 and 12 that encodes a variant polypeptide, wherein the variant polypeptide has the polypeptide sequence of a naturally occurring polypeptide variant.

In another aspect, the disclosure comprises an isolated nucleic acid molecule having a nucleic acid sequence encoding a polypeptide comprising an amino acid sequence selected from the group consisting of a mature form of the amino acid sequence given SEQ ID NO:2n, wherein n is an integer between 1 and 12, wherein the nucleic acid molecule differs by a single nucleotide from a nucleic acid sequence selected from the group consisting of SEQ ID NOS: 2n-1, wherein n is an integer between 1 and 12.

In another aspect, the disclosure includes an isolated nucleic acid molecule having a nucleic acid sequence encoding a polypeptide including an amino acid sequence selected from the group consisting of a mature form of the amino acid sequence given SEQ ID NO:2n, wherein n is an integer between 1 and 12, wherein the nucleic acid molecule comprises a nucleotide sequence selected from the group consisting of the nucleotide sequence selected from the group consisting of SEQ ID NO:2n-1, wherein n is an integer between 1 and 12; a nucleotide sequence wherein one or more nucleotides in the nucleotide sequence selected from the group consisting of SEQ ID NO:2n-1, wherein n is an integer between 1 and 12 is changed from that selected from the group consisting of the chosen sequence to a different nucleotide provided that no more than 15% of the nucleotides are so changed; a nucleic acid fragment of the sequence selected from the group consisting of SEQ ID NO:2n-1, wherein n is an integer between 1 and 12; and a nucleic acid fragment wherein one or more nucleotides in the nucleotide sequence selected from the group consisting of SEQ ID NO:2n-1, wherein n is an integer between 1 and 12 is changed from that selected from the group consisting of the chosen sequence to a different nucleotide provided that no more than 15% of the nucleotides are so changed.

In another aspect, the disclosure includes an isolated nucleic acid molecule having a nucleic acid sequence encoding a polypeptide including an amino acid sequence selected from the group consisting of a mature form of the amino acid sequence given SEQ ID NO:2n, wherein n is an integer between 1 and 12, wherein the nucleic acid molecule hybridizes under stringent conditions to the nucleotide sequence selected from the group consisting of SEQ ID NO:2n-1, wherein n is an integer between 1 and 12, or a complement of the nucleotide sequence.

In another aspect, the disclosure includes an isolated nucleic acid molecule having a nucleic acid sequence encoding a polypeptide including an amino acid sequence selected from the group consisting of a mature form of the amino acid sequence given SEQ ID NO:2n, wherein n is an integer between 1 and 12, wherein the nucleic acid molecule has a nucleotide sequence in which any nucleotide specified in the coding sequence of the chosen nucleotide sequence is changed from that selected from the group consisting of the chosen sequence to a different nucleotide provided that no more than 15% of the nucleotides in the chosen coding sequence are so changed, an isolated second polynucleotide that is a complement of the first polynucleotide, or a fragment of any of them.

In another aspect, the disclosure includes a vector involving the nucleic acid molecule having a nucleic acid sequence encoding a polypeptide including an amino acid sequence selected from the group consisting of a mature form of the amino acid sequence given SEQ ID NO:2n, wherein n is an integer between 1 and 12. This vector can have a promoter operably linked to the nucleic acid molecule. This vector can be located within a cell.

In another aspect, the disclosure involves a method for determining the presence or amount of a nucleic acid molecule having a nucleic acid sequence encoding a polypeptide including an amino acid sequence selected from the group consisting of a mature form of the amino acid sequence given SEQ ID NO:2n, wherein n is an integer between 1 and 12 in a sample, the method including providing the sample; introducing the sample to a probe that binds to the nucleic acid molecule; and determining the presence or amount of the probe bound to the nucleic acid molecule, thereby determining the presence or amount of the nucleic acid molecule in the sample. The presence or amount of the nucleic acid molecule is used as a marker for cell or tissue type. The cell type can be cancerous.

In another aspect, the disclosure involves a method for determining the presence of or predisposition for a disease associated with altered levels of a nucleic acid molecule having a nucleic acid sequence encoding a polypeptide including an amino acid sequence selected from the group consisting of a mature form of the amino acid sequence given SEQ ID NO:2n, wherein n is an integer between 1 and 12 in a first mammalian subject, the method including measuring the amount of the nucleic acid in a sample from the first mammalian subject; and comparing the amount of the nucleic acid in the sample of step (a) to the amount of the nucleic acid present in a control sample from a second mammalian subject known not to have or not be predisposed to, the disease; wherein an alteration in the level of the nucleic acid in the first subject as compared to the control sample indicates the presence of or predisposition to the disease.

The invention further provides an antibody that binds immunospecifically to a NOVX polypeptide. The NOVX antibody can be monoclonal, humanized, or a fully human antibody. Preferably, the antibody has a dissociation constant for the binding of the NOVX polypeptide to the antibody less than 1 x $10^{-9}$ M. More preferably, the NOVX antibody neutralizes the activity of the NOVX polypeptide.

In a further aspect, the invention provides for the use of a therapeutic in the manufacture of a medicament for treating a syndrome associated with a human disease, associated with a NOVX polypeptide. Preferably the therapeutic is a NOVX antibody.

In yet a further aspect, the invention provides a method of treating or preventing a NOVX-associated disorder, a method of treating a pathological state in a mammal, and a method of treating or preventing a pathology associated with a polypeptide by administering a NOVX antibody to a subject in an amount sufficient to treat or prevent the disorder.

In another embodiment, the invention involves an immunoconjugate. The immunoconjugates can be an antibody conjugated to a cytotoxic agent such as a chemotherapeutic agent, a toxin (e.g., an enzymatically active toxin of bacterial, fungal, plant, or animal origin, or fragments thereof), or a radioactive isotope (i.e., a radioconjugate).

The invention further provides methods for treating cancer by administering one or more antibodies or immunoconjugates to a patient. In particular the present invention provides an isolated human or humanised antibody or an immunologically active fragment thereof that immunospecifically binds to a human Hepatitis A Virus cellular receptor I (HAVcr-1) polypeptide comprising a sequence selected from SEQ-ID NO: 2, 4, 6, 8 and 39 for use in treating cancer in a subject selected from lunch cancer and ovarian cancer, wherein said antibody or fragment binds the HAVcr-1 polypeptide with an equilibrium dissociation constant that is less than or equal to 100_nM and wherein the antibody or immunologically active fragment thereof is conjugated to a cytotoxic agent selected from the group consisting of a chemotherapeutic agent, a toxin, and a radioactive isotope.

This disclosure also provides methods for treating inflammation by administering one or more antibodies or one or more immunoconjugates to a patient.

In another aspect, the disclosure involves protein-protein complexes. These complexes can involve any one of polypeptides NOV1a - NOV-1d complexed with any one of polypeptides NOV2a - NOV2h. Alternatively, the complex can involve a fragment of any one of polypeptides NOV1a- NOV-1d complexed with a fragment of any one of polypeptides NOV2a - NOV2h. The fragments can consist of specific domains of the polypeptides (*e.g.* the PKD domain of NOV2a-NOV2h, the mucin domain of NOV1a-NOV1d, or the unglycosylated mucin domain of NOV1a-NOV1d).

In another aspect, the disclosure involves antibodies which immunospecifically bind to any of the NOV1-NOV2 complexes.

In another aspect, the disclosure involves an antibody which disrupts or neutralizes the interaction between the protein-protein complexes of the invention thereby disassociating any NOV1-NOV2 complex or preventing the formation of any NOV1-NOV2 complex. For example, antibodies which disrupt or neutralize the interactions between the NOV polypeptides include antibodies which immunospecifically bind to the PKD domain of NOV2, the mucin domain of NOV1, or the unglycosylated mucin domain of NOV1.

Unless otherwise defined, all technical and scientific terms used herein have the same meaning as commonly understood by one of ordinary skill in the art to which this invention belongs.

In the case of conflict, the present specification, including definitions, will control.

Other features and advantages of the invention will be apparent from the following detailed description and claims.

**BRIEF DESCRIPTION OF THE DRAWINGS**

Figure 1 is a diagram showing proteins that interact with CG57008-02 (HAVCR-1), including CG51373 (nephrin 1 like), and LOC155465.

Figure 2 is a bar graph of the results of an ELISA assay of antibodies CR014.1.29, CR04.2.56.2, CR014.2.59.2, and CR014.2.45.1 against CG57008-02.

Figure 3 is a bar graph of the results of an ELISA assay of antibodies CR014.1.29, CR04.2.56.2, CR014.2.59.2, and CR014.2.45.1 against irrelevant protein.

Figures 4A and 4B are photographs showing staining of Renal Cell Cancer (left) and Pancreatic Cancer (right) with the anti-CG57008 2.59.2 monoclonal antibody.

Figure 5 is a bar graph of Clonogenic Assay results of anti-CG57008 monoclonal antibody mediated toxin killing in the ACHN kidney cancer cell line.

Figure 6 is a bar graph of Clonogenic Assay results of anti-CG57008 monoclonal antibody mediated toxin killing in the BT549 breast cancer cell line.

Figure 7 is a bar graph of the results of an ELISA assay showing that Monoclonal Antibodies 2.59.2, 2.56.2 and 2.45.1 significantly inhibited IL-4 release from Th1 cells compared to the control PK16.3 mAb.

Figure 8 is a bar graph of the results of an ELISA assay showing that Monoclonal Antibodies 2.59.2, and 2.45.1 significantly inhibited IL-4 release from Th2 cells compared to control PK16.3 mAb.

Figure 9 is a bar graph of the results of an ELISA assay showing that Monoclonal Antibodies 2.59.2 significantly inhibited IL-5 release from Th1 cells compared to control PK16.3 mAb.

Figure 10 is a bar graph of the results of an ELISA assay showing that Monoclonal Antibodies 2.59.2, and 1.29 significantly inhibited IL-5 release from Th2 cells compared to control PK16.3 mAb.

Figure 11 is a bar graph of the results of an ELISA assay showing that Monoclonal Antibodies 2.59.2, 1.29 and 2.56.2 significantly inhibited IL-10 release from Th1 cells compared to control PK16.3 mAb.

Figure 12 is a bar graph of the results of an ELISA assay showing that Monoclonal Antibodies 2.59.2, 1.29 and 2.45.1 significantly inhibited IL-10 release from Th2 cells compared to control PK16.3 mAb.

Figure 13 is a bar graph of the results of an ELISA assay showing that Monoclonal Antibodies 2.59.2, 1.29 and 2.56.2 significantly inhibited IL-13 release from Th1 cells compared to control PK16.3 mAb.

Figure 14 is a bar graph of the results of an ELISA assay showing that Monoclonal Antibodies mAbs 2.59.2 and 1.29 significantly inhibited IL-13 release from Th2 cells compared to control PK16.3 mAb.

Figure 15 is a bar graph of the results of an ELISA assay showing that Anti-CG57008-02 Monoclonal Antibodies did not inhibit IFN$\gamma$ release from Th1 cells compared to control PK16.3 mAb.

Figure 16 is a bar graph of the results of an ELISA assay showing that Monoclonal Antibodies 2.59.2 and 2.45.1 significantly inhibited IFN$\gamma$ release from Th2 cells compared to control PK16.3 mAb.

Figure 17 is a bar graph of the results of a clonogenic assay of CAKI-1 cells treated with Auristatin E (AE) conjugated antibodies.

Figure 18 is a bar graph of the results of a clonogenic assay of BT549 cells treated with Auristatin E (AE) conjugated antibodies.

## DETAILED DESCRIPTION OF THE INVENTION

HAVcr-1 (Hepatitis A Virus cellular receptor 1) is a putative adhesion protein involved in renal regeneration. The HAVcr-1 protein is a type 1 membrane protein that contains a novel six-cysteine immunoglobulin-like domain and a mucin domain. Ichimura et. al first identified this molecule, which they designated kidney injury molecule-1 (KIM-1), as being expressed at low levels in normal kidney with dramatically increased expression in post-ischemic kidney. (J Biol Chem. 1998 Feb 13;273(7):4135-42.) Feigelstock demonstrated that KIM-1 is the human ortholog of Hepatitis A virus receptor (J Virol. 1998 Aug;72(8):6621-8.) In polycystic kidney disease, KIM-1 expression is strongly associated with partial dedifferentiation of epithelial cells (Kuehn EW. Am J Physiol Renal Physiol 2002 Dec;283(6):F1326-36). Shedding of a soluble domain of KIM-1 is proposed to constitute an active mechanism allowing dedifferentiated regenerating kidney cells to scatter (Bailly V. J Biol Chem 2002 Oct 18;277(42):39739-48).

The HAVcr-1 protein has homology to P-type 'Trefoil' domains which have been shown to induce scattering and cellular invasion by kidney, colon and breast tumor cells. (Prest SJ. FASEB J. 2002 Feb 12). Trefoils may act via an anti-apoptotic mechanism by making cells resistant to anoikis, an anchorage-related apoptosis in epithelium. (Chen YH. Biochem Biophys Res Commun. 2000 Aug 11;274(3):576-82.)

Monoclonal antibodies directed against HAVcr-1 protein should block migration and induce apoptosis of kidney cancer cells. Since HAVcr-1 gene appears to be highly induced in tumors, the HAVcr-1 protein could also be the target of toxin-conjugate monoclonal antibodies.

Tim1, a mouse ortholog of HAVcr-1, has been deposited in Genbank and identified as Tapr, a major T cell regulatory locus that controls the development of airway hyperreactivity. (McIntire JJ. Nat Immunol. 2001 Dec;2(12):1 109-16.) The human HAVcr-1 gene also maps to a portion of Chromosome 5 (*Tapr*) that has been implicated in asthma. Tim1 is expressed by T cells and presumably interacts with an unknown ligand(s) on antigen presenting cells (Wills-Karp M. Nature Immunology 2, 1095 - 1096; McIntire JJ. Nature Immunology 2, 1109 - 1116).

Antibodies to HAVcr-1 protein would send a partially activating signal that would lead to T cell anergy, act as an antagonist to block T cells from differentiating into Th2 cells, or act as an agonist to trigger T cell differentiation. Blocking of Th2 effector T cell function would decrease inflammation associated with asthma, lupus, and emphysema.

The present invention provides novel nucleotides and polypeptides encoded thereby. Included in the invention are the novel nucleic acid sequences, their encoded polypeptides, antibodies, and other related compounds. The sequences are collectively referred to herein as "NOVX nucleic acids" or "NOVX polynucleotides" and the corresponding encoded polypeptides are referred to as "NOVX polypeptides" or "NOVX proteins." Unless indicated otherwise, "NOVX" is meant to refer to any of the novel sequences disclosed herein. Table A provides a summary of the NOVX nucleic acids and their encoded polypeptides.

**TABLE A. Sequences and Corresponding SEQ ID Numbers**

| NOVX Assignment | Internal Identification | SEQ ID NO (nucleic acid) | SEQ ID NO (amino acid) | Homology |
|---|---|---|---|---|
| NOV1a | CG57008-03 | 1 | 2 | Hepatitis A virus cellular receptor 1 - Homo sapiens |
| NOV1b | CG57008-01 | 3 | 4 | Hepatitis A virus cellular receptor 1 - Homo sapiens |
| NOV1c | CG57008-02 | 5 | 6 | Hepatitis A virus cellular receptor 1 - Homo sapiens |
| NOV1d | CG57008-04 | 7 | 8 | Hepatitis A virus cellular receptor 1 - Homo sapiens |
| NOV2a | CG51373-12 | 9 | 10 | Sequence 1 from Patent WO0200691 - Homo sapiens |
| NOV2b | CG51373-02 | 11 | 12 | Sequence 1 from Patent WO0200691 - Homo sapiens |
| NOV2c | CG51373-03 | 13 | 14 | Sequence 1 from Patent WO0200691 - Homo sapiens |
| NOV2d | CG51373-07 | 15 | 16 | Sequence 1 from Patent WO0200691 - Homo sapiens |
| NOV2e | CG51373-10 | 17 | 18 | Sequence 1 from Patent WO0200691 - Homo sapiens |
| NOV2f | CG51373-11 | 19 | 20 | Sequence 1 from Patent WO0200691 - Homo sapiens |

(continued)

| NOVX Assignment | Internal Identification | SEQ ID NO (nucleic acid) | SEQ ID NO (amino acid) | Homology |
|---|---|---|---|---|
| NOV2g | CG51373-13 | 21 | 22 | Sequence 1 from Patent WO0200691 - Homo sapiens |
| NOV2h | CG51373-14 | 23 | 24 | Sequence 1 from Patent WO0200691 - Homo sapiens |

Table A indicates the homology of NOVX polypeptides to known protein families. Thus, the nucleic acids and polypeptides, antibodies and related compounds according to the invention corresponding to a NOVX as identified in column 1 of Table A are useful in therapeutic and diagnostic applications implicated in, for example, pathologies and disorders associated with the known protein families identified in column 5 of Table A.

**Polynucleotide and Polypeptide Sequences, and Homology Data**

**NOV1:**

A NOV1 clone was analyzed, and the nucleotide and encoded polypeptide sequences are shown in Table 1A.

| Table 1A. NOV1 Sequence Analysis | | | |
|---|---|---|---|
| NOV1a, CG57008-03 DNA Sequence | SEQ ID NO: 1 | 1017 bp | |
| | ORF Start: at 1 | ORF Stop: end of sequence | |
| **TCT**GTAAAGGTTGGTGGAGAGGCAGGTCCATCTGTCACACTACCCTGCCACTACAGTGGAGCTGTCAC ATCAATGTGCTGGAATAGAGGCTCATGTTCTCTATTCACATGCCAAAATGGCATTGTCTGGACCAATG GAACCCACGTCACCTATCGGAAGGACACACGCTATAAGCTATTGGGGGACCTTTCAAGAAGGGATGTC TCTTTGACCATAGAAAATACAGCTGTGTCTGACAGTGGCGTATATTGTTGCCGTGTTGAGCACCGTGG GTGGTTCAATGACATGAAAATCACCGTATCATTGGAGATTGTGCCACCCAAGGTCACGACTACTCCAA TTGTCACAACTGTTCCAACCGTCACGACTGTTCGAACGAGCACCACTGTTCCAACGACAACGACTGTT CCAACGACAACTGTTCCAACAACAATGAGCATTCCAACGACAACGACTGTTCCGACGACAATGACTGT TTCAACGACAACGAGCGTTCCAACGACAACGAGCATTCCAACAACAACAAGTGTTCCAGTGACAACAA CGGTCTCTACCTTTGTTCCTCCAATGCCTTTGCCCAGGCAGAACCATGAACCAGTAGCCACTTCACCA TCTTCACCTCAGCCAGCAGAAACCCACCCTACGACACTGCAGGGAGCAATAAGGAGAGAACCCACCAG CTCACCATTGTACTCTTACACAACAGATGGGAATGACACCGTGACAGAGTCTTCAGATGGCCTTTGGA ATAACAATCAAACTCAACTGTTCCTAGAACATAGTCTACTGACGGCCAATACCACTAAAGGAATCTAT GCTGGAGTCTGTATTTCTGTCTTGGTGCTTCTTGCTCTTTTGGGTGTCATCATTGCCAAAAAGTATTT CTTCAAAAAGGAGGTTCAACAACTAAGTGTTTCATTTAGCAGCCTTCAAATTAAAGCTTTGCAAAATG CAGTTGAAAAGGAAGTCCAAGCAGAAGACAATATCTACATTGAGAATAGTCTTTATGCCACGGAC | | | |
| NOV1a, CG57008-03 Protein Sequence | SEQ ID NO: 2 | 339 aa | MW at 36608.1kD |
| SVKVGGEAGPSVTLPCHYSGAVTSMCWNRGSCSLFTCQNGIVWTNGTHVTYRKDTRYKLLGDLSRRDV | | | |
| SLTIENTAVSDSGVYCCRVEHRGWFNDMKITVSLEIVPPKVTTTPIVTTVPTVTTVRTSTTVPTTTTV PTTTVPTTMSIPTTTTVPTTMTVSTTTSVPTTTSIPTTTSVPVTTTVSTFVPPMPLPRQNHEPVATSP SSPQPAETHPTTLQGAIRREPTSSPLYSYTTDGNDTVTESSDGLWNNNQTQLFLEHSLLTANTTKGIY AGVCISVLVLLALLGVIIAKKYFFKKEVQQLSVSFSSLQIKALQNAVEKEVQAEDNIYIENSLYATD | | | |
| NOV1b, CG57008-01 DNA Sequence | SEQ ID NO: 3 | 1440 bp | |
| | ORF Start: ATG at 52 | ORF Stop: end of sequence | |

(continued)

| Table 1A. NOV1 Sequence Analysis | | | |
|---|---|---|---|
| <div>GTTACCCAGCATTGTGAGTGACAGAGCCTGGATCTGAACGCTGATCCCATA**ATG**CATCCTCAAGTGGT<br>CATCTTAAGCCTCATCCTACATCTGGCAGATTCTGTAGCTGGTTCTGTAAAGGTTGGTGGAGAGGCAG<br>GTCCATCTGTCACACTACCCTGCCACTACAGTGGAGCTGTCACATCAATGTGCTGGAATAGAGGCTCA<br>TGTTCTCTATTCACATGCCAAAATGGCATTGTCTGGACCAATGGAACCCACGTCACCTATCGGAAGGA<br>CACACGCTATAAGCTATTGGGGGACCTTTCAAGAAGGGATGTCTCTTTGACCATAGAAAATACAGCTG<br>TGTCTGACAGTGGCGTATATTGTTGCCGTGTTGAGCACCGTGGGTGGTTCAATGACATGAAAATCACC<br>GTATCATTGGAGATTGTGCCACCCAAGGTCACGACTACTCCAATTGTCACAACTGTTCCAACCGTCAC<br>GACTGTTCGAACGAGCACCACTGTTCCAACGACAACGACTGTTCCAACGACAACTGTTCCAACAACAA<br>TGAGCATTCCAACGACAACGACTGTTCCGACGACAATGACTGTTTCAACGACAACGAGCGTTCCAACG<br>ACAACGAGCATTCCAACAACAACAAGTGTTCCAGTGACAACAACGGTCTCTACCTTTGTTCCTCCAAT<br>GCCTTTGCCCAGGCAGAACCATGAACCAGTAGCCACTTCACCATCTTCACCTCAGCCAGCAGAAACCC<br>ACCCTACGACACTGCAGGGAGCAATAAGGAGAGAACCCACCAGCTCACCATTGTACTCTTACACAACA<br>GATGGGAATGACACCGTGACAGAGTCTTCAGATGGCCTTTGGAATAACAATCAAACTCAACTGTTCCT<br>AGAACATAGTCTACTGACGGCCAATACCACTAAAGGAATCTATGCTGGAGTCTGTATTTCTGTCTTGG<br>TGCTTCTTGCTCTTTTGGGTGTCATCATTGCCAAAAGTATTTCTTCAAAAAGGAGGTTCAACAACTA<br>AGTGTTTCATTTAGCAGCCTTCAAATTAAAGCTTTGCAAAATGCAGTTGAAAAGGAAGTCCAAGCAGA<br>AGACAATATCTACATTGAGAATAGTCTTTATGCCACGGAC**TAA**GACCCAGTGGTGCTCTTTGAGAGTT<br>TACGCCCATGACTGCAGAAGACTGAACAGGTATCAGCACATCAGATGTCTTTTAGACTCCAAGACAAT<br>TTTTCTGTTTCAGTTTCATCTGGCATTCCAACATGTCAGTGATACTGGGTAGAGTAACTCTCCCACTC<br>CAAACTGTGTATAGTCAACCTCATCATTAATGTAGTCCTAATTTGTTTTGCTAAAACTGGCTCAATCC<br>TTCTGATCATTGCAGAGTTTTCTCTCAAACATGAACACTTTAGAATTGTATGTTCTCTTTAGACCCCA<br>TAAATCCTGTAT</div> | | | |
| NOV1b, CG57008-01<br>Protein Sequence | SEQ ID NO: 4 | 359 aa | MW at 38703.6kD |
| <div>MHPQVVILSLILHLADSVAGSVKVGGEAGPSVTLPCHYSGAVTSMCWNRGSCSLFTCQNGIVWTNGTH<br>VTYRKDTRYKLLGDLSRRDVSLTIENTAVSDSGVYCCRVEHRGWFNDMKITVSLEIVPPKVTTTPIVT<br>TVPTVTTVRTSTTVPTTTTVPTTTVPTTMSIPTTTTVPTTMTVSTTTSVPTTTSIPTTTSVPVTTTVS<br>TFVPPMPLPRQNHEPVATSPSSPQPAETHPTTLQGAIRREPTSSPLYSYTTDGNDTVTESSDGLWNNN<br>QTQLFLEHSLLTANTTKGIYAGVCISVLVLLALLGVIIAKKYFFKKEVQQLSVSFSSLQIKALQNAVE<br>KEVQAEDNIYIENSLYATD</div> | | | |
| NOV1c, CG57008-02 DNA<br>Sequence | SEQ ID NO: 5 | 789 bp | |
| | ORF Start: at 1 | ORF Stop: end of sequence | |
| <div>**TCT**GTAAAGGTTGGTGGAGAGGCAGGTCCATCTGTCACACTACCCTGCCACTACAGTGGAGCTGTCAC<br>ATCAATGTGCTGGAATAGAGGCTCATGTTCTCTATTCACATGCCAAAATGGCATTGTCTGGACCAATG<br>GAACCCACGTCACCTATCGGAAGGACACACGCTATAAGCTATTGGGGGACCTTTCAAGAAGGGATGTC<br>TCTTTGACCATAGAAAATACAGCTGTGTCTGACAGTGGCGTATATTGTTGCCGTGTTGAGCACCGTGG<br>GTGGTTCAATGACATGAAAATCACCGTATCATTGGAGATTGTGCCACCCAAGGTCACGACTACTCCAA<br>TTGTCACAACTGTTCCAACCGTCACGACTGTTCGAACGAGCACCACTGTTCCAACGACAACGACTGTT<br>CCAACGACAACTGTTCCAACAACAATGAGCATTCCAACGACAACGACTGTTCCGACGACAATGACTGT<br>TTCAACGACAACGAGCGTTCCAACGACAACGAGCATTCCAACAACAACAAGTGTTCCAGTGACAACAA<br>CGGTCTCTACCTTTGTTCCTCCAATGCCTTTGCCCAGGCAGAACCATGAACCAGTAGCCACTTCACCA<br>TCTTCACCTCAGCCAGCAGAAACCCACCCTACGACACTGCAGGGAGCAATAAGGAGAGAACCCACCAG<br>CTCACCATTGTACTCTTACACAACAGATGGGAATGACACCGTGACAGAGTCTTCAGATGGCCTTTGGA<br>ATAACAATCAAACTCAACTGTTCCTAGAACATAGTCTACTG</div> | | | |
| NOV1c, CG57008-02<br>Protein Sequence | SEQ ID NO: 6 | 263 aa | MW at 28270.5kD |
| <div>SVKVGGEAGPSVTLPCHYSGAVTSMCWNRGSCSLFTCQNGIVWTNGTHVTYRKDTRYKLLGDLSRRDV<br>SLTIENTAVSDSGVYCCRVEHRGWFNDMKITVSLEIVPPKVTTTPIVTTVPTVTTVRTSTTVPTTTTV<br>PTTTVPTTMSIPTTTTVPTTMTVSTTTSVPTTTSIPTTTSVPVTTTVSTFVPPMPLPRQNHEPVATSP<br>SSPQPAETHPTTLQGAIRREPTSSPLYSYTTDGNDTVTESSDGLWNNNQTQLFLEHSLL</div> | | | |
| NOV1d, CG57008-04 DNA<br>Sequence | SEQ ID NO: 7 | 343 bp | |
| | ORF Start: at 11 | ORF Stop: end of sequence | |

(continued)

| Table 1A. NOV1 Sequence Analysis |
|---|
| CACCGGATCC**TCT**GTAAAGGTTGGTGGAGAGGCAGGTCCATCTGTCACACTACCCTGCCACTACAGTG GAGCTGTCACATCAATGTGCTGGAATAGAGGCTCATGTTCTCTATTCACATGCCAAAATGGCATTGTC TGGACCAATGGAACCCACGTCACCTATCGGAAGGACACACGCTATAAGCTATTGGGGGACCTTTCAAG AAGGGATGTCTCTTTGACCATAGAAAATACAGCTGTGTCTGACAGTGGCGTATATTGTTGCCGTGTTG AGCACCGTGGGTGGTTCAATGACATGAAAATCACCGTATCATTGGAGATTGTGCCACCCAAG**GTC**GAC GGC |

| NOV1d, CG57008-04 Protein Sequence | SEQ ID NO: 8 | 108 aa | MW at 11916.5kD |
|---|---|---|---|

| |
|---|
| SVKVGGEAGPSVTLPCHYSGAVTSMCWNRGSCSLFTCQNGIVWTNGTHVTYRKDTRYKLLGDLSRRDV SLTIENTAVSDSGVYCCRVEHRGWFNDMKITVSLEIVPPK |

A ClustalW comparison of the above protein sequences yields the following sequence alignment shown in Table 1B.

### Table 1B. Comparison of the NOV1 protein sequences.

```
NOV1a    --------------------SVKVGGEAGPSVTLPCHYSGAVTSMCWNRGSCSLFTCQNG
NOV1b    MHPQVVILSLILHLADSVAGSVKVGGEAGPSVTLPCHYSGAVTSMCWNRGSCSLFTCQNG
NOV1c    --------------------SVKVGGEAGPSVTLPCHYSGAVTSMCWNRGSCSLFTCQNG
NOV1d    --------------------SVKVGGEAGPSVTLPCHYSGAVTSMCWNRGSCSLFTCQNG

NOV1a    IVWTNGTHVTYRKDTRYKLLGDLSRRDVSLTIENTAVSDSGVYCCRVEHRGWFNDMKITV
NOV1b    IVWTNGTHVTYRKDTRYKLLGDLSRRDVSLTIENTAVSDSGVYCCRVEHRGWFNDMKITV
NOV1c    IVWTNGTHVTYRKDTRYKLLGDLSRRDVSLTIENTAVSDSGVYCCRVEHRGWFNDMKITV
NOV1d    IVWTNGTHVTYRKDTRYKLLGDLSRRDVSLTIENTAVSDSGVYCCRVEHRGWFNDMKITV

NOV1a    SLEIVPPKVTTTPIVTTVPTVTTVRTSTTVPTTTTVPTTTVPTTMSIPTTTTVPTTMTVS
NOV1b    SLEIVPPKVTTTPIVTTVPTVTTVRTSTTVPTTTTVPTTTVPTTMSIPTTTTVPTTMTVS
NOV1c    SLEIVPPKVTTTPIVTTVPTVTTVRTSTTVPTTTTVPTTTVPTTMSIPTTTTVPTTMTVS
NOV1d    SLEIVPPK----------------------------------------------------

NOV1a    TTTSVPTTTSIPTTTSVPVTTTVSTFVPPMPLPRQNHEPVATSPSSPQPAETHPTTLQGA
NOV1b    TTTSVPTTTSIPTTTSVPVTTTVSTFVPPMPLPRQNHEPVATSPSSPQPAETHPTTLQGA
NOV1c    TTTSVPTTTSIPTTTSVPVTTTVSTFVPPMPLPRQNHEPVATSPSSPQPAETHPTTLQGA
NOV1d    ------------------------------------------------------------

NOV1a    IRREPTSSPLYSYTTDGNDTVTESSDGLWNNNQTQLFLEHSLLTANTTKGIYAGVCISVL
NOV1b    IRREPTSSPLYSYTTDGNDTVTESSDGLWNNNQTQLFLEHSLLTANTTKGIYAGVCISVL
NOV1c    IRREPTSSPLYSYTTDGNDTVTESSDGLWNNNQTQLFLEHSLL-----------------
NOV1d    ------------------------------------------------------------

NOV1a    VLLALLGVIIAKKYFFKKEVQQLSVSFSSLQIKALQNAVEKEVQAEDNIYIENSLYATD
NOV1b    VLLALLGVIIAKKYFFKKEVQQLSVSFSSLQIKALQNAVEKEVQAEDNIYIENSLYATD
NOV1c    -----------------------------------------------------------
```

```
NOV1d   -----------------------------------------------------------

NOV1a  (SEQ ID NO:  2)
NOV1b  (SEQ ID NO:  4)
NOV1c  (SEQ ID NO:  6)
NOV1d  (SEQ ID NO:  8)
```

Further analysis of the NOV1a protein yielded the following properties shown in Table 1C.

| Table 1C. Protein Sequence Properties NOV1a | |
| --- | --- |
| SignalP analysis: | No Known Signal Sequence Predicted |
| PSORT II analysis: | |
| PSG: | a new signal peptide prediction method |
| | N-region: length 7; pos.chg 1; neg.chg 1 |
| | H-region: length 21; peak value 7.47 |
| | PSG score: 3.07 |
| GvH: | von Heijne's method for signal seq. recognition |
| | GvH score (threshold: -2.1): -7.40 |
| | possible cleavage site: between 24 and 25 |
| >>> Seems to have no N-terminal signal peptide | |
| ALOM: | Klein et al's method for TM region allocation |
| | Init position for calculation: 1 |
| | Tentative number of TMS(s) for the threshold 0.5: 1 |
| | Number of TMS(s) for threshold 0.5: 1 |
| | INTEGRAL Likelihood =-14.49 Transmembrane 275 - 291 |
| | PERIPHERAL Likelihood = 6.58 (at 176) |
| | ALOM score: -14.49 (number of TMSs: 1) |
| MTOP: | Prediction of membrane topology (Hartmann et al.) |
| | Center position for calculation: 282 |
| | Charge difference: 2.5 C( 3.0) - N( 0.5) |
| | C > N: C-terminal side is inside |
| >>>Caution: Inconsistent mtop result with signal peptide | |
| >>> Single TMS is located near the C-terminus | |
| >>> | membrane topology: type Nt (cytoplasmic tail 1 to 274) |
| MITDISC: discrimination of mitochondrial targeting seq | |
| | R content: 2 Hyd Moment (75) : 7.31 |
| | Hyd Moment (95) : 9.99 G content: 7 |
| | D/E content: 2 S/T content: 12 |
| | Score: -4.31 |
| Gavel: | prediction of cleavage sites for mitochondrial preseq |
| | R-2 motif at 62 YRK|DT |
| NUCDISC: discrimination of nuclear localization signals | |

(continued)

| Table 1C. Protein Sequence Properties NOV1a | |
|---|---|
| SignalP analysis: | No Known Signal Sequence Predicted |
| PSORT II analysis: | |

pat4: none
pat7: none
bipartite: none
content of basic residues: 6.8%
NLS Score: -0.47

KDEL:          ER retention motif in the C-terminus: none

ER Membrane Retention Signals: none

SKL:          peroxisomal targeting signal in the C-terminus: none

PTS2:          2nd peroxisomal targeting signal: none

VAC:          possible vacuolar targeting motif: none

RNA-binding motif: none

Actinin-type actin-binding motif:
type 1: none
type 2: none

NMYR:          N-myristoylation pattern : none

Prenylation motif: none

memYQRL:          transport motif from cell surface to Golgi: none

Tyrosines in the tail: too long tail

Dileucine motif in the tail: found
LL at 59
LL at 262

checking 63 PROSITE DNA binding motifs: none

checking 71 PROSITE ribosomal protein motifs: none

checking 33 PROSITE prokaryotic DNA binding motifs: none

NNCN:          Reinhardt's method for Cytoplasmic/Nuclear discrimination
Prediction: nuclear
Reliability: 55.5

COIL:          Lupas's algorithm to detect coiled-coil regions
total: 0 residues

------------------------

(continued)

| Table 1C. Protein Sequence Properties NOV1a | |
|---|---|
| SignalP analysis: | No Known Signal Sequence Predicted |
| PSORT II analysis: | |
| Final Results (k = 9/23): | |

30.4 %: nuclear
21.7 %: cytoplasmic
13.0 %: mitochondrial
13.0 %: Golgi
8.7 %: vesicles of secretory system
8.7 %: endoplasmic reticulum
4.3 %: peroxisomal

» prediction for CG57008-03 is nuc (k=23)

A search of the NOV1protein against the Geneseq database, a proprietary database that contains sequences published in patents and patent publication, yielded several homologous proteins shown in Table 1D.

| Table 1D. Geneseq Results for NOV1a | | | | |
|---|---|---|---|---|
| Geneseq Identifier | Protein/Organism/Length [Patent #, Date] | NOV1a Residues/ Match Residues | Identities/ Similarities for the Matched Region | Expect Value |
| AAW38336 | Human kidney injury related molecule (KIM) - Homo sapiens, 334 aa. [WO9744460-A1, 27-NOV-1997] | 1..303 21..323 | 303/303 (100%) 303/303 (100%) | e-177 |
| AAR92803 | Hepatitis A virus receptor - Cercopithecus aethiops, 451 aa. [WO9604376-A1, 15-FEB-1996] | 1..337 21..437 | 264/417 (63%) 302/417 (72%) | e-145 |
| AAW38334 | Rat kidney injury related molecule (KIM) - Rattus sp, 307 aa. [WO9744460-A1, 27-NOV-1997] | 4..331 25..298 | 135/332 (40%) 177/332 (52%) | 8e-52 |
| AAM39027 | Human polypeptide SEQ ID NO 2172 - Homo sapiens, 378 aa. [WO200153312-A1, 26-JUL-2001] | 9..214 33..222 | 86/209 (41%) 113/209 (53%) | 3e-32 |
| AAY25768 | Human secreted protein encoded from gene 58 - Homo sapiens, 379 aa. [WO9938881-A1, 05-AUG-1999] | 9..214 33..222 | 86/209 (41%) 113/209 (53%) | 3e-32 |

In a BLAST search of public sequence databases, the NOV1protein was found to have homology to the proteins shown in the BLASTP data in Table 1E.

| Table 1E. Public BLASTP Results for NOV1a | | | | |
|---|---|---|---|---|
| Protein Accession Number | Protein/Organism/Length | NOV1a Residues/ Match Residues | Identities/ Similarities for the Matched Portion | Expect Value |
| O43656 | Hepatitis A virus cellular receptor 1 - Homo sapiens (Human), 359 aa. | 1..339 21..359 | 339/339 (100%) 339/339 (100%) | 0.0 |

(continued)

| Protein Accession Number | Protein/Organism/Length | NOV1a Residues/ Match Residues | Identities/ Similarities for the Matched Portion | Expect Value |
|---|---|---|---|---|
| Q96D42 | Hypothetical protein - Homo sapiens (Human), 364 aa. | 1..339 21..364 | 338/344 (98%) 338/344 (98%) | 0.0 |
| O46598 | Hepatitis A virus cellular receptor 1 long form (Hepatitis A virus cellular receptor 1 short form) - Cercopithecus aethiops (Green monkey) (Grivet), 478 aa. | 1..337 26..464 | 261/439 (59%) 301/439 (68%) | e-139 |
| O18984 | Hepatitis A virus receptor - Cercopithecus aethiops (Green monkey) (Grivet), 460 aa. | 99..337 209..446 | 178/239 (74%) 203/239 (84%) | 6e-99 |
| O46597 | Hepatitis A virus cellular receptor 1 long form (Hepatitis A virus cellular receptor 1 short form) - Cercopithecus aethiops (Green monkey) (Grivet), 474 aa. | 99..337 223..460 | 177/239 (74%) 205/239 (85%) | 1e-98 |

*Table 1E. Public BLASTP Results for NOV1a*

PFam analysis predicts that the NOV1a protein contains the domains shown in the Table 1F.

**Table 1F. Domain Analysis of NOVla**

| Pfam Domain | NOV1a Match Region | Identities/ Similarities for the Matched Region | Expect Value |
|---|---|---|---|
| ig | 9..87 | 19/84 (23%) 54/84 (64%) | 0.0059 |

A full-length human kidney injury related molecule was recently cloned and is described in WO97/44460, which is incorporated by reference in its entirety.

**Table 1G. Brief description of relevant variants**

| CGUID-Variant | Description | Size |
|---|---|---|
| CG57008-01 (NOV1b) | Full length protein | 359 aa |
| CG57008-02 (NOV1c) | Soluble extracellular domain | 263 aa |
| CG57008-03 (NOV1a) | Mature Protein (first 20aa comprising the signal sequence has been removed) | 339 aa |
| CG57008-04 (NOV1d) | N-terminal extracellular fragment | 108aa |

**NOV2:**

A NOV2 clone was analyzed, and the nucleotide and encoded polypeptide sequences are shown in Table 2A.

**Table 2A. NOV2 Sequence Analysis**

| | SEQ ID NO: 9 | 2336 bp | |
|---|---|---|---|

(continued)

| Table 2A. NOV2 Sequence Analysis |
|---|

| NOV2a, CG51373-12 DNA Sequence | CACCAAGCTT**ATG**CTGAGCCTCCTCGTCTGGATCCTCACTCTCTCCGATACTTTCTCC CAAGGGACCCAGACCCGCTTCAGCCAGGAGCCAGCTGACCAGACGGTGGTGGCTGGAC AGCGGGCCGTGCTCCCCTGTGTGCTGCTCAACTACTCTGGAATTGTGCAATGGACCAA GGACGGGCTGGCCCTGGGCATGGGCCAGGGCCTCAAAGCCTGGCCACGGTACCGGGTT GTGGGCTCCGCAGACGCTGGGCAGTACAACCTGGAGATCACAGATGCTGAGCTCTCTG ACGACGCCTCTTACGAGTGCCAGGCCACGGAGGCCGCCCTGCGCTCTCGGCGGGCCAA ACTCACCGTGCTCATCCCCCCAGAGGACACCAGGATTGACGGAGGCCCTGTGATTCTA CTGCAGGCAGGCACCCCCCACAACCTCACATGCCGGGCCTTCAATGCGAAGCCTGCTG CCACCATCATCTGGTTCCGGGACGGGACGCAGCAGGAGGGCGCTGTGGCCAGCACGGA ATTGCTGAAGGATGGGAAGAGGGAGACCACCGTGAGCCAACTGCTTATTAACCCCACG GACCTGGACATAGGGCGTGTCTTCACTTGCCGAAGCATGAACGAAGCCATCCCTAGTG GCAAGGAGACTTCCATCGAGCTGGATGTGCACCACCCTCCTACAGTGACCCTGTCCAT TGAGCCACAGACGGTGCAGGAGGGTGAGCGTGTTGTCTTTACCTGCCAGGCCACAGCC AACCCCGAGATCTTGGGCTACAGGTGGGCCAAAGGGGGTTTCTTGATTGAAGACGCCC ACGAGAGTCGCTATGAGACAAATGTGGATTATTCCTTTTTCACGGAGCCTGTGTCTTG TGAGGTTCACAACAAAGTGGGAAGCACCAATGTCAGCACTTTAGTAAATGTCCACTTT GCTCCCCGGATTGTAGTTGACCCCAAACCCACAACCACAGACATTGGCTCTGATGTGA CCCTTACCTGTGTCTGGGTTGGGAATCCCCCCCTCACTCTCACCTGGACCAAAAAGGA CTCAAATATGGGGCCCAGGCCTCCTGGCTCCCCACCCGAGGCTGCTCTCTCTGCCCAG GTCCTGAGTAACAGCAACCAGCTGCTGCTGAAGTCGGTGACTCAGGCAGACGCTGGCA CCTACACCTGCCGGGCCATCGTGCCTCGAATCGGAGTGGCTGAGCGGGAGGTGCCGCT CTATGTGAACGGGCCCCCCATCATCTCCAGTGAGGCAGTGCAGTATGCTGTGAGGGGT GACGGTGGCAAGGTGGAGTGTTTCATTGGGAGCACACCACCCCCAGACCGCATAGCAT GGGCCTGGAAGGAGAACTTCTTGGAGGTGGGGACCCTGGAACGCTATACAGTGGAGAG GACCAACTCAGGCAGTGGGGTGCTATCCACGCTCACCATCAACAATGTCATGGAGGCC GACTTTCAGACTCACTACAACTGCACCGCCTGGAACAGCTTCGGGCCAGGCACAGCCA TCATCCAGCTGGAAGAGCGAGAGGTGTTACCTGTGGGCATCATAGCTGGGGCCACCAT CGGCGCGAGCATCCTGCTCATCTTCTTCTTCATCGCCTTGGTATTCTTCCTCTACCGG |

| | CGCCGCAAAGGCAGTCGCAAAGACGTGACCCTGAGGAAGCTGGATATCAAGGTGGAGA CAGTGAACCGAGAGCCACTTACGATGCATTCTGACCGGGAGGATGACACCGCCAGCGT CTCCACAGCAACCCGGGTCATGAAGGCCATCTACTCGTCGTTTAAGGATGATGTGGAT CTGAAGCAGGACCTGCGCTGCGACACCATCGACACCCGGGAGGAGTATGAGATGAAGG ACCCCACCAATGGCTACTACAACGTGCGTGCCCATGAAGACGCCCGTCTTCCAGGGC AGTGCTCTATGCTGACTACCGTGCCCCTGGCCCTGCCCGCTTCGACGGCCGCCCCTCA TCCCGTCTCTCCCACTCCAGCGGCTATGCCCAGCTCAACACCTATAGCCGGGGCCCTG CCTCTGACTATGGCCCTGAGCCCACACCCCCTGGCCCTGCTGCCCCAGCTGGCACTGA CACAACCAGCCAGCTGTCCTACGAGAACTATGAGAAGTTCAACTCCCATCCCTTCCCT GGGGCAGCTGGGTACCCCACCTACCGACTGGGCTACCCCCAGGCCCCACCCTCTGGCC TGGAGCGGACCCCATATGAGGCGTATGACCCCATTGGCAAGTACGCCACAGCCACTCG ATTCTCCTACACCTCCCAGCACTCGGACTACGGCCAGCGATTCCAGCAGCGCATGCAG ACTCACGTG**GTC**GACG |

| | ORF Start: ATG at 11 | | ORF Stop: at 2330 |
| | SEQ ID NO: 10 | 773 aa | MW at 85048.5kD |

# EP 1 576 169 B1

(continued)

| Table 2A. NOV2 Sequence Analysis | | | |
|---|---|---|---|
| NOV2a, CG51373-12 Protein Sequence | MLSLLVWILTLSDTFSQGTQTRFSQEPADQTVVAGQRAVLPCVLLNYSGIVQWTKDGL ALGMGQGLKAWPRYRVVGSADAGQYNLEITDAELSDDASYECQATEAALRSRRAKLTV LIPPEDTRIDGGPVILLQAGTPHNLTCRAFNAKPAATIIWFRDGTQQEGAVASTELLK DGKRETTVSQLLINPTDLDIGRVFTCRSMNEAIPSGKETSIELDVHHPPTVTLSIEPQ TVQEGERVVFTCQATANPEILGYRWAKGGFLIEDAHESRYETNVDYSFFTEPVSCEVH NKVGSTNVSTLVNVHFAPRIVVDPKPTTTDIGSDVTLTCVWVGNPPLTLTWTKKDSNM GPRPPGSPPEAALSAQVLSNSNQLLLKSVTQADAGTYTCRAIVPRIGVAEREVPLYVN GPPIISSEAVQYAVRGDGGKVECFIGSTPPPDRIAWAWKENFLEVGTLERYTVERTNS GSGVLSTLTINNVMEADFQTHYNCTAWNSFGPGTAIIQLEEREVLPVGIIAGATIGAS ILLIFFFIALVFFLYRRRKGSRKDVTLRKLDIKVETVNREPLTMHSDREDDTASVSTA TRVMKAIYSSFKDDVDLKQDLRCDTIDTREEYEMKDPTNGYYNVRAHEDRPSSRAVLY ADYRAPGPARFDGRPSSRLSHSSGYAQLNTYSRGPASDYGPEPTPPGPAAPAGTDTTS QLSYENYEKFNSHPFPGAAGYPTYRLGYPQAPPSGLERTPYEAYDPIGKYATATRFSY TSQHSDYGQRFQQRMQTHV | | |
| | SEQ ID NO: 11 | 3464 bp | |
| NOV2b, CG51373-02 DNA Sequence | **ATG**CATTTGACTCTGGAAGTCTTAAACCATGGCCCCTTCCCTCTAAACCTTTCCTCCA TTGCTTACAATCATGGAACTGTGTTTGGCCACTGGAAGAATAACGTCACTCGGGAAAC GCTGGTGAAAGTAAAAGATGCTGAAGATCAGTTGGGTGCACGAGTGGGTTACATCGAA CTGGATCTCAACAGCGGGAAGGAAACATTTCTGGTGAATGAGGAGGCAACGGGCGAGA CCTCAGGAGACAATGTTGTTCATTCTAGGAATCTGTCTCAGACAATCTTCATCACCCG GAAACGATGGGAGGGGACCCAGACCCGCTTCAGCCAGGAGCCAGCTGACCAGACGGTG GTGGCTGGACAGCGGGCCGTGCTCCCCTGTGTGCTGCTCAACTACTCTGGAATTGTGC AATGGACCAAGGACGGGCTGGCCCTGGGCATGGGCCAGGCCCTCAAAGCCTGGCCACG GTACCGGGTTGTGGGCTCCGCAGACGCTGGGCAGTACAACCTGGAGATCACAGATGCT GAGCTCTCTGACGACGCCTCTTACGAGTGCCAGGCCACGGAGGCCGCCCTGCGCTCTC GGCGGGCCAAACTCACCGTGCTCATCCCCCCAGAGGACACCAGGATTGACGGAGGCCC TGTGATTCTACTGCAGGCAGGCACCCCCCACAACCTCACATGCCGGGCCTTCAATGCG AAGCCTGCTGCCACCATCATCTGGTTCCGGGACGGGACGCAGCAGGAGGGCGCTGTGG CCAGCACGGAATTGCTGAAGGATGGGAAGAGGGAGACCACCGTGAGCCAACTGCTTAT TAACCCCACGGACCTGGACATAGGGCGTGTCTTCACTTGCCGAAGCATGAACGAAGCC ATCCCTAGTGGCAAGGAGACTTCCATCGAGCTGGATGTGCACCACCCTCCTACAGTGA CCCTGTCCATTGAGCCACAGACGGTGCAGGAGGGTGAGCGTGTTGTCTTTACCTGCCA GGCCACAGCCAACCCCGAGATCTTGGGCTACAGGTGGGCCAAAGGGGGTTTCTTGATT GAAGACGCCCACGAGAGTCGCTATGAGACAAATGTGGATTATTCCTTTTTCACGGAGC CTGTGTCTTGTGAGGTTCACAACAAAGTGGGAAGCACCAATGTCAGCACTTTAGTAAA TGTCCACTTTGCTCCCCGGATTGTAGTTGACCCCAAACCCACAACCACAGACATTGGC TCTGATGTGACCCTTACCTGTGTCTGGGTTGGGAATCCCCCCCTCACTCTCACCTGGA CCAAAAAGGACTCAAATATGGTCCTGAGTAACAGCAACCAGCTGCTGCTGAAGTCGGT GACTCAGGCAGACGCTGGCACCTACACCTGCCGGGCCATCGTGCCTCGAATCGGAGTG GCTGAGCGGGAGGTGCCGCTCTATGTGAACGGGCCCCCCATCATCTCCAGTGAGGCAG | | |

17

**Table 2A. NOV2 Sequence Analysis**

<table>
<tr><td>
TGCAGTATGCTGTGAGGGGTGACGGTGGCAAGGTGGAGTGTTTCATTGGGAGCACACC<br>
ACCCCCAGACCGCATAGCATGGGCCTGGAAGGAGAACTTCTTGGAGGTGGGGACCCTG<br>
GAACGCTATACAGTGGAGAGGACCAACTCAGGCAGTGGGGTGCTATCCACGCTCACCA<br>
TCAACAATGTCATGGAGGCCGACTTTCAGACTCACTACAACTGCACCGCCTGGAACAG<br>
CTTCGGGCCAGGCACAGCCATCATCCAGCTGGAAGAGCGAGAGGTGTTACCTGTGGGC<br>
ATCATAGCTGGGGCCACCATCGGCGCGAGCATCCTGCTCATCTTCTTCTTCATCGCCT<br>
TGGTATTCTTCCTCTACCGGCGCCGCAAAGGCAGTCGCAAAGACGTGACCCTGAGGAA<br>
GCTGGATATCAAGGTGGAGACAGTGAACCGAGAGCCACTTACGATGCATTCTGACCGG<br>
GAGGATGACACCGCCAGCGTCTCCACAGCAACCCGGGTCATGAAGGCCATCTACTCGT<br>
CGTTTAAGGATGATGTGGATCTGAAGCAGGACCTGCGCTGCGACACCATCGACACCCG<br>
GGAGGAGTATGAGATGAAGGACCCCACCAATGGCTACTACAACGTGCGTGCCCATGAA<br>
GACCGCCCGTCTTCCAGGGCAGTGCTCTATGCTGACTACCGTGCCCCTGGCCCTGCCC<br>
GCTTCGACGGCCGCCCCTCATCCCGTCTCTCCCACTCCAGCGGCTATGCCCAGCTCAA<br>
CACCTATAGCCGGGGCCCTGCCTCTGACTATGGCCCTGAGCCCACACCCCCTGGCCCT<br>
GCTGCCCCAGCTGGCACTGACACAACCAGCCAGCTGTCCTACGAGAACTATGAGAAGT<br>
TCAACTCCCATCCCTTCCCTGGGGCAGCTGGGTACCCCACCTACCGACTGGGCTACCC<br>
CCAGGCCCCACCCTCTGGCCTGGAGCGGACCCCATATGAGGCGTATGACCCCATTGGC<br>
AAGTACGCCACAGCCACTCGATTCTCCTACACCTCCCAGCACTCGGACTACGGCCAGC<br>
<u>GATTCCAGCAGCGCATGCAGACTCACGTG**TAG**GGGCCAGAGCCTGGCTGGGGCATCTC</u><br>
<u>TGCGGGGCAGAGGAGAAGGCTTTCGCAGCTGTTCCCTGATATTCAGGGACATTGCTCA</u><br>
<u>TTGCTCCCTTCTCGGACCAGCCTTCTTCCTCCCACCATGGCAGGTGGGGAGCAGGTCT</u><br>
<u>CCCAGAGACACCCCGTCCCGAGGATGGTGCTCTGTGCATGCCCCAGCCTCCTGGGCCT</u><br>
<u>GCCCTTCCCTCTTCTTCGGGAGGATGTGTCTCTTCTGACCTGCACTCTTGCCTGACCC</u><br>
<u>TAGAATGGGGACAGGGAAAGTGAAGGTTAGGGAAAGCAGAGGGGGGCACTTTTTAGCA</u><br>
<u>TTCCCTTTCTATCCCACCCCTCTGATCTCCCATAAGTGGAAATGGGGGTACCCAGGGA</u><br>
<u>TGGGCAGGCTTTGGCCTAGGGACATGAAGTATGGGAGTGGGTGGCTGTGGCACAGACA</u><br>
<u>GGTGGAAAACGGGATAGCCTGGCCAGTCCCTCTGTTGTCTGCATTCGTGCCCTGGGTG</u><br>
<u>CCTCTCTCCTTCCTCAGGGTACTGCAGAAGGGAGCGAACAGGGTACTGTTCGCTCTTG</u><br>
<u>TCTACAGAACAGCCCTGGCACTGCATTCAAATCCAGTCTTCATTCAGCTGGGATCAAA</u><br>
<u>ATGCCAGTCACCTTGGCTACCCACTGTGGACAGCTGTCTGTCAGCATGCAGAGGGATC</u><br>
<u>CAGGAATCCCCCCGGCAGCACGGCCCGCTTTCCTTCTCCTCCATGCTGGGCCAGCCAG</u><br>
<u>ATAAGTCAGGGTCCTGGTGGAGAAAGAAAGGCTAGGACCATGTCCTCATTGACCCAGA</u><br>
<u>TACTGCTGTGTGCTGCACAGCAGTGAACCAACACTAGAGGGAGCCACACAAGCCTCCT</u><br>
<u>CTCCCCAGTCTGCCCCACTTCCTGGCTTTAACTCTTGAGCTGGTTTGGGGAGTGGTGA</u><br>
<u>GGTAGGGGTGGGGGTGCTGTAGGCTCTTTTTCAAAAAAAAAC</u>
</td></tr>
</table>

| ORF Start: ATG at 1 | | ORF Stop: TAG at 2524 |
|---|---|---|
| SEQ ID NO: 12 | 841 aa | MW at 92988.2kD |
| NOV2b,<br>CG51373-02<br>Protein<br>Sequence | MHLTLEVLNHGPFPLNLSSIAYNHGTVFGHWKNNVTRETLVKVKDAEDQLGARVGYIE<br>LDLNSGKETFLVNEEATGETSGDNVVHSRNLSQTIFITRKRWEGTQTRFSQEPADQTV<br>VAGQRAVLPCVLLNYSGIVQWTKDGLALGMGQALKAWPRYRVVGSADAGQYNLEITDA<br>ELSDDASYECQATEAALRSRRAKLTVLIPPEDTRIDGGPVILLQAGTPHNLTCRAFNA<br>KPAATIIWFRDGTQQEGAVASTELLKDGKRETTVSQLLINPTDLDIGRVFTCRSMNEA<br>IPSGKETSIELDVHHPPTVTLSIEPQTVQEGERVVFTCQATANPEILGYRWAKGGFLI<br>EDAHESRYETNVDYSFFTEPVSCEVHNKVGSTNVSTLVNVHFAPRIVVDPKPTTTDIG<br>SDVTLTCVWVGNPPLTLTWTKKDSNMVLSNSNQLLLKSVTQADAGTYTCRAIVPRIGV<br>AEREVPLYVNGPPIISSEAVQYAVRGDGGKVECFIGSTPPPDRIAWAWKENFLEVGTL<br>ERYTVERTNSGSGVLSTLTINNVMEADFQTHYNCTAWNSFGPGTAIIQLEEREVLPVG<br>IIAGATIGASILLIFFFIALVFFLYRRRKGSRKDVTLRKLDIKVETVNREPLTMHSDR<br>EDDTASVSTATRVMKAIYSSFKDDVDLKQDLRCDTIDTREEYEMKDPTNGYYNVRAHE<br>DRPSSRAVLYADYRAPGPARFDGRPSSRLSHSSGYAQLNTYSRGPASDYGPEPTPPGP<br>AAPAGTDTTSQLSYENYEKFNSHPFPGAAGYPTYRLGYPQAPPSGLERTPYEAYDPIG<br>KYATATRFSYTSQHSDYGQRFQQRMQTHV | |
| | SEQ ID NO: 13 | 3379 bp | |
| NOV2c, | <u>CTCTCCGATACTTTCTCCCAAGGGTCAGCTGCTTCTTCATTCCAAGTGGACAAGGAGC</u><br><u>CAGCTGCTCACTGTCCTTGAGAGACTTCAGCGAGAGACCAGGGTGTCCAGGCTCCATG</u> | |

(continued)

| Table 2A. NOV2 Sequence Analysis | | |
|---|---|---|
| CG51373-03 DNA Sequence | CAGGAAAGCCATGCGTATAAATTCCACCTCTGAGCCAGGCCTCACCAGCAAGCCCACT<br>CTTAAGCCCTTGACTTGGGCTCCAGGGGCCATGGGAAGGAGAAACGGACCCAGACCCG<br>CTTCAGCCAGGAGCCAGCTGACCAGACGGTGGTGGCTGGACAGCGGGCCGTGCTCCCC<br>TGTGTGCTGCTCAACTACTCTGGAATTGTGCAATGGACCAAGGACGGGCTGGCCCTGG<br>GCATGGGCCAGGCCCTCAAAGCCTGGCCACGGTACCGGGTTGTGGGCTCCGCAGACGC<br>TGGGCAGTACAACCTGGAGATCACAGATGCTGAGCTCTCTGACGACGCCTCTTACGAG<br>TGCCAGGCCACGGAGGCCGCCCTGCGCTCTCGGCGGGCCAAACTCACCGTGCTCATCC<br>CCCCAGAGGACACCAGGATTGACGGAGGCCCTGTGATTCTACTGCAGGCAGGCACCCC<br>CCACAACCTCACATGCCGGGCCTTCAATGCGAAGCCTGCTGCCACCATCATCTGGTTC<br>CGGGACGGGACGCAGCAGGAGGGCGCTGTGGCCAGCACGGAATTGCTGAAGGATGGGA<br>AGAGGGAGACCACCGTGAGCCAACTGCTTATTAACCCCACGGACCTGGACATAGGGCG<br>TGTCTTCACTTGCCGAAGCATGAACGAAGCCATCCCTAGTGGCAAGGAGACTTCCATC<br>GAGCTGGATGTGCACCACCCTCCTACAGTGACCCTGTCCATTGAGCCACAGACGGTGC<br>AGGAGGGTGAGCGTGTTGTCTTTACCTGCCAGGCCACAGCCAACCCCGAGATCTTGGG<br>CTACAGGTGGGCCAAAGGGGGTTTCTTGATTGAAGACGCCCACGAGAGTCGCTATGAG<br>ACAAATGTGGATTATTCCTTTTTCACGGAGCCTGTGTCTTGTGAGGTTCACAACAAAG<br>TGGGAAGCACCAATGTCAGCACTTTAGTAAATGTCCACTTTGCTCCCCGGATTGTAGT<br>TGACCCCAAACCCACAACCACAGACATTGGCTCTGATGTGACCCTTACCTGTGTCTGG<br>GTTGGGAATCCCCCCCTCACTCTCACCTGGACCAAAAAGGACTCAAATATGGTCCTGA<br>GTAACAGCAACCAGCTGCTGCTGAAGTCGGTGACTCAGGCAGACGCTGGCACCTACAC<br>CTGCCGGGCCATCGTGCCTCGAATCGGAGTGGCTGAGCGGGAGGTGCCGCTCTATGTG<br>AACGGGCCCCCCATCATCTCCAGTGAGGCAGTGCAGTATGCTGTGAGGGGTGACGGTG<br>GCAAGGTGGAGTGTTTCATTGGGAGCACACCACCCCCAGACCGCATAGCATGGGCCTG<br>GAAGGAGAACTTCTTGGAGGTGGGGACCCTGGAACGCTATACAGTGGAGAGGACCAAC<br>TCAGGCAGTGGGGTGCTATCCACGCTCACCATCAACAATGTCATGGAGGCCGACTTTC<br>AGACTCACTACAACTGCACCGCCTGGAACAGCTTCGGGCCAGGCACAGCCATCATCCA<br>GCTGGAAGAGCGAGAGGTGTTACCTGTGGGCATCATAGCTGGGGCCACCATCGGCGCG<br>AGCATCCTGCTCATCTTCTTCTTCATCGCCTTGGTATTCTTCCTCTACCGGCGCCGCA<br>AAGGCAGTCGCAAAGACGTGACCCTGAGGAAGCTGGATATCAAGGTGGAGACAGTGAA<br>CCGAGAGCCACTTACGATGCATTCTGACCGGGAGGATGACACCGCCAGCGTCTCCACA<br>GCAACCCGGGTCATGAAGGCCATCTACTCGTCGTTTAAGGATGATGTGGATCTGAAGC<br>AGGACCTGCGCTGCGACACCATCGACACCCGGGAGGAGTATGAGATGAAGGACCCCAC<br>CAATGGCTACTACAACGTGCGTGCCCATGAAGACCGCCCGTCTTCCAGGGCAGTGCTC<br>TATGCTGACTACCGTGCCCCTGGCCCTGCCCGCTTCGACGGCCGCCCCTCATCCCGTC<br>TCTCCCACTCCAGCGGCTATGCCCAGCTCAACACCTATAGCCGGGGCCCTGCCTCTGA<br>CTATGGCCCTGAGCCCACACCCCCTGGCCCTGCTGCCCCAGCTGGCACTGACACAACC<br>AGCCAGCTGTCCTACGAGAACTATGAGAAGTTCAACTCCCATCCCTTCCCTGGGGCAG<br>CTGGGTACCCCACCTACCGACTGGGCTACCCCCAGGCCCCACCCTCTGGCCTGGAGCG<br>GACCCCATATGAGGCGTATGACCCCATTGGCAAGTACGCCACAGCCACTCGATTCTCC<br>TACACCTCCCAGCACTCGGACTACGGCCAGCGATTCCAGCAGCGCATGCAGACTCACG<br>TGTAGGGGCCAGAGCCTGGCTGGGGCATCTCTGCGGGGCAGAGGAGAAGGCTTTCGCA<br>GCTGTTCCCTGATATTCAGGGACATTGCTCATTGCTCCCTTCTCGGACCAGCCTTCTT<br>CCTCCCACCATGGCAGGTGGGGAGCAGGTCTCCCAGAGACACCCCGTCCCGAGGATGG<br>TGCTCTGTGCATGCCCCAGCCTCCTGGGCCTGCCCTTCCCTCTTCTTCGGGAGGATGT<br>GTCTCTTCTGACCTGCACTCTTGCCTGACCCTAGAATGGGGACAGGGAAAGTGAAGGT<br>TAGGGAAAGCAGAGGGGGGCACTTTTTAGCATTCCCTTTCTATCCCACCCCTCTGATC<br>TCCCATAAGTGGAAATGGGGGTACCCAGGGATGGGCAGGCTTTGGCCTAGGGACATGA<br>AGTATGGGAGTGGGTGGCTGTGGCACAGACAGGTGGAAAACGGGATAGCCTGGCCAGT<br>CCCTCTGTTGTCTGCATTCGTGCCCTGGGTGCCTCTCTCCTTCCTCAGGGTACTGCAG<br>AAGGGAGCGAACAGGGTACTGTTCGCTCTTGTCTACAGAACAGCCCTGGCACTGCATT<br>CAAATCCAGTCTTCATTCAGCTGGGATCAAAATGCCAGTCACCTTGGCTACCCACTGT<br>GGACAGCTGTCTGTCAGCATGCAGAGGGATCCAGGAATCCCCCGGCAGCACGGCCCG<br>CTTTCCTTCTCCTCCATGCTGGGCCAGCCAGATAAGTCAGGGTCCTGGTGGAGAAAGA<br>AAGGCTAGGACCATGTCCTCATTGACCCAGATACTGCTGTGTGCTGCACAGCAGTGAA<br>CCAACACTAGAGGGAGCCACACAAGCCTCCTCTCCCCAGTCTGCCCCACTTCCTGGCT<br>TTAACTCTTGAGCTGGTTTGGGGAGTGGTGAGGTAGGGGTGGGGGTGCTGTAGGCTCT<br>TTTTCAAAAAAAAAC |  |  |
| | ORF Start: ATG at 351 | | ORF Stop: TAG at 2439 |

(continued)

| Table 2A. NOV2 Sequence Analysis | | | |
|---|---|---|---|
| | SEQ ID NO: 14 | 696 aa | MW at 76928.3kD |
| NOV2c, CG51373-03 Protein Sequence | MGQALKAWPRYRVVGSADAGQYNLEITDAELSDDASYECQATEAAALRSRRAKLTVLIP PEDTRIDGGPVILLQAGTPHNLTCRAFNAKPAATIIWFRDGTQQEGAVASTELLKDGK RETTVSQLLINPTDLDIGRVFTCRSMNEAIPSGKETSIELDVHHPPTVTLSIEPQTVQ EGERVVFTCQATANPEILGYRWAKGGFLIEDAHESRYETNVDYSFFTEPVSCEVHNKV GSTNVSTLVNVHFAPRIVVDPKPTTTDIGSDVTLTCVWVGNPPLTLTWTKKDSNMVLS NSNQLLLKSVTQADAGTYTCRAIVPRIGVAEREVPLYVNGPPIISSEAVQYAVRGDGG KVECFIGSTPPPDRIAWAWKENFLEVGTLERYTVERTNSGSGVLSTLTINNVMEADFQ THYNCTAWNSFGPGTAIIQLEEREVLPVGIIAGATIGASILLIFFFIALVFFLYRRRK GSRKDVTLRKLDIKVETVNREPLTMHSDREDDTASVSTATRVMKAIYSSFKDDVDLKQ DLRCDTIDTREEYEMKDPTNGYYNVRAHEDRPSSRAVLYADYRAPGPARFDGRPSSRL SHSSGYAQLNTYSRGPASDYGPEPTPPGPAAPAGTDTTSQLSYENYEKFNSHPFPGAA GYPTYRLGYPQAPPSGLERTPYEAYDPIGKYATATRFSYTSQHSDYGQRFQQRMQTHV |
| | SEQ ID NO: 15 | | 1407 bp |
| NOV2d, CG51373-07 DNA Sequence | **CGC**TTCAGCCAGGAGCCAGCTGACCAGACGGTGGTGGCTGGACAGCGGGCCGTGCTCC CCTGTGTGCTGCTCAACTACTCTGGAATTGTGCAATGGACCAAGGACGGGCTGGCCCT GGGCATGGGCCAGGCCCTCAAAGCCTGGCCACGGTACCGGGTTGTGGGCTCCGCAGAC GCTGGGCAGTACAACCTGGAGATCACAGATGCTGAGCTCTCTGACGACGCCTCTTACG AGTGCCAGGCCACGGAGGCCGCCCTGCGCTCTCGGCGGGCCAAACTCACCGTGCTCAT CCCCCCAGAGGACACCAGGATTGACGGAGGCCCTGTGATTCTACTGCAGGCAGGCACC CCCCACAACCTCACATGCCGGGCCTTCAATGCGAAGCCTGCTGCCACCATCATCTGGT TCCGGGACGGGACGCAGCAGGAGGGCGCTGTGGCCAGCACGGAATTGCTGAAGGATGG GAAGAGGGAGACCACCGTGAGCCAACTGCTTATTAACCCCACGGACCTGGACATAGGG CGTGTCTTCACTTGCCGAAGCATGAACGAAGCCATCCCTAGTGGCAAGGAGACTTCCA TCGAGCTGGATGTGCACCACCCTCCTACAGTGACCCTGTCCATTGAGCCACAGACGGT GCAGGAGGGTGAGCGTGTTGTCTTTACCTGCCAGGCCACAGCCAACCCCGAGATCTTG GGCTACAGGTGGGCCAAAGGGGGTTTCTTGATTGAAGACGCCCACGAGAGTCGCTATG AGACAAATGTGGATTATTCCTTTTTCACGGAGCCTGTGTCTTGTGAGGTTCACAACAA AGTGGGAAGCACCAATGTCAGCACTTTAGTAAATGTCCACTTTGCTCCCCGGATTGTA GTTGACCCCAAACCCACAACCACAGACATTGGCTCTGATGTGACCCTTACCTGTGTCT GGGTTGGGAATCCCCCCCTCACTCTCACCTGGACCAAAAAGGACTCAAATATGGTCCT GAGTAACAGCAACCAGCTGCTGCTGAAGTCGGTGACTCAGGCAGACGCTGGCACCTAC ACCTGCCGGGCCATCGTGCCTCGAATCGGAGTGGCTGAGCGGGAGGTGCCGCTCTATG TGAACGGGCCCCCCATCATCTCCAGTGAGGCAGTGCAGTATGCTGTGAGGGGTGACGG TGGCAAGGTGGAGTGTTTCATTGGGAGCACACCACCCCCAGACCGCATAGCATGGGCC TGGAAGGAGAACTTCTTGGAGGTGGGGACCCTGGAACGCTATACAGTGGAGAGGACCA ACTCAGGCAGTGGGGTGCTATCCACGCTCACCATCAACAATGTCATGGAGGCCGACTT TCAGACTCACTACAACTGCACCGCCTGGAACAGCTTCGGGCCAGGCACAGCCATCATC CAGCTGGAAGAGCGA |
| | ORF Start: at 1 | | ORF Stop: end of sequence |
| | SEQ ID NO: 16 | 469 aa | MW at 51246.2kD |
| NOV2d, CG51373-07 Protein Sequence | RFSQEPADQTVVAGQRAVLPCVLLNYSGIVQWTKDGLALGMGQALKAWPRYRVVGSAD AGQYNLEITDAELSDDASYECQATEAAALRSRRAKLTVLIPPEDTRIDGGPVILLQAGT PHNLTCRAFNAKPAATIIWFRDGTQQEGAVASTELLKDGKRETTVSQLLINPTDLDIG RVFTCRSMNEAIPSGKETSIELDVHHPPTVTLSIEPQTVQEGERVVFTCQATANPEIL GYRWAKGGFLIEDAHESRYETNVDYSFFTEPVSCEVHNKVGSTNVSTLVNVHFAPRIV VDPKPTTTDIGSDVTLTCVWVGNPPLTLTWTKKDSNMVLSNSNQLLLKSVTQADAGTY TCRAIVPRIGVAEREVPLYVNGPPIISSEAVQYAVRGDGGKVECFIGSTPPPDRIAWA WKENFLEVGTLERYTVERTNSGSGVLSTLTINNVMEADFQTHYNCTAWNSFGPGTAII QLEER |
| | SEQ ID NO: 17 | 3430 bp | |
| NOV2e, | CTCTCCGATACTTTCTCCCAAGGGTCAGCTGCTTCTTCATTCCAAGTGGACAAGGAGC |

(continued)

| Table 2A. NOV2 Sequence Analysis |
|---|

| CG51373-10 DNA Sequence | CAGCTGCTCACTGTCCTTGAGAGACTTCAGCGAGAGACCAGGGTGTCCAGGCTCCATG<br>CAGGAAAGCCATGCGTATAAATTCCACCTCTGAGCCAGGCCTCACCAGCAAGCCCACT<br>CTTAAGCCCTTGACTTGGGCTCCAGGGGCCATGGGAAGGAGAAACGGACCCAGACCCG<br>CTTCAGCCAGGAGCCAGCTGACCAGACGGTGGTGGCTGGACAGCGGGCCGTGCTCCCC<br>TGTGTGCTGCTCAACTACTCTGGAATTGTGCAATGGACCAAGGACGGGCTGGCCCTGG<br>GC**ATG**GGCCAGGCCCTCAAAGCCTGGCCACGGTACCGGGTTGTGGGCTCCGCAGACGC<br>TGGGCAGTACAACCTGGAGATCACAGATGCTGAGCTCTCTGACGACGCCTCTTACGAG<br>TGCCAGGCCACGGAGGCCGCCCTGCGCTCTCGGCGGGCCAAACTCACCGTGCTCATCC<br>CCCCAGAGGACACCAGGATTGACGGAGGCCCTGTGATTCTACTGCAGGCAGGCACCCC<br>CCACAACCTCACATGCCGGGCCTTCAATGCGAAGCCTGCTGCCACCATCATCTGGTTC<br>CGGGACGGGACGCAGCAGGAGGGCGCTGTGGCCAGCACGGAATTGCTGAAGGATGGGA<br>AGAGGGAGACCACCGTGAGCCAACTGCTTATTAACCCCACGGACCTGGACATAGGGCG<br>TGTCTTCACTTGCCGAAGCATGAACGAAGCCATCCCTAGTGGCAAGGAGACTTCCATC<br>GAGCTGGATGTGCACCACCCTCCTACAGTGACCCTGTCCATTGAGCCACAGACGGTGC<br>AGGAGGGTGAGCGTGTTGTCTTTACCTGCCAGGCCACAGCCAACCCCGAGATCTTGGG<br>CTACAGGTGGGCCAAAGGGGGGTTTCTTGATTGAAGACGCCCACGAGAGTCGCTATGAG<br>ACAAATGTGGATTATTCCTTTTTCACGGAGCCTGTGTCTTGTGAGGTTCACAACAAAG<br>TGGGAAGCACCAATGTCAGCACTTTAGTAAATGTCCACTTTGCTCCCCGGATTGTAGT<br>TGACCCCAAACCCACAACCACAGACATTGGCTCTGATGTGACCCTTACCTGTGTCTGG<br>GTTGGGGAAATCCCCCCCCTCACTCTCACCTGGACCAAAAAGGACTCAAATATTGGGGC<br>CCTGGCTTCTTGGTTCCCCACCCGAGGCTGCTCTCTCTGCCCAGGTCCTGAGTAACAG<br>CAACCAGCTGCTGCTGAAGTCGGTGACTCAGGCAGACGCTGGCACCTACACCTGCCGG<br>GCCATCGTGCCTCGAATCGGAGTGGCTGAGCGGGAGGTGCCGCTCTATGTGAACGGGC<br>CCCCCATCATCTCCAGTGAGGCAGTGCAGTATGCTGTGAGGGGTGACGGTGGCAAGGT<br>GGAGTGTTTCATTGGGAGCACACCACCCCCAGACCGCATAGCATGGGCCTGGAAGGAG<br>AACTTCTTGGAGGTGGGGACCCTGGAACGCTATACAGTGGAGAGGACCAACTCAGGCA<br>GTGGGGTGCTATCCACGCTCACCATCAACAATGTCATGGAGGCCGACTTTCAGACTCA<br>CTACAACTGCACCGCCTGGAACAGCTTCGGGCCAGGCACAGCCATCATCCAGCTGGAA<br>GAGCGAGAGGTGTTACCTGTGGGCATCATAGCTGGGGCCACCATCGGCGCGAGCATCC<br>TGCTCATCTTCTTCTTCATCGCCTTGGTATTCTTCCTCTACCGGCGCCGCAAAGGCAG<br>TCGCAAAGACGTGACCCTGAGGAAGCTGGATATCAAGGTGGAGACAGTGAACCGAGAG<br>CCACTTACGATGCATTCTGACCGGGAGGATGACACCGCCAGCGTCTCCACAGCAACCC<br>GGGTCATGAAGGCCATCTACTCGTCGTTTAAGGATGATGTGGATCTGAAGCAGGACCT<br>GCGCTGCGACACCATCGACACCCGGGAGGAGTATGAGATGAAGGACCCCACCAATGGC<br>TACTACAACGTGCGTGCCCATGAAGACCGCCCGTCTTCCAGGGCAGTGCTCTATGCTG<br>ACTACCGTGCCCCTGGCCCTGCCCGCTTCGACGGCCGCCCCTCATCCCGTCTCTCCCA<br>CTCCAGCGGCTATGCCCAGCTCAACACCTATAGCCGGGGCCCTGCCTCTGACTATGGC<br>CCTGAGCCCACACCCCCTGGCCCTGCTGCCCCAGCTGGCACTGACACAACCAGCCAGC<br>TGTCCTACGAGAACTATGAGAAGTTCAACTCCCATCCCTTCCCTGGGGCAGCTGGGTA<br>CCCCACCTACCGACTGGGCTACCCCCAGGCCCCACCCTCTGGCCTGGAGCGGACCCCA<br>TATGAGGCGTATGACCCCATTGGCAAGTACGCCACAGCCACTCGATTCTCCTACACCT<br>CCCAGCACTCGGACTACGGCCAGCGATTCCAGCAGCGCATGCAGACTCACGTG**TAGGG**<br>GCCAGAGCCTGGCTGGGGCATCTCTGCGGGGCAGAGGAGAAGGCTTTCGCAGCTGTTC<br>CCTGATATTCAGGGACATTGCTCATTGCTCCCTTCTCGGACCAGCCTTCTTCCTCCCA<br>CCATGGCAGGTGGGGAGCAGGTCTCCCAGAGACACCCGTCCCGAGGATGGTGCTCTG<br>TGCATGCCCCAGCCTCCTGGGCCTGCCCTTCCCTCTTCTTCGGGAGGATGTGTCTCTT<br>CTGACCTGCACTCTTGCCTGACCCTAGAATGGGGACAGGGAAAGTGAAGGTTAGGGAA<br>AGCAGAGGGGGGCACTTTTTAGCATTCCCTTTCTATCCCACCCCTCTGATCTCCCATA<br>AGTGGAAATGGGGGTACCCAGGGATGGGCAGGCTTTGGCCTAGGGACATGAAGTATGG<br>GAGTGGGTGGCTGTGGCACAGACAGGTGGAAAACGGGATAGCCTGGCCAGTCCCTCTG<br>TTGTCTGCATTCGTGCCCTGGGTGCCTCTCTCCTTCCTCAGGGTACTGCAGAAGGGAG<br>CGAACAGGGTACTGTTCGCTCTTGTCTACAGAACAGCCCTGGCACTGCATTCAAATCC<br>AGTCTTCATTCAGCTGGGATCAAAATGCCAGTCACCTTGGCTACCCACTGTGGACAGC<br>TGTCTGTCAGCATGCAGAGGGATCCAGGAATCCCCCCGGCAGCACGGCCCGCTTTCCT<br>TCTCCTCCATGCTGGGCCAGCCAGATAAGTCAGGGTCCTGGTGGAGAAAGAAAGGCTA<br>GGACCATGTCCTCATTGACCCAGATACTGCTGTGTGCTGCACAGCAGTGAACCAACAC<br>TAGAGGGAGCCACACAAGCCTCCTCTCCCCAGTCTGCCCCACTTCCTGGCTTTAACTC |

(continued)

| Table 2A. NOV2 Sequence Analysis |||
|---|---|---|
| | TTGAGCTGGTTTGGGGAGTGGTGAGGTAGGGGTGGGGGTGCTGTAGGCTCTTTTTCAA AAAAAAAC ||
| | ORF Start: ATG at 351 | | ORF Stop: TAG at 2490 |
| | SEQ ID NO: 18 | 713 aa | MW at 78491.0kD |
| NOV2e, CG51373-10 Protein Sequence | MGQALKAWPRYRVVGSADAGQYNLEITDAELSDDASYECQATEAALRSRRAKLTVLIP PEDTRIDGGPVILLQAGTPHNLTCRAFNAKPAATIIWFRDGTQQEGAVASTELLKDGK RETTVSQLLINPTDLDIGRVFTCRSMNEAIPSGKETSIELDVHHPPTVTLSIEPQTVQ EGERVVFTCQATANPEILGYRWAKGGFLIEDAHESRYETNVDYSFFTEPVSCEVHNKV GSTNVSTLVNVHFAPRIVVDPKPTTTDIGSDVTLTCVWVGEIPPSLSPGPKRTQILGP WLLGSPPEAALSAQVLSNSNQLLLKSVTQADAGTYTCRAIVPRIGVAEREVPLYVNGP PIISSEAVQYAVRGDGGKVECFIGSTPPPDRIAWAWKENFLEVGTLERYTVERTNSGS GVLSTLTINNVMEADFQTHYNCTAWNSFGPGTAIIQLEEREVLPVGIIAGATIGASIL LIFFFIALVFFLYRRRKGSRKDVTLRKLDIKVETVNREPLTMHSDREDDTASVSTATR VMKAIYSSFKDDVDLKQDLRCDTIDTREEYEMKDPTNGYYNVRAHEDRPSSRAVLYAD YRAPGPARFDGRPSSRLSHSSGYAQLNTYSRGPASDYGPEPTPPGPAAPAGTDTTSQL SYENYEKFNSHPFPGAAGYPTYRLGYPQAPPSGLERTPYEAYDPIGKYATATRFSYTS QHSDYGQRFQQRMQTHV ||
| | SEQ ID NO: 19 | 3212 bp | |
| NOV2f, CG51373-11 DNA Sequence | **ATG**CTGAGCCTCCTCGTCTGGATCCTCACTCTCTCCGATACTTTCTCCCAAGGGACCC AGACCCGCTTCAGCCAGGAGCCAGCTGACCAGACGGTGGTGGCTGGACAGCGGGCCGT GCTCCCCTGTGTGCTGCTCAACTACTCTGGAATTGTGCAATGGACCAAGGACGGGCTG GCCCTGGGCATGGGCCAGGGCCTCAAAGCCTGGCCACGGTACCGGGTTGTGGGCTCCG CAGACGCTGGGCAGTACAACCTGGAGATCACAGATGCTGAGCTCTCTGACGACGCCTC TTACGAGTGCCAGGCCACGGAGGCCGCCCTGCGCTCTCGGCGGGCCAAACTCACCGTG CTCATCCCCCCAGAGGACACCAGGATTGACGGAGGCCCTGTGATTCTACTGCAGGCAG GCACCCCCCACAACCTCACATGCCGGGCCTTCAATGCGAAGCCTGCTGCCACCATCAT CTGGTTCCGGGACGGGACGCAGCAGGAGGGCGCTGTGGCCAGCACGGAATTGCTGAAG GATGGGAAGAGGGAGACCACCGTGAGCCAACTGCTTATTAACCCCACGGACCTGGACA TAGGGCGTGTCTTCACTTGCCGAAGCATGAACGAAGCCATCCCTAGTGGCAAGGAGAC TTCCATCGAGCTGGATGTGCACCACCTCCTACAGTGACCCTGTCCATTGAGCCACAG ACGGTGCAGGAGGGTGAGCGTGTTGTCTTTACCTGCCAGGCCACAGCCAACCCCGAGA TCTTGGGCTACAGGTGGGCCAAAGGGGGTTTCTTGATTGAAGACGCCCACGAGAGTCG CTATGAGACAAATGTGGATTATTCCTTTTTCACGGAGCCTGTGTCTTGTGAGGTTCAC AACAAAGTGGGAAGCACCAATGTCAGCACTTTAGTAAATGTCCACTTTGCTCCCCGGA TTGTAGTTGACCCCAAACCCACAACCACAGACATTGGCTCTGATGTGACCCTTACCTG TGTCTGGGTTGGGAATCCCCCCCTCACTCTCACCTGGACCAAAAAGGACTCAAATATG GTCCTGAGTAACAGCAACCAGCTGCTGCTGAAGTCGGTGACTCAGGCAGACGCTGGCA CCTACACCTGCCGGGCCATCGTGCCTCGAATCGGAGTGGCTGAGCGGGAGGTGCCGCT CTATGTGAACGGGCCCCCCATCATCTCCAGTGAGGCAGTGCAGTATGCTGTGAGGGGT GACGGTGGCAAGGTGGAGTGTTTCATTGGGAGCACACCACCCCCAGACCGCATAGCAT GGGCCTGGAAGGAGAACTTCTTGGAGGTGGGGACCCTGGAACGCTATACAGTGGAGAG GACCAACTCAGGCAGTGGGGTGCTATCCACGCTCACCATCAACAATGTCATGGAGGCC GACTTTCAGACTCACTACAACTGCACCGCCTGGAACAGCTTCGGGCCAGGCACAGCCA TCATCCAGCTGGAAGAGCGAGAGGTGTTACCTGTGGGCATCATAGCTGGGGCCACCAT CGGCGCGAGCATCCTGCTCATCTTCTTCTTCATCGCCTTGGTATTCTTCCTCTACCGG CGCCGCAAAGGCAGTCGCAAAGACGTGACCCTGAGGAAGCTGGATATCAAGGTGGAGA CAGTGAACCGAGAGCCACTTACGATGCATTCTGACCGGGAGGATGACACCGCCAGCGT CTCCACAGCAACCCGGGTCATGAAGGCCATCTACTCGTCGTTTAAGGATGATGTGGAT CTGAAGCAGGACCTGCGCTGCGACACCATCGACACCCGGGAGGAGTATGAGATGAAGG ACCCCACCAATGGCTACTACAACGTGCGTGCCCATGAAGACCGCCCGTCTTCCAGGGC AGTGCTCTATGCTGACTACCGTGCCCCTGGCCCTGCCCGCTTCGACGGCCGCCCCTCA TCCCGTCTCTCCCACTCCAGCGGCTATGCCCAGCTCAACACCTATAGCCGGGGCCCTG CCTCTGACTATGGCCCTGAGCCCACACCCCCTGGCCCTGCTGCCCCAGCTGGCACTGA CACAACCAGCCAGCTGTCCTACGAGAACTATGAGAAGTTCAACTCCCATCCCTTCCCT GGGGCAGCTGGGTACCCCACCTACCGACTGGGCTACCCCCAGGCCCCACCCTCTGGCC |

(continued)

**Table 2A. NOV2 Sequence Analysis**

TGGAGCGGACCCCATATGAGGCGTATGACCCCATTGGCAAGTACGCCACAGCCACTCG
ATTCTCCTACACCTCCCAGCACTCGGACTACGGCCAGCGATTCCAGCAGCGCATGCAG
ACTCACGTG**TAG**GGGGCCAGAGCCTGGCTGGGGCATCTCTGCGGGGCAGAGGAGAAGGC
TTTCGCAGCTGTTCCCTGATATTCAGGGACATTGCTCATTGCTCCCTTCTCGGACCAG
CCTTCTTCCTCCCACCATGGCAGGTGGGGAGCAGGTCTCCCAGAGACACCCCGTCCCG
AGGATGGTGCTCTGTGCATGCCCCAGCCTCCTGGGCCTGCCCTTCCCTCTTCTTCGGG
AGGATGTGTCTCTTCTGACCTGCACTCTTGCCTGACCCTAGAATGGGGACAGGGAAAG
TGAAGGTTAGGGAAAGCAGAGGGGGGCACTTTTTAGCATTCCCTTTCTATCCCACCCC
TCTGATCTCCCATAAGTGGAAATGGGGGTACCCAGGGATGGGCAGGCTTTGGCCTAGG
GACATGAAGTATGGGAGTGGGTGGCTGTGGCACAGACAGGTGGAAAACGGGATAGCCT
GGCCAGTCCCTCTGTTGTCTGCATTCGTGCCCTGGGTGCCTCTCCTTCCTCAGGGT
ACTGCAGAAGGGAGCGAACAGGGTACTGTTCGCTCTTGTCTACAGAACAGCCCTGGCA
CTGCATTCAAATCCAGTCTTCATTCAGCTGGGATCAAAATGCCAGTCACCTTGGCTAC
CCACTGTGGACAGCTGTCTGTCAGCATGCAGAGGGATCCAGGAATCCCCCCGGCAGCA
CGGCCCGCTTTCCTTCTCCTCCATGCTGGGCCAGCCAGATAAGTCAGGGTCCTGGTGG
AGAAAGAAAGGCTAGGACCATGTCCTCATTGACCCAGATACTGCTGTGTGCTGCACAG
CAGTGAACCAACACTAGAGGGAGCCACACAAGCCTCCTCTCCCAGTCTGCCCCACTT
CCTGGCTTTAACTCTTGAGCTGGTTTGGGGAGTGGTGAGGTAGGGGTGGGGGTGCTGT
AGGCTCTTTTTCAAAAAAAAC

| | | |
|---|---|---|
| ORF Start: ATG at 1 | | ORF Stop: TAG at 2272 |
| SEQ ID NO: 20 | 757 aa | MW at 83534.8kD |

| | |
|---|---|
| NOV2f, CG51373-11 Protein Sequence | MLSLLVWILTLSDTFSQGTQTRFSQEPADQTVVAGQRAVLPCVLLNYSGIVQWTKDGL ALGMGQGLKAWPRYRVVGSADAGQYNLEITDAELSDDASYECQATEAALRSRRAKLTV LIPPEDTRIDGGPVILLQAGTPHNLTCRAFNAKPAATIIWFRDGTQQEGAVASTELLK DGKRETTVSQLLINPTDLDIGRVFTCRSMNEAIPSGKETSIELDVHHPPTVTLSIEPQ TVQEGERVVFTCQATANPEILGYRWAKGGFLIEDAHESRYETNVDYSFFTEPVSCEVH NKVGSTNVSTLVNVHFAPRIVVDPKPTTTDIGSDVTLTCVWVGNPPLTLTWTKKDSNM VLSNSNQLLLKSVTQADAGTYTCRAIVPRIGVAEREVPLYVNGPPIISSEAVQYAVRG DGGKVECFIGSTPPPDRIAWAWKENFLEVGTLERYTVERTNSGSGVLSTLTINNVMEA DFQTHYNCTAWNSFGPGTAIIQLEEREVLPVGIIAGATIGASILLIFFFIALVFFLYR RRKGSRKDVTLRKLDIKVETVNREPLTMHSDREDDTASVSTATRVMKAIYSSFKDDVD LKQDLRCDTIDTREEYEMKDPTNGYYNVRAHEDRPSSRAVLYADYRAPGPARFDGRPS SRLSHSSGYAQLNTYSRGPASDYGPEPTPPGPAAPAGTDTTSQLSYENYEKFNSHPFP GAAGYPTYRLGYPQAPPSGLERTPYEAYDPIGKYATATRFSYTSQHSDYGQRFQQRMQ THV |

| | | |
|---|---|---|
| SEQ ID NO: 21 | 2290 bp | |

| | |
|---|---|
| NOV2g, CG51373-13 DNA Sequence | CACCAAGCTT**ATG**CTGAGCCTCCTCGTCTGGATCCTCACTCTCTCCGATACTTTCTCC CAAGGGACCCAGACCCGCTTCAGCCAGGAGCCAGCTGACCAGACGGTGGTGGCTGGAC AGCGGGCCGTGCTCCCCTGTGTGCTGCTCAACTACTCTGGAATTGTGCAATGGACCAA GGACGGGCTGGCCCTGGGCATGGGCCAGGGCCTCAAAGCCTGGCCACGGTACCGGGTT GTGGGCTCCGCAGACGCTGGGCAGTACAACCTGGAGATCACAGATGCTGAGCTCTCTG ACGACGCCTCTTACGAGTGCCAGGCCACGGAGGCCGCCCTGCGCTCTCGGCGGGCCAA ACTCACCGTGCTCATCCCCCCAGAGGACACCAGGATTGACGGAGGCCCTGTGATTCTA CTGCAGGCAGGCACCCCCCACAACCTCACATGCCGGGCCTTCAATGCGAAGCCTGCTG CCACCATCATCTGGTTCCGGGACGGGACGCAGCAGGAGGGCGCTGTGGCCAGCACGGA ATTGCTGAAGGATGGGAAGAGGGAGACCACCGTGAGCCAACTGCTTATTAACCCCACG GACCTGGACATAGGGCGTGTCTTCACTTGCCGAAGCATGAACGAAGCCATCCCTAGTG GCAAGGAGACTTCCATCGAGCTGGATGTGCACCACCCTCCTACAGTGACCCTGTCCAT TGAGCCACAGACGGTGCAGGAGGGTGAGCGTGTTGTCTTTACCTGCCAGGCCACAGCC AACCCGGAGATCTTGGGCTACAGGTGGGCCAAAGGGGGTTTCTTGATTGAAGACGCCC ACGAGAGTCGCTATGAGACAAATGTGGATTATTCCTTTTTCACGGAGCCTGTGTCTTG TGAGGTCCACAACAAAGTGGGAAGCACCAATGTCAGCACTTTAGTAAATGTCCACTTT GCTCCCCGGATTGTAGTTGACCCCAAACCCACAACCACAGACATTGGCTCTGATGTGA CCCTTACCTGTGTCTGGGTTGGGAATCCCCCCCTCACTCTCACCTGGACCAAAAAGGA CTCAAATATGGTCCTGAGTAACAGCAACCAGCTGCTGCTGAAGTCGGTGACTCAGGCA |

(continued)

| Table 2A. NOV2 Sequence Analysis |
|---|
| GACGCTGGCACCTACACCTGCCGGGCCATCGTGCCTCGAATCGGAGTGGCTGAGCGGG AGGTGCCGCTCTATGTGAACGGGCCCCCCATCATCTCCAGTGAGGCAGTGCAGTATGC TGTGAGGGGTGACGGTGGCAAGGTGGAGTGTTTCATTGGGAGCACACCACCCCCAGAC CGCATAGCATGGGCCTGGAAGGAGAACTTCTTGGAGGTGGGGACCCTGGAACGCTATA CAGTGGAGAGGACCAACTCAGGCAGTGGGGTGCTATCCACGCTCACCATCAACAATGT CATGGAGGCCGACTTTCAGACTCACTACAACTGCACCGCCTGGAACAGCTTCGGGCCA GGCACAGCCATCATCCAGCTGGAAGAGCGAGAGGTGTTACCTGTGGGCATCATAGCTG GGGCCACCATCGGCGCGAGCATCCTGCTCATCTTCTTCTTCATCGCCTTGGTATTCTT CCTCTACCGGCGCCGCAAAGGCAGTCGCAAAGACGTGACCCTGAGGAAGCTGGATATC AAGGTGGAGACAGTGAACCGAGAGCCACTTACGATGCATTCTGACCGGGAGGATGACA CCGCCAGCGTCTCCACAGCAACCCGGGTCATGAAGGCCATCTACTCGTCGTTTAAGGA TGATGTGGATCTGAAGCAGGACCTGCGCTGCGACACCATCGACACCCGGGAGGAGTAT GAGATGAAGGACCCCACCAATGGCTACTACAACGTGCGTGCCCATGAAGACCGCCCGT CTTCCAGGGCAGTGCTCTATGCTGACTACCGTGCCCCTGGCCCTGCCCGCTTCGACGG CCGCCCCTCATCCCGTCTCTCCCACTCCAGCGGCTATGCCCAGCTCAACACCTATAGC CGGGGCCCTGCCTCTGACTATGGCCCTGAGCCCACACCCCCTGGCCCTGCTGCCCCAG CTGGCACTGACACAACCAGCCAGCTGTCCTACGAGAACTATGAGAAGTTCAACTCCCA TCCCTTCCCTGGGGCAGCTGGGTACCCCACCTACCGACTGGGCTACCCCCAGGCCCCA CCCTCTGGCCTGGAGCGGACCCCATATGAGGCGTATGACCCCATTGGCAAGTACGCCA CAGCCACTCGATTCTCCTACACCTCCCAGCACTCGGACTACGGCCAGCGATTCCAGCA GCGCATGCAGACTCACGTG**GTC**GACGGC |

| | ORF Start: ATG at 11 | | ORF Stop: at 2282 |
|---|---|---|---|
| | SEQ ID NO: 22 | 757 aa | MW at 83534.8kD |
| NOV2g, CG51373-13 Protein Sequence | MLSLLVWILTLSDTFSQGTQTRFSQEPADQTVVAGQRAVLPCVLLNYSGIVQWTKDGL ALGMGQGLKAWPRYRVVGSADAGQYNLEITDAELSDDASYECQATEAALRSRRAKLTV LIPPEDTRIDGGPVILLQAGTPHNLTCRAFNAKPAATIIWFRDGTQQEGAVASTELLK DGKRETTVSQLLINPTDLDIGRVFTCRSMNEAIPSGKETSIELDVHHPPTVTLSIEPQ TVQEGERVVFTCQATANPEILGYRWAKGGFLIEDAHESRYETNVDYSFFTEPVSCEVH NKVGSTNVSTLVNVHFAPRIVVDPKPTTTDIGSDVTLTCVWVGNPPLTLTWTKKDSNM VLSNSNQLLLKSVTQADAGTYTCRAIVPRIGVAEREVPLYVNGPPIISSEAVQYAVRG DGGKVECFIGSTPPPDRIAWAWKENFLEVGTLERYTVERTNSGSGVLSTLTINNVMEA DFQTHYNCTAWNSFGPGTAIIQLEEREVLPVGIIAGATIGASILLIFFFIALVFFLYR RRKGSRKDVTLRKLDIKVETVNREPLTMHSDREDDTASVSTATRVMKAIYSSFKDDVD LKQDLRCDTIDTREEYEMKDPTNGYYNVRAHEDRPSSRAVLYADYRAPGPARFDGRPS SRLSHSSGYAQLNTYSRGPASDYGPEPTPPGPAAPAGTDTTSQLSYENYEKFNSHPFP GAAGYPTYRLGYPQAPPSGLERTPYEAYDPIGKYATATRFSYTSQHSDYGQRFQQRMQ THV |
| | SEQ ID NO: 23 | 2290 bp | |
| NOV2h, CG51373-14 DNA Sequence | CACCAAGCTT**CAA**GGGACCCAGACCCGCTTCAGCCAGGAGCCAGCTGACCAGACGGTG GTGGCTGGACAGCGGGCCGTGCTCCCCTGTGTGCTGCTCAACTACTCTGGAATTGTGC AATGGACCAAGGACGGGCTGGCCCTGGGCATGGGCCAGGGCCTCAAAGCCTGGCCACG GTACCGGGTTGTGGGCTCCGCAGACGCTGGGCAGTACAACCTGGAGATCACAGATGCT GAGCTCTCTGACGACGCCTCTTACGAGTGCCAGGCCACGGAGGCCGCCCTGCGCTCTC GGCGGGCCAAACTCACCGTGCTCATCCCCCCAGAGGACACCAGGATTGACGGAGGCCC TGTGATTCTACTGCAGGCAGGCACCCCCACAACCTCACATGCCGGGCCTTCAATGCG AAGCCTGCTGCCACCATCATCTGGTTCCGGGACGGGACGCAGCAGGAGGGCGCTGTGG CCAGCACGGAATTGCTGAAGGATGGGAAGAGGGAGACCACCGTGAGCCAACTGCTTAT TAACCCCACGGACCTGGACATAGGGCGTGTCTTCACTTGCCGAAGCATGAACGAAGCC ATCCCTAGTGGCAAGGAGACTTCCATCGAGCTGGATGTGCACCACCCTCCTACAGTGA CCCTGTCCATTGAGCCACAGACGGTGCAGGAGGGTGAGCGTGTTGTCTTTACCTGCCA GGCCACAGCCAACCCGGAGATCTTGGGCTACAGGTGGGCCAAAGGGGGTTTCTTGATT GAAGACGCCCACGAGAGTCGCTATGAGACAAATGTGGATTATTCCTTTTTCACGGAGC CTGTGTCTTGTGAGGTTCACAACAAAGTGGGAAGCACCAATGTCAGCACTTTAGTAAA TGTCCACTTTGCTCCCCGGATTGTAGTTGACCCCAAACCCACAACCACAGACATTGGC TCTGATGTGACCCTTACCTGTGTCTGGGTTGGGAATCCCCCCCCTCACTCTCACCTGGA |

(continued)

| Table 2A. NOV2 Sequence Analysis |||
| --- | --- | --- |
| | CCAAAAAGGACTCAAATATGGGGCCCAGGCCTCCTGGCTCCCCACCCGAGGCTGCTCT CTCTGCCCAGGTCCTGAGTAACAGCAACCAGCTGCTGCTGAAGTCGGTGACTCAGGCA GACGCTGGCACCTACACCTGCCGGGCCATCGTGCCTCGAATCGGGAGTGGCTGAGCGGG AGGTGCCGCTCTATGTGAACGGGCCCCCCATCATCTCCAGTGAGGCAGTGCAGTATGC TGTGAGGGGTGACGGTGGCAAGGTGGAGTGTTTCATTGGGAGCACACCACCCCCAGAC CGCATAGCATGGGCCTGGAAGGAGAACTTCTTGGAGGTGGGGACCCTGGAACGCTATA CAGTGGAGAGGACCAACTCAGGCAGTGGGGTGCTATCCACGCTCACCATCAACAATGT CATGGAGGCCGACTTTCAGACTCACTACAACTGCACCGCCTGGAACAGCTTCGGGCCA GGCACAGCCATCATCCAGCTGGAAGAGCGAGAGGTGTTACCTGTGGGCATCATAGCTG GGGCCACCATCGGCGCGAGCATCCTGCTCATCTTCTTCTTCATCGCCTTGGTATTCTT CCTCTACCGGCGCCGCAAAGGCAGTCGCAAAGACGTGACCCTGAGGAAGCTGGATATC AAGGTGGAGACAGTGAACCGAGAGCCACTTACGATGCATTCTGACCGGGAGGATGACA CCGCCAGCGTCTCCACAGCAACCCGGGTCATGAAGGCCATCTACTCGTCGTTTAAGGA TGATGTGGATCTGAAGCAGGACCTGCGCTGCGACACCATCGACACCCGGGAGGAGTAT GAGATGAAGGACCCCACCAATGGCTACTACAACGTGCGTGCCCATGAAGACCGCCCGT CTTCCAGGGCAGTGCTCTATGCTGACTACCGTGCCCCTGGCCCTGCCCGCTTCGACGG CCGCCCCTCATCCCGTCTCTCCCACTCCAGCGGCTATGCCCAGCTCAACACCTATAGC CGGGGCCCTGCCTCTGACTATGGCCCTGAGCCCACACCCCCTGGCCCTGCTGCCCCAG CTGGCACTGACACAACCAGCCAGCTGTCCTACGAGAACTATGAGAAGTTCAACTCCCA TCCCTTCCCTGGGGCAGCTGGGTACCCCACCTACCGACTGGGCTACCCCCAGGCCCCA CCCTCTGGCCTGGAGCGGACCCCATATGAGGCGTATGACCCCATTGGCAAGTACGCCA CAGCCACTCGATTCTCCTACACCTCCCAGCACTCGGACTACGGCCAGCGATTCCAGCA GCGCATGCAGACTCACGTG**GTC**GACGGC | | |
| | ORF Start: at 11 | | ORF Stop: at 2282 |
| | SEQ ID NO: 24 | 757 aa | MW at 83227.3kD |
| NOV2h, CG51373-14 Protein Sequence | QGTQTRFSQEPADQTVVAGQRAVLPCVLLNYSGIVQWTKDGLALGMGQGLKAWPRYRV VGSADAGQYNLEITDAELSDDASYECQATEAALRSRRAKLTVLIPPEDTRIDGGPVIL LQAGTPHNLTCRAFNAKPAATIIWFRDGTQQEGAVASTELLKDGKRETTVSQLLINPT DLDIGRVFTCRSMNEAIPSGKETSIELDVHHPPTVTLSIEPQTVQEGERVVFTCQATA NPEILGYRWAKGGFLIEDAHESRYETNVDYSFFTEPVSCEVHNKVGSTNVSTLVNVHF APRIVVDPKPTTTDIGSDVTLTCVWVGNPPLTLTWTKKDSNMGPRPPGSPPEAALSAQ VLSNSNQLLLKSVTQADAGTYTCRAIVPRIGVAEREVPLYVNGPPIISSEAVQYAVRG DGGKVECFIGSTPPPDRIAWAWKENFLEVGTLERYTVERTNSGSGVLSTLTINNVMEA DFQTHYNCTAWNSFGPGTAIIQLEEREVLPVGIIAGATIGASILLIFFFIALVFFLYR RRKGSRKDVTLRKLDIKVETVNREPLTMHSDREDDTASVSTATRVMKAIYSSFKDDVD LKQDLRCDTIDTREEYEMKDPTNGYYNVRAHEDRPSSRAVLYADYRAPGPARFDGRPS SRLSHSSGYAQLNTYSRGPASDYGPEPTPPGPAAPAGTDTTSQLSYENYEKFNSHPFP GAAGYPTYRLGYPQAPPSGLERTPYEAYDPIGKYATATRFSYTSQHSDYGQRFQQRMQ THV | | |

Sequence comparison of the above protein sequences yields the following sequence relationships shown in Table 2B.

| Table 2B. Comparison of NOV2a against NOV2b through NOV2h. |||
| --- | --- | --- |
| Protein Sequence | NOV2a Residues/ Match Residues | Identities/ Similarities for the Matched Region |
| NOV2b | 17..773 101..841 | 739/757 (97%) 740/757 (97%) |
| NOV2c | 62..773 1..696 | 695/712 (97%) 695/712 (97%) |
| NOV2d | 22..506 1..469 | 468/485 (96%) 468/485 (96%) |
| NOV2e | 62..773 | 696/713 (97%) |

(continued)

| Table 2B. Comparison of NOV2a against NOV2b through NOV2h. | | |
|---|---|---|
| Protein Sequence | NOV2a Residues/ Match Residues | Identities/ Similarities for the Matched Region |
| | 1..713 | 698/713 (97%) |
| NOV2f | 1..773 | 757/773 (97%) |
| | 1..757 | 757/773 (97%) |
| NOV2g | 1..773 | 757/773 (97%) |
| | 1..757 | 757/773 (97%) |
| NOV2h | 17..773 | 757/757 (100%) |
| | 1..757 | 757/757 (100%) |

Further analysis of the NOV2a protein yielded the following properties shown in Table 2C.

| Table 2C. Protein Sequence Properties NOV2a | |
|---|---|
| SignalP analysis: | Cleavage site between residues 17 and 18 |
| PSORT II analysis: | PSG: a new signal peptide prediction method |
| |     N-region: length 0; pos.chg 0; neg.chg 0 |
| |     H-region: length 12; peak value 10.45 |
| |     PSG score: 6.05 |
| | |
| | GvH: von Heijne's method for signal seq. recognition |
| |     GvH score (threshold: -2.1): 0.73 |
| |     possible cleavage site: between 16 and 17 |
| | |
| | >>> Seems to have a cleavable signal peptide (1 to 16) |
| | |
| | ALOM: Klein et al's method for TM region allocation |
| |     Init position for calculation: 17 |
| |     Tentative number of TMS(s) for the threshold 0.5: 1 |
| |     Number of TMS(s) for threshold 0.5: 1 |
| |     INTEGRAL Likelihood =-12.26 Transmembrane 519 - 535 |
| |     PERIPHERAL Likelihood = 3.61 (at 38) |
| |     ALOM score: -12.26 (number of TMSs: 1) |
| | |
| | MTOP: Prediction of membrane topology (Hartmann et al.) |
| |     Center position for calculation: 8 |
| |     Charge difference: -2.0 C(-1.0) - N(1.0) |
| |     N >= C: N-terminal side is inside |
| | |
| | >>> membrane topology: type 1a (cytoplasmic tail 536 to 773) |
| | |
| | MITDISC: discrimination of mitochondrial targeting seq |
| |     R content: 1          Hyd Moment (75) : 1.68 |
| |     Hyd Moment (95) : 2.60     G content: 1 |
| |     D/E content: 2          S/T content: 8 |
| |     Score: -5.22 |
| | |
| | Gavel: prediction of cleavage sites for mitochondrial preseq |
| |     R-2 motif at 32 TRF\|SQ |

(continued)

| Table 2C. Protein Sequence Properties NOV2a | |
|---|---|
| SignalP analysis: | Cleavage site between residues 17 and 18 |
| | NUCDISC: discrimination of nuclear localization signals<br>    pat4: RRRK (5) at 538<br>    pat7: none<br>    bipartite: none<br>    content of basic residues: 9.6%<br>    NLS Score: -0.16<br><br>KDEL: ER retention motif in the C-terminus: none<br><br>ER Membrane Retention Signals: none<br><br>SKL: peroxisomal targeting signal in the C-terminus: none<br><br>PTS2: 2nd peroxisomal targeting signal: none<br><br>VAC: possible vacuolar targeting motif: none<br><br>RNA-binding motif: none<br><br>Actinin-type actin-binding motif:<br>    type 1: none<br>    type 2: none<br><br>NMYR: N-myristoylation pattern : none<br><br>Prenylation motif: none<br><br>memYQRL: transport motif from cell surface to Golgi: none<br><br>Tyrosines in the tail: too long tail<br><br>Dileucine motif in the tail: none<br><br>checking 63 PROSITE DNA binding motifs: none<br><br>checking 71 PROSITE ribosomal protein motifs: none<br><br>checking 33 PROSITE prokaryotic DNA binding motifs: none<br><br>NNCN: Reinhardt's method for Cytoplasmic/Nuclear discrimination<br>    Prediction: cytoplasmic<br>    Reliability: 70.6 |
| COIL: | COIL: Lupas's algorithm to detect coiled-coil regions total: 0 residues |
| | Final Results (k = 9/23): |

(continued)

| Table 2C. Protein Sequence Properties NOV2a | |
|---|---|
| SignalP analysis: | Cleavage site between residues 17 and 18 |
| | 44.4 %: endoplasmic reticulum<br>22.2 %: Golgi<br>22.2 %: extracellular, including cell wall<br>11.1 %: plasma membrane<br><br>>> prediction for CG51373-12 is end (k=9) |

A search of the NOV2a protein against the Geneseq database, a proprietary database that contains sequences published in patents and patent publication, yielded several homologous proteins shown in Table 2D.

| Table 2D. Geneseq Results for NOV2a | | | | |
|---|---|---|---|---|
| Geneseq Identifier | Protein/Organism/Length [Patent #, Date] | NOV2a Residues/ Match Residues | Identities/ Similarities for the Matched Region | Expect Value |
| ABB05749 | Human G protein-coupled receptor NOV1a protein SEQ ID NO:2 - Homo sapiens, 841 aa. [WO200200691-A2, 03-JAN-2002] | 17..773<br>101..841 | 739/757 (97%)<br>740/757 (97%) | 0.0 |
| ABP66884 | Human polypeptide SEQ ID NO 605 - Homo sapiens, 749 aa. [US2002090672-A1, 1-JUL-2002] | 57..773<br>33..749 | 717/717 (100%)<br>717/717 (100%) | 0.0 |
| ABB 10297 | Human cDNA SEQ ID NO: 605 - Homo sapiens, 749 aa. [WO200154474-A2, 02-AUG-2001] | 57..773<br>33..749 | 717/717 (100%)<br>717/717 (100%) | 0.0 |
| ABG65107 | Human albumin fusion protein #1782 - Homo sapiens, 712 aa. [WO200177137-A1, 18-OCT-2001] | 62..773<br>1..712 | 712/712 (100%)<br>712/712 (100%) | 0.0 |
| AAE07070 | Human gene 20 encoded secreted protein HDTJG33, SEQ ID NO:87 - Homo sapiens, 712 aa. [WO200154708-A1, 02-AUG-2001] | 62..773<br>1..712 | 712/712 (100%)<br>712/712 (100%) | 0.0 |

In a BLAST search of public sequence databases, the NOV2a protein was found to have homology to the proteins shown in the BLASTP data in Table 2E.

| Table 2E. Public BLASTP Results for NOV2a | | | | |
|---|---|---|---|---|
| Protein Accession Number | Protein/Organism/Length | NOV2a Residues/ Match Residues | Identities/ Similarities for the Matched Portion | Expect Value |
| CAD23338 | Sequence 1 from Patent WO0200691 - Homo sapiens (Human), 841 aa. | 17..773<br>101..841 | 739/757 (97%)<br>740/757 (97%) | 0.0 |
| Q8CJ59 | NEPH1 - Mus musculus (Mouse), 789 aa. | 18..773<br>50..789 | 714/756 (94%)<br>726/756 (95%) | 0.0 |
| CAD23349 | Sequence 25 from Patent WO0200691 - Homo sapiens (Human), 696 aa. | 62..773<br>1..696 | 695/712 (97%)<br>695/712 (97%) | 0.0 |
| CAD23348 | Sequence 23 from Patent WO0200691 - Homo sapiens (Human), 713 aa. | 62..773<br>1..713 | 696/713 (97%)<br>698/713 (97%) | 0.0 |

(continued)

| Table 2E. Public BLASTP Results for NOV2a | | | | |
|---|---|---|---|---|
| Protein Accession Number | Protein/Organism/Length | NOV2a Residues/ Match Residues | Identities/ Similarities for the Matched Portion | Expect Value |
| Q96J84 | NEPH1 - Homo sapiens (Human), 605 aa. | 1..607 1..591 | 590/607 (97%) 591/607 (97%) | 0.0 |

PFam analysis predicts that the NOV2a protein contains the domains shown in the Table 2F.

| Table 2F. Domain Analysis of NOV2a | | | |
|---|---|---|---|
| Pfam Domain | NOV2a Match Region | Identities/ Similarities for the Matched Region | Expect Value |
| ig | 35..102 | 12/72 (17%) 47/72 (65%) | 1.3e-05 |
| ig | 136..202 | 14/69 (20%) 48/69 (70%) | 0.0023 |
| ig | 322..389 | 21/71 (30%) 55/71 (77%) | 7.2e-10 |

NOVX nucleic acids and their encoded polypeptides are useful in a variety of applications and contexts. The various NOVX nucleic acids and polypeptides according to the invention are useful as novel members of the protein families according to the presence of domains and sequence relatedness to previously described proteins. Additionally, NOVX nucleic acids and polypeptides can also be used to identify proteins that are members of the family to which the NOVX polypeptides belong.

Consistent with other known members of the family of proteins, identified in column 5 of Table A, the NOVX polypeptides of the present invention show homology to, and contain domains that are characteristic of, other members of such protein families.

The NOVX nucleic acids and polypeptides can also be used to screen for molecules, which inhibit or enhance NOVX activity or function. Specifically, the nucleic acids and polypeptides according to the invention can be used as targets for the identification of small molecules that modulate or inhibit diseases associated with the protein families listed in Table A.

The NOVX nucleic acids and polypeptides are also useful for detecting specific cell types. Details of the expression analysis for each NOVX are presented in Example 7. Accordingly, the NOVX nucleic acids, polypeptides, antibodies and related compounds according to the invention have diagnostic and therapeutic applications in the detection of a variety of diseases with differential expression in normal vs. diseased tissues, e.g. detection of a variety of cancers.

Additional utilities for NOVX nucleic acids and polypeptides according to the invention are disclosed herein.

**NOVX clones**

NOVX nucleic acids and their encoded polypeptides are useful in a variety of applications and contexts. The various NOVX nucleic acids and polypeptides according to the invention are useful as novel members of the protein families according to the presence of domains and sequence relatedness to previously described proteins. Additionally, NOVX nucleic acids and polypeptides can also be used to identify proteins that are members of the family to which the NOVX polypeptides belong.

The NOVX genes and their corresponding encoded proteins are useful for preventing, treating or ameliorating medical conditions, e.g., by protein or gene therapy. Pathological conditions can be diagnosed by determining the amount of the new protein in a sample or by determining the presence of mutations in the new genes. Specific uses are described for each of the NOVX genes, based on the tissues in which they are most highly expressed. Uses include developing products for the diagnosis or treatment of a variety of diseases and disorders.

The NOVX nucleic acids and proteins of the invention are useful in potential diagnostic and therapeutic applications and as a research tool. These include serving as a specific or selective nucleic acid or protein diagnostic and/or prognostic marker, wherein the presence or amount of the nucleic acid or the protein are to be assessed, as well as potential therapeutic applications such as the following: (i) a protein therapeutic, (ii) a small molecule drug target, (iii) an antibody target (therapeutic, diagnostic, drug targeting/cytotoxic antibody), (iv) a nucleic acid useful in gene therapy (gene delivery/gene ablation), and (v) a composition promoting tissue regeneration *in vitro* and *in vivo* (vi) a biological defense weapon.

In one specific embodiment, the invention includes an isolated polypeptide comprising an amino acid sequence selected from the group consisting of: (a) a mature form of the amino acid sequence selected from the group consisting of SEQ ID NO: 2n, wherein n is an integer between 1 and 12; (b) a variant of a mature form of the amino acid sequence selected from the group consisting of SEQ ID NO: 2n, wherein n is an integer between 1 and 12, wherein any amino acid in the mature form is changed to a different amino acid, provided that no more than 15% of the amino acid residues in the sequence of the mature form are so changed; (c) an amino acid sequence selected from the group consisting of SEQ ID NO: 2n, wherein n is an integer between 1 and 12; (d) a variant of the amino acid sequence selected from the group consisting of SEQ ID NO:2n, wherein n is an integer between 1 and 12 wherein any amino acid specified in the chosen sequence is changed to a different amino acid, provided that no more than 15% of the amino acid residues in the sequence are so changed; and (e) a fragment of any of (a) through (d).

In another specific embodiment, the invention includes an isolated nucleic acid molecule comprising a nucleic acid sequence encoding a polypeptide comprising an amino acid sequence selected from the group consisting of: (a) a mature form of the amino acid sequence given SEQ ID NO: 2n, wherein n is an integer between 1 and 12; (b) a variant of a mature form of the amino acid sequence selected from the group consisting of SEQ ID NO: 2n, wherein n is an integer between 1 and 12 wherein any amino acid in the mature form of the chosen sequence is changed to a different amino acid, provided that no more than 15% of the amino acid residues in the sequence of the mature form are so changed; (c) the amino acid sequence selected from the group consisting of SEQ ID NO: 2n, wherein n is an integer between 1 and 12; (d) a variant of the amino acid sequence selected from the group consisting of SEQ ID NO: 2n, wherein n is an integer between 1 and 12, in which any amino acid specified in the chosen sequence is changed to a different amino acid, provided that no more than 15% of the amino acid residues in the sequence are so changed; (e) a nucleic acid fragment encoding at least a portion of a polypeptide comprising the amino acid sequence selected from the group consisting of SEQ ID NO: 2n, wherein n is an integer between 1 and 12 or any variant of said polypeptide wherein any amino acid of the chosen sequence is changed to a different amino acid, provided that no more than 10% of the amino acid residues in the sequence are so changed; and (f) the complement of any of said nucleic acid molecules.

In yet another specific embodiment, the invention includes an isolated nucleic acid molecule, wherein said nucleic acid molecule comprises a nucleotide sequence selected from the group consisting of: (a) the nucleotide sequence selected from the group consisting of SEQ ID NO: 2n-1, wherein n is an integer between 1 and 12; (b) a nucleotide sequence wherein one or more nucleotides in the nucleotide sequence selected from the group consisting of SEQ ID NO: 2n-1, wherein n is an integer between 1 and 12 is changed from that selected from the group consisting of the chosen sequence to a different nucleotide provided that no more than 15% of the nucleotides are so changed; (c) a nucleic acid fragment of the sequence selected from the group consisting of SEQ ID NO: 2n-1, wherein n is an integer between 1 and 12; and (d) a nucleic acid fragment wherein one or more nucleotides in the nucleotide sequence selected from the group consisting of SEQ ID NO: 2n-1, wherein n is an integer between 1 and 12 is changed from that selected from the group consisting of the chosen sequence to a different nucleotide provided that no more than 15% of the nucleotides are so changed.

**NOVX Nucleic Acids and Polypeptides**

One aspect of the invention pertains to isolated nucleic acid molecules that encode NOVX polypeptides or biologically active portions thereof. Also included in the invention are nucleic acid fragments sufficient for use as hybridization probes to identify NOVX-encoding nucleic acids (e.g., NOVX mRNAs) and fragments for use as PCR primers for the amplification and/or mutation of NOVX nucleic acid molecules. As used herein, the term "nucleic acid molecule" is intended to include DNA molecules (*e.g.*, cDNA or genomic DNA), RNA molecules (*e.g.*, mRNA), analogs of the DNA or RNA generated using nucleotide analogs, and derivatives, fragments and homologs thereof. The nucleic acid molecule can be single-stranded or double-stranded, but preferably is comprised double-stranded DNA.

A NOVX nucleic acid can encode a mature NOVX polypeptide. As used herein, a "mature" form of a polypeptide or protein disclosed in the present invention is the product of a naturally occurring polypeptide or precursor form or proprotein. The naturally occurring polypeptide, precursor or proprotein includes, by way of nonlimiting example, the full-length gene product encoded by the corresponding gene. Alternatively, it can be defined as the polypeptide, precursor or proprotein encoded by an ORF described herein. The product "mature" form arises, by way of nonlimiting example, as a result of one or more naturally occurring processing steps that may take place within the cell (e.g., host cell) in which the gene product arises. Examples of such processing steps leading to a "mature" form of a polypeptide or protein include the cleavage of the N-terminal methionine residue encoded by the initiation codon of an ORF, or the proteolytic cleavage of a signal peptide or leader sequence. Thus a mature form arising from a precursor polypeptide or protein that has residues 1 to N, where residue 1 is the N-terminal methionine, would have residues 2 through N remaining after removal of the N-terminal methionine. Alternatively, a mature form arising from a precursor polypeptide or protein having residues 1 to N, in which an N-terminal signal sequence from residue 1 to residue M is cleaved, would have the residues from residue M+1 to residue N remaining. Further as used herein, a "mature" form of a polypeptide or protein may arise from a step of post-translational modification other than a proteolytic cleavage event. Such additional processes include, by way of non-limiting example, glycosylation, myristylation or phosphorylation. In general, a mature polypeptide or protein may result from the operation of only one of these processes, or a combination of any of them.

The term "probe", as utilized herein, refers to nucleic acid sequences of variable length, preferably between at least about 10 nucleotides (nt), about 100 nt, or as many as approximately, *e.g.*, 6,000 nt, depending upon the specific use. Probes are used in the detection of identical, similar, or complementary nucleic acid sequences. Longer length probes are generally obtained from a natural or recombinant source, are highly specific, and much slower to hybridize than shorter-length oligomer probes. Probes can be single-stranded or double-stranded and designed to have specificity in PCR, membrane-based hybridization technologies, or ELISA-like technologies.

The term "isolated" nucleic acid molecule, as used herein, is a nucleic acid that is separated from other nucleic acid molecules which are present in the natural source of the nucleic acid. Preferably, an "isolated" nucleic acid is free of sequences which naturally flank the nucleic acid (*i.e.,* sequences located at the 5'- and 3'-termini of the nucleic acid) in the genomic DNA of the organism from which the nucleic acid is derived. For example, in various embodiments, the isolated NOVX nucleic acid molecules can contain less than about 5 kb, 4 kb, 3 kb, 2 kb, 1 kb, 0.5 kb or 0.1 kb of nucleotide sequences which naturally flank the nucleic acid molecule in genomic DNA of the cell/tissue from which the nucleic acid is derived (*e.g.,* brain, heart, liver, spleen, *etc*). Moreover, an "isolated" nucleic acid molecule, such as a cDNA molecule, can be substantially free of other cellular material, or culture medium, or of chemical precursors or other chemicals.

A nucleic acid molecule of the invention, *e.g.*, a nucleic acid molecule having the nucleotide sequence of SEQ ID NO:2$n$-1, wherein $n$ is an integer between 1 and 12, or a complement of this nucleotide sequence, can be isolated using standard molecular biology techniques and the sequence information provided herein. Using all or a portion of the nucleic acid sequence of SEQ ID NO:2$n$-1, wherein $n$ is an integer between 1 and 12, as a hybridization probe, NOVX molecules can be isolated using standard hybridization and cloning techniques (*e.g.,* as described in Sambrook, et al., (eds.), MOLECULAR CLONING: A LABORATORY MANUAL 2nd Ed., Cold Spring Harbor Laboratory Press, Cold Spring Harbor, NY, 1989; and Ausubel, et al., (eds.), CURRENT PROTOCOLS IN MOLECULAR BIOLOGY, John Wiley & Sons, New York, NY, 1993.)

A nucleic acid of the invention can be amplified using cDNA, mRNA or alternatively, genomic DNA, as a template with appropriate oligonucleotide primers according to standard PCR amplification techniques. The nucleic acid so amplified can be cloned into an appropriate vector and characterized by DNA sequence analysis. Furthermore, oligonucleotides corresponding to NOVX nucleotide sequences can be prepared by standard synthetic techniques, *e.g.*, using an automated DNA synthesizer.

As used herein, the term "oligonucleotide" refers to a series of linked nucleotide residues. A short oligonucleotide sequence can be based on, or designed from, a genomic or cDNA sequence and is used to amplify, confirm, or reveal the presence of an identical, similar or complementary DNA or RNA in a particular cell or tissue. Oligonucleotides comprise a nucleic acid sequence having about 10 nt, 50 nt, or 100 nt in length, preferably about 15 nt to 30 nt in length. In one embodiment of the invention, an oligonucleotide comprising a nucleic acid molecule less than 100 nt in length would further comprise at least 6 contiguous nucleotides of SEQ ID NO:2$n$-1, wherein n is an integer between 1 and 12, or a complement thereof. Oligonucleotides can be chemically synthesized and can also be used as probes.

In another embodiment, an isolated nucleic acid molecule of the invention comprises a nucleic acid molecule that is a complement of the nucleotide sequence shown in SEQ ID NO:2$n$-1, wherein n is an integer between 1 and 12, or a portion of this nucleotide sequence (*e.g.*, a fragment that can be used as a probe or primer or a fragment encoding a biologically-active portion of a NOVX polypeptide). A nucleic acid molecule that is complementary to the nucleotide sequence of SEQ ID NO:2$n$-1, wherein n is an integer between 1 and 12, is one that is sufficiently complementary to the nucleotide sequence of SEQ ID NO:2$n$-1, wherein $n$ is an integer between 1 and 12, that it can hydrogen bond with few or no mismatches to the nucleotide sequence shown in SEQ ID NO:2$n$-1, wherein $n$ is an integer between 1 and 12, thereby forming a stable duplex.

As used herein, the term "complementary" refers to Watson-Crick or Hoogsteen base pairing between nucleotides units of a nucleic acid molecule, and the term "binding" means the physical or chemical interaction between two polypeptides or compounds or associated polypeptides or compounds or combinations thereof. Binding includes ionic, non-ionic, van der Waals, hydrophobic interactions, and the like. A physical interaction can be either direct or indirect. Indirect interactions may be through or due to the effects of another polypeptide or compound. Direct binding refers to interactions that do not take place through, or due to, the effect of another polypeptide or compound, but instead are without other substantial chemical intermediates.

A "fragment" provided herein is defined as a sequence of at least 6 (contiguous) nucleic acids or at least 4 (contiguous) amino acids, a length sufficient to allow for specific hybridization in the case of nucleic acids or for specific recognition of an epitope in the case of amino acids, and is at most some portion less than a full length sequence. Fragments can be derived from any contiguous portion of a nucleic acid or amino acid sequence of choice.

A full-length NOVX clone is identified as containing an ATG translation start codon and an in-frame stop codon. Any disclosed NOVX nucleotide sequence lacking an ATG start codon therefore encodes a truncated C-terminal fragment of the respective NOVX polypeptide, and requires that the corresponding full-length cDNA extend in the 5' direction of the disclosed sequence. Any disclosed NOVX nucleotide sequence lacking an in-frame stop codon similarly encodes a truncated N-terminal fragment of the respective NOVX polypeptide, and requires that the corresponding full-length cDNA extend in the 3' direction of the disclosed sequence.

A "derivative" is a nucleic acid sequence or amino acid sequence formed from the native compounds either directly, by modification or partial substitution. An "analog" is a nucleic acid sequence or amino acid sequence that has a structure similar to, but not identical to, the native compound, *e.g.* they differs from it in respect to certain components or side chains. Analogs can be synthetic or derived from a different evolutionary origin and may have a similar or opposite metabolic activity compared to wild type. A "homolog" is a nucleic acid sequence or amino acid sequence of a particular gene that is derived from different species.

Derivatives and analogs can be full length or other than full length. Derivatives or analogs of the nucleic acids or proteins of the invention include, but are not limited to, molecules comprising regions that are substantially homologous to the nucleic acids or proteins of the invention, in various embodiments, by at least about 70%, 80%, or 95% identity (with a preferred identity of 80-95%) over a nucleic acid or amino acid sequence of identical size or when compared to an aligned sequence in which the alignment is done by a computer homology program known in the art, or whose encoding nucleic acid is capable of hybridizing to the complement of a sequence encoding the proteins under stringent, moderately stringent, or low stringent conditions. *See e.g.* Ausubel, et al., CURRENT PROTOCOLS IN MOLECULAR BIOLOGY, John Wiley & Sons, New York, NY, 1993, and below.

A "homologous nucleic acid sequence" or "homologous amino acid sequence," or variations thereof, refer to sequences characterized by a homology at the nucleotide level or amino acid level as discussed above. Homologous nucleotide sequences include those sequences coding for isoforms of NOVX polypeptides. Isoforms can be expressed in different tissues of the same organism as a result of, for example, alternative splicing of RNA. Alternatively, isoforms can be encoded by different genes. In the invention, homologous nucleotide sequences include nucleotide sequences encoding for a NOVX polypeptide of species other than humans, including, but not limited to: vertebrates, and thus can include, *e.g.,* frog, mouse, rat, rabbit, dog, cat cow, horse, and other organisms. Homologous nucleotide sequences also include, but are not limited to, naturally occurring allelic variations and mutations of the nucleotide sequences set forth herein. A homologous nucleotide sequence does not, however, include the exact nucleotide sequence encoding human NOVX protein. Homologous nucleic acid sequences include those nucleic acid sequences that encode conservative amino acid substitutions (see below) in SEQ ID NO:$2n$-1, wherein $n$ is an integer between 1 and 12, as well as a polypeptide possessing NOVX biological activity. Various biological activities of the NOVX proteins are described below.

A NOVX polypeptide is encoded by the open reading frame ("ORF") of a NOVX nucleic acid. An ORF corresponds to a nucleotide sequence that could potentially be translated into a polypeptide. A stretch of nucleic acids comprising an ORF is uninterrupted by a stop codon. An ORF that represents the coding sequence for a full protein begins with an ATG "start" codon and terminates with one of the three "stop" codons, namely, TAA, TAG, or TGA. For the purposes of this invention, an ORF can be any part of a coding sequence, with or without a start codon, a stop codon, or both. For an ORF to be considered as a good candidate for coding for a *bona fide* cellular protein, a minimum size requirement is often set, *e.g.,* a stretch of DNA that would encode a protein of 50 amino acids or more.

The nucleotide sequences determined from the cloning of the human NOVX genes allows for the generation of probes and primers designed for use in identifying and/or cloning NOVX homologues in other cell types, e.g. from other tissues, as well as NOVX homologues from other vertebrates. The probe/primer typically comprises substantially purified oligonucleotide. The oligonucleotide typically comprises a region of nucleotide sequence that hybridizes under stringent conditions to at least about 12, 25, 50, 100, 150, 200, 250, 300, 350 or 400 consecutive sense strand nucleotide sequence of SEQ ID NO:$2n$-1, wherein $n$ is an integer between 1 and 12; or an anti-sense strand nucleotide sequence of SEQ ID NO:$2n$-1, wherein n is an integer between 1 and 12; or of a naturally occurring mutant of SEQ ID NO:$2n$-1, wherein n is an integer between 1 and 12.

Probes based on the human NOVX nucleotide sequences can be used to detect transcripts or genomic sequences encoding the same or homologous proteins. In various embodiments, the probe has a detectable label attached, e.g. the label can be a radioisotope, a fluorescent compound, an enzyme, or an enzyme co-factor. Such probes can be used as a part of a diagnostic test kit for identifying cells or tissues which mis-express a NOVX protein, such as by measuring a level of a NOVX-encoding nucleic acid in a sample of cells from a subject *e.g.,* detecting NOVX mRNA levels or determining whether a genomic NOVX gene has been mutated or deleted.

"A polypeptide having a biologically-active portion of a NOVX polypeptide" refers to polypeptides exhibiting activity similar, but not necessarily identical to, an activity of a polypeptide of the invention, including mature forms, as measured in a particular biological assay, with or without dose dependency. A nucleic acid fragment encoding a "biologically-active portion of NOVX" can be prepared by isolating a portion of SEQ ID NO:2*n*-1, wherein *n* is an integer between 1 and 12, that encodes a polypeptide having a NOVX biological activity (the biological activities of the NOVX proteins are described below), expressing the encoded portion of NOVX protein (*e.g.,* by recombinant expression *in vitro*) and assessing the activity of the encoded portion of NOVX.

**NOVX Nucleic Acid and Polypeptide Variants**

The invention further encompasses nucleic acid molecules that differ from the nucleotide sequences of SEQ ID NO:2*n*-1, wherein *n* is an integer between 1 and 12, due to degeneracy of the genetic code and thus encode the same NOVX proteins as that encoded by the nucleotide sequences of SEQ ID NO:2*n*-1, wherein *n* is an integer between 1 and 12. In another embodiment, an isolated nucleic acid molecule of the invention has a nucleotide sequence encoding a protein having an amino acid sequence of SEQ ID NO:2*n*, wherein n is an integer between 1 and 12.

In addition to the human NOVX nucleotide sequences of SEQ ID NO:2*n*-1, wherein *n* is an integer between 1 and 12, it will be appreciated by those skilled in the art that DNA sequence polymorphisms that lead to changes in the amino acid sequences of the NOVX polypeptides may exist within a population (*e.g.*, the human population). Such genetic polymorphism in the NOVX genes may exist among individuals within a population due to natural allelic variation. As used herein, the terms "gene" and "recombinant gene" refer to nucleic acid molecules comprising an open reading frame (ORF) encoding a NOVX protein, preferably a vertebrate NOVX protein. Such natural allelic variations can typically result in 1-5% variance in the nucleotide sequence of the NOVX genes. Any and all such nucleotide variations and resulting amino acid polymorphisms in the NOVX polypeptides, which are the result of natural allelic variation and that do not alter the functional activity of the NOVX polypeptides, are intended to be within the scope of the invention.

Moreover, nucleic acid molecules encoding NOVX proteins from other species, and thus that have a nucleotide sequence that differs from a human SEQ ID NO:2*n*-1, wherein n is an integer between 1 and 12, are intended to be within the scope of the invention. Nucleic acid molecules corresponding to natural allelic variants and homologues of the NOVX cDNAs of the invention can be isolated based on their homology to the human NOVX nucleic acids disclosed herein using the human cDNAs, or a portion thereof, as a hybridization probe according to standard hybridization techniques under stringent hybridization conditions.

Accordingly, in another embodiment, an isolated nucleic acid molecule of the invention is at least 6 nucleotides in length and hybridizes under stringent conditions to the nucleic acid molecule comprising the nucleotide sequence of SEQ ID NO:2*n*-1, wherein n is an integer between 1 and 12. In another embodiment, the nucleic acid is at least 10, 25, 50, 100, 250, 500, 750, 1000, 1500, or 2000 or more nucleotides in length. In yet another embodiment, an isolated nucleic acid molecule of the invention hybridizes to the coding region. As used herein, the term "hybridizes under stringent conditions" is intended to describe conditions for hybridization and washing under which nucleotide sequences at least about 65% homologous to each other typically remain hybridized to each other.

Homologs (*i.e.,* nucleic acids encoding NOVX proteins derived from species other than human) or other related sequences (*e.g.*, paralogs) can be obtained by low, moderate or high stringency hybridization with all or a portion of the particular human sequence as a probe using methods well known in the art for nucleic acid hybridization and cloning.

As used herein, the phrase "stringent hybridization conditions" refers to conditions under which a probe, primer or oligonucleotide will hybridize to its target sequence, but to no other sequences. Stringent conditions are sequence-dependent and will be different in different circumstances. Longer sequences hybridize specifically at higher temperatures than shorter sequences. Generally, stringent conditions are selected to be about 5 °C lower than the thermal melting point (Tm) for the specific sequence at a defined ionic strength and pH. The Tm is the temperature (under defined ionic strength, pH and nucleic acid concentration) at which 50% of the probes complementary to the target sequence hybridize to the target sequence at equilibrium. Since the target sequences are generally present at excess, at Tm, 50% of the probes are occupied at equilibrium. Typically, stringent conditions are those in which the salt concentration is less than about 1.0 M sodium ion, typically about 0.01 to 1.0 M sodium ion (or other salts) at pH 7.0 to 8.3 and the temperature is at least about 30 °C for short probes, primers or oligonucleotides (e.g., 10 nt to 50 nt) and at least about 60 °C for longer probes, primers and oligonucleotides. Stringent conditions can also be achieved with the addition of destabilizing agents, such as formamide.

Stringent conditions are known to those skilled in the art and can be found in Ausubel, et al., (eds.), CURRENT PROTOCOLS IN MOLECULAR BIOLOGY, John Wiley & Sons, N.Y. (1989), 6.3.1-6.3.6. Preferably, the conditions are such that sequences at least about 65%, 70%, 75%, 85%, 90%, 95%, 98%, or 99% homologous to each other typically remain hybridized to each other. A non-limiting example of stringent hybridization conditions are hybridization in a high salt buffer comprising 6X SSC, 50 mM Tris-HCl (pH 7.5), 1 mM EDTA, 0.02% PVP, 0.02% Ficoll, 0.02% BSA, and 500 mg/ml denatured salmon sperm DNA at 65°C, followed by one or more washes in 0.2X SSC, 0.01% BSA at 50°C. An isolated nucleic acid molecule of the invention that hybridizes under stringent conditions to a sequence of SEQ ID NO:2*n*-1, wherein *n* is an integer between 1 and 12, corresponds to a naturally-occurring nucleic acid molecule. As used herein, a "naturally-occurring" nucleic acid molecule refers to an RNA or DNA molecule having a nucleotide sequence that occurs in nature (*e.g.*, encodes a natural protein).

In a second embodiment, a nucleic acid sequence that is hybridizable to the nucleic acid molecule comprising the nucleotide sequence of SEQ ID NO:2*n*-1, wherein *n* is an integer between 1 and 12, or fragments, analogs or derivatives thereof, under conditions of moderate stringency is provided. A non-limiting example of moderate stringency hybridization conditions are hybridization in 6X SSC, 5X Reinhardt's solution, 0.5% SDS and 100 mg/ml denatured salmon sperm DNA at 55 °C, followed by one or more washes in 1X SSC, 0.1% SDS at 37 °C. Other conditions of moderate stringency that can be used are well-known within the art. *See, e.g.,* Ausubel, et al. (eds.), 1993, CURRENT PROTOCOLS IN MOLECULAR BIOLOGY, John Wiley & Sons, NY, and Krieger, 1990; GENE TRANSFER AND EXPRESSION, A LABORATORY MANUAL, Stockton Press, NY.

In a third embodiment, a nucleic acid that is hybridizable to the nucleic acid molecule comprising the nucleotide sequences of SEQ ID NO:2*n*-1, wherein *n* is an integer between 1 and 12, or fragments, analogs or derivatives thereof, under conditions of low stringency, is provided. A non-limiting example of low stringency hybridization conditions are hybridization in 35% formamide, 5X SSC, 50 mM Tris-HCl (pH 7.5), 5 mM EDTA, 0.02% PVP, 0.02% Ficoll, 0.2% BSA, 100 mg/ml denatured salmon sperm DNA, 10% (wt/vol) dextran sulfate at 40°C, followed by one or more washes in 2X SSC, 25 mM Tris-HCl (pH 7.4), 5 mM EDTA, and 0.1% SDS at 50°C. Other conditions of low stringency that can be used are well known in the art (*e.g.,* as employed for cross-species hybridizations). *See, e.g.,* Ausubel, et al. (eds.), 1993, CURRENT PROTOCOLS IN MOLECULAR BIOLOGY, John Wiley & Sons, NY, and Kriegler, 1990, GENE TRANSFER AND EXPRESSION, A LABORATORY MANUAL, Stockton Press, NY; Shilo and Weinberg, 1981. Proc Natl Acad Sci USA 78: 6789-6792.

**Conservative Mutations**

In addition to naturally-occurring allelic variants of NOVX sequences that may exist in the population, the skilled artisan will further appreciate that changes can be introduced by mutation into the nucleotide sequences of SEQ ID NO:2*n*-1, wherein n is an integer between 1 and 12, thereby leading to changes in the amino acid sequences of the encoded NOVX protein, without altering the functional ability of that NOVX protein. For example, nucleotide substitutions leading to amino acid substitutions at "non-essential" amino acid residues can be made in the sequence of SEQ ID NO:2n, wherein *n* is an integer between 1 and 12. A "non-essential" amino acid residue is a residue that can be altered from the wild-type sequences of the NOVX proteins without altering their biological activity, whereas an "essential" amino acid residue is required for such biological activity. For example, amino acid residues that are conserved among the NOVX proteins of the invention are predicted to be particularly non-amenable to alteration. Amino acids for which conservative substitutions can be made are well-known within the art.

Another aspect of the invention pertains to nucleic acid molecules encoding NOVX proteins that contain changes in amino acid residues that are not essential for activity. Such NOVX proteins differ in amino acid sequence from SEQ ID NO:2*n*-1, wherein *n* is an integer between 1 and 12, yet retain biological activity. In one embodiment, the isolated nucleic acid molecule comprises a nucleotide sequence encoding a protein, wherein the protein comprises an amino acid sequence at least about 40% homologous to the amino acid sequences of SEQ ID NO:2n, wherein *n* is an integer between 1 and 12. Preferably, the protein encoded by the nucleic acid molecule is at least about 60% homologous to SEQ ID NO:2*n*, wherein *n* is an integer between 1 and 12; more preferably at least about 70% homologous to SEQ ID NO:2*n*, wherein *n* is an integer between 1 and 12; still more preferably at least about 80% homologous to SEQ ID NO:2*n*, wherein *n* is an integer between 1 and 12; even more preferably at least about 90% homologous to SEQ ID NO:2*n*, wherein *n* is an integer between 1 and 12; and most preferably at least about 95% homologous to SEQ ID NO:2*n*, wherein n is an integer between 1 and 12.

An isolated nucleic acid molecule encoding a NOVX protein homologous to the protein of SEQ ID NO:2*n*, wherein *n* is an integer between 1 and 12, can be created by introducing one or more nucleotide substitutions, additions or deletions into the nucleotide sequence of SEQ ID NO:2*n*-1, wherein n is an integer between 1 and 12, such that one or more amino acid substitutions, additions or deletions are introduced into the encoded protein.

Mutations can be introduced any one of SEQ ID NO:2*n*-1, wherein *n* is an integer between 1 and 12, by standard techniques, such as site-directed mutagenesis and PCR-mediated mutagenesis. Preferably, conservative amino acid substitutions are made at one or more predicted, non-essential amino acid residues. A "conservative amino acid substitution" is one in which the amino acid residue is replaced with an amino acid residue having a similar side chain. Families of amino acid residues having similar side chains have been defined within the art. These families include amino acids with basic side chains (*e.g.,* lysine, arginine, histidine), acidic side chains (*e.g.,* aspartic acid, glutamic acid), uncharged polar side chains (e.g., glycine, asparagine, glutamine, serine, threonine, tyrosine, cysteine), nonpolar side chains (*e.g.*, alanine, valine, leucine, isoleucine, proline, phenylalanine, methionine, tryptophan), beta-branched side chains (*e.g.*, threonine, valine, isoleucine) and aromatic side chains (*e.g.,* tyrosine, phenylalanine, tryptophan, histidine). Thus, a predicted non-essential amino acid residue in the NOVX protein is replaced with another amino acid residue from the same side chain family. Alternatively, in another embodiment, mutations can be introduced randomly along all or part of a NOVX coding sequence, such as by saturation mutagenesis, and the resultant mutants can be screened for NOVX biological activity to identify mutants that retain activity. Following mutagenesis of a nucleic acid of SEQ ID NO:2$n$-1, wherein $n$ is an integer between 1 and 12, the encoded protein can be expressed by any recombinant technology known in the art and the activity of the protein can be determined.

The relatedness of amino acid families can also be determined based on side chain interactions. Substituted amino acids can be fully conserved "strong" residues or fully conserved "weak" residues. The "strong" group of conserved amino acid residues can be any one of the following groups: STA, NEQK, NHQK, NDEQ, QHRK, MILV, MILF, HY, FYW, wherein the single letter amino acid codes are grouped by those amino acids that can be substituted for each other. Likewise, the "weak" group of conserved residues can be any one of the following: CSA, ATV, SAG, STNK, STPA, SGND, SNDEQK, NDEQHK, NEQHRK, HFY, wherein the letters within each group represent the single letter amino acid code.

In one embodiment, a mutant NOVX protein can be assayed for (i) the ability to form protein:protein interactions with other NOVX proteins, other cell-surface proteins, or biologically-active portions thereof, (*ii*) complex formation between a mutant NOVX protein and a NOVX ligand; or (*iii*) the ability of a mutant NOVX protein to bind to an intracellular target protein or biologically-active portion thereof; (*e.g.* avidin proteins).

In yet another embodiment, a mutant NOVX protein can be assayed for the ability to regulate a specific biological function (*e.g.,* regulation of insulin release).

## Interfering RNA

In one aspect of the invention, NOVX gene expression can be attenuated by RNA interference. One approach well-known in the art is short interfering RNA (siRNA) mediated gene silencing where expression products of a NOVX gene are targeted by specific double stranded NOVX derived siRNA nucleotide sequences that are complementary to at least a 19-25 nt long segment of the NOVX gene transcript, including the 5' untranslated (UT) region, the ORF, or the 3' UT region. *See, e.g.,* PCT applications WO00/44895, WO99/32619, WO01/75164, WO01/9251 WO 01/29058, WO01/89304, WO02/16620, and WO02/29858, each incorporated by reference herein in their entirety. Targeted genes can be a NOVX gene, or an upstream or downstream modulator of the NOVX gene. Nonlimiting examples of upstream or downstream modulators of a NOVX gene include, *e.g.*, a transcription factor that binds the NOVX gene promoter, a kinase or phosphatase that interacts with a NOVX polypeptide, and polypeptides involved in a NOVX regulatory pathway.

According to the methods of the present invention, NOVX gene expression is silenced using short interfering RNA. A NOVX polynucleotide according to the invention includes a siRNA polynucleotide. Such a NOVX siRNA can be obtained using a NOVX polynucleotide sequence, for example, by processing the NOVX ribopolynucleotide sequence in a cell-free system, such as but not limited to a Drosophila extract, or by transcription of recombinant double stranded NOVX RNA or by chemical synthesis of nucleotide sequences homologous to a NOVX sequence. *See, e.g.,* Tuschl, Zamore, Lehmann, Bartel and Sharp (1999), Genes & Dev. 13: 3191-3197, incorporated herein by reference in its entirety. When synthesized, a typical 0.2 micromolar-scale RNA synthesis provides about 1 milligram of siRNA, which is sufficient for 1000 transfection experiments using a 12-well tissue culture plate format.

The most efficient silencing is generally observed with siRNA duplexes composed of a 21-nt sense strand and a 21-nt antisense strand, paired in a manner to have a 2-nt 3' overhang. The sequence of the 2-nt 3' overhang makes an additional small contribution to the specificity of siRNA target recognition. The contribution to specificity is localized to the unpaired nucleotide adjacent to the first paired bases. In one embodiment, the nucleotides in the 3' overhang are ribonucleotides. In an alternative embodiment, the nucleotides in the 3' overhang are deoxyribonucleotides. Using 2'-deoxyribonucleotides in the 3' overhangs is as efficient as using ribonucleotides, but deoxyribonucleotides are often cheaper to synthesize and are most likely more nuclease resistant.

A contemplated recombinant expression vector of the invention comprises a NOVX DNA molecule cloned into an expression vector comprising operatively-linked regulatory sequences flanking the NOVX sequence in a manner that allows for expression (by transcription of the DNA molecule) of both strands. An RNA molecule that is antisense to NOVX mRNA is transcribed by a first promoter (*e.g.,* a promoter sequence 3' of the cloned DNA) and an RNA molecule that is the sense strand for the NOVX mRNA is transcribed by a second promoter (*e.g.,* a promoter sequence 5' of the cloned DNA). The sense and antisense strands may hybridize in vivo to generate siRNA constructs for silencing of the NOVX gene. Alternatively, two constructs can be utilized to create the sense and anti-sense strands of a siRNA construct. Finally, cloned DNA can encode a construct having secondary structure, wherein a single transcript has both the sense and complementary antisense sequences from the target gene or genes. In an example of this embodiment, a hairpin RNAi product is homologous to all or a portion of the target gene. In another example, a hairpin RNAi product is a siRNA. The regulatory sequences flanking the NOVX sequence may be identical or may be different, such that their expression may be modulated independently, or in a temporal or spatial manner.

In a specific embodiment, siRNAs are transcribed intracellularly by cloning the NOVX gene templates into a vector containing, *e.g.*, a RNA pol III transcription unit from the smaller nuclear RNA (snRNA) U6 or the human RNase P RNA H1. One example of a vector system is the GeneSuppressor™ RNA Interference kit (commercially available from Imgenex). The U6 and H1 promoters are members of the type III class of Pol III promoters. The +1 nucleotide of the U6-like promoters is always guanosine, whereas the +1 for H1 promoters is adenosine. The termination signal for these promoters is defined by five consecutive thymidines. The transcript is typically cleaved after the second uridine. Cleavage at this position generates a 3' UU overhang in the expressed siRNA, which is similar to the 3' overhangs of synthetic siRNAs. Any sequence less than 400 nucleotides in length can be transcribed by these promoter, therefore they are ideally suited for the expression of around 21-nucleotide siRNAs in, *e.g.,* an approximately 50-nucleotide RNA stem-loop transcript.

A siRNA vector appears to have an advantage over synthetic siRNAs where long term knock-down of expression is desired. Cells transfected with a siRNA expression vector would experience steady, long-term mRNA inhibition. In contrast, cells transfected with exogenous synthetic siRNAs typically recover from mRNA suppression within seven days or ten rounds of cell division. The long-term gene silencing ability of siRNA expression vectors may provide for applications in gene therapy.

In general, siRNAs are chopped from longer dsRNA by an ATP-dependent ribonuclease called DICER. DICER is a member of the RNase III family of double-stranded RNA-specific endonucleases. The siRNAs assemble with cellular proteins into an endonuclease complex. *In vitro* studies in Drosophila suggest that the siRNAs/protein complex (siRNP) is then transferred to a second enzyme complex, called an RNA-induced silencing complex (RISC), which contains an endoribonuclease that is distinct from DICER. RISC uses the sequence encoded by the antisense siRNA strand to find and destroy mRNAs of complementary sequence. The siRNA thus acts as a guide, restricting the ribonuclease to cleave only mRNAs complementary to one of the two siRNA strands.

A NOVX mRNA region to be targeted by siRNA is generally selected from a desired NOVX sequence beginning 50 to 100 nt downstream of the start codon. Alternatively, 5' or 3' UTRs and regions nearby the start codon can be used but are generally avoided, as these may be richer in regulatory protein binding sites. UTR-binding proteins and/or translation initiation complexes may interfere with binding of the siRNP or RISC endonuclease complex. An initial BLAST homology search for the selected siRNA sequence is done against an available nucleotide sequence library to ensure that only one gene is targeted. Specificity of target recognition by siRNA duplexes indicate that a single point mutation located in the paired region of an siRNA duplex is sufficient to abolish target mRNA degradation. See, Elbashir et al. 2001 EMBO J. 20(23):6877-88. Hence, consideration should be taken to accommodate SNPs, polymorphisms, allelic variants or species-specific variations when targeting a desired gene.

In one embodiment, a complete NOVX siRNA experiment includes the proper negative control. A negative control siRNA generally has the same nucleotide composition as the NOVX siRNA but lack significant sequence homology to the genome. Typically, one would scramble the nucleotide sequence of the NOVX siRNA and do a homology search to make sure it lacks homology to any other gene.

Two independent NOVX siRNA duplexes can be used to knock-down a target NOVX gene. This helps to control for specificity of the silencing effect. In addition, expression of two independent genes can be simultaneously knocked down by using equal concentrations of different NOVX siRNA duplexes, *e.g.,* a NOVX siRNA and an siRNA for a regulator of a NOVX gene or polypeptide. Availability of siRNA-associating proteins is believed to be more limiting than target mRNA accessibility.

A targeted NOVX region is typically a sequence of two adenines (AA) and two thymidines (TT) divided by a spacer region of nineteen (N19) residues (*e.g.,* AA(N19)TT). A desirable spacer region has a G/C-content of approximately 30% to 70%, and more preferably of about 50%. If the sequence AA(N19)TT is not present in the target sequence, an alternative target region would be AA(N21). The sequence of the NOVX sense siRNA corresponds to (N19)TT or N21, respectively. In the latter case, conversion of the 3' end of the sense siRNA to TT can be performed if such a sequence does not naturally occur in the NOVX polynucleotide. The rationale for this sequence conversion is to generate a symmetric duplex with respect to the sequence composition of the sense and antisense 3' overhangs. Symmetric 3' overhangs may help to ensure that the siRNPs are formed with approximately equal ratios of sense and antisense target RNA-cleaving siRNPs. *See, e.g.,* Elbashir, Lendeckel and Tuschl (2001). Genes & Dev. 15: 188-200, incorporated by reference herein in its entirely. The modification of the overhang of the sense sequence of the siRNA duplex is not expected to affect targeted mRNA recognition, as the antisense siRNA strand guides target recognition.

Alternatively, if the NOVX target mRNA does not contain a suitable AA(N21) sequence, one can search for the sequence NA(N21). Further, the sequence of the sense strand and antisense strand may still be synthesized as 5' (N19)TT, as it is believed that the sequence of the 3'-most nucleotide of the antisense siRNA does not contribute to specificity. Unlike antisense or ribozyme technology, the secondary structure of the target mRNA does not appear to have a strong effect on silencing. *See,* Harborth, et al. (2001) J. Cell Science 114: 4557-4565, incorporated by reference in its entirety.

Transfection of NOVX siRNA duplexes can be achieved using standard nucleic acid transfection methods, for example, OLIGOFECTAMINE Reagent (commercially available from Invitrogen). An assay for NOVX gene silencing is generally performed approximately 2 days after transfection. No NOVX gene silencing has been observed in the absence of transfection reagent, allowing for a comparative analysis of the wild-type and silenced NOVX phenotypes. In a specific embodiment, for one well of a 24-well plate, approximately 0.84 μg of the siRNA duplex is generally sufficient. Cells are typically seeded the previous day, and are transfected at about 50% confluence. The choice of cell culture media and conditions are routine to those of skill in the art, and will vary with the choice of cell type. The efficiency of transfection may depend on the cell type, but also on the passage number and the confluency of the cells. The time and the manner of formation of siRNA-liposome complexes (*e.g.* inversion versus vortexing) are also critical. Low transfection efficiencies are the most frequent cause of unsuccessful NOVX silencing. The efficiency of transfection needs to be carefully examined for each new cell line to be used. Preferred cell are derived from a mammal, more preferably from a rodent such as a rat or mouse, and most preferably from a human. Where used for therapeutic treatment, the cells are preferentially autologous, although non-autologous cell sources are also contemplated as within the scope of the present invention.

For a control experiment, transfection of 0.84 μg single-stranded sense NOVX siRNA will have no effect on NOVX silencing, and 0.84 μg antisense siRNA has a weak silencing effect when compared to 0.84 μg of duplex siRNAs. Control experiments again allow for a comparative analysis of the wild-type and silenced NOVX phenotypes. To control for transfection efficiency, targeting of common proteins is typically performed, for example targeting of lamin A/C or transfection of a CMV-driven EGFP-expression plasmid (*e.g.* commercially available from Clontech). In the above example, a determination of the fraction of lamin A/C knockdown in cells is determined the next day by such techniques as immunofluorescence, Western blot, Northern blot or other similar assays for protein expression or gene expression. Lamin A/C monoclonal antibodies can be obtained from Santa Cruz Biotechnology.

Depending on the abundance and the half life (or turnover) of the targeted NOVX polynucleotide in a cell, a knock-down phenotype may become apparent after 1 to 3 days, or even later. In cases where no NOVX knock-down phenotype is observed, depletion of the NOVX polynucleotide may be observed by immunofluorescence or Western blotting. If the NOVX polynucleotide is still abundant after 3 days, cells need to be split and transferred to a fresh 24-well plate for re-transfection. If no knock-down of the targeted protein is observed, it may be desirable to analyze whether the target mRNA (NOVX or a NOVX upstream or downstream gene) was effectively destroyed by the transfected siRNA duplex. Two days after transfection, total RNA is prepared, reverse transcribed using a target-specific primer, and PCR-amplified with a primer pair covering at least one exon-exon junction in order to control for amplification of pre-mRNAs. RT/PCR of a non-targeted mRNA is also needed as control. Effective depletion of the mRNA yet undetectable reduction of target protein can indicate that a large reservoir of stable NOVX protein may exist in the cell. Multiple transfection in sufficiently long intervals may be necessary until the target protein is finally depleted to a point where a phenotype may become apparent. If multiple transfection steps are required, cells are split 2 to 3 days after transfection. The cells may be transfected immediately after splitting.

An inventive therapeutic method of the invention contemplates administering a NOVX siRNA construct as therapy to compensate for increased or aberrant NOVX expression or activity. The NOVX ribopolynucleotide is obtained and processed into siRNA fragments, or a NOVX siRNA is synthesized, as described above. The NOVX siRNA is administered to cells or tissues using known nucleic acid transfection techniques, as described above. A NOVX siRNA specific for a NOVX gene will decrease or knockdown NOVX transcription products, which leads to reduced NOVX polypeptide production, resulting in reduced NOVX polypeptide activity in the cells or tissues.

The present invention also encompasses a method of treating a disease or condition associated with the presence of a NOVX protein in an individual comprising administering to the individual an RNAi construct that targets the mRNA of the protein (the mRNA that encodes the protein) for degradation. A specific RNAi construct includes a siRNA or a double stranded gene transcript that is processed into siRNAs. Upon treatment, the target protein is not produced or is not produced to the extent it would be in the absence of the treatment.

Where the NOVX gene function is not correlated with a known phenotype, a control sample of cells or tissues from healthy individuals provides a reference standard for determining NOVX expression levels. Expression levels are detected using the assays described, *e.g.,* RT-PCR, Northern blotting, Western blotting, ELISA, and the like. A subject sample of cells or tissues is taken from a mammal, preferably a human subject, suffering from a disease state. The NOVX ribopolynucleotide is used to produce siRNA constructs, that are specific for the NOVX gene product. These cells or tissues are treated by administering NOVX siRNA's to the cells or tissues by methods described for the transfection of nucleic acids into a cell or tissue, and a change in NOVX polypeptide or polynucleotide expression is observed in the subject sample relative to the control sample, using the assays described. This NOVX gene knockdown approach provides a rapid method for determination of a NOVX minus (NOVX⁻) phenotype in the treated subject sample. The NOVX⁻ phenotype observed in the treated subject sample thus serves as a marker for monitoring the course of a disease state during treatment.

In specific embodiments, a NOVX siRNA is used in therapy. Methods for the generation and use of a NOVX siRNA are known to those skilled in the art. Example techniques are provided below.

**Production of RNAs**

Sense RNA (ssRNA) and antisense RNA (asRNA) of NOVX are produced using known methods such as transcription in RNA expression vectors. In the initial experiments, the sense and antisense RNA are about 500 bases in length each. The produced ssRNA and asRNA (0.5 μM) in 10 mM Tris-HCl (pH 7.5) with 20 mM NaCl were heated to 95° C for 1 min then cooled and annealed at room temperature for 12 to 16 h. The RNAs are precipitated and resuspended in lysis buffer (below). To monitor annealing, RNAs are electrophoresed in a 2% agarose gel in TBE buffer and stained with ethidium bromide. See, *e.g.,* Sambrook et al., Molecular Cloning. Cold Spring Harbor Laboratory Press, Plainview, N.Y. (1989).

**Lysate Preparation**

Untreated rabbit reticulocyte lysate (Ambion) are assembled according to the manufacturer's directions. dsRNA is incubated in the lysate at 30° C for 10 min prior to the addition of mRNAs. Then NOVX mRNAs are added and the incubation continued for an additional 60 min. The molar ratio of double stranded RNA and mRNA is about 200:1. The NOVX mRNA is radiolabeled (using known techniques) and its stability is monitored by gel electrophoresis.

In a parallel experiment made with the same conditions, the double stranded RNA is internally radiolabeled with a $^{32}$P-ATP. Reactions are stopped by the addition of 2 X proteinase K buffer and deproteinized as described previously (Tuschl et al., Genes Dev., 13:3191-3197 (1999)). Products are analyzed by electrophoresis in 15% or 18% polyacrylamide sequencing gels using appropriate RNA standards. By monitoring the gels for radioactivity, the natural production of 10 to 25 nt RNAs from the double stranded RNA can be determined.

The band of double stranded RNA, about 21-23 bps, is eluded. The efficacy of these 21-23 mers for suppressing NOVX transcription is assayed in vitro using the same rabbit reticulocyte assay described above using 50 nanomolar of double stranded 21-23 mer for each assay. The sequence of these 21-23 mers is then determined using standard nucleic acid sequencing techniques.

**RNA Preparation**

21 nt RNAs, based on the sequence determined above, are chemically synthesized using Expedite RNA phosphoramidites and thymidine phosphoramidite (Proligo, Germany). Synthetic oligonucleotides are deprotected and gel-purified (Elbashir, Lendeckel, & Tuschl, Genes & Dev. 15, 188-200 (2001)), followed by Sep-Pak C18 cartridge (Waters, Milford, Mass., USA) purification (Tuschl, et al., Biochemistry, 32:11658-11668 (1993)).

These RNAs (20 μM) single strands are incubated in annealing buffer (100 mM potassium acetate, 30 mM HEPES-KOH at pH 7.4, 2 mM magnesium acetate) for 1 min at 90° C followed by 1 h at 37° C.

**Cell Culture**

A cell culture known in the art to regularly express NOVX is propagated using standard conditions. 24 hours before transfection, at approx. 80% confluency, the cells are trypsinized and diluted 1:5 with fresh medium without antibiotics (1-3 X 105 cells/ml) and transferred to 24-well plates (500 ml/well). Transfection is performed using a commercially available lipofection kit and NOVX expression is monitored using standard techniques with positive and negative control. A positive control is cells that naturally express NOVX while a negative control is cells that do not express NOVX. Base-paired 21 and 22 nt siRNAs with overhanging 3' ends mediate efficient sequence-specific mRNA degradation in lysates and in cell culture. Different concentrations of siRNAs are used. An efficient concentration for suppression in vitro in mammalian culture is between 25 nM to 100 nM final concentration. This indicates that siRNAs are effective at concentrations that are several orders of magnitude below the concentrations applied in conventional antisense or ribozyme gene targeting experiments.

The above method provides a way both for the deduction of NOVX siRNA sequence and the use of such siRNA for in vitro suppression. In vivo suppression may be performed using the same siRNA using well known in vivo transfection or gene therapy transfection techniques.

**Antisense Nucleic Acids**

Another aspect of the invention pertains to isolated antisense nucleic acid molecules that are hybridizable to or complementary to the nucleic acid molecule comprising the nucleotide sequence of SEQ ID NO:2*n*-1, wherein *n* is an integer between 1 and 12, or fragments, analogs or derivatives thereof. An "antisense" nucleic acid comprises a nucleotide sequence that is complementary to a "sense" nucleic acid encoding a protein (e.g., complementary to the coding strand of a double-stranded cDNA molecule or complementary to an mRNA sequence). In specific aspects, antisense nucleic acid molecules are provided that comprise a sequence complementary to at least about 10, 25, 50, 100, 250 or 500 nucleotides or an entire NOVX coding strand, or to only a portion thereof. Nucleic acid molecules encoding fragments, homologs, derivatives and analogs of a NOVX protein of SEQ ID NO:$2n$, wherein $n$ is an integer between 1 and 12, or antisense nucleic acids complementary to a NOVX nucleic acid sequence of SEQ ID NO:$2n$-1, wherein $n$ is an integer between 1 and 12, are additionally provided.

In one embodiment, an antisense nucleic acid molecule is antisense to a "coding region" of the coding strand of a nucleotide sequence encoding a NOVX protein. The term "coding region" refers to the region of the nucleotide sequence comprising codons which are translated into amino acid residues. In another embodiment, the antisense nucleic acid molecule is antisense to a "noncoding region" of the coding strand of a nucleotide sequence encoding the NOVX protein. The term "noncoding region" refers to 5' and 3' sequences which flank the coding region that are not translated into amino acids (*i.e.,* also referred to as 5' and 3' untranslated regions).

Given the coding strand sequences encoding the NOVX protein disclosed herein, antisense nucleic acids of the invention can be designed according to the rules of Watson and Crick or Hoogsteen base pairing. The antisense nucleic acid molecule can be complementary to the entire coding region of NOVX mRNA, but more preferably is an oligonucleotide that is antisense to only a portion of the coding or noncoding region of NOVX mRNA. For example, the antisense oligonucleotide can be complementary to the region surrounding the translation start site of NOVX mRNA. An antisense oligonucleotide can be, for example, about 5, 10, 15, 20, 25, 30, 35, 40, 45 or 50 nucleotides in length. An antisense nucleic acid of the invention can be constructed using chemical synthesis or enzymatic ligation reactions using procedures known in the art. For example, an antisense nucleic acid (*e.g.,* an antisense oligonucleotide) can be chemically synthesized using naturally-occurring nucleotides or variously modified nucleotides designed to increase the biological stability of the molecules or to increase the physical stability of the duplex formed between the antisense and sense nucleic acids (*e.g.,* phosphorothioate derivatives and acridine substituted nucleotides can be used).

Examples of modified nucleotides that can be used to generate the antisense nucleic acid include: 5-fluorouracil, 5-bromouracil, 5-chlorouracil, 5-iodouracil, hypoxanthine, xanthine, 4-acetylcytosine, 5-carboxymethylaminomethyl-2-thiouridine, 5-(carboxyhydroxylmethyl) uracil, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 5-methoxyuracil, 3-methylcytosine, 5-methylcytosine, N6-adenine, 7-methylguanine, 5-methylaminomethyluracil, 5-methoxyaminomethyl-2-thiouracil, 2-thiouracil, 4-thiouracil, beta-D-mannosylqueosine, 5'-methoxycarboxymethyluracil, 2-methylthio-N6-isopentenyladenine, uracil-5-oxyacetic acid (v), wybutoxosine, pseudouracil, queosine, 2-thiocytosine, 5-methyl-2-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine. Alternatively, the antisense nucleic acid can be produced biologically using an expression vector into which a nucleic acid has been subcloned in an antisense orientation (*i.e.,* RNA transcribed from the inserted nucleic acid will be of an antisense orientation to a target nucleic acid of interest, described further in the following subsection).

The antisense nucleic acid molecules of the invention are typically administered to a subject or generated *in situ* such that they hybridize with or bind to cellular mRNA and/or genomic DNA encoding a NOVX protein to thereby inhibit expression of the protein (*e.g.*, by inhibiting transcription and/or translation). The hybridization can be by conventional nucleotide complementarity to form a stable duplex, or, for example, in the case of an antisense nucleic acid molecule that binds to DNA duplexes, through specific interactions in the major groove of the double helix. An example of a route of administration of antisense nucleic acid molecules of the invention includes direct injection at a tissue site. Alternatively, antisense nucleic acid molecules can be modified to target selected cells and then administered systemically. For example, for systemic administration, antisense molecules can be modified such that they specifically bind to receptors or antigens expressed on a selected cell surface (*e.g.,* by linking the antisense nucleic acid molecules to peptides or antibodies that bind to cell surface receptors or antigens). The antisense nucleic acid molecules can also be delivered to cells using the vectors described herein. To achieve sufficient nucleic acid molecules, vector constructs in which the antisense nucleic acid molecule is placed under the control of a strong pol II or pol III promoter are preferred.

In yet another embodiment, the antisense nucleic acid molecule of the invention is an $\alpha$-anomeric nucleic acid molecule. An $\alpha$-anomeric nucleic acid molecule forms specific double-stranded hybrids with complementary RNA in which, contrary to the usual $\beta$-units, the strands run parallel to each other. *See, e.g.,* Gaultier, et al., 1987. Nucl. Acids Res. 15: 6625-6641. The antisense nucleic acid molecule can also comprise a 2'-o-methylribonucleotide (*See, e.g.,* Inoue, et al. 1987. Nucl. Acids Res. 15: 6131-6148) or a chimeric RNA-DNA analogue (*See, e.g.,* Inoue, et al., 1987. FEBS Lett. 215: 327-330.

**Ribozymes and PNA Moieties**

Nucleic acid modifications include, by way of non-limiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject.

In one embodiment, an antisense nucleic acid of the invention is a ribozyme. Ribozymes are catalytic RNA molecules with ribonuclease activity that are capable of cleaving a single-stranded nucleic acid, such as an mRNA, to which they have a complementary region. Thus, ribozymes (*e.g.,* hammerhead ribozymes as described in Haseloff and Gerlach 1988. Nature 334: 585-591) can be used to catalytically cleave NOVX mRNA transcripts to thereby inhibit translation of NOVX mRNA. A ribozyme having specificity for a NOVX-encoding nucleic acid can be designed based upon the nucleotide sequence of a NOVX cDNA disclosed herein (*i.e.,* SEQ ID NO:2$n$-1, wherein $n$ is an integer between 1 and 12). For example, a derivative of a *Tetrahymena* L-19 IVS RNA can be constructed in which the nucleotide sequence of the active site is complementary to the nucleotide sequence to be cleaved in a NOVX-encoding mRNA. *See, e.g.,* U.S. Patent 4,987,071 to Cech, et al. and U.S. Patent 5,116,742 to Cech, et al. NOVX mRNA can also be used to select a catalytic RNA having a specific ribonuclease activity from a pool of RNA molecules. *See, e.g.,* Bartel et al., (1993) Science 261:1411-1418.

Alternatively, NOVX gene expression can be inhibited by targeting nucleotide sequences complementary to the regulatory region of the NOVX nucleic acid (e.g., the NOVX promoter and/or enhancers) to form triple helical structures that prevent transcription of the NOVX gene in target cells. *See, e.g.,* Helene, 1991. Anticancer Drug Des. 6: 569-84; Helene, et al. 1992. Ann. N.Y. Acad. Sci. 660: 27-36; Maher, 1992. Bioassays 14: 807-15.

In various embodiments, the NOVX nucleic acids can be modified at the base moiety, sugar moiety or phosphate backbone to improve, *e.g.*, the stability, hybridization, or solubility of the molecule. For example, the deoxyribose phosphate backbone of the nucleic acids can be modified to generate peptide nucleic acids. *See, e.g.,* Hyrup, et al., 1996. Bioorg Med Chem 4: 5-23. As used herein, the terms "peptide nucleic acids" or "PNAs" refer to nucleic acid mimics (*e.g.,* DNA mimics) in which the deoxyribose phosphate backbone is replaced by a pseudopeptide backbone and only the four natural nucleotide bases are retained. The neutral backbone of PNAs has been shown to allow for specific hybridization to DNA and RNA under conditions of low ionic strength. The synthesis of PNA oligomer can be performed using standard solid phase peptide synthesis protocols as described in Hyrup, et al., 1996. *supra;* Perry-O'Keefe, et al., 1996. Proc. Natl. Acad. Sci. USA 93: 14670-14675.

PNAs of NOVX can be used in therapeutic and diagnostic applications. For example, PNAs can be used as antisense or antigene agents for sequence-specific modulation of gene expression by, *e.g.,* inducing transcription or translation arrest or inhibiting replication. PNAs of NOVX can also be used, for example, in the analysis of single base pair mutations in a gene (*e.g.*, PNA directed PCR clamping; as artificial restriction enzymes when used in combination with other enzymes, *e.g.,* S$_1$ nucleases (*See,* Hyrup, *et al., 1996.supra*); or as probes or primers for DNA sequence and hybridization (*See,* Hyrup, *et al.,* 1996, *supra;* Perry-O'Keefe, *et al.,* 1996. *supra*).

In another embodiment, PNAs of NOVX can be modified, *e.g.,* to enhance their stability or cellular uptake, by attaching lipophilic or other helper groups to PNA, by the formation of PNA-DNA chimeras, or by the use of liposomes or other techniques of drug delivery known in the art. For example, PNA-DNA chimeras of NOVX can be generated that may combine the advantageous properties of PNA and DNA. Such chimeras allow DNA recognition enzymes (*e.g.,* RNase H and DNA polymerases) to interact with the DNA portion while the PNA portion would provide high binding affinity and specificity. PNA-DNA chimeras can be linked using linkers of appropriate lengths selected in terms of base stacking, number of bonds between the nucleotide bases, and orientation (*see,* Hyrup, et al., 1996. *supra*). The synthesis of PNA-DNA chimeras can be performed as described in Hyrup, *et al.,* 1996. *supra* and Finn, et al., 1996. Nucl Acids Res 24: 3357-3363. For example, a DNA chain can be synthesized on a solid support using standard phosphoramidite coupling chemistry, and modified nucleoside analogs, *e.g.,* 5'-(4-methoxytrityl)amino-5'-deoxy-thymidine phosphoramidite, can be used between the PNA and the 5' end of DNA. *See, e.g.,* Mag, et al., 1989. Nucl Acid Res 17: 5973-5988. PNA monomers are then coupled in a stepwise manner to produce a chimeric molecule with a 5' PNA segment and a 3' DNA segment. *See, e.g.,* Finn, *et al.,* 1996. *supra.* Alternatively, chimeric molecules can be synthesized with a 5' DNA segment and a 3' PNA segment. *See, e.g.,* Petersen, et al., 1975. Bioorg. Med. Chem. Lett. 5: 1119-11124.

In other embodiments, the oligonucleotide may include other appended groups such as peptides (*e.g.,* for targeting host cell receptors *in vivo*), or agents facilitating transport across the cell membrane (*see, e.g.,* Letsinger, et al., 1989. Proc. Natl. Acad. Sci. U.S.A. 86: 6553-6556; Lemaitre, et al., 1987. Proc. Natl. Acad. Sci. 84: 648-652; PCT Publication No. WO88/09810) or the blood-brain barrier (*see, e.g.,* PCT Publication No. WO 89/10134). In addition, oligonucleotides can be modified with hybridization triggered cleavage agents (*see, e.g.,* Krol, et al., 1988. BioTechniques 6:958-976) or intercalating agents (*see, e.g.,* Zon, 1988. Pharm. Res. 5: 539-549). To this end, the oligonucleotide may be conjugated to another molecule, *e.g.*, a peptide, a hybridization triggered cross-linking agent, a transport agent, a hybridization-triggered cleavage agent, and the like.

**NOVX Polypeptides**

A polypeptide according to the invention includes a polypeptide including the amino acid sequence of NOVX polypeptides whose sequences are provided in any one of SEQ ID NO:2$n$, wherein $n$ is an integer between 1 and 12.

The invention also includes a mutant or variant protein any of whose residues may be changed from the corresponding residues shown in any one of SEQ ID NO:2*n*, wherein *n* is an integer between 1 and 12, while still encoding a protein that maintains its NOVX activities and physiological functions, or a functional fragment thereof.

In general, a NOVX variant that preserves NOVX-like function includes any variant in which residues at a particular position in the sequence have been substituted by other amino acids, and further include the possibility of inserting an additional residue or residues between two residues of the parent protein as well as the possibility of deleting one or more residues from the parent sequence. Any amino acid substitution, insertion, or deletion is encompassed by the invention. In favorable circumstances, the substitution is a conservative substitution as defined above.

One aspect of the invention pertains to isolated NOVX proteins, and biologically-active portions thereof, or derivatives, fragments, analogs or homologs thereof. Also provided are polypeptide fragments suitable for use as immunogens to raise anti-NOVX antibodies. In one embodiment, native NOVX proteins can be isolated from cells or tissue sources by an appropriate purification scheme using standard protein purification techniques. In another embodiment, NOVX proteins are produced by recombinant DNA techniques. Alternative to recombinant expression, a NOVX protein or polypeptide can be synthesized chemically using standard peptide synthesis techniques.

An "isolated" or "purified" polypeptide or protein or biologically-active portion thereof is substantially free of cellular material or other contaminating proteins from the cell or tissue source from which the NOVX protein is derived, or substantially free from chemical precursors or other chemicals when chemically synthesized. The language "substantially free of cellular material" includes preparations of NOVX proteins in which the protein is separated from cellular components of the cells from which it is isolated or recombinantly-produced. In one embodiment, the language "substantially free of cellular material" includes preparations of NOVX proteins having less than about 30% (by dry weight) of non-NOVX proteins (also referred to herein as a "contaminating protein"), more preferably less than about 20% of non-NOVX proteins, still more preferably less than about 10% of non-NOVX proteins, and most preferably less than about 5% of non-NOVX proteins. When the NOVX protein or biologically-active portion thereof is recombinantly-produced, it is also preferably substantially free of culture medium, *i.e.,* culture medium represents less than about 20%, more preferably less than about 10%, and most preferably less than about 5% of the volume of the NOVX protein preparation.

The language "substantially free of chemical precursors or other chemicals" includes preparations of NOVX proteins in which the protein is separated from chemical precursors or other chemicals that are involved in the synthesis of the protein. In one embodiment, the language "substantially free of chemical precursors or other chemicals" includes preparations of NOVX proteins having less than about 30% (by dry weight) of chemical precursors or non-NOVX chemicals, more preferably less than about 20% chemical precursors or non-NOVX chemicals, still more preferably less than about 10% chemical precursors or non-NOVX chemicals, and most preferably less than about 5% chemical precursors or non-NOVX chemicals.

Biologically-active portions of NOVX proteins include peptides comprising amino acid sequences sufficiently homologous to or derived from the amino acid sequences of the NOVX proteins (*e.g.*, the amino acid sequence of SEQ ID NO:2*n*, wherein *n* is an integer between 1 and 12) that include fewer amino acids than the full-length NOVX proteins, and exhibit at least one activity of a NOVX protein. Typically, biologically-active portions comprise a domain or motif with at least one activity of the NOVX protein. A biologically-active portion of a NOVX protein can be a polypeptide which is, for example, 10, 25, 50, 100 or more amino acid residues in length.

Moreover, other biologically-active portions, in which other regions of the protein are deleted, can be prepared by recombinant techniques and evaluated for one or more of the functional activities of a native NOVX protein.

In an embodiment, the NOVX protein has an amino acid sequence of SEQ ID NO:2*n*, wherein *n* is an integer between 1 and 12. In other embodiments, the NOVX protein is substantially homologous to SEQ ID NO:2*n*, wherein *n* is an integer between 1 and 12, and retains the functional activity of the protein of SEQ ID NO:2*n*, wherein *n* is an integer between 1 and 12, yet differs in amino acid sequence due to natural allelic variation or mutagenesis, as described in detail, below. Accordingly, in another embodiment, the NOVX protein is a protein that comprises an amino acid sequence at least about 45% homologous to the amino acid sequence of SEQ ID NO:2*n*, wherein *n* is an integer between 1 and 12, and retains the functional activity of the NOVX proteins of SEQ ID NO:2*n*, wherein *n* is an integer between 1 and 12.

**Determining Homology Between Two or More Sequences**

To determine the percent homology of two amino acid sequences or of two nucleic acids, the sequences are aligned for optimal comparison purposes (e.g., gaps can be introduced in the sequence of a first amino acid or nucleic acid sequence for optimal alignment with a second amino or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, then the molecules are homologous at that position (*i.e.,* as used herein amino acid or nucleic acid "homology" is equivalent to amino acid or nucleic acid "identity").

The nucleic acid sequence homology may be determined as the degree of identity between two sequences.

The homology may be determined using computer programs known in the art, such as GAP software provided in the GCG program package. *See,* Needleman and Wunsch, 1970. J Mol Biol 48: 443-453. Using GCG GAP software with the following settings for nucleic acid sequence comparison: GAP creation penalty of 5.0 and GAP extension penalty of 0.3, the coding region of the analogous nucleic acid sequences referred to above exhibits a degree of identity preferably of at least 70%, 75%, 80%, 85%, 90%, 95%, 98%, or 99%, with the CDS (encoding) part of the DNA sequence of SEQ ID NO:2*n*-1, wherein *n* is an integer between 1 and 12.

The term "sequence identity" refers to the degree to which two polynucleotide or polypeptide sequences are identical on a residue-by-residue basis over a particular region of comparison. The term "percentage of sequence identity" is calculated by comparing two optimally aligned sequences over that region of comparison, determining the number of positions at which the identical nucleic acid base (*e.g.*, A, T, C, G, U, or I, in the case of nucleic acids) occurs in both sequences to yield the number of matched positions, dividing the number of matched positions by the total number of positions in the region of comparison (*i.e.,* the window size), and multiplying the result by 100 to yield the percentage of sequence identity. The term "substantial identity" as used herein denotes a characteristic of a polynucleotide sequence, wherein the polynucleotide comprises a sequence that has at least 80 percent sequence identity, preferably at least 85 percent identity and often 90 to 95 percent sequence identity, more usually at least 99 percent sequence identity as compared to a reference sequence over a comparison region.

## Chimeric and Fusion Proteins

The invention also provides NOVX chimeric or fusion proteins. As used herein, a NOVX "chimeric protein" or "fusion protein" comprises a NOVX polypeptide operatively-linked to a non-NOVX polypeptide. An "NOVX polypeptide" refers to a polypeptide having an amino acid sequence corresponding to a NOVX protein of SEQ ID NO:2*n*, wherein *n* is an integer between 1 and 12, whereas a "non-NOVX polypeptide" refers to a polypeptide having an amino acid sequence corresponding to a protein that is not substantially homologous to the NOVX protein, *e.g.*, a protein that is different from the NOVX protein and that is derived from the same or a different organism. Within a NOVX fusion protein the NOVX polypeptide can correspond to all or a portion of a NOVX protein. In one embodiment, a NOVX fusion protein comprises at least one biologically-active portion of a NOVX protein. In another embodiment, a NOVX fusion protein comprises at least two biologically-active portions of a NOVX protein. In yet another embodiment, a NOVX fusion protein comprises at least three biologically-active portions of a NOVX protein. Within the fusion protein, the term "operatively-linked" is intended to indicate that the NOVX polypeptide and the non-NOVX polypeptide are fused in-frame with one another. The non-NOVX polypeptide can be fused to the N-terminus or C-terminus of the NOVX polypeptide.

In one embodiment, the fusion protein is a GST-NOVX fusion protein in which the NOVX sequences are fused to the C-terminus of the GST (glutathione S-transferase) sequences. Such fusion proteins can facilitate the purification of recombinant NOVX polypeptides.

In another embodiment, the fusion protein is a NOVX protein containing a heterologous signal sequence at its N-terminus. In certain host cells (*e.g.,* mammalian host cells), expression and/or secretion of NOVX can be increased through use of a heterologous signal sequence.

In yet another embodiment, the fusion protein is a NOVX-immunoglobulin fusion protein in which the NOVX sequences are fused to sequences derived from a member of the immunoglobulin protein family. The NOVX-immunoglobulin fusion proteins of the invention can be incorporated into pharmaceutical compositions and administered to a subject to inhibit an interaction between a NOVX ligand and a NOVX protein on the surface of a cell, to thereby suppress NOVX-mediated signal transduction *in vivo.* The NOVX-immunoglobulin fusion proteins can be used to affect the bioavailability of a NOVX cognate ligand. Inhibition of the NOVX ligand/NOVX interaction may be useful therapeutically for both the treatment of proliferative and differentiative disorders, as well as modulating (*e.g.* promoting or inhibiting) cell survival. Moreover, the NOVX-immunoglobulin fusion proteins of the invention can be used as immunogens to produce anti-NOVX antibodies in a subject, to purify NOVX ligands, and in screening assays to identify molecules that inhibit the interaction of NOVX with a NOVX ligand.

A NOVX chimeric or fusion protein of the invention can be produced by standard recombinant DNA techniques. For example, DNA fragments coding for the different polypeptide sequences are ligated together in-frame in accordance with conventional techniques, *e.g.*, by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of gene fragments can be carried out using anchor primers that give rise to complementary overhangs between two consecutive gene fragments that can subsequently be annealed and reamplified to generate a chimeric gene sequence (*see, e.g.,* Ausubel, et al. (eds.) CURRENT PROTOCOLS IN MOLECULAR BIOLOGY, John Wiley & Sons, 1992). Moreover, many expression vectors are commercially available that already encode a fusion moiety (*e.g.,* a GST polypeptide). A NOVX-encoding nucleic acid can be cloned into such an expression vector such that the fusion moiety is linked in-frame to the NOVX protein.

## NOVX Agonists and Antagonists

The invention also pertains to variants of the NOVX proteins that function as either NOVX agonists (*i.e.,* mimetics) or as NOVX antagonists. Variants of the NOVX protein can be generated by mutagenesis (*e.g.,* discrete point mutation or truncation of the NOVX protein). An agonist of the NOVX protein can retain substantially the same, or a subset of, the biological activities of the naturally occurring form of the NOVX protein. An antagonist of the NOVX protein can inhibit one or more of the activities of the naturally occurring form of the NOVX protein by, for example, competitively binding to a downstream or upstream member of a cellular signaling cascade which includes the NOVX protein. Thus, specific biological effects can be elicited by treatment with a variant of limited function. In one embodiment, treatment of a subject with a variant having a subset of the biological activities of the naturally occurring form of the protein has fewer side effects in a subject relative to treatment with the naturally occurring form of the NOVX proteins.

Variants of the NOVX proteins that function as either NOVX agonists (*i.e.,* mimetics) or as NOVX antagonists can be identified by screening combinatorial libraries of mutants (*e.g.,* truncation mutants) of the NOVX proteins for NOVX protein agonist or antagonist activity. In one embodiment, a variegated library of NOVX variants is generated by combinatorial mutagenesis at the nucleic acid level and is encoded by a variegated gene library. A variegated library of NOVX variants can be produced by, for example, enzymatically ligating a mixture of synthetic oligonucleotides into gene sequences such that a degenerate set of potential NOVX sequences is expressible as individual polypeptides, or alternatively, as a set of larger fusion proteins (*e.g.,* for phage display) containing the set of NOVX sequences therein. There are a variety of methods which can be used to produce libraries of potential NOVX variants from a degenerate oligonucleotide sequence. Chemical synthesis of a degenerate gene sequence can be performed in an automatic DNA synthesizer, and the synthetic gene then ligated into an appropriate expression vector. Use of a degenerate set of genes allows for the provision, in one mixture, of all of the sequences encoding the desired set of potential NOVX sequences. Methods for synthesizing degenerate oligonucleotides are well-known within the art. *See, e.g.,* Narang, 1983. Tetrahedron 39: 3; Itakura, et al., 1984. Annu. Rev. Biochem. 53: 323; Itakura, et al., 1984. Science 198: 1056; Ike, et al., 1983. Nucl. Acids Res. 11: 477.

## Polypeptide Libraries

In addition, libraries of fragments of the NOVX protein coding sequences can be used to generate a variegated population of NOVX fragments for screening and subsequent selection of variants of a NOVX protein. In one embodiment, a library of coding sequence fragments can be generated by treating a double stranded PCR fragment of a NOVX coding sequence with a nuclease under conditions wherein nicking occurs only about once per molecule, denaturing the double stranded DNA, renaturing the DNA to form double-stranded DNA that can include sense/antisense pairs from different nicked products, removing single stranded portions from reformed duplexes by treatment with $S_1$ nuclease, and ligating the resulting fragment library into an expression vector. By this method, expression libraries can be derived which encodes N-terminal and internal fragments of various sizes of the NOVX proteins.

Various techniques are known in the art for screening gene products of combinatorial libraries made by point mutations or truncation, and for screening cDNA libraries for gene products having a selected property. Such techniques are adaptable for rapid screening of the gene libraries generated by the combinatorial mutagenesis of NOVX proteins. The most widely used techniques, which are amenable to high throuput analysis, for screening large gene libraries typically include cloning the gene library into replicable expression vectors, transforming appropriate cells with the resulting library of vectors, and expressing the combinatorial genes under conditions in which detection of a desired activity facilitates isolation of the vector encoding the gene whose product was detected. Recursive ensemble mutagenesis (REM), a new technique that enhances the frequency of functional mutants in the libraries, can be used in combination with the screening assays to identify NOVX variants. *See, e.g.,* Arkin and Yourvan, 1992. Proc. Natl. Acad. Sci. USA 89: 7811-7815; Delgrave, et al., 1993. Protein Engineering 6:327-331.

## Anti-NOVX Antibodies

Included in the invention are antibodies to NOVX proteins, or fragments of NOVX proteins. The term "antibody" as used herein refers to immunoglobulin molecules and immunologically active portions of immunoglobulin (Ig) molecules, *i.e.,* molecules that contain an antigen binding site that specifically binds (immunoreacts with) an antigen. Such antibodies include, but are not limited to, polyclonal, monoclonal, chimeric, single chain, $F_{ab}$, $F_{ab'}$ and $F_{(ab')2}$ fragments, and an $F_{ab}$ expression library. In general, antibody molecules obtained from humans relates to any of the classes IgG, IgM, IgA, IgE and IgD, which differ from one another by the nature of the heavy chain present in the molecule. Certain classes have subclasses as well, such as $IgG_1$, $IgG_2$, and others. Furthermore, in humans, the light chain may be a kappa chain or a lambda chain. Reference herein to antibodies includes a reference to all such classes, subclasses and types of human antibody species.

An isolated protein of the invention intended to serve as an antigen, or a portion or fragment thereof, can be used as an immunogen to generate antibodies that immunospecifically bind the antigen, using standard techniques for polyclonal and monoclonal antibody preparation. The full-length protein can be used or, alternatively, the invention provides antigenic peptide fragments of the antigen for use as immunogens. An antigenic peptide fragment comprises at least 6 amino acid residues of the amino acid sequence of the full length protein, such as an amino acid sequence of SEQ ID NO:2$n$, wherein $n$ is an integer between 1 and 12, and encompasses an epitope thereof such that an antibody raised against the peptide forms a specific immune complex with the full length protein or with any fragment that contains the epitope. Preferably, the antigenic peptide comprises at least 10 amino acid residues, or at least 15 amino acid residues, or at least 20 amino acid residues, or at least 30 amino acid residues. Preferred epitopes encompassed by the antigenic peptide are regions of the protein that are located on its surface; commonly these are hydrophilic regions.

In certain embodiments of the invention, at least one epitope encompassed by the antigenic peptide is a region of NOVX that is located on the surface of the protein, *e.g.*, a hydrophilic region. A hydrophobicity analysis of the human NOVX protein sequence indicates which regions of a NOVX polypeptide are particularly hydrophilic and, therefore, are likely to encode surface residues useful for targeting antibody production. As a means for targeting antibody production, hydropathy plots showing regions of hydrophilicity and hydrophobicity may be generated by any method well known in the art, including, for example, the Kyte Doolittle or the Hopp Woods methods, either with or without Fourier transformation. See, *e.g.*, Hopp and Woods, 1981, Proc. Nat. Acad. Sci. USA 78: 3824-3828; Kyte and Doolittle 1982, J. Mol. Biol. 157: 105-142, each incorporated herein by reference in their entirety. Antibodies that are specific for one or more domains within an antigenic protein, or derivatives, fragments, analogs or homologs thereof, are also provided herein.

The term "epitope" includes any protein determinant capable of specific binding to an immunoglobulin or T-cell receptor. Epitopic determinants usually consist of chemically active surface groupings of molecules such as amino acids or sugar side chains and usually have specific three dimensional structural characteristics, as well as specific charge characteristics. A NOVX polypeptide or a fragment thereof comprises at least one antigenic epitope. An anti-NOVX antibody of the present invention is said to specifically bind to antigen NOVX when the equilibrium binding constant ($K_D$) is $\leq 1$ $\mu$M, preferably $\leq$ 100 nM, more preferably $\leq$ 10 nM, and most preferably $\leq$ 100 pM to about 1 pM, as measured by assays such as radioligand binding assays or similar assays known to those skilled in the art.

A protein of the invention, or a derivative, fragment, analog, homolog or ortholog thereof, may be utilized as an immunogen in the generation of antibodies that immunospecifically bind these protein components.

Various procedures known within the art may be used for the production of polyclonal or monoclonal antibodies directed against a protein of the invention, or against derivatives, fragments, analogs homologs or orthologs thereof (see, for example, Antibodies: A Laboratory Manual, Harlow E, and Lane D, 1988, Cold Spring Harbor Laboratory Press, Cold Spring Harbor, NY, incorporated herein by reference). Some of these antibodies are discussed below.

**Polyclonal Antibodies**

For the production of polyclonal antibodies, various suitable host animals (*e.g.,* rabbit, goat, mouse or other mammal) may be immunized by one or more injections with the native protein, a synthetic variant thereof, or a derivative of the foregoing. An appropriate immunogenic preparation can contain, for example, the naturally occurring immunogenic protein, a chemically synthesized polypeptide representing the immunogenic protein, or a recombinantly expressed immunogenic protein. Furthermore, the protein may be conjugated to a second protein known to be immunogenic in the mammal being immunized. Examples of such immunogenic proteins include but are not limited to keyhole limpet hemocyanin, serum albumin, bovine thyroglobulin, and soybean trypsin inhibitor. The preparation can further include an adjuvant. Various adjuvants used to increase the immunological response include, but are not limited to, Freund's (complete and incomplete), mineral gels (*e.g.,* aluminum hydroxide), surface active substances (*e.g.,* lysolecithin, pluronic polyols, polyanions, peptides, oil emulsions, dinitrophenol, *etc.*), adjuvants usable in humans such as Bacille Calmette-Guerin and Corynebacterium parvum, or similar immunostimulatory agents. Additional examples of adjuvants which can be employed include MPL-TDM adjuvant (monophosphoryl Lipid A, synthetic trehalose dicorynomycolate).

The polyclonal antibody molecules directed against the immunogenic protein can be isolated from the mammal (*e.g.,* from the blood) and further purified by well known techniques, such as affinity chromatography using protein A or protein G, which provide primarily the IgG fraction of immune serum. Subsequently, or alternatively, the specific antigen which is the target of the immunoglobulin sought, or an epitope thereof, may be immobilized on a column to purify the immune specific antibody by immunoaffinity chromatography. Purification of immunoglobulins is discussed, for example, by D. Wilkinson (The Scientist, published by The Scientist, Inc., Philadelphia PA, Vol. 14, No. 8 (April 17, 2000), pp. 25-28).

**Monoclonal Antibodies**

The term "monoclonal antibody" (MAb) or "monoclonal antibody composition", as used herein, refers to a population of antibody molecules that contain only one molecular species of antibody molecule consisting of a unique light chain gene product and a unique heavy chain gene product. In particular, the complementarity determining regions (CDRs) of the monoclonal antibody are identical in all the molecules of the population. MAbs thus contain an antigen binding site capable of immunoreacting with a particular epitope of the antigen characterized by a unique binding affinity for it.

Monoclonal antibodies can be prepared using hybridoma methods, such as those described by Kohler and Milstein, Nature, 256:495 (1975). In a hybridoma method, a mouse, hamster, or other appropriate host animal, is typically immunized with an immunizing agent to elicit lymphocytes that produce or are capable of producing antibodies that specifically bind to the immunizing agent. Alternatively, the lymphocytes can be immunized in vitro.

The immunizing agent typically includes the protein antigen, a fragment thereof or a fusion protein thereof. Generally, either peripheral blood lymphocytes are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell (Goding, Monoclonal Antibodies: Principles and Practice, Academic Press, (1986) pp. 59-103). Immortalized cell lines are usually transformed mammalian cells, particularly myeloma cells of rodent, bovine and human origin. Usually, rat or mouse myeloma cell lines are employed. The hybridoma cells can be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically includes hypoxanthine, aminopterin, and thymidine ("HAT medium"), which substances prevent the growth of HGPRT-deficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, California and the American Type Culture Collection, Manassas, Virginia. Human myeloma and mouse-human heteromyeloma cell lines also have been described for the production of human monoclonal antibodies (Kozbor, J. Immunol., 133:3001 (1984); Brodeur et al., Monoclonal Antibody Production Techniques and Applications, Marcel Dekker, Inc., New York, (1987) pp. 51-63).

The culture medium in which the hybridoma cells are cultured can then be assayed for the presence of monoclonal antibodies directed against the antigen. Preferably, the binding specificity of monoclonal antibodies produced by the hybridoma cells is determined by immunoprecipitation or by an in vitro binding assay, such as radioimmunoassay (RIA) or enzyme-linked immunoabsorbent assay (ELISA). Such techniques and assays are known in the art. The binding affinity of the monoclonal antibody can, for example, be determined by the Scatchard analysis of Munson and Pollard, Anal. Biochem., 107:220 (1980). It is an objective, especially important in therapeutic applications of monoclonal antibodies, to identify antibodies having a high degree of specificity and a high binding affinity for the target antigen.

After the desired hybridoma cells are identified, the clones can be subcloned by limiting dilution procedures and grown by standard methods (Goding, 1986). Suitable culture media for this purpose include, for example, Dulbecco's Modified Eagle's Medium and RPMI-1640 medium. Alternatively, the hybridoma cells can be grown in vivo as ascites in a mammal.

The monoclonal antibodies secreted by the subclones can be isolated or purified from the culture medium or ascites fluid by conventional immunoglobulin purification procedures such as, for example, protein A-Sepharose, hydroxylapatite chromatography, gel electrophoresis, dialysis, or affinity chromatography.

The monoclonal antibodies can also be made by recombinant DNA methods, such as those described in U.S. Patent No. 4,816,567. DNA encoding the monoclonal antibodies of the invention can be readily isolated and sequenced using conventional procedures (*e.g.,* by using oligonucleotide probes that are capable of binding specifically to genes encoding the heavy and light chains of murine antibodies). The hybridoma cells of the invention serve as a preferred source of such DNA. Once isolated, the DNA can be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also can be modified, for example, by substituting the coding sequence for human heavy and light chain constant domains in place of the homologous murine sequences (U.S. Patent No. 4,816,567; Morrison, Nature 368, 812-13 (1994)) or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to create a chimeric bivalent antibody.

**Humanized Antibodies**

The antibodies directed against the protein antigens of the invention can further comprise humanized antibodies or human antibodies. These antibodies are suitable for administration to humans without engendering an immune response by the human against the administered immunoglobulin. Humanized forms of antibodies are chimeric immunoglobulins, immunoglobulin chains or fragments thereof (such as Fv, Fab, Fab', F(ab')$_2$ or other antigen-binding subsequences of antibodies) that are principally comprised of the sequence of a human immunoglobulin, and contain minimal sequence derived from a non-human immunoglobulin. Humanization can be performed following the method of Winter and co-workers (Jones et al., Nature, 321:522-525 (1986); Riechmann et al., Nature, 332:323-327 (1988); Verhoeyen et al., Science, 239:1534-1536 (1988)), by substituting rodent CDRs or CDR sequences for the corresponding sequences of a human antibody. (See also U.S. Patent No. 5,225,539.) In some instances, Fv framework residues of the human immunoglobulin are replaced by corresponding non-human residues. Humanized antibodies can also comprise residues which are found neither in the recipient antibody nor in the imported CDR or framework sequences. In general, the humanized antibody comprises substantially all of at least one, and typically two, variable domains, in which all or substantially all of the CDR regions correspond to those of a non-human immunoglobulin and all or substantially all of the framework regions are those of a human immunoglobulin consensus sequence. The humanized antibody optimally also comprises at least a portion of an immunoglobulin constant region (Fc), typically that of a human immunoglobulin (Jones et al., 1986; Riechmann et al., 1988; and Presta, Curr. Op. Struct. Biol., 2:593-596 (1992)).

**Human Antibodies**

Fully human antibodies essentially relate to antibody molecules in which the entire sequence of both the light chain and the heavy chain, including the CDRs, arise from human genes. Such antibodies are termed "human antibodies", or "fully human antibodies" herein. Human monoclonal antibodies can be prepared by the trioma technique; the human B-cell hybridoma technique (see Kozbor, et al., 1983 Immunol Today 4: 72) and the EBV hybridoma technique to produce human monoclonal antibodies (see Cole, et al., 1985 In: MONOCLONAL ANTIBODIES AND CANCER THERAPY, Alan R. Liss, Inc., pp. 77-96). Human monoclonal antibodies may be utilized in the practice of the present invention and may be produced by using human hybridomas (see Cote, et al., 1983. Proc Natl Acad Sci USA 80: 2026-2030) or by transforming human B-cells with Epstein Barr Virus in vitro (see Cole, et al., 1985 In: MONOCLONAL ANTIBODIES AND CANCER THERAPY, Alan R. Liss, Inc., pp. 77-96).
In addition, human antibodies can also be produced using additional techniques, including phage display libraries (Hoogenboom and Winter, J. Mol. Biol., 227:381 (1991); Marks et al., J. Mol. Biol., 222:581 (1991)). Similarly, human antibodies can be made by introducing human immunoglobulin loci into transgenic animals, *e.g.*, mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; 5,661,016, and in Marks et al. (Bio/Technology 10, 779-783 (1992)); Lonberg et al. (Nature 368 856-859 (1994)); Morrison (Nature 368, 812-13 (1994)); Fishwild et al, (Nature Biotechnology 14, 845-51 (1996)); Neuberger (Nature Biotechnology 14, 826 (1996)); and Lonberg and Huszar (Intern. Rev. Immunol. 13 65-93 (1995)).
Human antibodies may additionally be produced using transgenic nonhuman animals which are modified so as to produce fully human antibodies rather than the animal's endogenous antibodies in response to challenge by an antigen. (See PCT publication WO94/02602). The endogenous genes encoding the heavy and light immunoglobulin chains in the nonhuman host have been incapacitated, and active loci encoding human heavy and light chain immunoglobulins are inserted into the host's genome. The human genes are incorporated, for example, using yeast artificial chromosomes containing the requisite human DNA segments. An animal which provides all the desired modifications is then obtained as progeny by crossbreeding intermediate transgenic animals containing fewer than the full complement of the modifications. The preferred embodiment of such a nonhuman animal is a mouse, and is termed the Xenomouse™ as disclosed in PCT publications WO 96/33735 and WO 96/34096. This animal produces B cells which secrete fully human immunoglobulins. The antibodies can be obtained directly from the animal after immunization with an immunogen of interest, as, for example, a preparation of a polyclonal antibody, or alternatively from immortalized B cells derived from the animal, such as hybridomas producing monoclonal antibodies. Additionally, the genes encoding the immunoglobulins with human variable regions can be recovered and expressed to obtain the antibodies directly, or can be further modified to obtain analogs of antibodies such as, for example, single chain Fv molecules.
An example of a method of producing a nonhuman host, exemplified as a mouse, lacking expression of an endogenous immunoglobulin heavy chain is disclosed in U.S. Patent No. 5,939,598. It can be obtained by a method including deleting the J segment genes from at least one endogenous heavy chain locus in an embryonic stem cell to prevent rearrangement of the locus and to prevent formation of a transcript of a rearranged immunoglobulin heavy chain locus, the deletion being effected by a targeting vector containing a gene encoding a selectable marker; and producing from the embryonic stem cell a transgenic mouse whose somatic and germ cells contain the gene encoding the selectable marker.

A method for producing an antibody of interest, such as a human antibody, is disclosed in U.S. Patent No. 5,916,771. It includes introducing an expression vector that contains a nucleotide sequence encoding a heavy chain into one mammalian host cell in culture, introducing an expression vector containing a nucleotide sequence encoding a light chain into another mammalian host cell, and fusing the two cells to form a hybrid cell. The hybrid cell expresses an antibody containing the heavy chain and the light chain.

In a further improvement on this procedure, a method for identifying a clinically relevant epitope on an immunogen, and a correlative method for selecting an antibody that binds immunospecifically to the relevant epitope with high affinity, are disclosed in PCT publication WO 99/53049.

### $F_{ab}$ Fragments and Single Chain Antibodies

According to the invention, techniques can be adapted for the production of single-chain antibodies specific to an antigenic protein of the invention (see *e.g.,* U.S. Patent No. 4,946,778). In addition, methods can be adapted for the construction of $F_{ab}$ expression libraries (see *e.g.,* Huse, et al., 1989 Science 246: 1275-1281) to allow rapid and effective identification of monoclonal $F_{ab}$ fragments with the desired specificity for a protein or derivatives, fragments, analogs or homologs thereof. Antibody fragments that contain the idiotypes to a protein antigen may be produced by techniques known in the art including, but not limited to: (i) an $F_{(ab')2}$ fragment produced by pepsin digestion of an antibody molecule; (ii) an $F_{ab}$ fragment generated by reducing the disulfide bridges of an $F_{(ab')2}$ fragment; (iii) an $F_{ab}$ fragment generated by the treatment of the antibody molecule with papain and a reducing agent and (iv) $F_v$ fragments.

### Bispecific Antibodies

Bispecific antibodies are monoclonal, preferably human or humanized, antibodies that have binding specificities for at least two different antigens. In the present case, one of the binding specificities is for an antigenic protein of the invention. The second binding target is any other antigen, and advantageously is a cell-surface protein or receptor or receptor subunit.

Methods for making bispecific antibodies are known in the art. Traditionally, the recombinant production of bispecific antibodies is based on the co-expression of two immunoglobulin heavy-chain/light-chain pairs, where the two heavy chains have different specificities (Milstein and Cuello, Nature, 305:537-539 (1983)). Because of the random assortment of immunoglobulin heavy and light chains, these hybridomas (quadromas) produce a potential mixture of ten different antibody molecules, of which only one has the correct bispecific structure. The purification of the correct molecule is usually accomplished by affinity chromatography steps. Similar procedures are disclosed in WO 93/08829, published 13 May 1993, and in Traunecker et al., EMBO J., 10:3655-3659 (1991).

Antibody variable domains with the desired binding specificities (antibody-antigen combining sites) can be fused to immunoglobulin constant domain sequences. The fusion preferably is with an immunoglobulin heavy-chain constant domain, comprising at least part of the hinge, CH2, and CH3 regions. It is preferred to have the first heavy-chain constant region (CH1) containing the site necessary for light-chain binding present in at least one of the fusions. DNAs encoding the immunoglobulin heavy-chain fusions and, if desired, the immunoglobulin light chain, are inserted into separate expression vectors, and are co-transfected into a suitable host organism. For further details of generating bispecific antibodies see, for example, Suresh et al., Methods in Enzymology, 121:210 (1986).

According to another approach described in WO 96/27011, the interface between a pair of antibody molecules can be engineered to maximize the percentage of heterodimers which are recovered from recombinant cell culture. The preferred interface comprises at least a part of the CH3 region of an antibody constant domain. In this method, one or more small amino acid side chains from the interface of the first antibody molecule are replaced with larger side chains (*e.g.* tyrosine or tryptophan). Compensatory "cavities" of identical or similar size to the large side chain(s) are created on the interface of the second antibody molecule by replacing large amino acid side chains with smaller ones (*e.g.* alanine or threonine). This provides a mechanism for increasing the yield of the heterodimer over other unwanted end-products such as homodimers.

Bispecific antibodies can be prepared as full length antibodies or antibody fragments (*e.g.* $F_{(ab')2}$ bispecific antibodies). Techniques for generating bispecific antibodies from antibody fragments have been described in the literature. For example, bispecific antibodies can be prepared using chemical linkage. Brennan et al., Science 229:81 (1985) describe a procedure wherein intact antibodies are proteolytically cleaved to generate $F_{(ab')2}$ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Additionally, Fab' fragments can be directly recovered from E. coli and chemically coupled to form bispecific antibodies. Shalaby et al., J. Exp. Med. 175:217-225 (1992) describe the production of a fully humanized bispecific antibody F(ab')$_2$ molecule. Each Fab' fragment was separately secreted from E. coli and subjected to directed chemical coupling in vitro to form the bispecific antibody. The bispecific antibody thus formed was able to bind to cells overexpressing the ErbB2 receptor and normal human T cells, as well as trigger the lytic activity of human cytotoxic lymphocytes against human breast tumor targets.

Various techniques for making and isolating bispecific antibody fragments directly from recombinant cell culture have also been described. For example, bispecific antibodies have been produced using leucine zippers. Kostelny et al., J. Immunol. 148(5): 1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA 90:6444-6448 (1993) has provided an alternative mechanism for making bispecific antibody fragments. The fragments comprise a heavy-chain variable domain ($V_H$) connected to a light-chain variable domain ($V_L$) by a linker which is too short to allow pairing between the two domains on the same chain. Accordingly, the $V_H$ and $V_L$ domains of one fragment are forced to pair with the complementary $V_L$ and $V_H$ domains of another fragment, thereby forming two antigen-binding sites. Another strategy for making bispecific antibody fragments by the use of single-chain Fv (sFv) dimers has also been reported. See, Gruber et al., J. Immunol. 152:5368 (1994).

Antibodies with more than two valencies are contemplated. For example, trispecific antibodies can be prepared. Tutt et al., J. Immunol. 147:60 (1991).

Exemplary bispecific antibodies can bind to two different epitopes, at least one of which originates in the protein antigen of the invention. Alternatively, an anti-antigenic arm of an immunoglobulin molecule can be combined with an arm which binds to a triggering molecule on a leukocyte such as a T-cell receptor molecule (e.g. CD2, CD3, CD28, or B7), or Fc receptors for IgG (FcγR), such as FcγRI (CD64), FcγRII (CD32) and FcγRIII (CD16) so as to focus cellular defense mechanisms to the cell expressing the particular antigen. Bispecific antibodies can also be used to direct cytotoxic agents to cells which express a particular antigen. These antibodies possess an antigen-binding arm and an arm which binds a cytotoxic agent or a radionuclide chelator, such as EOTUBE, DPTA, DOTA, or TETA. Another bispecific antibody of interest binds the protein antigen described herein and further binds tissue factor (TF).

## Heteroconjugate Antibodies

Heteroconjugate antibodies are also within the scope of the present invention. Heteroconjugate antibodies are composed of two covalently joined antibodies. Such antibodies have, for example, been proposed to target immune system cells to unwanted cells (U.S. Patent No. 4,676,980), and for treatment of HIV infection (WO 91/00360; WO 92/200373; EP 03089). It is contemplated that the antibodies can be prepared in vitro using known methods in synthetic protein chemistry, including those involving crosslinking agents. For example, immunotoxins can be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980.

## Effector Function Engineering

It can be desirable to modify the antibody of the invention with respect to effector function, so as to enhance, *e.g.*, the effectiveness of the antibody in treating cancer. For example, cysteine residue(s) can be introduced into the Fc region, thereby allowing interchain disulfide bond formation in this region. The homodimeric antibody thus generated can have improved internalization capability and/or increased complement-mediated cell killing and antibody-dependent cellular cytotoxicity (ADCC). See Caron et al., J. Exp Med., 176: 1191-1195 (1992) and Shopes, J. Immunol., 148: 2918-2922 (1992). Homodimeric antibodies with enhanced anti-tumor activity can also be prepared using heterobifunctional cross-linkers as described in Wolff et al. Cancer Research, 53: 2560-2565 (1993). Alternatively, an antibody can be engineered that has dual Fc regions and can thereby have enhanced complement lysis and ADCC capabilities. See Stevenson et al., Anti-Cancer Drug Design, 3: 219-230 (1989).

## Immunoconjugates

The invention also pertains to immunoconjugates comprising an antibody conjugated to a cytotoxic agent such as a chemotherapeutic agent, toxin (e.g., an enzymatically active toxin of bacterial, fungal, plant, or animal origin, or fragments thereof), or a radioactive isotope *(i.e.,* a radioconjugate).

Chemotherapeutic agents useful in the generation of such immunoconjugates have been described above. Enzymatically active toxins and fragments thereof that can be used include diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes. A variety of radionuclides are available for the production of radioconjugated antibodies. Examples include $^{212}$Bi, $^{131}$I, $^{131}$In, $^{9o}$Y, and $^{186}$Re.

Conjugates of the antibody and cytotoxic agent are made using a variety of bifunctional protein-coupling agents such as N-succinimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al.. Science, 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026.

In another embodiment, the antibody can be conjugated to a "receptor" (such streptavidin) for utilization in tumor pretargeting wherein the antibody-receptor conjugate is administered to the patient, followed by removal of unbound conjugate from the circulation using a clearing agent and then administration of a "ligand" (*e.g.,* avidin) that is in turn conjugated to a cytotoxic agent.

**Immunoliposomes**

The antibodies disclosed herein can also be formulated as immunoliposomes. Liposomes containing the antibody are prepared by methods known in the art, such as described in Epstein et al., Proc. Natl. Acad. Sci. USA, 82: 3688 (1985); Hwang et al., Proc. Natl Acad. Sci. USA, 77: 4030 (1980); and U.S. Pat. Nos. 4,485,045 and 4,544,545. Liposomes with enhanced circulation time are disclosed in U.S. Patent No. 5,013,556.

Particularly useful liposomes can be generated by the reverse-phase evaporation method with a lipid composition comprising phosphatidylcholine, cholesterol, and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin et al ., J. Biol. Chem., 257: 286-288 (1982) via a disulfide-interchange reaction. A chemotherapeutic agent (such as Doxorubicin) is optionally contained within the liposome. See Gabizon et al., J. National Cancer Inst., 81(19): 1484 (1989).

**Diagnostic Applications of Antibodies Directed Against the Proteins of the Invention**

In one embodiment, methods for the screening of antibodies that possess the desired specificity include, but are not limited to, enzyme linked immunosorbent assay (ELISA) and other immunologically mediated techniques known within the art. In a specific embodiment, selection of antibodies that are specific to a particular domain of an NOVX protein is facilitated by generation of hybridomas that bind to the fragment of an NOVX protein possessing such a domain. Thus, antibodies that are specific for a desired domain within an NOVX protein, or derivatives, fragments, analogs or homologs thereof, are also provided herein.

Antibodies directed against a NOVX protein of the invention may be used in methods known within the art relating to the localization and/or quantitation of a NOVX protein (*e.g.,* for use in measuring levels of the NOVX protein within appropriate physiological samples, for use in diagnostic methods, for use in imaging the protein, and the like). In a given embodiment, antibodies specific to a NOVX protein, or derivative, fragment, analog or homolog thereof, that contain the antibody derived antigen binding domain, are utilized as pharmacologically active compounds (referred to hereinafter as "Therapeutics").

An antibody specific for a NOVX protein of the invention (e.g., a monoclonal antibody or a polyclonal antibody) can be used to isolate a NOVX polypeptide by standard techniques, such as immunoaffinity, chromatography or immunoprecipitation. An antibody to a NOVX polypeptide can facilitate the purification of a natural NOVX antigen from cells, or of a recombinantly produced NOVX antigen expressed in host cells. Moreover, such an anti-NOVX antibody can be used to detect the antigenic NOVX protein (*e.g.,* in a cellular lysate or cell supernatant) in order to evaluate the abundance and pattern of expression of the antigenic NOVX protein. Antibodies directed against a NOVX protein can be used diagnostically to monitor protein levels in tissue as part of a clinical testing procedure, *e.g.,* to, for example, determine the efficacy of a given treatment regimen. Detection can be facilitated by coupling (*i.e.,* physically linking) the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include $^{125}$I, $^{131}$I, $^{35}$S or $^{3}$H.

**Antibody Therapeutics**

Antibodies of the invention, including polyclonal, monoclonal, humanized and fully human antibodies, may used as therapeutic agents. Such agents are generally employed to treat or prevent a disease or pathology in a subject. An antibody preparation, preferably one having high specificity and high affinity for its target antigen, is administered to the subject and generally has an effect due to its binding with the target. Such an effect may be one of two kinds, depending on the specific nature of the interaction between the given antibody molecule and the target antigen in question. In the first instance, administration of the antibody may abrogate or inhibit the binding of the target with an endogenous ligand to which it naturally binds. In this case, the antibody binds to the target and masks a binding site of the naturally occurring ligand, wherein the ligand serves as an effector molecule. Thus the receptor mediates a signal transduction pathway for which ligand is responsible.

Alternatively, the effect may be one in which the antibody elicits a physiological result by virtue of binding to an effector binding site on the target molecule. In this case the target, a receptor having an endogenous ligand which may be absent or defective in the disease or pathology, binds the antibody as a surrogate effector ligand, initiating a receptor-based signal transduction event by the receptor.

A therapeutically effective amount of an antibody of the invention relates generally to the amount needed to achieve a therapeutic objective. As noted above, this may be a binding interaction between the antibody and its target antigen that, in certain cases, interferes with the functioning of the target, and in other cases, promotes a physiological response. The amount required to be administered will furthermore depend on the binding affinity of the antibody for its specific antigen, and will also depend on the rate at which an administered antibody is depleted from the free volume other subject to which it is administered. Common ranges for therapeutically effective dosing of an antibody or antibody fragment of the invention may be, by way of nonlimiting example, from about 0.1 mg/kg body weight to about 50 mg/kg body weight. Common dosing frequencies may range, for example, from twice daily to once a week.

**Pharmaceutical Compositions of Antibodies**

Antibodies specifically binding a protein of the invention, as well as other molecules identified by the screening assays disclosed herein, can be administered for the treatment of various disorders in the form of pharmaceutical compositions. Principles and considerations involved in preparing such compositions, as well as guidance in the choice of components are provided, for example, in Remington : The Science And Practice Of Pharmacy 19th ed. (Alfonso R. Gennaro, et al., editors) Mack Pub. Co., Easton, Pa. : 1995; Drug Absorption Enhancement: Concepts, Possibilities, Limitations, And Trends, Harwood Academic Publishers, Langhorne, Pa., 1994; and Peptide And Protein Drug Delivery (Advances In Parenteral Sciences, Vol. 4), 1991, M. Dekker, New York.

If the antigenic protein is intracellular and whole antibodies are used as inhibitors, internalizing antibodies are preferred. However, liposomes can also be used to deliver the antibody, or an antibody fragment, into cells. Where antibody fragments are used, the smallest inhibitory fragment that specifically binds to the binding domain of the target protein is preferred. For example, based upon the variable-region sequences of an antibody, peptide molecules can be designed that retain the ability to bind the target protein sequence. Such peptides can be synthesized chemically and/or produced by recombinant DNA technology. See, *e.g.,* Marasco et al., Proc. Natl. Acad. Sci. USA, 90: 7889-7893 (1993). The formulation herein can also contain more than one active compound as necessary for the particular indication being treated, preferably those with complementary activities that do not adversely affect each other. Alternatively, or in addition, the composition can comprise an agent that enhances its function, such as, for example, a cytotoxic agent, cytokine, chemotherapeutic agent, or growth-inhibitory agent. Such molecules are suitably present in combination in amounts that are effective for the purpose intended.

The active ingredients can also be entrapped in microcapsules prepared, for example, by coacervation techniques or by interfacial polymerization, for example, hydroxymethylcellulose or gelatin-microcapsules and poly-(methylmethacrylate) microcapsules, respectively, in colloidal drug delivery systems (for example, liposomes, albumin microspheres, microemulsions, nano-particles, and nanocapsules) or in macroemulsions.

The formulations to be used for in vivo administration must be sterile. This is readily accomplished by filtration through sterile filtration membranes.

Sustained-release preparations can be prepared. Suitable examples of sustained-release preparations include semipermeable matrices of solid hydrophobic polymers containing the antibody, which matrices are in the form of shaped articles, e.g., films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and γ ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods.

**ELISA Assay**

An agent for detecting an analyte protein is an antibody capable of binding to an analyte protein, preferably an antibody with a detectable label. Antibodies can be polyclonal, or more preferably, monoclonal. An intact antibody, or a fragment thereof (*e.g.*, $F_{ab}$ or $F_{(ab)2}$) can be used. The term "labeled", with regard to the probe or antibody, is intended to encompass direct labeling of the probe or antibody by coupling (*i.e.*, physically linking) a detectable substance to the probe or antibody, as well as indirect labeling of the probe or antibody by reactivity with another reagent that is directly labeled. Examples of indirect labeling include detection of a primary antibody using a fluorescently-labeled secondary antibody and end-labeling of a DNA probe with biotin such that it can be detected with fluorescently-labeled streptavidin. The term "biological sample" is intended to include tissues, cells and biological fluids isolated from a subject, as well as tissues, cells and fluids present within a subject. Included within the usage of the term "biological sample", therefore, is blood and a fraction or component of blood including blood serum, blood plasma, or lymph. That is, the detection method of the invention can be used to detect an analyte mRNA, protein, or genomic DNA in a biological sample *in vitro* as well as *in vivo.* For example, *in vitro* techniques for detection of an analyte mRNA include Northern hybridizations and *in situ* hybridizations. *In vitro* techniques for detection of an analyte protein include enzyme linked immunosorbent assays (ELISAs), Western blots, immunoprecipitations, and immunofluorescence. *In vitro* techniques for detection of an analyte genomic DNA include Southern hybridizations. Procedures for conducting immunoassays are described, for example in "ELISA: Theory and Practice: Methods in Molecular Biology", Vol. 42, J. R. Crowther (Ed.) Human Press, Totowa, NJ, 1995; "Immunoassay", E. Diamandis and T. Christopoulus, Academic Press, Inc., San Diego, CA, 1996; and "Practice and Theory of Enzyme Immunoassays", P. Tijssen, Elsevier Science Publishers, Amsterdam, 1985. Furthermore, *in vivo* techniques for detection of an analyte protein include introducing into a subject a labeled anti-an analyte protein antibody. For example, the antibody can be labeled with a radioactive marker whose presence and location in a subject can be detected by standard imaging techniques.

**NOVX Recombinant Expression Vectors and Host Cells**

Another aspect of the invention pertains to vectors, preferably expression vectors, containing a nucleic acid encoding a NOVX protein, or derivatives, fragments, analogs or homologs thereof. As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (*e.g.,* bacterial vectors having a bacterial origin of replication and episomal mammalian vectors). Other vectors (*e.g.*, non-episomal mammalian vectors) are integrated into the genome of a host cell upon introduction into the host cell, and thereby are replicated along with the host genome. Moreover, certain vectors are capable of directing the expression of genes to which they are operatively-linked. Such vectors are referred to herein as "expression vectors". In general, expression vectors of utility in recombinant DNA techniques are often in the form of plasmids. In the present specification, "plasmid" and "vector" can be used interchangeably as the plasmid is the most commonly used form of vector. However, the invention is intended to include such other forms of expression vectors, such as viral vectors (*e.g.*, replication defective retroviruses, adenoviruses and adeno-associated viruses), which serve equivalent functions.

The recombinant expression vectors of the invention comprise a nucleic acid of the invention in a form suitable for expression of the nucleic acid in a host cell, which means that the recombinant expression vectors include one or more regulatory sequences, selected on the basis of the host cells to be used for expression, that is operatively-linked to the nucleic acid sequence to be expressed. Within a recombinant expression vector, "operably-linked" is intended to mean that the nucleotide sequence of interest is linked to the regulatory sequence(s) in a manner that allows for expression of the nucleotide sequence (*e.g.,* in an *in vitro* transcription/translation system or in a host cell when the vector is introduced into the host cell).

The term "regulatory sequence" is intended to includes promoters, enhancers and other expression control elements (*e.g.*, polyadenylation signals). Such regulatory sequences are described, for example, in Goeddel, GENE EXPRESSION TECHNOLOGY: METHODS IN ENZYMOLOGY 185, Academic Press, San Diego, Calif. (1990). Regulatory sequences include those that direct constitutive expression of a nucleotide sequence in many types of host cell and those that direct expression of the nucleotide sequence only in certain host cells (e.g., tissue-specific regulatory sequences). It will be appreciated by those skilled in the art that the design of the expression vector can depend on such factors as the choice of the host cell to be transformed, the level of expression of protein desired, *etc.* The expression vectors of the invention can be introduced into host cells to thereby produce proteins or peptides, including fusion proteins or peptides, encoded by nucleic acids as described herein (e.g., NOVX proteins, mutant forms of NOVX proteins, fusion proteins, *etc*.).

The recombinant expression vectors of the invention can be designed for expression of NOVX proteins in prokaryotic or eukaryotic cells. For example, NOVX proteins can be expressed in bacterial cells such as *Escherichia coli,* insect cells (using baculovirus expression vectors) yeast cells or mammalian cells. Suitable host cells are discussed further in Goeddel, GENE EXPRESSION TECHNOLOGY: METHODS IN ENZYMOLOGY 185, Academic Press, San Diego, Calif. (1990). Alternatively, the recombinant expression vector can be transcribed and translated *in vitro,* for example using T7 promoter regulatory sequences and T7 polymerase.

Expression of proteins in prokaryotes is most often carried out in *Escherichia coli* with vectors containing constitutive or inducible promoters directing the expression of either fusion or non-fusion proteins. Fusion vectors add a number of amino acids to a protein encoded therein, usually to the amino terminus of the recombinant protein. Such fusion vectors typically serve three purposes: (*i*) to increase expression of recombinant protein; (*ii*) to increase the solubility of the recombinant protein; and (*iii*) to aid in the purification of the recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Typical fusion expression vectors include pGEX (Pharmacia Biotech Inc; Smith and Johnson, 1988. Gene 67: 31-40), pMAL (New England Biolabs, Beverly, Mass.) and pRIT5 (Pharmacia, Piscataway, N.J.) that fuse glutathione S-transferase (GST), maltose E binding protein, or protein A, respectively, to the target recombinant protein.

Examples of suitable inducible non-fusion *E. coli* expression vectors include pTrc (Amrann et al., (1988) Gene 69:301-315) and pET 11d (Studier et al., GENE EXPRESSION TECHNOLOGY: METHODS IN ENZYMOLOGY 185, Academic Press, San Diego, Calif. (1990) 60-89).

One strategy to maximize recombinant protein expression in *E. coli* is to express the protein in a host bacteria with an impaired capacity to proteolytically cleave the recombinant protein. *See, e.g.,* Gottesman, GENE EXPRESSION TECHNOLOGY: METHODS IN ENZYMOLOGY 185, Academic Press, San Diego, Calif. (1990) 119-128. Another strategy is to alter the nucleic acid sequence of the nucleic acid to be inserted into an expression vector so that the individual codons for each amino acid are those preferentially utilized in *E. coli (see, e.g.,* Wada, et al., 1992. Nucl. Acids Res. 20: 2111-2118). Such alteration of nucleic acid sequences of the invention can be carried out by standard DNA synthesis techniques.

In another embodiment, the NOVX expression vector is a yeast expression vector. Examples of vectors for expression in yeast *Saccharomyces cerivisae* include pYepSec1 1 (Baldari, et al., 1987. EMBO J. 6: 229-234), pMFa (Kurjan and Herskowitz, 1982. Cell 30: 933-943), pJRY88 (Schultz et al., 1987. Gene 54: 113-123), pYES2 (Invitrogen Corporation, San Diego, Calif.), and picZ (InVitrogen Corp, San Diego, Calif.).

Alternatively, NOVX can be expressed in insect cells using baculovirus expression vectors. Baculovirus vectors available for expression of proteins in cultured insect cells (*e.g.,* SF9 cells) include the pAc series (Smith, et al., 1983. Mol. Cell. Biol. 3: 2156-2165) and the pVL series (Lucklow and Summers, 1989. Virology 170: 31-39).

In yet another embodiment, a nucleic acid of the invention is expressed in mammalian cells using a mammalian expression vector. Examples of mammalian expression vectors include pCDM8 (Seed, 1987. Nature 329: 840) and pMT2PC (Kaufman, et al., 1987. EMBO J. 6: 187-195). When used in mammalian cells, the expression vector's control functions are often provided by viral regulatory elements. For example, commonly used promoters are derived from polyoma, adenovirus 2, cytomegalovirus, and simian virus 40. For other suitable expression systems for both prokaryotic and eukaryotic cells see, *e.g.,* Chapters 16 and 17 of Sambrook, et al., MOLECULAR CLONING: A LABORATORY MANUAL. 2nd ed., Cold Spring Harbor Laboratory, Cold Spring Harbor Laboratory Press, Cold Spring Harbor, N.Y., 1989.

In another embodiment, the recombinant mammalian expression vector is capable of directing expression of the nucleic acid preferentially in a particular cell type *(e.g.,* tissue-specific regulatory elements are used to express the nucleic acid). Tissue-specific regulatory elements are known in the art. Non-limiting examples of suitable tissue-specific promoters include the albumin promoter (liver-specific; Pinkert, et al., 1987. Genes Dev. 1: 268-277), lymphoid-specific promoters (Calame and Eaton, 1988. Adv. Immunol. 43: 235-275), in particular promoters of T cell receptors (Winoto and Baltimore, 1989. EMBO J. 8: 729-733) and immunoglobulins (Banerji, et al., 1983. Cell 33: 729-740; Queen and Baltimore, 1983. Cell 33: 741-748), neuron-specific promoters *(e.g.,* the neurofilament promoter; Byrne and Ruddle, 1989. Proc. Natl. Acad. Sci. USA 86: 5473-5477), pancreas-specific promoters (Edlund, et al., 1985. Science 230: 912-916), and mammary gland-specific promoters (*e.g.,* milk whey promoter; U.S. Pat. No. 4,873,316 and European Application Publication No. 264,166). Developmentally-regulated promoters are also encompassed, *e.g.*, the murine hox promoters (Kessel and Gruss, 1990. Science 249: 374-379) and the $\alpha$-fetoprotein promoter (Campes and Tilghman, 1989. Genes Dev. 3: 537-546).

The invention further provides a recombinant expression vector comprising a DNA molecule of the invention cloned into the expression vector in an antisense orientation. That is, the DNA molecule is operatively-linked to a regulatory sequence in a manner that allows for expression (by transcription of the DNA molecule) of an RNA molecule that is antisense to NOVX mRNA. Regulatory sequences operatively linked to a nucleic acid cloned in the antisense orientation can be chosen that direct the continuous expression of the antisense RNA molecule in a variety of cell types, for instance viral promoters and/or enhancers, or regulatory sequences can be chosen that direct constitutive, tissue specific or cell type specific expression of antisense RNA. The antisense expression vector can be in the form of a recombinant plasmid, phagemid or attenuated virus in which antisense nucleic acids are produced under the control of a high efficiency regulatory region, the activity of which can be determined by the cell type into which the vector is introduced. For a discussion of the regulation of gene expression using antisense genes *see, e.g.,* Weintraub, et al., "Antisense RNA as a molecular tool for genetic analysis," Reviews-Trends in Genetics, Vol. 1(1) 1986.

Another aspect of the invention pertains to host cells into which a recombinant expression vector of the invention has been introduced. The terms "host cell" and "recombinant host cell" are used interchangeably herein. It is understood that such terms refer not only to the particular subject cell but also to the progeny or potential progeny of such a cell. Because certain modifications may occur in succeeding generations due to either mutation or environmental influences, such progeny may not, in fact, be identical to the parent cell, but are still included within the scope of the term as used herein.

A host cell can be any prokaryotic or eukaryotic cell. For example, NOVX protein can be expressed in bacterial cells such as *E. coli,* insect cells, yeast or mammalian cells (such as Chinese hamster ovary cells (CHO) or COS cells). Other suitable host cells are known to those skilled in the art.

Vector DNA can be introduced into prokaryotic or eukaryotic cells via conventional transformation or transfection techniques. As used herein, the terms "transformation" and "transfection" are intended to refer to a variety of art-recognized techniques for introducing foreign nucleic acid (*e.g.,* DNA) into a host cell, including calcium phosphate or calcium chloride co-precipitation, DEAE-dextran-mediated transfection, lipofection, or electroporation. Suitable methods for transforming or transfecting host cells can be found in Sambrook, et al. (MOLECULAR CLONING: A LABORATORY MANUAL. 2nd ed., Cold Spring Harbor Laboratory, Cold Spring Harbor Laboratory Press, Cold Spring Harbor, N.Y., 1989), and other laboratory manuals.

For stable transfection of mammalian cells, it is known that, depending upon the expression vector and transfection technique used, only a small fraction of cells may integrate the foreign DNA into their genome. In order to identify and select these integrants, a gene that encodes a selectable marker (*e.g.,* resistance to antibiotics) is generally introduced into the host cells along with the gene of interest. Various selectable markers include those that confer resistance to drugs, such as G418, hygromycin and methotrexate. Nucleic acid encoding a selectable marker can be introduced into a host cell on the same vector as that encoding NOVX or can be introduced on a separate vector. Cells stably transfected with the introduced nucleic acid can be identified by drug selection (*e.g.,* cells that have incorporated the selectable marker gene will survive, while the other cells die).

A host cell of the invention, such as a prokaryotic or eukaryotic host cell in culture, can be used to produce (*i.e.,* express) NOVX protein. Accordingly, the invention further provides methods for producing NOVX protein using the host cells of the invention. In one embodiment, the method comprises culturing the host cell of invention (into which a recombinant expression vector encoding NOVX protein has been introduced) in a suitable medium such that NOVX protein is produced. In another embodiment, the method further comprises isolating NOVX protein from the medium or the host cell.

**Transgenic NOVX Animals**

The host cells of the invention can also be used to produce non-human transgenic animals. For example, in one embodiment, a host cell of the invention is a fertilized oocyte or an embryonic stem cell into which NOVX protein-coding sequences have been introduced. Such host cells can then be used to create non-human transgenic animals in which exogenous NOVX sequences have been introduced into their genome or homologous recombinant animals in which endogenous NOVX sequences have been altered. Such animals are useful for studying the function and/or activity of NOVX protein and for identifying and/or evaluating modulators of NOVX protein activity. As used herein, a "transgenic animal" is a non-human animal, preferably a mammal, more preferably a rodent such as a rat or mouse, in which one or more of the cells of the animal includes a transgene. Other examples of transgenic animals include non-human primates, sheep, dogs, cows, goats, chickens, amphibians, *etc.* A transgene is exogenous DNA that is integrated into the genome of a cell from which a transgenic animal develops and that remains in the genome of the mature animal, thereby directing the expression of an encoded gene product in one or more cell types or tissues of the transgenic animal. As used herein, a "homologous recombinant animal" is a non-human animal, preferably a mammal, more preferably a mouse, in which an endogenous NOVX gene has been altered by homologous recombination between the endogenous gene and an exogenous DNA molecule introduced into a cell of the animal, *e.g.*, an embryonic cell of the animal, prior to development of the animal.

A transgenic animal of the invention can be created by introducing NOVX-encoding nucleic acid into the male pronuclei of a fertilized oocyte (*e.g.*, by microinjection, retroviral infection) and allowing the oocyte to develop in a pseudopregnant female foster animal. The human NOVX cDNA sequences, *i.e.*, any one of SEQ ID NO:2n-1, wherein *n* is an integer between 1 and 12, can be introduced as a transgene into the genome of a non-human animal. Alternatively, a non-human homologue of the human NOVX gene, such as a mouse NOVX gene, can be isolated based on hybridization to the human NOVX cDNA (described further *supra*) and used as a transgene. Intronic sequences and polyadenylation signals can also be included in the transgene to increase the efficiency of expression of the transgene. A tissue-specific regulatory sequence(s) can be operably-linked to the NOVX transgene to direct expression of NOVX protein to particular cells. Methods for generating transgenic animals via embryo manipulation and microinjection, particularly animals such as mice, have become conventional in the art and are described, for example, in U.S. Patent Nos. 4,736,866; 4,870,009; and 4,873,191; and Hogan, 1986. In: MANIPULATING THE MOUSE EMBRYO, Cold Spring Harbor Laboratory Press, Cold Spring Harbor, N.Y. Similar methods are used for production of other transgenic animals. A transgenic founder animal can be identified based upon the presence of the NOVX transgene in its genome and/or expression of NOVX mRNA in tissues or cells of the animals. A transgenic founder animal can then be used to breed additional animals carrying the transgene. Moreover, transgenic animals carrying a transgene-encoding NOVX protein can further be bred to other transgenic animals carrying other transgenes.

To create a homologous recombinant animal, a vector is prepared which contains at least a portion of a NOVX gene into which a deletion, addition or substitution has been introduced to thereby alter, *e.g.*, functionally disrupt, the NOVX gene. The NOVX gene can be a human gene (*e.g.,* the cDNA of any one of SEQ ID NO:2n-1, wherein *n* is an integer between 1 and 12), but more preferably, is a non-human homologue of a human NOVX gene. For example, a mouse homologue of human NOVX gene of SEQ ID NO:2n-1, wherein *n* is an integer between 1 and 12, can be used to construct a homologous recombination vector suitable for altering an endogenous NOVX gene in the mouse genome. In one embodiment, the vector is designed such that, upon homologous recombination, the endogenous NOVX gene is functionally disrupted *(i.e.,* no longer encodes a functional protein; also referred to as a "knock out" vector).

Alternatively, the vector can be designed such that, upon homologous recombination, the endogenous NOVX gene is mutated or otherwise altered but still encodes functional protein (*e.g.,* the upstream regulatory region can be altered to thereby alter the expression of the endogenous NOVX protein). In the homologous recombination vector, the altered portion of the NOVX gene is flanked at its 5'- and 3'-termini by additional nucleic acid of the NOVX gene to allow for homologous recombination to occur between the exogenous NOVX gene carried by the vector and an endogenous NOVX gene in an embryonic stem cell. The additional flanking NOVX nucleic acid is of sufficient length for successful homologous recombination with the endogenous gene. Typically, several kilobases of flanking DNA (both at the 5'- and 3'-termini) are included in the vector. *See, e.g.,* Thomas, et al., 1987. Cell 51: 503 for a description of homologous recombination vectors. The vector is ten introduced into an embryonic stem cell line *(e.g.,* by electroporation) and cells in which the introduced NOVX gene has homologously-recombined with the endogenous NOVX gene are selected. *See, e.g.,* Li, et al., 1992. Cell 69: 915.

The selected cells are then injected into a blastocyst of an animal (*e.g.,* a mouse) to form aggregation chimeras. *See, e.g.,* Bradley, 1987. In: TERATOCARCINOMAS AND EMBRYONIC STEM CELLS: A PRACTICAL APPROACH, Robertson, ed. IRL, Oxford, pp. 113-152. A chimeric embryo can then be implanted into a suitable pseudopregnant female foster animal and the embryo brought to term. Progeny harboring the homologously-recombined DNA in their germ cells can be used to breed animals in which all cells of the animal contain the homologously-recombined DNA by germline transmission of the transgene. Methods for constructing homologous recombination vectors and homologous recombinant animals are described further in Bradley, 1991. Curr. Opin. Biotechnol. 2: 823-829; PCT International Publication Nos.: WO 90/11354; WO 91/01140; WO 92/0968; and WO 93/04169.

In another embodiment, transgenic non-humans animals can be produced that contain selected systems that allow for regulated expression of the transgene. One example of such a system is the cre/loxP recombinase system of bacteriophage P1. For a description of the cre/loxP recombinase system, *See, e.g.,* Lakso, et al., 1992. Proc. Natl. Acad. Sci. USA 89: 6232-6236. Another example of a recombinase system is the FLP recombinase system of *Saccharomyces cerevisiae. See,* O'Gorman, et al., 1991. Science 251:1351-1355. If a cre/loxP recombinase system is used to regulate expression of the transgene, animals containing transgenes encoding both the Cre recombinase and a selected protein are required. Such animals can be provided through the construction of "double" transgenic animals, *e.g.*, by mating two transgenic animals, one containing a transgene encoding a selected protein and the other containing a transgene encoding a recombinase.

Clones of the non-human transgenic animals described herein can also be produced according to the methods described in Wilmut, et al., 1997. Nature 385: 810-813. In brief, a cell *(e.g.,* a somatic cell) from the transgenic animal can be isolated and induced to exit the growth cycle and enter Go phase. The quiescent cell can then be fused, *e.g.,* through the use of electrical pulses, to an enucleated oocyte from an animal of the same species from which the quiescent cell is isolated. The reconstructed oocyte is then cultured such that it develops to morula or blastocyte and then transferred to pseudopregnant female foster animal. The offspring borne of this female foster animal will be a clone of the animal from which the cell *(e.g.,* the somatic cell) is isolated.

**Pharmaceutical Compositions**

The NOVX nucleic acid molecules, NOVX proteins, and anti-NOVX antibodies (also referred to herein as "active compounds") of the invention, and derivatives, fragments, analogs and homologs thereof, can be incorporated into pharmaceutical compositions suitable for administration. Such compositions typically comprise the nucleic acid molecule, protein, or antibody and a pharmaceutically acceptable carrier. As used herein, "pharmaceutically acceptable carrier" is intended to include any and all solvents, dispersion media, coatings, antibacterial and antifungal agents, isotonic and absorption delaying agents, and the like, compatible with pharmaceutical administration. Suitable carriers are described in the most recent edition of Remington's Pharmaceutical Sciences, a standard reference text in the field, which is incorporated herein by reference. Preferred examples of such carriers or diluents include, but are not limited to, water, saline, finger's solutions, dextrose solution, and 5% human serum albumin. Liposomes and non-aqueous vehicles such as fixed oils may also be used. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

A pharmaceutical composition of the invention is formulated to be compatible with its intended route of administration. Examples of routes of administration include parenteral, *e.g.,* intravenous, intradermal, subcutaneous, oral *(e.g.,* inhalation), transdermal *(i.e.,* topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid (EDTA); buffers such as acetates, citrates or phosphates, and agents for the adjustment of tonicity such as sodium chloride or dextrose. The pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, N.J.) or phosphate buffered saline (PBS). In all cases, the composition must be sterile and should be fluid to the extent that easy syringeability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it is be preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound *(e.g.,* a NOVX protein or anti-NOVX antibody) in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, methods of preparation are vacuum drying and freeze-drying that yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, *e.g.,* a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (*e.g.,* with conventional suppository bases such as cocoa butter and other glycerides) or retention enemas for rectal delivery.

In one embodiment, the active compounds are prepared with carriers that protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No. 4,522,811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

The nucleic acid molecules of the invention can be inserted into vectors and used as gene therapy vectors. Gene therapy vectors can be delivered to a subject by, for example, intravenous injection, local administration (*see, e.g.,* U.S. Patent No. 5,328,470) or by stereotactic injection (*see, e.g.,* Chen, et al., 1994. Proc. Natl. Acad. Sci. USA 91: 3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, *e.g.,* retroviral vectors, the pharmaceutical preparation can include one or more cells that produce the gene delivery system.

The pharmaceutical compositions can be included in a container, pack, or dispenser together with instructions for administration.

**Screening and Detection Methods**

The isolated nucleic acid molecules of the invention can be used to express NOVX protein (*e.g.,* via a recombinant expression vector in a host cell in gene therapy applications), to detect NOVX mRNA (*e.g.,* in a biological sample) or a genetic lesion in a NOVX gene, and to modulate NOVX activity, as described further, below. In addition, the NOVX proteins can be used to screen drugs or compounds that modulate the NOVX protein activity or expression as well as to treat disorders characterized by insufficient or excessive production of NOVX protein or production of NOVX protein forms that have decreased or aberrant activity compared to NOVX wild-type protein (*e.g.;* diabetes (regulates insulin release); obesity (binds and transport lipids); metabolic disturbances associated with obesity, the metabolic syndrome X as well as anorexia and wasting disorders associated with chronic diseases and various cancers, and infectious disease(possesses anti-microbial activity) and the various dyslipidemias. In addition, the anti-NOVX antibodies of the invention can be used to detect and isolate NOVX proteins and modulate NOVX activity. In yet a further aspect, the invention can be used in methods to influence appetite, absorption of nutrients and the disposition of metabolic substrates in both a positive and negative fashion.

The invention further pertains to novel agents identified by the screening assays described herein and uses thereof for treatments as described, *supra.*

**Screening Assays**

The invention provides a method (also referred to herein as a "screening assay") for identifying modulators, *i.e.,* candidate or test compounds or agents (*e.g.*, peptides, peptidomimetics, small molecules or other drugs) that bind to NOVX proteins or have a stimulatory or inhibitory effect on, *e.g.,* NOVX protein expression or NOVX protein activity. The invention also includes compounds identified in the screening assays described herein.

In one embodiment, the invention provides assays for screening candidate or test compounds which bind to or modulate the activity of the membrane-bound form of a NOVX protein or polypeptide or biologically-active portion thereof. The test compounds of the invention can be obtained using any of the numerous approaches in combinatorial library methods known in the art, including: biological libraries; spatially addressable parallel solid phase or solution phase libraries; synthetic library methods requiring deconvolution; the "one-bead one-compound" library method; and synthetic library methods using affinity chromatography selection. The biological library approach is limited to peptide libraries, while the other four approaches are applicable to peptide, non-peptide oligomer or small molecule libraries of compounds. *See, e.g.,* Lam, 1997. Anticancer Drug Design 12: 145.

A "small molecule" as used herein, is meant to refer to a composition that has a molecular weight of less than about 5 kD and most preferably less than about 4 kD. Small molecules can be, *e.g.*, nucleic acids, peptides, polypeptides, peptidomimetics, carbohydrates, lipids or other organic or inorganic molecules. Libraries of chemical and/or biological mixtures, such as fungal, bacterial, or algal extracts, are known in the art and can be screened with any of the assays of the invention.

Examples of methods for the synthesis of molecular libraries can be found in the art, for example in: DeWitt, et al., 1993. Proc. Natl. Acad. Sci. U.S.A. 90: 6909; Erb, et al., 1994. Proc. Natl. Acad. Sci. U.S.A. 91: 11422; Zuckermann, et al., 1994. J. Med. Chem. 37: 2678; Cho, et al., 1993. Science 261: 1303; Carrell, et al., 1994. Angew. Chem. Int. Ed. Engl. 33: 2059; Carell, et al., 1994. Angew. Chem. Int. Ed. Engl. 33: 2061; and Gallop, et al., 1994. J. Med. Chem. 37: 1233.

Libraries of compounds may be presented in solution (*e.g.,* Houghten, 1992. Biotechniques 13: 412-421), or on beads (Lam, 1991. Nature 354: 82-84), on chips (Fodor, 1993. Nature 364: 555-556), bacteria (Ladner, U.S. Patent No. 5,223,409), spores (Ladner, U.S. Patent 5,233,409), plasmids (Cull, et al., 1992. Proc. Natl. Acad. Sci. USA 89: 1865-1869) or on phage (Scott and Smith, 1990. Science 249: 386-390; Devlin, 1990. Science 249: 404-406; Cwirla, et al., 1990. Proc. Natl. Acad. Sci. U.S.A. 87: 6378-6382; Felici, 1991. J. Mol. Biol. 222: 301-310; Ladner, U.S. Patent No. 5,233,409.).

In one embodiment, an assay is a cell-based assay in which a cell which expresses a membrane-bound form of NOVX protein, or a biologically-active portion thereof, on the cell surface is contacted with a test compound and the ability of the test compound to bind to a NOVX protein determined. The cell, for example, can of mammalian origin or a yeast cell. Determining the ability of the test compound to bind to the NOVX protein can be accomplished, for example, by coupling the test compound with a radioisotope or enzymatic label such that binding of the test compound to the NOVX protein or biologically-active portion thereof can be determined by detecting the labeled compound in a complex. For example, test compounds can be labeled with $^{125}$I, $^{35}$S, $^{14}$C, or $^{3}$H, either directly or indirectly, and the radioisotope detected by direct counting of radioemission or by scintillation counting. Alternatively, test compounds can be enzymatically-labeled with, for example, horseradish peroxidase, alkaline phosphatase, or luciferase, and the enzymatic label detected by determination of conversion of an appropriate substrate to product. In one embodiment, the assay comprises contacting a cell which expresses a membrane-bound form of NOVX protein, or a biologically-active portion thereof, on the cell surface with a known compound which binds NOVX to form an assay mixture, contacting the assay mixture with a test compound, and determining the ability of the test compound to interact with a NOVX protein, wherein determining the ability of the test compound to interact with a NOVX protein comprises determining the ability of the test compound to preferentially bind to NOVX protein or a biologically-active portion thereof as compared to the known compound.

In another embodiment, an assay is a cell-based assay comprising contacting a cell expressing a membrane-bound form of NOVX protein, or a biologically-active portion thereof, on the cell surface with a test compound and determining the ability of the test compound to modulate (*e.g.*, stimulate or inhibit) the activity of the NOVX protein or biologically-active portion thereof. Determining the ability of the test compound to modulate the activity of NOVX or a biologically-active portion thereof can be accomplished, for example, by determining the ability of the NOVX protein to bind to or interact with a NOVX target molecule. As used herein, a "target molecule" is a molecule with which a NOVX protein binds or interacts in nature, for example, a molecule on the surface of a cell which expresses a NOVX interacting protein, a molecule on the surface of a second cell, a molecule in the extracellular milieu, a molecule associated with the internal surface of a cell membrane or a cytoplasmic molecule. A NOVX target molecule can be a non-NOVX molecule or a NOVX protein or polypeptide of the invention. In one embodiment, a NOVX target molecule is a component of a signal transduction pathway that facilitates transduction of an extracellular signal (*e.g.* a signal generated by binding of a compound to a membrane-bound NOVX molecule) through the cell membrane and into the cell. The target, for example, can be a second intercellular protein that has catalytic activity or a protein that facilitates the association of downstream signaling molecules with NOVX.

Determining the ability of the NOVX protein to bind to or interact with a NOVX target molecule can be accomplished by one of the methods described above for determining direct binding. In one embodiment, determining the ability of the NOVX protein to bind to or interact with a NOVX target molecule can be accomplished by determining the activity of the target molecule. For example, the activity of the target molecule can be determined by detecting induction of a cellular second messenger of the target (*i.e.* intracellular $Ca^{2+}$, diacylglycerol, $IP_3$, *etc*.), detecting catalytic/enzymatic activity of the target an appropriate substrate, detecting the induction of a reporter gene (comprising a NOVX-responsive regulatory element operatively linked to a nucleic acid encoding a detectable marker, *e.g.,* luciferase), or detecting a cellular response, for example, cell survival, cellular differentiation, or cell proliferation.

In yet another embodiment, an assay of the invention is a cell-free assay comprising contacting a NOVX protein or biologically-active portion thereof with a test compound and determining the ability of the test compound to bind to the NOVX protein or biologically-active portion thereof. Binding of the test compound to the NOVX protein can be determined either directly or indirectly as described above. In one such embodiment, the assay comprises contacting the NOVX protein or biologically-active portion thereof with a known compound which binds NOVX to form an assay mixture, contacting the assay mixture with a test compound, and determining the ability of the test compound to interact with a NOVX protein, wherein determining the ability of the test compound to interact with a NOVX protein comprises determining the ability of the test compound to preferentially bind to NOVX or biologically-active portion thereof as compared to the known compound.

In still another embodiment, an assay is a cell-free assay comprising contacting NOVX protein or biologically-active portion thereof with a test compound and determining the ability of the test compound to modulate (*e.g.* stimulate or inhibit) the activity of the NOVX protein or biologically-active portion thereof. Determining the ability of the test compound to modulate the activity of NOVX can be accomplished, for example, by determining the ability of the NOVX protein to bind to a NOVX target molecule by one of the methods described above for determining direct binding. In an alternative embodiment, determining the ability of the test compound to modulate the activity of NOVX protein can be accomplished by determining the ability of the NOVX protein further modulate a NOVX target molecule. For example, the catalytic/enzymatic activity of the target molecule on an appropriate substrate can be determined as described, *supra.*

In yet another embodiment, the cell-free assay comprises contacting the NOVX protein or biologically-active portion thereof with a known compound which binds NOVX protein to form an assay mixture, contacting the assay mixture with a test compound, and determining the ability of the test compound to interact with a NOVX protein, wherein determining the ability of the test compound to interact with a NOVX protein comprises determining the ability of the NOVX protein to preferentially bind to or modulate the activity of a NOVX target molecule.

The cell-free assays of the invention are amenable to use of both the soluble form or the membrane-bound form of NOVX protein. In the case of cell-free assays comprising the membrane-bound form of NOVX protein, it may be desirable to utilize a solubilizing agent such that the membrane-bound form of NOVX protein is maintained in solution. Examples of such solubilizing agents include non-ionic detergents such as n-octylglucoside, n-dodecylglucoside, n-dodecylmaltoside, octanoyl-N-methylglucamide, decanoyl-N-methylglucamide, Triton® X-100, Triton® X-114, Thesit®, Isotridecypoly(ethylene glycol ether)$_n$, N-dodecyl--N,N-dimethyl-3-ammonio-1-propane sulfonate, 3-(3-cholamidopropyl) dimethylamminiol-1-propane sulfonate (CHAPS), or 3-(3-cholamidopropyl)dimethylamminiol-2-hydroxy-1-propane sulfonate (CHAPSO).

In more than one embodiment of the above assay methods of the invention, it may be desirable to immobilize either NOVX protein or its target molecule to facilitate separation of complexed from uncomplexed forms of one or both of the proteins, as well as to accommodate automation of the assay. Binding of a test compound to NOVX protein, or interaction of NOVX protein with a target molecule in the presence and absence of a candidate compound, can be accomplished in any vessel suitable for containing the reactants. Examples of such vessels include microtiter plates, test tubes, and micro-centrifuge tubes. In one embodiment, a fusion protein can be provided that adds a domain that allows one or both of the proteins to be bound to a matrix. For example, GST-NOVX fusion proteins or GST-target fusion proteins can be adsorbed onto glutathione sepharose beads (Sigma Chemical, St. Louis, MO) or glutathione derivatized microtiter plates, that are then combined with the test compound or the test compound and either the non-adsorbed target protein or NOVX protein, and the mixture is incubated under conditions conducive to complex formation (*e.g.*, at physiological conditions for salt and pH). Following incubation, the beads or microtiter plate wells are washed to remove any unbound components, the matrix immobilized in the case of beads, complex determined either directly or indirectly, for example, as described, *supra.* Alternatively, the complexes can be dissociated from the matrix, and the level of NOVX protein binding or activity determined using standard techniques.

Other techniques for immobilizing proteins on matrices can also be used in the screening assays of the invention. For example, either the NOVX protein or its target molecule can be immobilized utilizing conjugation of biotin and streptavidin. Biotinylated NOVX protein or target molecules can be prepared from biotin-NHS (N-hydroxy-succinimide) using techniques well-known within the art (*e.g.*, biotinylation kit, Pierce Chemicals, Rockford, Ill.), and immobilized in the wells of streptavidin-coated 96 well plates (Pierce Chemical). Alternatively, antibodies reactive with NOVX protein or target molecules, but which do not interfere with binding of the NOVX protein to its target molecule, can be derivatized to the wells of the plate, and unbound target or NOVX protein trapped in the wells by antibody conjugation. Methods for detecting such complexes, in addition to those described above for the GST-immobilized complexes, include immunodetection of complexes using antibodies reactive with the NOVX protein or target molecule, as well as enzyme-linked assays that rely on detecting an enzymatic activity associated with the NOVX protein or target molecule.

In another embodiment, modulators of NOVX protein expression are identified in a method wherein a cell is contacted with a candidate compound and the expression of NOVX mRNA or protein in the cell is determined. The level of expression of NOVX mRNA or protein in the presence of the candidate compound is compared to the level of expression of NOVX mRNA or protein in the absence of the candidate compound. The candidate compound can then be identified as a modulator of NOVX mRNA or protein expression based upon this comparison. For example, when expression of NOVX mRNA or protein is greater (i.e., statistically significantly greater) in the presence of the candidate compound than in its absence, the candidate compound is identified as a stimulator of NOVX mRNA or protein expression. Alternatively, when expression of NOVX mRNA or protein is less (statistically significantly less) in the presence of the candidate compound than in its absence, the candidate compound is identified as an inhibitor of NOVX mRNA or protein expression. The level of NOVX mRNA or protein expression in the cells can be determined by methods described herein for detecting NOVX mRNA or protein.

In yet another aspect of the invention, the NOVX proteins can be used as "bait proteins" in a two-hybrid assay or three hybrid assay (see, e.g., U.S. Patent No. 5,283,317; Zervos, et al., 1993. Cell 72: 223-232; Madura, et al., 1993. J. Biol. Chem. 268: 12046-12054; Bartel, et al., 1993. Biotechniques 14: 920-924; Iwabuchi, et al., 1993. Oncogene 8: 1693-1696; and Brent WO 94/10300), to identify other proteins that bind to or interact with NOVX ("NOVX-binding proteins" or "NOVX-bp") and modulate NOVX activity. Such NOVX-binding proteins are also involved in the propagation of signals by the NOVX proteins as, for example, upstream or downstream elements of the NOVX pathway.

The two-hybrid system is based on the modular nature of most transcription factors, which consist of separable DNA-binding and activation domains. Briefly, the assay utilizes two different DNA constructs. In one construct, the gene that codes for NOVX is fused to a gene encoding the DNA binding domain of a known transcription factor (e.g., GAL-4). In the other construct, a DNA sequence, from a library of DNA sequences, that encodes an unidentified protein ("prey" or "sample") is fused to a gene that codes for the activation domain of the known transcription factor. If the "bait" and the "prey" proteins are able to interact, in vivo, forming a NOVX-dependent complex, the DNA-binding and activation domains of the transcription factor are brought into close proximity. This proximity allows transcription of a reporter gene (e.g., LacZ) that is operably linked to a transcriptional regulatory site responsive to the transcription factor. Expression of the reporter gene can be detected and cell colonies containing the functional transcription factor can be isolated and used to obtain the cloned gene that encodes the protein which interacts with NOVX.

The invention further pertains to novel agents identified by the aforementioned screening assays and uses thereof for treatments as described herein.

### Detection Assays

Portions or fragments of the cDNA sequences identified herein (and the corresponding complete gene sequences) can be used in numerous ways as polynucleotide reagents. By way of example, and not of limitation, these sequences can be used to: (i) map their respective genes on a chromosome; and, thus, locate gene regions associated with genetic disease; (ii) identify an individual from a minute biological sample (tissue typing); and (iii) aid in forensic identification of a biological sample. Some of these applications are described in the subsections, below.

### Chromosome Mapping

Once the sequence (or a portion of the sequence) of a gene has been isolated, this sequence can be used to map the location of the gene on a chromosome. This process is called chromosome mapping. Accordingly, portions or fragments of the NOVX sequences of SEQ ID NO:2$n$-1, wherein $n$ is an integer between 1 and 12, or fragments or derivatives thereof, can be used to map the location of the NOVX genes, respectively, on a chromosome. The mapping of the NOVX sequences to chromosomes is an important first step in correlating these sequences with genes associated with disease.

Briefly, NOVX genes can be mapped to chromosomes by preparing PCR primers (preferably 15-25 bp in length) from the NOVX sequences. Computer analysis of the NOVX, sequences can be used to rapidly select primers that do not span more than one exon in the genomic DNA, thus complicating the amplification process. These primers can then be used for PCR screening of somatic cell hybrids containing individual human chromosomes. Only those hybrids containing the human gene corresponding to the NOVX sequences will yield an amplified fragment.

Somatic cell hybrids are prepared by fusing somatic cells from different mammals (e.g., human and mouse cells). As hybrids of human and mouse cells grow and divide, they gradually lose human chromosomes in random order, but retain the mouse chromosomes. By using media in which mouse cells cannot grow, because they lack a particular enzyme, but in which human cells can, the one human chromosome that contains the gene encoding the needed enzyme will be retained. By using various media, panels of hybrid cell lines can be established. Each cell line in a panel contains either a single human chromosome or a small number of human chromosomes, and a full set of mouse chromosomes, allowing easy mapping of individual genes to specific human chromosomes. See, e.g., D'Eustachio, et al., 1983. Science 220: 919-924. Somatic cell hybrids containing only fragments of human chromosomes can also be produced by using human chromosomes with translocations and deletions.

PCR mapping of somatic cell hybrids is a rapid procedure for assigning a particular sequence to a particular chromosome. Three or more sequences can be assigned per day using a single thermal cycler. Using the NOVX sequences to design oligonucleotide primers, sub-localization can be achieved with panels of fragments from specific chromosomes.

Fluorescence *in situ* hybridization (FISH) of a DNA sequence to a metaphase chromosomal spread can further be used to provide a precise chromosomal location in one step. Chromosome spreads can be made using cells whose division has been blocked in metaphase by a chemical like colcemid that disrupts the mitotic spindle. The chromosomes can be treated briefly with trypsin, and then stained with Giemsa. A pattern of light and dark bands develops on each chromosome, so that the chromosomes can be identified individually. The FISH technique can be used with a DNA sequence as short as 500 or 600 bases. However, clones larger than 1,000 bases have a higher likelihood of binding to a unique chromosomal location with sufficient signal intensity for simple detection. Preferably 1,000 bases, and more preferably 2,000 bases, will suffice to get good results at a reasonable amount of time. For a review of this technique, *see,* Verma, et al., HUMAN CHROMOSOMES: A MANUAL OF BASIC TECHNIQUES (Pergamon Press, New York 1988).

Reagents for chromosome mapping can be used individually to mark a single chromosome or a single site on that chromosome, or panels of reagents can be used for marking multiple sites and/or multiple chromosomes. Reagents corresponding to noncoding regions of the genes actually are preferred for mapping purposes. Coding sequences are more likely to be conserved within gene families, thus increasing the chance of cross hybridizations during chromosomal mapping.

Once a sequence has been mapped to a precise chromosomal location, the physical position of the sequence on the chromosome can be correlated with genetic map data. Such data are found, *e.g.*, in McKusick, MENDELIAN INHERITANCE IN MAN, available on-line through Johns Hopkins University Welch Medical Library). The relationship between genes and disease, mapped to the same chromosomal region, can then be identified through linkage analysis (co-inheritance of physically adjacent genes), described in, *e.g.,* Egeland, et al., 1987. Nature, 325: 783-787.

Moreover, differences in the DNA sequences between individuals affected and unaffected with a disease associated with the NOVX gene, can be determined. If a mutation is observed in some or all of the affected individuals but not in any unaffected individuals, then the mutation is likely to be the causative agent of the particular disease. Comparison of affected and unaffected individuals generally involves first looking for structural alterations in the chromosomes, such as deletions or translocations that are visible from chromosome spreads or detectable using PCR based on that DNA sequence. Ultimately, complete sequencing of genes from several individuals can be performed to confirm the presence of a mutation and to distinguish mutations from polymorphisms.

**Tissue Typing**

The NOVX sequences of the invention can also be used to identify individuals from minute biological samples. In this technique, an individual's genomic DNA is digested with one or more restriction enzymes, and probed on a Southern blot to yield unique bands for identification. The sequences of the invention are useful as additional DNA markers for RFLP ("restriction fragment length polymorphisms," described in U.S. Patent No. 5,272,057).

Furthermore, the sequences of the invention can be used to provide an alternative technique that determines the actual base-by-base DNA sequence of selected portions of an individual's genome. Thus, the NOVX sequences described herein can be used to prepare two PCR primers from the 5'- and 3'-termini of the sequences. These primers can then be used to amplify an individual's DNA and subsequently sequence it.

Panels of corresponding DNA sequences from individuals, prepared in this manner, can provide unique individual identifications, as each individual will have a unique set of such DNA sequences due to allelic differences. The sequences of the invention can be used to obtain such identification sequences from individuals and from tissue. The NOVX sequences of the invention uniquely represent portions of the human genome. Allelic variation occurs to some degree in the coding regions of these sequences, and to a greater degree in the noncoding regions. It is estimated that allelic variation between individual humans occurs with a frequency of about once per each 500 bases. Much of the allelic variation is due to single nucleotide polymorphisms (SNPs), which include restriction fragment length polymorphisms (RFLPs).

Each of the sequences described herein can, to some degree, be used as a standard against which DNA from an individual can be compared for identification purposes. Because greater numbers of polymorphisms occur in the noncoding regions, fewer sequences are necessary to differentiate individuals. The noncoding sequences can comfortably provide positive individual identification with a panel of perhaps 10 to 1,000 primers that each yield a noncoding amplified sequence of 100 bases. If coding sequences, such as those of SEQ ID NO:$2n$-1, wherein $n$ is an integer between 1 and 12, are used, a more appropriate number of primers for positive individual identification would be 500-2,000.

**Predictive Medicine**

The invention also pertains to the field of predictive medicine in which diagnostic assays, prognostic assays, pharmacogenomics, and monitoring clinical trials are used for prognostic (predictive) purposes to thereby treat an individual prophylactically. Accordingly, one aspect of the invention relates to diagnostic assays for determining NOVX protein and/or nucleic acid expression as well as NOVX activity, in the context of a biological sample (*e.g.*, blood, serum, cells, tissue) to thereby determine whether an individual is afflicted with a disease or disorder, or is at risk of developing a disorder, associated with aberrant NOVX expression or activity. The disorders include metabolic disorders, diabetes, obesity, infectious disease, anorexia, cancer-associated cachexia, cancer, neurodegenerative disorders, Alzheimer's Disease, Parkinson's Disorder, immune disorders, and hematopoietic disorders, and the various dyslipidemias, metabolic disturbances associated with obesity, the metabolic syndrome X and wasting disorders associated with chronic diseases and various cancers. The invention also provides for prognostic (or predictive) assays for determining whether an individual is at risk of developing a disorder associated with NOVX protein, nucleic acid expression or activity. For example, mutations in a NOVX gene can be assayed in a biological sample. Such assays can be used for prognostic or predictive purpose to thereby prophylactically treat an individual prior to the onset of a disorder characterized by or associated with NOVX protein, nucleic acid expression, or biological activity.

Another aspect of the invention provides methods for determining NOVX protein, nucleic acid expression or activity in an individual to thereby select appropriate therapeutic or prophylactic agents for that individual (referred to herein as "pharmacogenomics"). Pharmacogenomics allows for the selection of agents (*e.g.,* drugs) for therapeutic or prophylactic treatment of an individual based on the genotype of the individual (*e.g.,* the genotype of the individual examined to determine the ability of the individual to respond to a particular agent.)

Yet another aspect of the invention pertains to monitoring the influence of agents (*e.g.*, drugs, compounds) on the expression or activity of NOVX in clinical trials.

These and other agents are described in further detail in the following sections.

**Diagnostic Assays**

An exemplary method for detecting the presence or absence of NOVX in a biological sample involves obtaining a biological sample from a test subject and contacting the biological sample with a compound or an agent capable of detecting NOVX protein or nucleic acid (*e.g.*, mRNA, genomic DNA) that encodes NOVX protein such that the presence of NOVX is detected in the biological sample. An agent for detecting NOVX mRNA or genomic DNA is a labeled nucleic acid probe capable of hybridizing to NOVX mRNA or genomic DNA. The nucleic acid probe can be, for example, a full-length NOVX nucleic acid, such as the nucleic acid of SEQ ID NO:$2n$-1, wherein $n$ is an integer between 1 and 12, or a portion thereof, such as an oligonucleotide of at least 15, 30, 50, 100, 250 or 500 nucleotides in length and sufficient to specifically hybridize under stringent conditions to NOVX mRNA or genomic DNA. Other suitable probes for use in the diagnostic assays of the invention are described herein.

An agent for detecting NOVX protein is an antibody capable of binding to NOVX protein, preferably an antibody with a detectable label. Antibodies can be polyclonal, or more preferably, monoclonal. An intact antibody, or a fragment thereof (*e.g*., Fab or F(ab')$_2$) can be used. The term "labeled", with regard to the probe or antibody, is intended to encompass direct labeling of the probe or antibody by coupling (*i.e.,* physically linking) a detectable substance to the probe or antibody, as well as indirect labeling of the probe or antibody by reactivity with another reagent that is directly labeled. Examples of indirect labeling include detection of a primary antibody using a fluorescently-labeled secondary antibody and end-labeling of a DNA probe with biotin such that it can be detected with fluorescently-labeled streptavidin. The term "biological sample" is intended to include tissues, cells and biological fluids isolated from a subject, as well as tissues, cells and fluids present within a subject. That is, the detection method of the invention can be used to detect NOVX mRNA, protein, or genomic DNA in a biological sample *in vitro* as well as *in vivo.* For example, *in vitro* techniques for detection of NOVX mRNA include Northern hybridizations and *in situ* hybridizations. *In vitro* techniques for detection of NOVX protein include enzyme linked immunosorbent assays (ELISAs), Western blots, immunoprecipitations, and immunofluorescence. *In vitro* techniques for detection of NOVX genomic DNA include Southern hybridizations. Furthermore, *in vivo* techniques for detection of NOVX protein include introducing into a subject a labeled anti-NOVX antibody. For example, the antibody can be labeled with a radioactive marker whose presence and location in a subject can be detected by standard imaging techniques.

In one embodiment, the biological sample contains protein molecules from the test subject. Alternatively, the biological sample can contain mRNA molecules from the test subject or genomic DNA molecules from the test subject. A preferred biological sample is a peripheral blood leukocyte sample isolated by conventional means from a subject.

In another embodiment, the methods further involve obtaining a control biological sample from a control subject, contacting the control sample with a compound or agent capable of detecting NOVX protein, mRNA, or genomic DNA, such that the presence of NOVX protein, mRNA or genomic DNA is detected in the biological sample, and comparing the presence of NOVX protein, mRNA or genomic DNA in the control sample with the presence of NOVX protein, mRNA or genomic DNA in the test sample.

The invention also encompasses kits for detecting the presence of NOVX in a biological sample. For example, the kit can comprise: a labeled compound or agent capable of detecting NOVX protein or mRNA in a biological sample; means for determining the amount of NOVX in the sample; and means for comparing the amount of NOVX in the sample with a standard. The compound or agent can be packaged in a suitable container. The kit can further comprise instructions for using the kit to detect NOVX protein or nucleic acid.

**Prognostic Assays**

The diagnostic methods described herein can furthermore be utilized to identify subjects having or at risk of developing a disease or disorder associated with aberrant NOVX expression or activity. For example, the assays described herein, such as the preceding diagnostic assays or the following assays, can be utilized to identify a subject having or at risk of developing a disorder associated with NOVX protein, nucleic acid expression or activity. Alternatively, the prognostic assays can be utilized to identify a subject having or at risk for developing a disease or disorder. Thus, the invention provides a method for identifying a disease or disorder associated with aberrant NOVX expression or activity in which a test sample is obtained from a subject and NOVX protein or nucleic acid (*e.g.*, mRNA, genomic DNA) is detected, wherein the presence of NOVX protein or nucleic acid is diagnostic for a subject having or at risk of developing a disease or disorder associated with aberrant NOVX expression or activity. As used herein, a "test sample" refers to a biological sample obtained from a subject of interest. For example, a test sample can be a biological fluid (*e.g.*, serum), cell sample, or tissue.

Furthermore, the prognostic assays described herein can be used to determine whether a subject can be administered an agent (*e.g.*, an agonist, antagonist, peptidomimetic, protein, peptide, nucleic acid, small molecule, or other drug candidate) to treat a disease or disorder associated with aberrant NOVX expression or activity. For example, such methods can be used to determine whether a subject can be effectively treated with an agent for a disorder. Thus, the invention provides methods for determining whether a subject can be effectively treated with an agent for a disorder associated with aberrant NOVX expression or activity in which a test sample is obtained and NOVX protein or nucleic acid is detected (*e.g.*, wherein the presence of NOVX protein or nucleic acid is diagnostic for a subject that can be administered the agent to treat a disorder associated with aberrant NOVX expression or activity).

The methods of the invention can also be used to detect genetic lesions in a NOVX gene, thereby determining if a subject with the lesioned gene is at risk for a disorder characterized by aberrant cell proliferation and/or differentiation. In various embodiments, the methods include detecting, in a sample of cells from the subject, the presence or absence of a genetic lesion characterized by at least one of an alteration affecting the integrity of a gene encoding a NOVX-protein, or the misexpression of the NOVX gene. For example, such genetic lesions can be detected by ascertaining the existence of at least one of: (*i*) a deletion of one or more nucleotides from a NOVX gene; (*ii*) an addition of one or more nucleotides to a NOVX gene; (*iii*) a substitution of one or more nucleotides of a NOVX gene, (*iv*) a chromosomal rearrangement of a NOVX gene; (*v*) an alteration in the level of a messenger RNA transcript of a NOVX gene, (*vi*) aberrant modification of a NOVX gene, such as of the methylation pattern of the genomic DNA, (*vii*) the presence of a non-wild-type splicing pattern of a messenger RNA transcript of a NOVX gene, (*viii*) a non-wild-type level of a NOVX protein, (*ix*) allelic loss of a NOVX gene, and (x) inappropriate post-translational modification of a NOVX protein. As described herein, there are a large number of assay techniques known in the art which can be used for detecting lesions in a NOVX gene. A preferred biological sample is a peripheral blood leukocyte sample isolated by conventional means from a subject. However, any biological sample containing nucleated cells may be used, including, for example, buccal mucosal cells.

In certain embodiments, detection of the lesion involves the use of a probe/primer in a polymerase chain reaction (PCR) (*see, e.g.,* U.S. Patent Nos. 4,683,195 and 4,683,202), such as anchor PCR or RACE PCR, or, alternatively, in a ligation chain reaction (LCR) (*see, e.g.,* Landegran, et al., 1988. Science 241: 1077-1080; and Nakazawa, et al., 1994. Proc. Natl. Acad. Sci. USA 91: 360-364), the latter of which can be particularly useful for detecting point mutations in the NOVX-gene (*see,* Abravaya, et al., 1995. Nucl. Acids Res. 23: 675-682). This method can include the steps of collecting a sample of cells from a patient, isolating nucleic acid (*e.g.*, genomic, mRNA or both) from the cells of the sample, contacting the nucleic acid sample with one or more primers that specifically hybridize to a NOVX gene under conditions such that hybridization and amplification of the NOVX gene (if present) occurs, and detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein.

Alternative amplification methods include: self sustained sequence replication (*see,* Guatelli, et al., 1990. Proc. Natl. Acad. Sci. USA 87: 1874-1878), transcriptional amplification system *(see,* Kwoh, et al., 1989. Proc. Natl. Acad. Sci. USA 86: 1173-1177); Qβ Replicase *(see,* Lizardi, et al, 1988. BioTechnology 6: 1197), or any other nucleic acid amplification method, followed by the detection of the amplified molecules using techniques well known to those of skill in the art. These detection schemes are especially useful for the detection of nucleic acid molecules if such molecules are present in very low numbers.

In an alternative embodiment, mutations in a NOVX gene from a sample cell can be identified by alterations in restriction enzyme cleavage patterns. For example, sample and control DNA is isolated, amplified (optionally), digested with one or more restriction endonucleases, and fragment length sizes are determined by gel electrophoresis and compared. Differences in fragment length sizes between sample and control DNA indicates mutations in the sample DNA. Moreover, the use of sequence specific ribozymes (*see, e.g.,* U.S. Patent No. 5,493,531) can be used to score for the presence of specific mutations by development or loss of a ribozyme cleavage site.

In other embodiments, genetic mutations in NOVX can be identified by hybridizing a sample and control nucleic acids, *e.g.*, DNA or RNA, to high-density arrays containing hundreds or thousands of oligonucleotides probes. *See, e.g.,* Cronin, et al., 1996. Human Mutation 7: 244-255; Kozal, et al., 1996. Nat. Med. 2: 753-759. For example, genetic mutations in NOVX can be identified in two dimensional arrays containing light-generated DNA probes as described in Cronin, *et al., supra.* Briefly, a first hybridization array of probes can be used to scan through long stretches of DNA in a sample arid control to identify base changes between the sequences by making linear arrays of sequential overlapping probes. This step allows the identification of point mutations. This is followed by a second hybridization array that allows the characterization of specific mutations by using smaller, specialized probe arrays complementary to all variants or mutations detected. Each mutation array is composed of parallel probe sets, one complementary to the wild-type gene and the other complementary to the mutant gene.

In yet another embodiment, any of a variety of sequencing reactions known in the art can be used to directly sequence the NOVX gene and detect mutations by comparing the sequence of the sample NOVX with the corresponding wild-type (control) sequence. Examples of sequencing reactions include those based on techniques developed by Maxim and Gilbert, 1977. Proc. Natl. Acad. Sci. USA 74: 560 or Sanger, 1977. Proc. Natl. Acad. Sci. USA 74: 5463. It is also contemplated that any of a variety of automated sequencing procedures can be utilized when performing the diagnostic assays (*see, e.g.,* Naeve, et al., 1995. Biotechniques 19: 448), including sequencing by mass spectrometry (see, *e.g.,* PCT International Publication No. WO 94/16101; Cohen, et al., 1996. Adv. Chromatography 36: 127-162; and Griffin, et al., 1993. Appl. Biochem. Biotechnol. 38: 147-159).

Other methods for detecting mutations in the NOVX gene include methods in which protection from cleavage agents is used to detect mismatched bases in RNA/RNA or RNA/DNA heteroduplexes. *See, e.g.,* Myers, et al., 1985. Science 230: 1242. In general, the art technique of "mismatch cleavage" starts by providing heteroduplexes of formed by hybridizing (labeled) RNA or DNA containing the wild-type NOVX sequence with potentially mutant RNA or DNA obtained from a tissue sample. The double-stranded duplexes are treated with an agent that cleaves single-stranded regions of the duplex such as may exist due to basepair mismatches between the control and sample strands. For instance, RNA/DNA duplexes can be treated with RNase and DNA/DNA hybrids treated with $S_1$ nuclease to enzymatically digesting the mismatched regions. In other embodiments, either DNA/DNA or RNA/DNA duplexes can be treated with hydroxylamine or osmium tetroxide and with piperidine in order to digest mismatched regions. After digestion of the mismatched regions, the resulting material is then separated by size on denaturing polyacrylamide gels to determine the site of mutation. *See, e.g.,* Cotton, et al., 1988. Proc. Natl. Acad. Sci. USA 85: 4397; Saleeba, et al., 1992. Methods Enzymol. 217: 286-295. In an embodiment, the control DNA or RNA can be labeled for detection.

In still another embodiment, the mismatch cleavage reaction employs one or more proteins that recognize mismatched base pairs in double-stranded DNA (so called "DNA mismatch repair" enzymes) in defined systems for detecting and mapping point mutations in NOVX cDNAs obtained from samples of cells. For example, the mutY enzyme of *E. coli* cleaves A at G/A mismatches and the thymidine DNA glycosylase from HeLa cells cleaves T at G/T mismatches. *See, e.g.,* Hsu, et al., 1994. Carcinogenesis 15: 1657-1662. According to an exemplary embodiment, a probe based on a NOVX sequence, *e.g.*, a wild-type NOVX sequence, is hybridized to a cDNA or other DNA product from a test cell(s). The duplex is treated with a DNA mismatch repair enzyme, and the cleavage products, if any, can be detected from electrophoresis protocols or the *like. See, e.g.,* U.S. Patent No. 5,459,039.

In other embodiments, alterations in electrophoretic mobility can be used to identify mutations in NOVX genes. For example, single strand conformation polymorphism (SSCP) may be used to detect differences in electrophoretic mobility between mutant and wild type nucleic *acids. See, e.g.,* Orita, et al., 1989. Proc. Natl. Acad. Sci. USA: 86: 2766; Cotton, 1993. Mutat. Res. 285: 125-144; Hayashi, 1992. Genet. Anal. Tech. Appl. 9: 73-79. Single-stranded DNA fragments of sample and control NOVX nucleic acids will be denatured and allowed to renature. The secondary structure of single-stranded nucleic acids varies according to sequence, the resulting alteration in electrophoretic mobility enables the detection of even a single base change. The DNA fragments can be labeled or detected with labeled probes. The sensitivity of the assay can be enhanced by using RNA (rather than DNA), in which the secondary structure is more sensitive to a change in sequence. In one embodiment, the subject method utilizes heteroduplex analysis to separate double stranded heteroduplex molecules on the basis of changes in electrophoretic mobility. *See, e.g.,* Keen, et al., 1991. Trends Genet. 7: 5.

In yet another embodiment, the movement of mutant or wild-type fragments in polyacrylamide gels containing a gradient of denaturant is assayed using denaturing gradient gel electrophoresis (DGGE). *See, e.g.,* Myers, et al., 1985. Nature 313: 495. When DGGE is used as the method of analysis, DNA will be modified to insure that it does not completely denature, for example by adding a GC clamp of approximately 40 bp of high-melting GC-rich DNA by PCR. In a further embodiment, a temperature gradient is used in place of a denaturing gradient to identify differences in the mobility of control and sample DNA. *See, e.g.,* Rosenbaum and Reissner, 1987. Biophys. Chem. 265: 12753.

Examples of other techniques for detecting point mutations include, but are not limited to, selective oligonucleotide hybridization, selective amplification, or selective primer extension. For example, oligonucleotide primers can be prepared in which the known mutation is placed centrally and then hybridized to target DNA under conditions that permit hybridization only if a perfect match is found. *See, e.g.,* Saiki, et al., 1986. Nature 324: 163; Saiki, et al., 1989. Proc. Natl. Acad. Sci. USA 86: 6230. Such allele specific oligonucleotides are hybridized to PCR amplified target DNA or a number of different mutations when the oligonucleotides are attached to the hybridizing membrane and hybridized with labeled target DNA.

Alternatively, allele specific amplification technology that depends on selective PCR amplification can be used in conjunction with the instant invention. Oligonucleotides used as primers for specific amplification can carry the mutation of interest in the center of the molecule (so that amplification depends on differential hybridization; *see, e.g.,* Gibbs, et al., 1989. Nucl. Acids Res. 17: 2437-2448) or at the extreme 3'-terminus of one primer where, under appropriate conditions, mismatch can prevent, or reduce polymerase extension *(see, e.g.,* Prossner, 1993. Tibtech. 11: 238). In addition it may be desirable to introduce a novel restriction site in the region of the mutation to create cleavage-based detection. *See, e.g.,* Gasparini, et al., 1992. Mol. Cell Probes 6: 1. It is anticipated that in certain embodiments amplification may also be performed using *Taq* ligase for amplification. *See, e.g.,* Barany, 1991. Proc. Natl. Acad. Sci. USA 88: 189. In such cases, ligation will occur only if there is a perfect match at the 3'-terminus of the 5' sequence, making it possible to detect the presence of a known mutation at a specific site by looking for the presence or absence of amplification.

The methods described herein may be performed, for example, by utilizing pre-packaged diagnostic kits comprising at least one probe nucleic acid or antibody reagent described herein, which may be conveniently used, *e.g.*, in clinical settings to diagnose patients exhibiting symptoms or family history of a disease or illness involving a NOVX gene.

Furthermore, any cell type or tissue, preferably peripheral blood leukocytes, in which NOVX is expressed can be utilized in the prognostic assays described herein. However, any biological sample containing nucleated cells may be used, including, for example, buccal mucosal cells.

**Pharmacogenomics**

Agents, or modulators that have a stimulatory or inhibitory effect on NOVX activity (*e.g.*, NOVX gene expression), as identified by a screening assay described herein can be administered to individuals to treat (prophylactically or therapeutically) disorders. The disorders include but are not limited to, *e.g.,* those diseases, disorders and conditions listed above, and more particularly include those diseases, disorders, or conditions associated with homologs of a NOVX protein, such as those summarized in Table A.

In conjunction with such treatment, the pharmacogenomics (*i.e.,* the study of the relationship between an individual's genotype and that individual's response to a foreign compound or drug) of the individual may be considered. Differences in metabolism of therapeutics can lead to severe toxicity or therapeutic failure by altering the relation between dose and blood concentration of the pharmacologically active drug. Thus, the pharmacogenomics of the individual permits the selection of effective agents (*e.g.,* drugs) for prophylactic or therapeutic treatments based on a consideration of the individual's genotype. Such pharmacogenomics can further be used to determine appropriate dosages and therapeutic regimens. Accordingly, the activity of NOVX protein, expression of NOVX nucleic acid, or mutation content of NOVX genes in an individual can be determined to thereby select appropriate agent(s) for therapeutic or prophylactic treatment of the individual.

Pharmacogenomics deals with clinically significant hereditary variations in the response to drugs due to altered drug disposition and abnormal action in affected persons. See *e.g.,* Eichelbaum, 1996. Clin. Exp. Pharmacol. Physiol., 23: 983-985; Linder, 1997. Clin. Chem., 43: 254-266. In general, two types of pharmacogenetic conditions can be differentiated. Genetic conditions transmitted as a single factor altering the way drugs act on the body (altered drug action) or genetic conditions transmitted as single factors altering the way the body acts on drugs (altered drug metabolism). These pharmacogenetic conditions can occur either as rare defects or as polymorphisms. For example, glucose-6-phosphate dehydrogenase (G6PD) deficiency is a common inherited enzymopathy in which the main clinical complication is hemolysis after ingestion of oxidant drugs (anti-malarials, sulfonamides, analgesics, nitrofurans) and consumption of fava beans.

As an illustrative embodiment, the activity of drug metabolizing enzymes is a major determinant of both the intensity and duration of drug action. The discovery of genetic polymorphisms of drug metabolizing enzymes (*e.g.*, N-acetyltransferase 2 (NAT 2) and cytochrome pregnancy zone protein precursor enzymes CYP2D6 and CYP2C 19) has provided an explanation as to why some patients do not obtain the expected drug effects or show exaggerated drug response and serious toxicity after taking the standard and safe dose of a drug. These polymorphisms are expressed in two phenotypes in the population, the extensive metabolizer (EM) and poor metabolizer (PM). The prevalence of PM is different among different populations. For example, the gene coding for CYP2D6 is highly polymorphic and several mutations have been identified in PM, which all lead to the absence of functional CYP2D6. Poor metabolizers of CYP2D6 and CYP2C19 quite frequently experience exaggerated drug response and side effects when they receive standard doses. If a metabolite is the active therapeutic moiety, PM show no therapeutic response, as demonstrated for the analgesic effect of codeine mediated by its CYP2D6-formed metabolite morphine. At the other extreme are the so called ultra-rapid metabolizers who do not respond to standard doses. Recently, the molecular basis of ultra-rapid metabolism has been identified to be due to CYP2D6 gene amplification.

Thus, the activity of NOVX protein, expression of NOVX nucleic acid, or mutation content of NOVX genes in an individual can be determined to thereby select appropriate agent(s) for therapeutic or prophylactic treatment of the individual. In addition, pharmacogenetic studies can be used to apply genotyping of polymorphic alleles encoding drug-metabolizing enzymes to the identification of an individual's drug responsiveness phenotype. This knowledge, when applied to dosing or drug selection, can avoid adverse reactions or therapeutic failure and thus enhance therapeutic or prophylactic efficiency when treating a subject with a NOVX modulator, such as a modulator identified by one of the exemplary screening assays described herein.

## Monitoring of Effects During Clinical Trials

Monitoring the influence of agents (*e.g.,* drugs, compounds) on the expression or activity of NOVX (*e.g.,* the ability to modulate aberrant cell proliferation and/or differentiation) can be applied not only in basic drug screening, but also in clinical trials. For example, the effectiveness of an agent determined by a screening assay as described herein to increase NOVX gene expression, protein levels, or upregulate NOVX activity, can be monitored in clinical trails of subjects exhibiting decreased NOVX gene expression, protein levels, or downregulated NOVX activity. Alternatively, the effectiveness of an agent determined by a screening assay to decrease NOVX gene expression, protein levels, or downregulate NOVX activity, can be monitored in clinical trails of subjects exhibiting increased NOVX gene expression, protein levels, or upregulated NOVX activity. In such clinical trials, the expression or activity of NOVX and, preferably, other genes that have been implicated in, for example, a cellular proliferation or immune disorder can be used as a "read out" or markers of the immune responsiveness of a particular cell.

By way of example, and not of limitation, genes, including NOVX, that are modulated in cells by treatment with an agent (*e.g.*, compound, drug or small molecule) that modulates NOVX activity (*e.g.*, identified in a screening assay as described herein) can be identified. Thus, to study the effect of agents on cellular proliferation disorders, for example, in a clinical trial, cells can be isolated and RNA prepared and analyzed for the levels of expression of NOVX and other genes implicated in the disorder. The levels of gene expression (*i.e.,* a gene expression pattern) can be quantified by Northern blot analysis or RT-PCR, as described herein, or alternatively by measuring the amount of protein produced, by one of the methods as described herein, or by measuring the levels of activity of NOVX or other genes. In this manner, the gene expression pattern can serve as a marker, indicative of the physiological response of the cells to the agent. Accordingly, this response state can be determined before, and at various points during, treatment of the individual with the agent.

In one embodiment, the invention provides a method for monitoring the effectiveness of treatment of a subject with an agent (*e.g.*, an agonist, antagonist, protein, peptide, peptidomimetic, nucleic acid, small molecule, or other drug candidate identified by the screening assays described herein) comprising the steps of (i) obtaining a pre-administration sample from a subject prior to administration of the agent; (*ii*) detecting the level of expression of a NOVX protein, mRNA, or genomic DNA in the preadministration sample; (*iii*) obtaining one or more post-administration samples from the subject; (*iv*) detecting the level of expression or activity of the NOVX protein, mRNA, or genomic DNA in the post-administration samples; (*v*) comparing the level of expression or activity of the NOVX protein, mRNA, or genomic DNA in the pre-administration sample with the NOVX protein, mRNA, or genomic DNA in the post administration sample or samples; and (*vi*) altering the administration of the agent to the subject accordingly. For example, increased administration of the agent may be desirable to increase the expression or activity of NOVX to higher levels than detected, *i.e.,* to increase the effectiveness of the agent. Alternatively, decreased administration of the agent may be desirable to decrease expression or activity of NOVX to lower levels than detected, *i.e.*, to decrease the effectiveness of the agent.

## Methods of Treatment

The invention provides for both prophylactic and therapeutic methods of treating a subject at risk of (or susceptible to) a disorder or having a disorder associated with aberrant NOVX expression or activity. The disorders include but are not limited to, *e.g.,* those diseases, disorders and conditions listed above, and more particularly include those diseases, disorders, or conditions associated with homologs of a NOVX protein, such as those summarized in Table A.

These methods of treatment will be discussed more fully, below.

### Diseases and Disorders

Diseases and disorders that are characterized by increased (relative to a subject not suffering from the disease or disorder) levels or biological activity can be treated with Therapeutics that antagonize (*i.e.,* reduce or inhibit) activity. Therapeutics that antagonize activity can be administered in a therapeutic or prophylactic manner. Therapeutics that can be utilized include, but are not limited to: (*i*) an aforementioned peptide, or analogs, derivatives, fragments or homologs thereof; (*ii*) antibodies to an aforementioned peptide; (*iii*) nucleic acids encoding an aforementioned peptide; (*iv*) administration of antisense nucleic acid and nucleic acids that are "dysfunctional" (*i.e.,* due to a heterologous insertion within the coding sequences of coding sequences to an aforementioned peptide) that are utilized to "knockout" endogenous function of an aforementioned peptide by homologous recombination (*see, e.g.,* Capecchi, 1989. Science 244: 1288-1292); or (*v*) modulators ( *i.e.,* inhibitors, agonists and antagonists, including additional peptide mimetic of the invention or antibodies specific to a peptide of the invention) that alter the interaction between an aforementioned peptide and its binding partner.

Diseases and disorders that are characterized by decreased (relative to a subject not suffering from the disease or disorder) levels or biological activity can be treated with Therapeutics that increase (*i.e.,* are agonists to) activity. Therapeutics that upregulate activity can be administered in a therapeutic or prophylactic manner. Therapeutics that can be utilized include, but are not limited to, an aforementioned peptide, or analogs, derivatives, fragments or homologs thereof; or an agonist that increases bioavailability.

Increased or decreased levels can be readily detected by quantifying peptide and/or RNA, by obtaining a patient tissue sample (*e.g.,* from biopsy tissue) and assaying it *in vitro* for RNA or peptide levels, structure and/or activity of the expressed peptides (or mRNAs of an aforementioned peptide). Methods that are well-known within the art include, but are not limited to, immunoassays (*e.g.,* by Western blot analysis, immunoprecipitation followed by sodium dodecyl sulfate (SDS) polyacrylamide gel electrophoresis, immunocytochemistry, *etc.*) and/or hybridization assays to detect expression of mRNAs (*e.g.,* Northern assays, dot blots, *in situ* hybridization, and the like).

### Prophylactic Methods

In one aspect, the invention provides a method for preventing, in a subject, a disease or condition associated with an aberrant NOVX expression or activity, by administering to the subject an agent that modulates NOVX expression or at least one NOVX activity. Subjects at risk for a disease that is caused or contributed to by aberrant NOVX expression or activity can be identified by, for example, any or a combination of diagnostic or prognostic assays as described herein. Administration of a prophylactic agent can occur prior to the manifestation of symptoms characteristic of the NOVX aberrancy, such that a disease or disorder is prevented or, alternatively, delayed in its progression. Depending upon the type of NOVX aberrancy, for example, a NOVX agonist or NOVX antagonist agent can be used for treating the subject. The appropriate agent can be determined based on screening assays described herein. The prophylactic methods of the invention are further discussed in the following subsections.

### Therapeutic Methods

Another aspect of the invention pertains to methods of modulating NOVX expression or activity for therapeutic purposes. The modulatory method of the invention involves contacting a cell with an agent that modulates one or more of the activities of NOVX protein activity associated with the cell. An agent that modulates NOVX protein activity can be an agent as described herein, such as a nucleic acid or a protein, a naturally-occurring cognate ligand of a NOVX protein, a peptide, a NOVX peptidomimetic, or other small molecule. In one embodiment, the agent stimulates one or more NOVX protein activity. Examples of such stimulatory agents include active NOVX protein and a nucleic acid molecule encoding NOVX that has been introduced into the cell. In another embodiment, the agent inhibits one or more NOVX protein activity. Examples of such inhibitory agents include antisense NOVX nucleic acid molecules and anti-NOVX antibodies. These modulatory methods can be performed *in vitro* (*e.g.,* by culturing the cell with the agent) or, alternatively, *in vivo* (*e.g.,* by administering the agent to a subject). As such, the invention provides methods of treating an individual afflicted with a disease or disorder characterized by aberrant expression or activity of a NOVX protein or nucleic acid molecule. In one embodiment, the method involves administering an agent (*e.g.*, an agent identified by a screening assay described herein), or combination of agents that modulates (*e.g.*, up-regulates or down-regulates) NOVX expression or activity. In another embodiment, the method involves administering a NOVX protein or nucleic acid molecule as therapy to compensate for reduced or aberrant NOVX expression or activity.

Stimulation of NOVX activity is desirable *in situ*ations in which NOVX is abnormally downregulated and/or in which increased NOVX activity is likely to have a beneficial effect. One example of such a situation is where a subject has a disorder characterized by aberrant cell proliferation and/or differentiation (*e.g.*, cancer or immune associated disorders). Another example of such a situation is where the subject has a gestational disease (*e.g.*, preclampsia).

**Determination of the Biological Effect of the Therapeutic**

In various embodiments of the invention, suitable *in vitro* or *in vivo* assays are performed to determine the effect of a specific Therapeutic and whether its administration is indicated for treatment of the affected tissue.

In various specific embodiments, *in vitro* assays can be performed with representative cells of the type(s) involved in the patient's disorder, to determine if a given Therapeutic exerts the desired effect upon the cell type(s). Compounds for use in therapy can be tested in suitable animal model systems including, but not limited to rats, mice, chicken, cows, monkeys, rabbits, and the like, prior to testing in human subjects. Similarly, for *in vivo* testing, any of the animal model system known in the art may be used prior to administration to human subjects.

**Prophylactic and Therapeutic Uses of the Compositions of the Invention**

The NOVX nucleic acids and proteins of the invention are useful in potential prophylactic and therapeutic applications implicated in a variety of disorders. The disorders include but are not limited to, *e.g.*, those diseases, disorders and conditions listed above, and more particularly include those diseases, disorders, or conditions associated with homologs of a NOVX protein, such as those summarized in Table A.

As an example, a cDNA encoding the NOVX protein of the invention may be useful in gene therapy, and the protein may be useful when administered to a subject in need thereof. By way of non-limiting example, the compositions of the invention will have efficacy for treatment of patients suffering from diseases, disorders, conditions and the like, including but not limited to those listed herein.

Both the novel nucleic acid encoding the NOVX protein, and the NOVX protein of the invention, or fragments thereof, may also be useful in diagnostic applications, wherein the presence or amount of the nucleic acid or the protein are to be assessed. A further use could be as an anti-bacterial molecule (*i.e.,* some peptides have been found to possess anti-bacterial properties). These materials are further useful in the generation of antibodies, which immunospecifically-bind to the novel substances of the invention for use in therapeutic or diagnostic methods.

The invention will be further described in the following examples, which do not limit the scope of the invention described in the claims.

**EXAMPLES**

**Example 1. Molecular Cloning of CG57008**

The open reading frame of CG57008 (SEQ ID NO: 4) codes for a 359 amino acid long, Type I transmembrane protein with a predicted N-terminal signal sequence represented by the first 20 residues. The predicted transmembrane domain starts at residue 287.

**Cloning the mature CG57008**

Oligonucleotide primers were designed to PCR amplify a DNA segment, representing an ORF, coding for the mature form of CG57008 (NOV1). The forward primer includes, a BamHI restriction site while the reverse primer contains an, in frame, XhoI restriction site for further subcloning purposes. The sequences of the primers are shown below:

HAVcr-1 FORW:

GGATCCTCTGTAAAGGTTGGTGGAGAGGCAGGTCC (SEQ ID NO:25),

HAVcr-1 FL-REV:

CTCGAGGTCCGTGGCATAAAGACTATTCTCAATG (SEQ ID NO:26).

PCR reactions were set up using a total of 5 ng cDNA template containing equal parts of cDNA samples derived from human testis, human mammary, human skeletal muscle , and fetal brain; 1 microM of each of the HAVcr-1 FORW and HAVcr-1 FL-REV primers, 5 micromoles dNTP (Clontech Laboratories, Palo Alto CA) and 1 microliter of 50xAdvantage-HF 2 polymerase (Clontech Laboratories, Palo Alto CA) in 50 microliter volume. The following reaction conditions were used:

a) 96°C 3 minutes
b) 96°C 30 seconds denaturation
c) 70°C 30 seconds, primer annealing. This temperature was gradually decreased by 1°C/cycle
d) 72°C 2 minute extension.
Repeat steps b-d 10 times
e) 96°C 30 seconds denaturation
f) 60°C 30 seconds annealing
g) 72°C 2 minute extension
Repeat steps e-g 25 times
h) 72°C 5 minutes final extension

An approximately 1 kbp large amplified product was isolated from agarose gel and ligated to pCR2.1 vector (Invitrogen, Carlsbad, CA). The cloned insert was sequenced, using vector specific, M13 Forward (-40) and M13 Reverse primers and verified as an open reading frame coding for the mature form of CG57008. The clone is called pCR2.1-CG57008-03-S843_15B.

### Table 35. The nucleotide sequence of the insert in pCR2.1- CG57008-03-S843_15B

```
TCTGTAAAGGTTGGTGGAGAGGCAGGTCCATCTGTCACACTACCCTGCCACTACAGTGGA
GCTGTCACATCAATGTGCTGGAATAGAGGCTCATGTTCTCTATTCACATGCCAAAATGGC
ATTGTCTGGACCAATGGAACCCACGTCACCTATCGGAAGGACACACGCTATAAGCTATTG
GGGGACCTTTCAAGAAGGGATGTCTCTTTGACCATAGAAAATACAGCTGTGTCTGACAGT
GGCGTATATTGTTGCCGTGTTGAGCACCGTGGGTGGTTCAATGACATGAAAATCACCGTA
TCATTGGAGATTGTGCCACCCAAGGTCACGACTACTCCAATTGTCACAACTGTTCCAACC
GTCACGACTGTTCGAACGAGCACCACTGTTCCAACGACAACGACTGTTCCAACGACAACT
GTTCCAACAACAATGAGCATTCCAACGACAACGACTGTTCCGACGACAATGACTGTTTCA
ACGACAACGAGCGTTCCAACGACAACGAGCATTCCAACAACAACAAGTGTTCCAGTGACA
ACAACGGTCTCTACCTTTGTTCCTCCAATGCCTTTGCCCAGGCAGAACCATGAACCAGTA
GCCACTTCACCATCTTCACCTCAGCCAGCAGAAACCCACCCTACGACACTGCAGGGAGCA
ATAAGGAGAGAACCCACCAGCTCACCATTGTACTCTTACACAACAGATGGGAATGACACC
GTGACAGAGTCTTCAGATGGCCTTTGGAATAACAATCAAACTCAACTGTTCCTAGAACAT
AGTCTACTGACGGCCAATACCACTAAAGGAATCTATGCTGGAGTCTGTATTTCTGTCTTG
GTGCTTCTTGCTCTTTTGGGTGTCATCATTGCCAAAAAGTATTTCTTCAAAAAGGAGGTT
CAACAACTAAGTGTTTCATTTAGCAGCCTTCAAATTAAAGCTTTGCAAAATGCAGTTGAA
AAGGAAGTCCAAGCAGAAGACAATATCTACATTGAGAATAGTCTTTATGCCACGGAC     (SEQ ID NO:27)
```

### Table 36. The polypeptide sequence of the protein coded by the insert in pCR2.1-CG57008-03-S843_15B

```
SVKVGGEAGPSVTLPCHYSGAVTSMCWNRGSCSLFTCQNGIVWTNGTHVTYRKDTRYKLL
GDLSRRDVSLTIENTAVSDSGVYCCRVEHRGWFNDMKITVSLEIVPPKVTTTPIVTTVPT
VTTVRTSTTVPTTTTVPTTTVPTTMSIPTTTTVPTTMTVSTTTSVPTTTSIPTTTSVPVT
TTVSTFVPPMPLPRQNHEPVATSPSSPQPAETHPTTLQGAIRREPTSSPLYSYTTDGNDT
VTESSDGLWNNNQTQLFLEHSLLTANTTKGIYAGVCISVLVLLALLGVIIAKKYFFKKEV
QQLSVSFSSLQIKALQNAVEKEVQAEDNIYIENSLYATD     (SEQ ID NO:28)
```

## Example 2. Cloning of the extracellular domain of CG57008

Oligonucleotide primers were designed to PCR amplify a DNA segment, representing an ORF, coding for the mature form of the extracellular domain of CG57008 (NOV1), between residues 21 and 286. The forward primer includes, a BamHI restriction site while the reverse primer contains an in-frame, XhoI restriction site for further subcloning purposes. The sequences of the primers are the following:

HAVcr-1 FORW:

GGATCCTCTGTAAAGGTTGGTGGAGAGGCAGGTCC (SEQ ID NO:29),

HAVcr-1 SolubleREV:

CTCGAGCAGTAGACTATGTTCTAGGAACAGTTGAG (SEQ ID NO:30).

PCR reactions were set up using a total of 5 ng cDNA template containing equal parts of cDNA samples derived from human testis, human mammary, human skeletal muscle, and fetal brain; 1 μM of each of the HAVcr-1 FORW and HAVcr-1 SolubleREV primers, 5 micromoles dNTP (Clontech Laboratories, Palo Alto CA) and 1 microliter of 50xAdvantage-HF 2 polymerase (Clontech Laboratories, Palo Alto CA) in 50 microliter volume. The following reaction conditions were used:

a) 96°C 3 minutes
b) 96°C 30 seconds denaturation
c) 70°C 30 seconds, primer annealing. This temperature was gradually decreased by 1°C/cycle
d) 72°C 2 minutes extension.
Repeat steps b-d 10 times
e) 96°C 30 seconds denaturation
f) 60°C 30 seconds annealing
g) 72°C 2 minutes extension
Repeat steps e-g 25 times
h) 72°C 5 minutes final extension

An approximately 750 bp large amplified product was isolated from agarose gel and ligated to pCR2.1 vector (Invitrogen, Carlsbad, CA). The cloned insert was sequenced, using vector specific, M13 Forward(-40) and M13 Reverse primers and verified as an open reading frame coding for the mature form of the extracellular domain of CG57008. The clone is called pCR2.1- CG57008-02-S841_13A.

### Table 3. The nucleotide sequence of the insert in pCR2.1- CG57008-02-S841_13A.

```
TCTGTAAAGGTTGGTGGAGAGGCAGGTCCATCTGTCACACTACCCTGCCACTACAGTGGA
GCTGTCACATCAATGTGCTGGAATAGAGGCTCATGTTCTCTATTCACATGCCAAAATGGC
ATTGTCTGGACCAATGGAACCCACGTCACCTATCGGAAGGACACACGCTATAAGCTATTG
GGGGACCTTTCAAGAAGGGATGTCTCTTTGACCATAGAAAATACAGCTGTGTCTGACAGT
GGCGTATATTGTTGCCGTGTTGAGCACCGTGGGTGGTTCAATGACATGAAAATCACCGTA
TCATTGGAGATTGTGCCACCCAAGGTCACGACTACTCCAATTGTCACAACTGTTCCAACC
GTCACGACTGTTCGAACGAGCACCACTGTTCCAACGACAACGACTGTTCCAACGACAACT
GTTCCAACAACAATGAGCATTCCAACGACAACGACTGTTCCGACGACAATGACTGTTTCA
ACGACAACGAGCGTTCCAACGACAACGAGCATTCCAACAACAACAAGTGTTCCAGTGACA
ACAACGGTCTCTACCTTTGTTCCTCCAATGCCTTTGCCCAGGCAGAACCATGAACCAGTA
GCCACTTCACCATCTTCACCTCAGCCAGCAGAAACCCACCCTACGACACTGCAGGGAGCA
ATAAGGAGAGAACCCACCAGCTCACCATTGTACTCTTACACAACAGATGGGAATGACACC
GTGACAGAGTCTTCAGATGGCCTTTGGAATAACAATCAAACTCAACTGTTCCTAGAACAT
AGTCTACTG    (SEQ ID NO:31)
```

### Table 4. The polypeptide sequence of the protein coded by the insert in  pCR2.1- CG57008-02-S841_13A

```
SVKVGGEAGPSVTLPCHYSGAVTSMCWNRGSCSLFTCQNGIVWTNGTHVTYRKDTRYKLL
GDLSRRDVSLTIENTAVSDSGVYCCRVEHRGWFNDMKITVSLEIVPPKVTTTPIVTTVPT
VTTVRTSTTVPTTTTVPTTTVPTTMSIPTTTTVPTTMTVSTTTSVPTTTSIPTTTSVPVT
TTVSTFVPPMPLPRQNHEPVATSPSSPQPAETHPTTLQGAIRREPTSSPLYSYTTDGNDT
VTESSDGLWNNNQTQLFLEHSLL    (SEQ ID NO:32)
```

### Example 3. Expression of CG57008-02 in Insect Cells

A 0.79 kb BamHI-XhoI fragment containing the CG57008-02 (NOV1c) sequence was subcloned to the baculovirus expression vector, pMe1V5His (CuraGen Corporation) to generate plasmid 646. Expression studies were performed using the pBlueBac baculovirus expression system (Invitrogen Corporation) following the manufacturer's recommendation. The conditioned media was analyzed 48 hours after post-infection by Western blot using an anti-V5 antibody (Invitrogen).

**Example 4. Expression of CG57008-02 in human embryonic kidney 293 cells**

A 0.79 kb BamHI-XhoI fragment containing the CG57008-02 (NOV1c) sequence was subcloned into BamHI-XhoI digested pCEP4/Sec to generate plasmid 754. The resulting plasmid 754 was transfected into 293 cells using the LipofectaminePlus reagent following the manufacturer's instructions (Gibco/BRL). The cell pellet and supernatant were harvested 72h post transfection and examined for CG57008-02 expression by Western blot (reducing conditions) using an anti-V5 antibody.

**Example 5. Expression of CG57008-02 in human embryonic kidney 293 cells**

The insert from pCR2.1- CG57008-02-S841_13A (see Example 2) was subcloned to the pEE14.4Sec mammalian expression vector. The vector carries the glutamine synthase selective marker that allows the selection of stable clones in the presence of methionine sulfoximine. Following selection, stable clones were established. The CG57008-02 expression and secretion, in two independent clones, was demonstrated by Western blot analysis.

**Example 6. Identification of Single Nucleotide Polymorphisms in NOVX nucleic acid sequences**

Variant sequences are also included in this application. A variant sequence can include a single nucleotide polymorphism (SNP). A SNP can, in some instances, be referred to as a "cSNP" to denote that the nucleotide sequence containing the SNP originates as a cDNA. A SNP can arise in several ways. For example, a SNP can be due to a substitution of one nucleotide for another at the polymorphic site. Such a substitution can be either a transition or a transversion. A SNP can also arise from a deletion of a nucleotide or an insertion of a nucleotide, relative to a reference allele. In this case, the polymorphic site is a site at which one allele bears a gap with respect to a particular nucleotide in another allele. SNPs occurring within genes can result in an alteration of the amino acid encoded by the gene at the position of the SNP. Intragenic SNPs can also be silent, when a codon including a SNP encodes the same amino acid as a result of the redundancy of the genetic code. SNPs occurring outside the region of a gene, or in an intron within a gene, do not result in changes in any amino acid sequence of a protein but can result in altered regulation of the expression pattern. Examples include alteration in temporal expression, physiological response regulation, cell type expression regulation, intensity of expression, and stability of transcribed message.

SeqCalling assemblies produced by the exon linking process were selected and extended using the following criteria. Genomic clones having regions with 98% identity to all or part of the initial or extended sequence were identified by BLASTN searches using the relevant sequence to query human genomic databases. The genomic clones that resulted were selected for further analysis because this identity indicates that these clones contain the genomic locus for these SeqCalling assemblies. These sequences were analyzed for putative coding regions as well as for similarity to the known DNA and protein sequences. Programs used for these analyses include Grail, Genscan, BLAST, HMMER, FASTA, Hybrid and other relevant programs.

Some additional genomic regions may have also been identified because selected SeqCalling assemblies map to those regions. Such SeqCalling sequences may have overlapped with regions defined by homology or exon prediction. They may also be included because the location of the fragment was in the vicinity of genomic regions identified by similarity or exon prediction that had been included in the original predicted sequence. The sequence so identified was manually assembled and then may have been extended using one or more additional sequences taken from CuraGen Corporation's human SeqCalling database. SeqCalling fragments suitable for inclusion were identified by the CuraTools™ program SeqExtend or by identifying SeqCalling fragments mapping to the appropriate regions of the genomic clones analyzed.

The regions defined by the procedures described above were then manually integrated and corrected for apparent inconsistencies that may have arisen, for example, from miscalled bases in the original fragments or from discrepancies between predicted exon junctions, EST locations and regions of sequence similarity, to derive the final sequence disclosed herein. When necessary, the process to identify and analyze SeqCalling assemblies and genomic clones was reiterated to derive the full length sequence (Alderborn et al., Determination of Single Nucleotide Polymorphisms by Real-time Pyrophosphate DNA Sequencing. Genome Research. 10 (8) 1249-1265, 2000).

Variants are reported individually but any combination of all or a select subset of variants are also included as contemplated NOVX embodiments of the invention.

**SNP data for CG57008-02:**

Four polymorphic variants of CG57008-02 have been identified and are shown below.

**Table B:**

| Variant | Nucleotides | | | Amino Acids | | |
|---------|-------------|---------|----------|-------------|---------|----------|
| | Position | Initial | Modified | Position | Initial | Modified |
| 13382206 | 203 | C | T | 51 | Ser | Leu |
| 13382207 | 338 | C | T | 96 | Ala | Val |
| 13378266 | 572 | C | T | 174 | Pro | Leu |
| 13378265 | 655 | A | G | 202 | Thr | Ala |

**Example 7. Quantitative expression analysis of CG57008-02 in various cells and tissues**

The quantitative expression of various clones was assessed using microtiter plates containing RNA samples from a variety of normal and pathology-derived cells, cell lines and tissues using real time quantitative PCR (RTQ PCR). RTQ PCR was performed on an Applied Biosystems ABI PRISM® 7700 or an ABI PRISM® 7900 HT Sequence Detection System. Various collections of samples are assembled on the plates, and referred to as Panel 1 (containing normal tissues and cancer cell lines), Panel 2 (containing samples derived from tissues from normal and cancer sources), Panel 3 (containing cancer cell lines), Panel 4 (containing cells and cell lines from normal tissues and cells related to inflammatory conditions), Panel 5D/5I (containing human tissues and cell lines with an emphasis on metabolic diseases), AI_comprehensive_panel (containing normal tissue and samples from autoinflammatory diseases), Panel CNSD.01 (containing samples from normal and diseased brains) and CNS_neurodegeneration_panel (containing samples from normal and Alzheimer's diseased brains).

RNA integrity from all samples is controlled for quality by visual assessment of agarose gel electropherograms using 28S and 18S ribosomal RNA staining intensity ratio as a guide (2:1 to 2.5:1 28s:18s) and the absence of low molecular weight RNAs that would be indicative of degradation products. Samples are controlled against genomic DNA contamination by RTQ PCR reactions run in the absence of reverse transcriptase using probe and primer sets designed to amplify across the span of a single exon.

First, the RNA samples were normalized to reference nucleic acids such as constitutively expressed genes (for example, β-actin and GAPDH). Normalized RNA (5 ul) was converted to cDNA and analyzed by RTQ-PCR using One Step RT-PCR Master Mix Reagents (Applied Biosystems; Catalog No. 4309169) and gene-specific primers according to the manufacturer's instructions.

In other cases, non-normalized RNA samples were converted to single strand cDNA (sscDNA) using Superscript II (Invitrogen Corporation; Catalog No. 18064-147) and random hexamers according to the manufacturer's instructions. Reactions containing up to 10 $\mu$g of total RNA were performed in a volume of 20 $\mu$l and incubated for 60 minutes at 42°C. This reaction can be scaled up to 50 $\mu$g of total RNA in a final volume of 100 $\mu$l. sscDNA samples are then normalized to reference nucleic acids as described previously, using 1X TaqMan® Universal Master mix (Applied Biosystems; catalog No. 4324020), following the manufacturer's instructions.

Probes and primers were designed for each assay according to Applied Biosystems Primer Express Software package (version I for Apple Computer's Macintosh Power PC) or a similar algorithm using the target sequence as input. Default settings were used for reaction conditions and the following parameters were set before selecting primers: primer concentration = 250 nM, primer melting temperature (Tm) range = 58°-60°C, primer optimal Tm = 59°C, maximum primer difference = 2°C, probe does not have 5'G, probe Tm must be 10°C greater than primer Tm, amplicon size 75bp to 100bp. The probes and primers selected (see below) were synthesized by Synthegen (Houston, TX, USA). Probes were double purified by HPLC to remove uncoupled dye and evaluated by mass spectroscopy to verify coupling of reporter and quencher dyes to the 5' and 3' ends of the probe, respectively. Their final concentrations were: forward and reverse primers, 900nM each, and probe, 200nM.

PCR conditions: When working with RNA samples, normalized RNA from each tissue and each cell line was spotted in each well of either a 96 well or a 384-well PCR plate (Applied Biosystems). PCR cocktails included either a single gene specific probe and primers set, or two multiplexed probe and primers sets (a set specific for the target clone and another gene-specific set multiplexed with the target probe). PCR reactions were set up using TaqMan® One-Step RT-PCR Master Mix (Applied Biosystems, Catalog No. 4313803) following manufacturer's instructions. Reverse transcription was performed at 48°C for 30 minutes followed by amplification/PCR cycles as follows: 95°C 10 min, then 40 cycles of 95°C for 15 seconds, 60°C for 1 minute. Results were recorded as CT values (cycle at which a given sample crosses a threshold level of fluorescence) using a log scale, with the difference in RNA concentration between a given sample and the sample with the lowest CT value being represented as 2 to the power of delta CT. The percent relative expression is then obtained by taking the reciprocal of this RNA difference and multiplying by 100.

When working with sscDNA samples, normalized sscDNA was used as described previously for RNA samples. PCR reactions containing one or two sets of probe and primers were set up as described previously, using 1X TaqMan® Universal Master mix (Applied Biosystems; catalog No. 4324020), following the manufacturer's instructions. PCR amplification was performed as follows: 95°C 10 min, then 40 cycles of 95°C for 15 seconds, 60°C for 1 minute. Results were analyzed and processed as described previously.

**Panels 1, 1.1, 1.2, and 1.3D**

The plates for Panels 1, 1.1, 1.2 and 1.3D include 2 control wells (genomic DNA control and chemistry control) and 94 wells containing cDNA from various samples. The samples in these panels are broken into 2 classes: samples derived from cultured cell lines and samples derived from primary normal tissues. The cell lines are derived from cancers of the following types: lung cancer, breast cancer, melanoma, colon cancer, prostate cancer, CNS cancer, squamous cell carcinoma, ovarian cancer, liver cancer, renal cancer, gastric cancer and pancreatic cancer. Cell lines used in these panels are widely available through the American Type Culture Collection (ATCC), a repository for cultured cell lines, and were cultured using the conditions recommended by the ATCC. The normal tissues found on these panels are comprised of samples derived from all major organ systems from single adult individuals or fetuses. These samples are derived from the following organs: adult skeletal muscle, fetal skeletal muscle, adult heart, fetal heart, adult kidney, fetal kidney, adult liver, fetal liver, adult lung, fetal lung, various regions of the brain, the spleen, bone marrow, lymph node, pancreas, salivary gland, pituitary gland, adrenal gland, spinal cord, thymus, stomach, small intestine, colon, bladder, trachea, breast, ovary, uterus, placenta, prostate, testis and adipose.

In the results for Panels 1, 1.1, 1.2 and 1.3D, the following abbreviations are used:

ca. = carcinoma,
\* = established from metastasis,
met = metastasis,
s cell var = small cell variant,
non-s = non-sm = non-small,
squam = squamous,
pl. eff = pl effusion = pleural effusion,
glio = glioma,
astro = astrocytoma, and
neuro = neuroblastoma.

**General_screening_panel-v1.4, v1.5, v1.6 and 1.7**

The plates for Panels 1.4, 1.5, 1.6 and 1.7 include 2 control wells (genomic DNA control and chemistry control) and 88 to 94 wells containing cDNA from various samples. The samples in Panels 1.4, 1.5, 1.6 and 1.7 are broken into 2 classes: samples derived from cultured cell lines and samples derived from primary normal tissues. The cell lines are derived from cancers of the following types: lung cancer, breast cancer, melanoma, colon cancer, prostate cancer, CNS cancer, squamous cell carcinoma, ovarian cancer, liver cancer, renal cancer, gastric cancer and pancreatic cancer. Cell lines used in Panels 1.4, 1.5, 1.6 and 1.7 are widely available through the American Type Culture Collection (ATCC), a repository for cultured cell lines, and were cultured using the conditions recommended by the ATCC. The normal tissues found on Panels 1.4, 1.5, 1.6 and 1.7 are comprised of pools of samples derived from all major organ systems from 2 to 5 different adult individuals or fetuses. These samples are derived from the following organs: adult skeletal muscle, fetal skeletal muscle, adult heart, fetal heart, adult kidney, fetal kidney, adult liver, fetal liver, adult lung, fetal lung, various regions of the brain, the spleen, bone marrow, lymph node, pancreas, salivary gland, pituitary gland, adrenal gland, spinal cord, thymus, stomach, small intestine, colon, bladder, trachea, breast, ovary, uterus, placenta, prostate, testis and adipose. Abbreviations are as described for Panels 1, 1.1, 1.2, and 1.3D.

**Panels 2D, 2.2, 2.3 and 2.4**

The plates for Panels 2D, 2.2, 2.3 and 2.4 generally include 2 control wells and 94 test samples composed of RNA or cDNA isolated from human tissue procured by surgeons working in close cooperation with the National Cancer Institute's Cooperative Human Tissue Network (CHTN) or the National Disease Research Initiative (NDRI) or from Ardais or Clinomics). The tissues are derived from human malignancies and in cases where indicated many malignant tissues have "matched margins" obtained from noncancerous tissue just adjacent to the tumor. These are termed normal adjacent tissues and are denoted "NAT" in the results below. The tumor tissue and the "matched margins" are evaluated by two independent pathologists (the surgical pathologists and again by a pathologist at NDRI/ CHTN/Ardais/Clinomics). Unmatched RNA samples from tissues without malignancy (normal tissues) were also obtained from Ardais or Clinomics. This analysis provides a gross histopathological assessment of tumor differentiation grade. Moreover, most samples include the original surgical pathology report that provides information regarding the clinical stage of the patient. These matched margins are taken from the tissue surrounding (i.e. immediately proximal) to the zone of surgery (designated "NAT", for normal adjacent tissue, in Table RR). In addition, RNA and cDNA samples were obtained from various human tissues derived from autopsies performed on elderly people or sudden death victims (accidents, etc.). These tissues were ascertained to be free of disease and were purchased from various commercial sources such as Clontech (Palo Alto, CA), Research Genetics, and Invitrogen.

### HASS Panel v 1.0

The HASS panel v 1.0 plates are comprised of 93 cDNA samples and two controls. Specifically, 81 of these samples are derived from cultured human cancer cell lines that had been subjected to serum starvation, acidosis and anoxia for different time periods as well as controls for these treatments, 3 samples of human primary cells, 9 samples of malignant brain cancer (4 medulloblastomas and 5 glioblastomas) and 2 controls. The human cancer cell lines are obtained from ATCC (American Type Culture Collection) and fall into the following tissue groups: breast cancer, prostate cancer, bladder carcinomas, pancreatic cancers and CNS cancer cell lines. These cancer cells are all cultured under standard recommended conditions. The treatments used (serum starvation, acidosis and anoxia) have been previously published in the scientific literature. The primary human cells were obtained from Clonetics (Walkersville, MD) and were grown in the media and conditions recommended by Clonetics. The malignant brain cancer samples are obtained as part of a collaboration (Henry Ford Cancer Center) and are evaluated by a pathologist prior to CuraGen receiving the samples . RNA was prepared from these samples using the standard procedures. The genomic and chemistry control wells have been described previously.

### ARDAIS Panel v 1.0

The plates for ARDAIS panel v 1.0 generally include 2 control wells and 22 test samples composed of RNA isolated from human tissue procured by surgeons working in close cooperation with Ardais Corporation. The tissues are derived from human lung malignancies (lung adenocarcinoma or lung squamous cell carcinoma) and in cases where indicated many malignant samples have "matched margins" obtained from noncancerous lung tissue just adjacent to the tumor. These matched margins are taken from the tissue surrounding (i.e. immediately proximal) to the zone of surgery (designated "NAT", for normal adjacent tissue) in the results below. The tumor tissue and the "matched margins" are evaluated by independent pathologists (the surgical pathologists and again by a pathologist at Ardais). Unmatched malignant and non-malignant RNA samples from lungs were also obtained from Ardais. Additional information from Ardais provides a gross histopathological assessment of tumor differentiation grade and stage. Moreover, most samples include the original surgical pathology report that provides information regarding the clinical state of the patient.

### ARDAIS Prostate/Kidney/Lung v 1.0

The plates for ARDAIS prostate, kidney, and lung 1.0 respectively, generally include 2 control wells and 68 test samples composed of RNA isolated from human tissue procured by surgeons working in close cooperation with Ardais Corporation. The tissues are derived from human prostate, kidney, or lung malignancies and in cases where indicated malignant samples have "matched margins" obtained from noncancerous prostate, kidney, or lung tissue just adjacent to the tumor. These matched margins are taken from the tissue surrounding (i.e. immediately proximal) to the zone of surgery (designated "NAT", for normal adjacent tissue) in the results below. The tumor tissue and the "matched margins" are evaluated by independent pathologists (the surgical pathologists and again by a pathologist at Ardais). RNA from unmatched malignant and non-malignant prostate, kidney, or lung samples were also obtained from Ardais. Additional information from Ardais provides a gross histopathological assessment of tumor differentiation grade and stage. Moreover, most samples include the original surgical pathology report that provides information regarding the clinical state of the patient.

### Panel 3D, 3.1 and 3.2

The plates of Panel 3D, 3.1, and 3.2 are comprised of 94 cDNA samples and two control samples. Specifically, 92 of these samples are derived from cultured human cancer cell lines, 2 samples of human primary cerebellar tissue and 2 controls. The human cell lines are generally obtained from ATCC (American Type Culture Collection), NCI or the German tumor cell bank and fall into the following tissue groups: Squamous cell carcinoma of the tongue, breast cancer, prostate cancer, melanoma, epidermoid carcinoma, sarcomas, bladder carcinomas, pancreatic cancers, kidney cancers, leukemias/lymphomas, ovarian/uterine/cervical, gastric, colon, lung and CNS cancer cell lines. In addition, there are two independent samples of cerebellum. These cells are all cultured under standard recommended conditions and RNA extracted using the standard procedures. The cell lines in panel 3D, 3.1, 3.2, 1, 1.1., 1.2, 1.3D, 1.4, 1.5, and 1.6 are of the most common cell lines used in the scientific literature.

## Panels 4D, 4R, and 4.1D

Panel 4 includes samples on a 96 well plate (2 control wells, 94 test samples) composed of RNA (Panel 4R) or cDNA (Panels 4D/4.1D) isolated from various human cell lines or tissues related to inflammatory conditions. Total RNA from control normal tissues such as colon and lung (Stratagene, La Jolla, CA) and thymus and kidney (Clontech) was employed. Total RNA from liver tissue from cirrhosis patients and kidney from lupus patients was obtained from BioChain (Biochain Institute, Inc., Hayward, CA). Intestinal tissue for RNA preparation from patients diagnosed as having Crohn's disease and ulcerative colitis was obtained from the National Disease Research Interchange (NDRI) (Philadelphia, PA).

Astrocytes, lung fibroblasts, dermal fibroblasts, coronary artery smooth muscle cells, small airway epithelium, bronchial epithelium, microvascular dermal endothelial cells, microvascular lung endothelial cells, human pulmonary aortic endothelial cells, human umbilical vein endothelial cells were all purchased from Clonetics (Walkersville, MD) and grown in the media supplied for these cell types by Clonetics. These primary cell types were activated with various cytokines or combinations of cytokines for 6 and/or 12-14 hours, as indicated. The following cytokines were used; IL-1 beta at approximately 1-5ng/ml, TNF alpha at approximately 5-10ng/ml, IFN gamma at approximately 20-50ng/ml, IL-4 at approximately 5-10ng/ml, IL-9 at approximately 5-10ng/ml, IL-13 at approximately 5-10ng/ml. Endothelial cells were sometimes starved for various times by culture in the basal media from Clonetics with 0.1% serum.

Mononuclear cells were prepared from blood of employees at CuraGen Corporation, using Ficoll. LAK cells were prepared from these cells by culture in DMEM 5% FCS (Hyclone), $100\mu$M non essential amino acids (Gibco/Life Technologies, Rockville, MD), 1mM sodium pyruvate (Gibco), mercaptoethanol $5.5\times10^{-5}$M (Gibco), and 10mM Hepes (Gibco) and Interleukin 2 for 4-6 days. Cells were then either activated with 10-20ng/ml PMA and 1-2$\mu$g/ml ionomycin, IL-12 at 5-10ng/ml, IFN gamma at 20-50ng/ml and IL-18 at 5-10ng/ml for 6 hours. In some cases, mononuclear cells were cultured for 4-5 days in DMEM 5% FCS (Hyclone), $100\mu$M non essential amino acids (Gibco), 1mM sodium pyruvate (Gibco), mercaptoethanol $5.5\times10^{-5}$M (Gibco), and 10mM Hepes (Gibco) with PHA (phytohemagglutinin) or PWM (pokeweed mitogen) at approximately $5\mu$g/ml. Samples were taken at 24, 48 and 72 hours for RNA preparation. MLR (mixed lymphocyte reaction) samples were obtained by taking blood from two donors, isolating the mononuclear cells using Ficoll and mixing the isolated mononuclear cells 1:1 at a final concentration of approximately $2\times10^{6}$ cells/ml in DMEM 5% FCS (Hyclone), $100\mu$M non essential amino acids (Gibco), 1mM sodium pyruvate (Gibco), mercaptoethanol $(5.5\times10^{-5}$M) (Gibco), and 10mM Hepes (Gibco). The MLR was cultured and samples taken at various time points ranging from 1- 7 days for RNA preparation.

Monocytes were isolated from mononuclear cells using CD14 Miltenyi Beads, +ve VS selection columns and a Vario Magnet according to the manufacturer's instructions. Monocytes were differentiated into dendritic cells by culture in DMEM 5% fetal calf serum (FCS) (Hyclone, Logan, UT), $100\mu$M non essential amino acids (Gibco), 1mM sodium pyruvate (Gibco), mercaptoethanol $5.5\times10^{-5}$M (Gibco), and 10mM Hepes (Gibco), 50ng/ml GMCSF and 5ng/ml IL-4 for 5-7 days. Macrophages were prepared by culture of monocytes for 5-7 days in DMEM 5% FCS (Hyclone), $100\mu$M non essential amino acids (Gibco), 1mM sodium pyruvate (Gibco), mercaptoethanol $5.5\times10^{-5}$M (Gibco), 10mM Hepes (Gibco) and 10% AB Human Serum or MCSF at approximately 50ng/ml. Monocytes, macrophages and dendritic cells were stimulated for 6 and 12-14 hours with lipopolysaccharide (LPS) at 100ng/ml. Dendritic cells were also stimulated with anti-CD40 monoclonal antibody (Pharmingen) at $10\mu$g/ml for 6 and 12-14 hours.

CD4 lymphocytes, CD8 lymphocytes and NK cells were also isolated from mononuclear cells using CD4, CD8 and CD56 Miltenyi beads, positive VS selection columns and a Vario Magnet according to the manufacturer's instructions. CD45RA and CD45RO CD4 lymphocytes were isolated by depleting mononuclear cells of CD8, CD56, CD14 and CD19 cells using CD8, CD56, CD14 and CD19 Miltenyi beads and positive selection. CD45RO beads were then used to isolate the CD45RO CD4 lymphocytes with the remaining cells being CD45RA CD4 lymphocytes. CD45RA CD4, CD45RO CD4 and CD8 lymphocytes were placed in DMEM 5% FCS (Hyclone), $100\mu$M non essential amino acids (Gibco), 1mM sodium pyruvate (Gibco), mercaptoethanol $5.5\times10^{-5}$M (Gibco), and 10mM Hepes (Gibco) and plated at $10^{6}$cells/ml onto Falcon 6 well tissue culture plates that had been coated overnight with $0.5\mu$g/ml anti-CD28 (Pharmingen) and 3ug/ml anti-CD3 (OKT3, ATCC) in PBS. After 6 and 24 hours, the cells were harvested for RNA preparation. To prepare chronically activated CD8 lymphocytes, we activated the isolated CD8 lymphocytes for 4 days on anti-CD28 and anti-CD3 coated plates and then harvested the cells and expanded them in DMEM 5% FCS (Hyclone), $100\mu$M non essential amino acids (Gibco), 1mM sodium pyruvate (Gibco), mercaptoethanol $5.5\times10^{-5}$M (Gibco), and 10mM Hepes (Gibco) and IL-2. The expanded CD8 cells were then activated again with plate bound anti-CD3 and anti-CD28 for 4 days and expanded as before. RNA was isolated 6 and 24 hours after the second activation and after 4 days of the second expansion culture. The isolated NK cells were cultured in DMEM 5% FCS (Hyclone), 100$\mu$M non essential amino acids (Gibco), 1mM sodium pyruvate (Gibco), mercaptoethanol 5.5x10$^{-5}$M (Gibco), and 10mM Hepes (Gibco) and IL-2 for 4-6 days before RNA was prepared.

To obtain B cells, tonsils were procured from NDRI. The tonsil was cut up with sterile dissecting scissors and then passed through a sieve. Tonsil cells were then spun down and resupended at 10$^6$ cells/ml in DMEM 5% FCS (Hyclone), 100$\mu$M non essential amino acids (Gibco), 1mM sodium pyruvate (Gibco), mercaptoethanol 5.5x10$^{-5}$ (Gibco), and 10mM Hepes (Gibco). To activate the cells, we used PWM at 5$\mu$g/ml or anti-CD40 (Pharmingen) at approximately 10$\mu$g/ml and IL-4 at 5-10ng/ml. Cells were harvested for RNA preparation at 24,48 and 72 hours.

To prepare the primary and secondary Th1/Th2 and Tr1 cells, six-well Falcon plates were coated overnight with 10$\mu$g/ml anti-CD28 (Pharmingen) and 2$\mu$g/ml OKT3 (ATCC), and then washed twice with PBS. Umbilical cord blood CD4 lymphocytes (Poietic Systems, German Town, MD) were cultured at 10$^5$-10$^6$ cells/ml in DMEM 5% FCS (Hyclone), 100$\mu$M non essential amino acids (Gibco), 1mM sodium pyruvate (Gibco), mercaptoethanol 5.5x10$^{-5}$M (Gibco), 10mM Hepes (Gibco) and IL-2 (4ng/ml). IL-12 (5ng/ml) and anti-IL4 (1$\mu$g/ml) were used to direct to Th1, while IL-4 (5ng/ml) and anti-IFN gamma (1$\mu$g/ml) were used to direct to Th2 and IL-10 at 5ng/ml was used to direct to Tr1. After 4-5 days, the activated Th1, Th2 and Tr1 lymphocytes were washed once in DMEM and expanded for 4-7 days in DMEM 5% FCS (Hyclone), 100$\mu$M non essential amino acids (Gibco), 1mM sodium pyruvate (Gibco), mercaptoethanol 5.5x10$^{-5}$M (Gibco), 10mM Hepes (Gibco) and IL-2 (1ng/ml). Following this, the activated Th1, Th2 and Tr1 lymphocytes were re-stimulated for 5 days with anti-CD28/OKT3 and cytokines as described above, but with the addition of anti-CD95L (1$\mu$g/ml) to prevent apoptosis. After 4-5 days, the Th1, Th2 and Tr1 lymphocytes were washed and then expanded again with IL-2 for 4-7 days. Activated Th1 and Th2 lymphocytes were maintained in this way for a maximum of three cycles. RNA was prepared from primary and secondary Th1, Th2 and Tr1 after 6 and 24 hours following the second and third activations with plate bound anti-CD3 and anti-CD28 mAbs and 4 days into the second and third expansion cultures in Interleukin 2.

The following leukocyte cells lines were obtained from the ATCC: Ramos, EOL-1, KU-812. EOL cells were further differentiated by culture in 0.1mM dbcAMP at 5x10$^5$cells/ml for 8 days, changing the media every 3 days and adjusting the cell concentration to 5x10$^5$cells/ml. For the culture of these cells, we used DMEM or RPMI (as recommended by the ATCC), with the addition of 5% FCS (Hyclone), 100$\mu$M non essential amino acids (Gibco), 1mM sodium pyruvate (Gibco), mercaptoethanol 5.5x10$^{-5}$M (Gibco), 10mM Hepes (Gibco). RNA was either prepared from resting cells or cells activated with PMA at 10ng/ml and ionomycin at 1$\mu$g/ml for 6 and 14 hours. Keratinocyte line CCD106 and an airway epithelial tumor line NCI-H292 were also obtained from the ATCC. Both were cultured in DMEM 5% FCS (Hyclone), 100$\mu$M non essential amino acids (Gibco), 1mM sodium pyruvate (Gibco), mercaptoethanol 5.5x10$^{-5}$M (Gibco), and 10mM Hepes (Gibco). CCD1106 cells were activated for 6 and 14 hours with approximately 5 ng/ml TNF alpha and 1ng/ml IL-1 beta, while NCI-H292 cells were activated for 6 and 14 hours with the following cytokines: 5ng/ml IL-4, 5ng/ml IL-9, 5ng/ml IL-13 and 25ng/ml IFN gamma.

For these cell lines and blood cells, RNA was prepared by lysing approximately 10$^7$cells/ml using Trizol (Gibco BRL). Briefly, 1/10 volume of bromochloropropane (Molecular Research Corporation) was added to the RNA sample, vortexed and after 10 minutes at room temperature, the tubes were spun at 14,000 rpm in a Sorvall SS34 rotor. The aqueous phase was removed and placed in a 15ml Falcon Tube. An equal volume of isopropanol was added and left at -20°C overnight. The precipitated RNA was spun down at 9,000 rpm for 15 min in a Sorvall SS34 rotor and washed in 70% ethanol. The pellet was redissolved in 300$\mu$l of RNAse-free water and 35$\mu$l buffer (Promega) 5$\mu$l DTT, 7$\mu$l RNAsin and 8$\mu$l DNAse were added. The tube was incubated at 37°C for 30 minutes to remove contaminating genomic DNA, extracted once with phenol chloroform and reprecipitated with 1/10 volume of 3M sodium acetate and 2 volumes of 100% ethanol. The RNA was spun down and placed in RNAse free water. RNA was stored at -80°C.

**AI_comprehensive panel_v1.0**

The plates for AI_comprehensive panel_v1.0 include two control wells and 89 test samples comprised of cDNA isolated from surgical and postmortem human tissues obtained from the Backus Hospital and Clinomics (Frederick, MD). Total RNA was extracted from tissue samples from the Backus Hospital in the Facility at CuraGen. Total RNA from other tissues was obtained from Clinomics.

Joint tissues including synovial fluid, synovium, bone and cartilage were obtained from patients undergoing total knee or hip replacement surgery at the Backus Hospital. Tissue samples were immediately snap frozen in liquid nitrogen to ensure that isolated RNA was of optimal quality and not degraded. Additional samples of osteoarthritis and rheumatoid arthritis joint tissues were obtained from Clinomics. Normal control tissues were supplied by Clinomics and were obtained during autopsy of trauma victims.

Surgical specimens of psoriatic tissues and adjacent matched tissues were provided as total RNA by Clinomics. Two male and two female patients were selected between the ages of 25 and 47. None of the patients were taking prescription drugs at the time samples were isolated.

Surgical specimens of diseased colon from patients with ulcerative colitis and Crohns disease and adjacent matched tissues were obtained from Clinomics. Bowel tissue from three female and three male Crohn's patients between the ages of 41-69 were used. Two patients were not on prescription medication while the others were taking dexamethasone, phenobarbital, or tylenol. Ulcerative colitis tissue was from three male and four female patients. Four of the patients were taking lebvid and two were on phenobarbital.

Total RNA from post mortem lung tissue from trauma victims with no disease or with emphysema, asthma or COPD was purchased from Clinomics. Emphysema patients ranged in age from 40-70 and all were smokers, this age range was chosen to focus on patients with cigarette-linked emphysema and to avoid those patients with alpha-1antitrypsin deficiencies. Asthma patients ranged in age from 36-75, and excluded smokers to prevent those patients that could also have COPD. COPD patients ranged in age from 35-80 and included both smokers and non-smokers. Most patients were taking corticosteroids, and bronchodilators.

In the labels employed to identify tissues in the AI_comprehensive panel_v1.0 panel, the following abbreviations are used:

AI = Autoimmunity
Syn = Synovial
Normal = No apparent disease
Rep22 /Rep20 = individual patients
RA = Rheumatoid arthritis
Backus = From Backus Hospital
OA = Osteoarthritis
(SS) (BA) (MF) = Individual patients
Adj = Adjacent tissue
Match control = adjacent tissues
-M = Male
-F = Female
COPD = Chronic obstructive pulmonary disease

### AI.05 chondrosarcoma

The AI.05 chondrosarcoma plates are comprised of SW1353 cells that had been subjected to serum starvation and treatment with cytokines that are known to induce MMP (1, 3 and 13) synthesis (*e.g.* IL1beta). These treatments include: IL-1beta (10 ng/ml), IL-1beta + TNF-alpha (50 ng/ml), IL-1beta + Oncostatin (50 ng/ml) and PMA (100 ng/ml). The SW1353 cells were obtained from the ATCC (American Type Culture Collection) and were all cultured under standard recommended conditions. The SW1353 cells were plated at $3 \times 10^5$ cells/ml (in DMEM medium-10 % FBS) in 6-well plates. The treatment was done in triplicate, for 6 and 18 h. The supernatants were collected for analysis of MMP 1, 3 and 13 production and for RNA extraction. RNA was prepared from these samples using the standard procedures.

### Panels 5D and 51

The plates for Panel 5D and 51 include two control wells and a variety of cDNAs isolated from human tissues and cell lines with an emphasis on metabolic diseases. Metabolic tissues were obtained from patients enrolled in the Gestational Diabetes study. Cells were obtained during different stages in the differentiation of adipocytes from human mesenchymal stem cells. Human pancreatic islets were also obtained.

In the Gestational Diabetes study, subjects are young (18 - 40 years), otherwise healthy women with and without gestational diabetes undergoing routine (elective) Caesarean section. After delivery of the infant, when the surgical incisions were being repaired/closed, the obstetrician removed a small sample (less than 1 cc) of the exposed metabolic tissues during the closure of each surgical level. The biopsy material was rinsed in sterile saline, blotted and fast frozen within 5 minutes from the time of removal. The tissue was then flash frozen in liquid nitrogen and stored, individually, in sterile screw-top tubes and kept on dry ice for shipment to or to be picked up by CuraGen. The metabolic tissues of interest include uterine wall (smooth muscle), visceral adipose, skeletal muscle (rectus) and subcutaneous adipose. Patient descriptions are as follows:

Patient 2: Diabetic Hispanic, overweight, not on insulin
Patient 7-9: Nondiabetic Caucasian and obese (BMI>30)
Patient 10: Diabetic Hispanic, overweight, on insulin
Patient 11: Nondiabetic African American and overweight

Patient 12: Diabetic Hispanic on insulin

Adipocyte differentiation was induced in donor progenitor cells obtained from Osirus (a division of Clonetics/BioWhittaker) in triplicate, except for Donor 3U which had only two replicates. Scientists at Clonetics isolated, grew and differentiated human mesenchymal stem cells (HuMSCs) for CuraGen based on the published protocol found in Mark F. Pittenger, et al., Multilineage Potential of Adult Human Mesenchymal Stem Cells Science Apr 2 1999: 143-147. Clonetics provided Trizol lysates or frozen pellets suitable for mRNA isolation and ds cDNA production. A general description of each donor is as follows:

Donor 2 and 3 U: Mesenchymal Stem cells, Undifferentiated Adipose
Donor 2 and 3 AM: Adipose, AdiposeMidway Differentiated
Donor 2 and 3 AD: Adipose, Adipose Differentiated

Human cell lines were generally obtained from ATCC (American Type Culture Collection), NCI or the German tumor cell bank and fall into the following tissue groups: kidney proximal convoluted tubule, uterine smooth muscle cells, small intestine, liver HepG2 cancer cells, heart primary stromal cells, and adrenal cortical adenoma cells. These cells are all cultured under standard recommended conditions and RNA extracted using the standard procedures. All samples were processed at CuraGen to produce single stranded cDNA.

Panel 51 contains all samples previously described with the addition of pancreatic islets from a 58 year old female patient obtained from the Diabetes Research Institute at the University of Miami School of Medicine. Islet tissue was processed to total RNA at an outside source and delivered to CuraGen for addition to panel 51.

In the labels employed to identify tissues in the 5D and 5I panels, the following abbreviations are used:

GO Adipose = Greater Omentum Adipose
SK = Skeletal Muscle
UT = Uterus
PL = Placenta
AD = Adipose Differentiated
AM = Adipose Midway Differentiated
U = Undifferentiated Stem Cells

**Human Metabolic RTQ-PCR Panel**

The plates for the Human Metabolic RTQ-PCR Panel include two control wells (genomic DNA control and chemistry control) and 211 cDNAs isolated from human tissues and cell lines with an emphasis on metabolic diseases. This panel is useful for establishing the tissue and cellular expression profiles for genes believed to play a role in the etiology and pathogenesis of obesity and/or diabetes and to confirm differential expression of such genes derived from other methods. Metabolic tissues were obtained from patients enrolled in the CuraGen Gestational Diabetes study and from autopsy tissues from Type II diabetics and age, sex and race-matched control patients. One or more of the following were used to characterize the patients: body mass index [BMI = wt (kg) / ht (m$^2$)], serum glucose, HgbA1c. Cell lines used in this panel are widely available through the American Type Culture Collection (ATCC), a repository for cultured cell lines. RNA from human Pancreatic Islets was also obtained.

In the Gestational Diabetes study, subjects are young (18-40 years), otherwise healthy women with and without gestational diabetes undergoing routine (elective) Caesarian section. After delivery of the infant, when the surgical incisions were being repaired/closed, the obstetrician removed a small sample (less than 1cc) of the exposed metabolic tissues during the closure of each surgical level. The biopsy material was rinsed in sterile saline, blotted, and then flash frozen in liquid nitrogen and stored, individually, in sterile screw-top tubes and kept on dry ice for shipment to or to be picked up by CuraGen. The metabolic tissues of interest include uterine wall (smooth muscle), visceral adipose, skeletal muscle (rectus), and subcutaneous adipose. Patient descriptions are as follows:

Patient 7 - Non-diabetic Caucasian and obese
Patient 8 - Non-diabetic Caucasian and obese
Patient 12 - Diabetic Caucasian with unknown BMI and on insulin
Patient 13 - Diabetic Caucasian, overweight, not on insulin
Patient 15 - Diabetic Caucasian, obese, not on insulin
Patient 17 - Diabetic Caucasian, normal weight, not on insulin
Patient 18 - Diabetic Hispanic, obese, not on insulin
Patient 19 - Non-diabetic Caucasian and normal weight

Patient 20 - Diabetic Caucasian, overweight, and on insulin
Patient 21 - Non-diabetic Caucasian and overweight
Patient 22 - Diabetic Caucasian, normal weight, on insulin
Patient 23 - Non-diabetic Caucasian and overweight
Patient 25 - Diabetic Caucasian, normal weight, not on insulin
Patient 26 - Diabetic Caucasian, obese, on insulin
Patient 27 - Diabetic Caucasian, obese, on insulin

Total RNA was isolated from metabolic tissues of 12 Type II diabetic patients and 12 matched control patients included hypothalamus, liver, pancreas, small intestine, psoas muscle, diaphragm muscle, visceral adipose, and subcutaneous adipose. The diabetics and non-diabetics were matched for age, sex, ethnicity, and BMI where possible.

The panel also contains pancreatic islets from a 22 year old male patient (with a BMI of 35) obtained from the Diabetes Research Institute at the University of Miami School of Medicine. Islet tissue was processed to total RNA at CuraGen.

Cell lines used in this panel are widely available through the American Type Culture Collection (ATCC), a repository for cultured cell lines, and were cultured at an outside facility. The RNA was extracted at CuraGen according to CuraGen protocols. All samples were then processed at CuraGen to produce single stranded cDNA.

In the labels used to identify tissues in the Human Metabolic panel, the following abbreviations are used:

PI = placenta
Go = greater omentum
Sk = skeletal muscle
Ut = uterus
CC = Caucasian
HI = Hispanic
AA = African American
AS = Asian
Diab = Type II diabetic
Norm = Non-diabetic
Overwt = Overweight; med BMI
Obese = Hi BMI
Low BM = 20-25
Med BM = 26-30
Hi BMI = Greater than 30
M = Male
# = Patient identifier
Vis. = Visceral
SubQ = Subcutaneous

Panel **CNSD.01**

The plates for Panel CNSD.01 include two control wells and 94 test samples comprised of cDNA isolated from postmortem human brain tissue obtained from the Harvard Brain Tissue Resource Center. Brains are removed from calvaria of donors between 4 and 24 hours after death, sectioned by neuroanatomists, and frozen at -80°C in liquid nitrogen vapor. All brains are sectioned and examined by neuropathologists to confirm diagnoses with clear associated neuropathology.

Disease diagnoses are taken from patient records. The panel contains two brains from each of the following diagnoses: Alzheimer's disease, Parkinson's disease, Huntington's disease, Progressive Supernuclear Palsy, Depression, and "Normal controls". Within each of these brains, the following regions are represented: cingulate gyrus, temporal pole, globus palladus, substantia nigra, Brodman Area 4 (primary motor strip), Brodman Area 7 (parietal cortex), Brodman Area 9 (prefrontal cortex), and Brodman area 17 (occipital cortex). Not all brain regions are represented in all cases; e.g., Huntington's disease is characterized in part by neurodegeneration in the globus palladus, thus this region is impossible to obtain from confirmed Huntington's cases. Likewise Parkinson's disease is characterized by degeneration of the substantia nigra making this region more difficult to obtain. Normal control brains were examined for neuropathology and found to be free of any pathology consistent with neurodegeneration.

In the labels employed to identify tissues in the CNS panel, the following abbreviations are used:

PSP = Progressive supranuclear palsy

Sub Nigra = Substantia nigra
Glob Palladus= Globus palladus
Temp Pole = Temporal pole
Cing Gyr = Cingulate gyrus
BA 4 = Brodman Area 4

**Panel CNS_Neurodegeneration_V1.0**

The plates for Panel CNS_Neurodegeneration_V1.0 include two control wells and 47 test samples comprised of cDNA isolated from postmortem human brain tissue obtained from the Harvard Brain Tissue Resource Center (McLean Hospital) and the Human Brain and Spinal Fluid Resource Center (VA Greater Los Angeles Healthcare System). Brains are removed from calvaria of donors between 4 and 24 hours after death, sectioned by neuroanatomists, and frozen at -80°C in liquid nitrogen vapor. All brains are sectioned and examined by neuropathologists to confirm diagnoses with clear associated neuropathology.

Disease diagnoses are taken from patient records. The panel contains six brains from Alzheimer's disease (AD) patients, and eight brains from "Normal controls" who showed no evidence of dementia prior to death. The eight normal control brains are divided into two categories: Controls with no dementia and no Alzheimer's like pathology (Controls) and controls with no dementia but evidence of severe Alzheimer's like pathology, (specifically senile plaque load rated as level 3 on a scale of 0-3; 0 = no evidence of plaques, 3 = severe AD senile plaque load). Within each of these brains, the following regions are represented: hippocampus, temporal cortex (Brodman Area 21), parietal cortex (Brodman area 7), and occipital cortex (Brodman area 17). These regions were chosen to encompass all levels of neurodegeneration in AD. The hippocampus is a region of early and severe neuronal loss in AD; the temporal cortex is known to show neurodegeneration in AD after the hippocampus; the parietal cortex shows moderate neuronal death in the late stages of the disease; the occipital cortex is spared in AD and therefore acts as a "control" region within AD patients. Not all brain regions are represented in all cases.

In the labels employed to identify tissues in the CNS_Neurodegeneration_V1.0 panel, the following abbreviations are used:

AD = Alzheimer's disease brain; patient was demented and showed AD-like pathology upon autopsy
Control = Control brains; patient not demented, showing no neuropathology Control (Path) = Control brains; pateint not demented but showing sever AD-like pathology
SupTemporal Ctx = Superior Temporal Cortex
Inf Temporal Ctx = Inferior Temporal Cortex

**Panel CNS_Neurodegeneration_V2.0**

The plates for Panel CNS_Neurodegeneration_V2.0 include two control wells and 47 test samples comprised of cDNA isolated from postmortem human brain tissue obtained from the Harvard Brain Tissue Resource Center (McLean Hospital) and the Human Brain and Spinal Fluid Resource Center (VA Greater Los Angeles Healthcare System). Brains are removed from calvaria of donors between 4 and 24 hours after death, sectioned by neuroanatomists, and frozen at -80°C in liquid nitrogen vapor. All brains are sectioned and examined by neuropathologists to confirm diagnoses with clear associated neuropathology.

Disease diagnoses are taken from patient records. The panel contains sixteen brains from Alzheimer's disease (AD) patients, and twenty-nine brains from "Normal controls" who showed no evidence of dementia prior to death. The twenty-nine normal control brains are divided into two categories: Fourteen controls with no dementia and no Alzheimer's like pathology (Controls) and fifteen controls with no dementia but evidence of severe Alzheimer's like pathology, (specifically senile plaque load rated as level 3 on a scale of 0-3; 0 = no evidence of plaques, 3 = severe AD senile plaque load). Tissue from the temporal cotex (Broddmann Area 21) was selected for all samples from the Harvard Brain Tissue Resource Center; from the two sample from the Human Brain and Spinal Fluid Resource Center (samples 1 and 2) tissue from the inferior and superior temporal cortex was used; each sample on the panel represents a pool of inferior and superior temporal cortex from an individual patient. The temporal cortex was chosen as it shows a loss of neurons in the intermediate stages of the disease. Selection of a region which is affected in the early stages of Alzheimer's disease (e.g., hippocampus or entorhinal cortex) could potentially result in the examination of gene expression after vulnerable neurons are lost, and missing genes involved in the actual neurodegeneration process.

In the labels employed to identify tissues in the CNS_Neurodegeneration_V2.0 panel, the following abbreviations are used:

AD = Alzheimer's disease brain; patient was demented and showed AD-like pathology upon autopsy

Control = Control brains; patient not demented, showing no neuropathology

AH3 = Control brains; patient not demented but showing severe AD-like pathology Inf & Sup Temp Ctx Pool = Pool of inferior and superior temporal cortex for a given individual

## A. CG57008-02: HAVcr-1

Expression of gene CG57008-02 was assessed using the primer-probe set Ag821, described in Table 5. Results of the RTQ-PCR runs are shown in Tables 6 - 17

**Table 5. Probe Name Ag821**

| Primers | Sequences | Length | Start Position | SEQ ID No |
|---------|-----------|--------|----------------|-----------|
| Forward | 5'-tcctcaagtggtcatcttaagc-3' | 22 | 57 | 33 |
| Probe | TET-5'-tcctacatctggcagattctgtagctg-3'-TAMRA | 27 | 83 | 34 |
| Reverse | 5'-tctccaccaacctttacagaac-3' | 22 | 110 | 35 |

**Table 6. AI_comprehensive panel_v1.0**

| Column A - Rel. Exp.(%) Ag821, Run 259498355 | | | |
|---|---|---|---|
| Tissue Name | A | Tissue Name | A |
| 110967 COPD-F | 16.7 | 112427 Match Control Psoriasis-F | 31.4 |
| 110980 COPD-F | 7.2 | 112418 Psoriasis-M | 6.6 |
| 110968 COPD-M | 6.6 | 112723 Match Control Psoriasis-M | 13.3 |
| 110977 COPD-M | 0.0 | 112419 Psoriasis-M | 10.7 |
| 110989 Emphysema-F | 25.3 | 112424 Match Control Psoriasis-M | 12.0 |
| 110992 Emphysema-F | 65.1 | 112420 Psoriasis-M | 46.7 |
| 110993 Emphysema-F | 10.9 | 112425 Match Control Psoriasis-M | 38.2 |
| 110994 Emphysema-F | 8.0 | 104689 (MF) OA Bone-Backus | 12.4 |
| 110995 Emphysema-F | 100.0 | 104690 (MF) Adj "Normal" Bone-Backus | 12.7 |
| 110996 Emphysema-F | 41.2 | 104691 (MF) OA Synovium-Backus | 14.4 |
| 110997 Asthma-M | 3.4 | 104692 (BA) OA Cartilage-Backus | 5.0 |
| 111001 Asthma-F | 11.1 | 104694 (BA) OA Bone-Backus | 3.8 |
| 111002 Asthma-F | 5.4 | 104695 (BA) Adj "Normal" Bone-Backus | 6.0 |
| 111003 Atopic Asthma-F | 16.0 | 104696 (BA) OA Synovium-Backus | 6.3 |
| 111004 Atopic Asthma-F | 14.6 | 104700 (SS) OA Bone-Backus | 6.5 |
| 111005 Atopic Asthma-F | 9.6 | 104701 (SS) Adj "Normal" Bone-Backus | 8.4 |
| 111006 Atopic Asthma-F | 2.7 | 104702 (SS) OA Synovium-Backus | 15.8 |
| 111417 Allergy-M | 10.8 | 117093 OA Cartilage Rep7 | 40.6 |
| 112347 Allergy-M | 0.8 | 112672 OA Bone5 | 35.4 |
| 112349 Normal Lung-F | 1.5 | 112673 OA Synovium5 | 19.3 |
| 112357 Normal Lung-F | 23.2 | 112674 OA Synovial Fluid cells5 | 12.2 |
| 112354 Normal Lung-M | 14.0 | 117100 OA Cartilage Rep14 | 1.5 |
| 112374 Crohns-F | 12.8 | 112756 OA Bone9 | 6.3 |
| 112389 Match Control Crohns-F | 3.6 | 112757 OA Synovium9 | 4.7 |

(continued)

| Column A - Rel. Exp.(%) Ag821, Run 259498355 | | | | |
|---|---|---|---|---|
| Tissue Name | A | Tissue Name | A | |
| 112375 Crohns-F | 8.6 | 1112758 OA Synovial Fluid Cells9 | 9.3 | |
| 112732 Match Control Crohns-F | 3.2 | 117125 RA Cartilage Rep2 | 9.5 | |
| 112725 Crohns-M | 2.6 | 113492 Bone2 RA | 8.1 | |
| 112387 Match Control Crohns-M | 5.0 | 113493 Synovium2 RA | 2.7 | |
| 112378 Crohns-M | 0.9 | 113494 Syn Fluid Cells RA | 12.0 | |
| 112390 Match Control Crohns-M | 85.9 | 113499 Cartilage4 RA | 11.0 | |
| 112726 Crohns-M | 11.7 | 113500 Bone4 RA | 21.3 | |
| 112731 Match Control Crohns-M | 10.8 | 113501 Synovium4 RA | 12.9 | |
| 112380 Ulcer Col-F | 12.2 | 113502 Syn Fluid Cells4 RA | 9.3 | |
| 112734 Match Control Ulcer Col-F | 5.4 | 113495 Cartilage3 RA | 8.5 | |
| 112384 Ulcer Col-F | 52.9 | 113496 Bone3 RA | 10.4 | |
| 112737 Match Control Ulcer Col-F | 2.5 | ,113497 Synovium3 RA | 5.8 | |
| 112386 Ulcer Col-F | 5.2 | 1113498 Syn Fluid Cells3 RA | 10.5 | |
| 112738 Match Control Ulcer Col-F | 1.1 | 117106 Normal Cartilage Rep20 | 0.9 | |
| 112381 Ulcer Col-M | 3.0 | 113663 Bone3 Normal | 3.1 | |
| 112735 Match Control Ulcer Col-M | 9.9 | 113664 Synovium3 Normal | 0.0 | |
| 112382 Ulcer Col-M | 8.7 | 113665 Syn Fluid Cells3 Normal | 1.0 | |
| 112394 Match Control Ulcer Col-M | 2.8 | 117107 Normal Cartilage Rep22 | 8.3 | |
| 112383 Ulcer Col-M | 37.6 | 113667 Bonn4 Normal | 11.9 | |
| 112736 Match Control Ulcer Col-M | 2.2 | 113668 Synovium4 Normal | 23.0 | |
| 112423 Psoriasis-F | 8.9 | 113669 Syn Fluid Cells4 Normal | 20.9 | |

**Table 7. Ardais Panel 1.1**

| Column A - Rel. Exp.(%) Ag821, Run 325595050 | | | |
|---|---|---|---|
| Tissue Name | A | Tissue Name | A |
| Lung adenocarcinoma SI A | 3.7 | Lung SCC SI A | 6.3 |
| Lung adenocarcinoma SI B | 13.4 | Lung SCC SI B NAT | 7.5 |
| Lung adenocarcinoma SI B NAT | 9.5 | Lung SCC SI C | 0.8 |
| Lung adenocarcinoma SI C | 0.6 | Lung SCC SI C NAT | 13.3 |
| Lung adenocarcinoma SI C NAT | 6.0 | Lung SCC SI D | 9.9 |
| Lung adenocarcinoma SII A | 6.3 | Lung SCC SI D NAT | 1.7 |
| Lung adenocarcinoma SII A NAT | 6.8 | Lung SCC SII A | 3.7 |
| Lung adenocarcinoma SII C NAT | 28.1 | Lung SCC SII B | 6.4 |
| Lung adenocarcinoma SIII A | 10.7 | Lung SCC SIII A | 1.8 |
| Lung adenocarcinoma SIII B | 2.5 | Lung SCC SIII A NAT | 3.0 |
| Lung adenocarcinoma SIII C | **100.0** | | |

**Table 8. Ardais Panel v.1.0**

| Column A - Rel. Exp. (%) Ag821, Run 263526496 | | | |
|---|---|---|---|
| **Tissue Name** | **A** | **Tissue Name** | **A** |
| 136799_Lung cancer(362) | 6.1 | 136787_lung cancer(356) | 45.1 |
| 136800_Lung NAT(363) | 0.8 | 136788_lungNAT(357) | 17.2 |
| 136813_Lung cancer(372) | 19.3 | 136804_Lung cancer(369) | **100.0** |
| 136814_Lung NAT(373) | 8.5 | 136805_Lung NAT(36A) | 11.1 |
| 136815_Lung cancer(374) | 10.6 | 136806_Lung cancer(36B) | 13.8 |
| 136816 Lung NAT(375) | 19.5 | 136807_Lung NAT(36C) | 6.3 |
| 136791_Lung cancer(35A) | 34.9 | 136789_lung cancer(358) | 59.5 |
| 136795_Lung cancer(35E) | 9.3 | 136802_Lung cancer(365) | 9.0 |
| 136797_Lung cancer(360) | 11.1 | 136803_Lung cancer(368) | 6.9 |
| 136794_lung NAT(35D) | 26.1 | 136811_Lung cancer(370) | 80.7 |
| 136818_Lung NAT(377) | 5.1 | 136810_LungNAT(36F) | 2.4 |

**Table 9. CNS_neurodegeneration_v1.0**

| Column A - Rel. Exp.(%) Ag821, Run 271695186 | | | |
|---|---|---|---|
| **Tissue Name** | **A** | **Tissue Name** | **A** |
| AD1 Hippo | 9.7 | Control (Path) 3 Temporal Ctx | 6.2 |
| AD 2 Hippo | 14.5 | Control (Path) 4 Temporal Ctx | 22.5 |
| AD 3 Hippo | 7.3 | AD 1 Occipital Ctx | 10.9 |
| AD 4 Hippo | 9.1 | AD 2 Occipital Ctx (Missing) | 0.0 |
| AD 5 Hippo | 29.1 | AD 3 Occipital Ctx | 6.7 |
| AD 6 Hippo | 97.3 | AD 4 Occipital Ctx | 9.1 |
| Control 2 Hippo | 32.3 | AD 5 Occipital Ctx | 9.6 |
| Control 4 Hippo | 19.2 | AD 6 Occipital Ctx | 20.6 |
| Control (Path) 3 Hippo | 6.4 | Control 1 Occipital Ctx | 3.4 |
| AD 1 Temporal Ctx | 25.2 | Control 2 Occipital Ctx | 36.6 |
| AD 2 Temporal Ctx | 37.9 | Control 3 Occipital Ctx | 9.7 |
| AD 3 Temporal Ctx | 4.1 | Control 4 Occipital Ctx | 6.8 |
| AD 4 Temporal Ctx | 15.0 | Control (Path) 1 Occipital Ctx | 80.7 |
| AD 5 Inf Temporal Ctx | 60.7 | Control (Path) 2 Occipital Ctx | 12.2 |
| AD 5 Sup Temporal Ctx | 28.5 | Control (Path) 3 Occipital Ctx | 5.4 |
| AD 6 Inf Temporal Ctx | 97.3 | Control (Path) 4 Occipital Ctx | 14.8 |
| AD 6 Sup Temporal Ctx | **100.0** | Control 1 Parietal Ctx | 2.5 |
| Control 1 Temporal Ctx | 4.9 | Control 2 Parietal Ctx | 25.0 |
| Control 2 Temporal Ctx | 14.5 | Control 3 Parietal Ctx | 9.9 |
| Control 3 Temporal Ctx | 7.5 | Control (Path) 1 Parietal Ctx | 40.9 |
| Control 3 Temporal Ctx | 15.9 | Control (Path) 2 Parietal Ctx | 27.5 |

(continued)

| Column A - Rel. Exp.(%) Ag821, Run 271695186 | | | |
|---|---|---|---|
| **Tissue Name** | **A** | **Tissue Name** | **A** |
| Control (Path) 1 Temporal Ctx | 41.2 | Control (Path) 3 Parietal Ctx | 2.2 |
| Control (Path) 2 Temporal Ctx | 35.6 | Control (Path) 4 Parietal Ctx | 53.6 |

**Table 10. General_screening_panel_v1.6**

| Column A - Rel. Exp.(%) Ag821, Run 278391652 | | | |
|---|---|---|---|
| **Tissue Name** | **A** | **Tissue Name** | **A** |
| Adipose | 0.0 | Renal ca. TK-10 | 31.4 |
| Melanoma* Hs688(A).T | 0.0 | Bladder | 0.2 |
| Melanoma* Hs688(B).T | 0.0 | Gastric ca. (liver met.) NCI-N87 | 0.0 |
| Melanoma* M14 | 0.0 | Gastric ca. KATO III | |
| Melanoma* LOXIMVI | 0.0 | Colon ca. SW-948 | 0.0 |
| Melanoma* SK-MEL-5 | 0.0 | Colon ca. SW480 | 0.0 |
| Squamous cell carcinoma SCC-4 | 0.0 | Colon ca.* (SW480 met) SW620 | 0.0 |
| Testis Pool | 0.1 | Colon ca. HT29 | 0.4 |
| Prostate ca.* (bone met) PC-3 | 0.0 | Colon ca. HCT-116 | 0.0 |
| Prostate Pool | 0.0 | Colon ca. CaCo-2 | 97.3 |
| Placenta | 0.0 | Colon cancer tissue | 0.0 |
| Uterus Pool | 0.0 | Colon ca. SW1116 | 0.0 |
| Ovarian ca. OVCAR-3 | 0.0 | Colon ca. Colo-205 | 0.0 |
| Ovarian ca. SK-OV-3 | 4.1 | Colon ca. SW-48 | 0.0 |
| Ovarian ca. OVCAR-4 | 0.0 | Pool | 0.1 |
| Ovarian ca. OSCAR-5 | 35.6 | Small Intestine Pool | 0.1 |
| Ovarian ca. IGROV-1 | 6.5 | Stomach Pool | 0.1 |
| Ovarian ca. OVCAR-8 | 0.0 | Bone Marrow Pool | 0.0 |
| Ovary | 0.0 | Fetal Heart | 0.1 |
| Breast ca. MCF-7 | 0.0 | Heart Pool | 0.0 |
| Breast ca. MDA-MB-231 | 0.0 | Lymph Node Pool | 0.1 |
| Breast ca. BT 549 | 0.1 | Fetal Skeletal Muscle | 0.1 |
| Breast ca. T47D | 0.0 | Muscle Pool | 0.0 |
| Breast ca. MDA-N | 0.0 | Spleen Pool | 0.1 |
| Breast Pool | 0.1 | Thymus Pool | 0.1 |
| Trachea | 0.0 | CNS cancer (glio/astro) U87-MG | 0.0 |
| Lung | 0.0 | CNS cancer (glio/astro) U-118-MG | 0.0 |
| Fetal Lung | 0.1 | CNS cancer (neuro;met) SK-N-AS | 0.0 |
| Lung ca. NCI-N417 | 0.0 | CNS cancer (astro) SF-539 | 0.0 |
| Lung ca. LX-1 | 0.1 | CNS cancer (astro) SNB-75 | 0.1 |

(continued)

| Column A - Rel. Exp.(%) Ag821, Run 278391652 | | | |
|---|---|---|---|
| Tissue Name | A | Tissue Name | A |
| Lung ca. NCI-H146 | 0.0 | CNS cancer (glio) SNB-19 | 6.3 |
| Lung ca. SHP-77 | 0.0 | CNS cancer (glio) SF-295 | 0.0 |
| Lung ca. A549 | 28.3 | Brain (Amygdala) Pool | 0.1 |
| Lung ca. NCI-H526 | 0.0 | Brain (cerebellum) | 0.2 |
| Lung ca. NCI-H23 | 0.0 | Brain (fetal) | 0.2 |
| Lung ca. NCI-H460 | 0.0 | Brain (Hippocampus) Pool | 0.1 |
| Lung ca. HOP-62 | 0.1 | Cerebral Cortex Pool | 0.1 |
| Lung ca. NCI-H522 | 0.0 | Brain (Substantia nigra) Pool | 0.1 |
| Liver | 0.0 | Brain (Thalamus) Pool | 0.2 |
| Fetal Liver | 0.5 | Brain (whole) | 0.2 |
| Liver ca. HepG2 | 1.7 | Spinal Cord Pool | 0.1 |
| Kidney Pool | 0.1 | Adrenal Gland | 0.0 |
| Fetal Kidney | 0.4 | Pituitary gland Pool | 0.0 |
| Renal ca. 786-0 | 100.0 | Salivary Gland | 0.0 |
| Renal ca. A498 | 18.9 | Thyroid (female) | 0.0 |
| Renal ca. ACHN | 59.0 | Pancreatic ca. CAPAN2 | 0.0 |
| Renal ca. UO-31 | 82.9 | Pancreas Pool | 0.1 |

**Table 11. HASS Panel v1.0**

| Column A - Rel. Exp.(%) Ag821, Run 248122719 | | | |
|---|---|---|---|
| Tissue Name | A | Tissue Name | A |
| MCF-7 C1 | 0.0 | U87-MG F1 (B) | 0.0 |
| IMCF-7 C2 | 0.0 | U87-MG F2 | 0.0 |
| MCF-7 C3 | 0.0 | U87-MG F3 | 0.0 |
| MCF-7 C4 | 0.0 | U87-MG F4 | 0.0 |
| MCF-7 C5 | 0.1 | U87-MG F5 | 0.0 |
| MCF-7 C6 | 0.0 | U87-MG F6 | 0.0 |
| MCF-7 C7 | 0.0 | 0.0 U87-MG F7 | 0.0 |
| MCF-7 C9 | 0.0 | U87-MG F8 | 0.0 |
| MCF-7 C10 | 0.0 | U87-MG F9 | 0.0 |
| MCF-7 C11 | 0.0 | U87-MG F10 | 0.0 |
| MCF-7 C12 | 0.0 | U87-MGF11 | 0.0 |
| MCF-7 C 13 | 0.0 | U87-MG F12 | 0.0 |
| MCF-7C15 0.0 | 0.0 | U87-MGF13 | 0.0 |
| MCF-7C16 | 0.0 | U87-MG F14 | 0.0 |
| MCF-7 C17 | 0.0 | U87-MG F15 | 0.0 |

(continued)

| Column A - Rel. Exp.(%) Ag821, Run 248122719 | | | |
|---|---|---|---|
| Tissue Name | A | Tissue Name | A |
| T24 D1 | 0.0 | U87-MG F16 | 0.0 |
| T24 D2 | 0.0 | U87-MG F17 | 0.0 |
| T24 D3 | 0.0 | LnCAP A1 | 0.0 |
| T24 D4 | 0.0 | LnCAP A2 | 0.0 |
| T24 D5 | 0.0 | LnCAP A3 | 0.0 |
| T24 D6 | 0.0 | LnCAP A4 | 0.0 |
| T24 D7 | 0.0 | LnCAP A5 | 0.0 |
| T24 D9 | 0.0 | LnCAP A6 | 0.0 |
| T24 D10 | 0.0 | LnCAP A7 | 0.0 |
| T24 D11 | 0.0 | LnCAP A8 | 0.0 |
| T24 D12 | 0.0 | LnCAP A9 | 0.0 |
| T24 D13 | 0.0 | LnCAP A10 | 0.0 |
| T24 D15 | 0.0 | LnCAP A11 | 0.0 |
| T24 D16 | 0.0 | LnCAP A12 | 0.0 |
| T24 D17 | 0.0 | LnCAP A13 | 0.0 |
| CAPaN B1 | 0.0 | LnCAP A14 | 0.0 |
| CAPaN B2 | 0.0 | LnCAP A15 | 0.0 |
| CAPaN B3 | 0.0 | LnCAP A16 | 0.0 |
| CAPaN B4 | 0.0 | LnCAP A17 | 0.0 |
| CAPaN B5 | 0.0 | Primary Astrocytes | 0.0 |
| CAPaN B6 | 0.0 | Primary Renal Proximal Tubule Epithelial cell A2 | 100.0 |
| CAPaN B7 | 0.0 | Primary melanocytes A5 | 0.0 |
| CAPaN B8 | 0.0 | 126443 - 341 medullo | 0.0 |
| CAPaN B9 | 0.0 | 126444 - 487 medullo | 0.1 |
| CAPaN B10 | 0.0 | 126445 - 425 medullo | 0.1 |
| CAPaN B11 | 0.0 | 126446 - 690 medullo | 0.2 |
| CAPaN B12 | 0.0 | 126447 - 54 adult glioma | 0.1 |
| CAPaN B13 | 0.0 | 126448 - 245 adult glioma | 0.1 |
| CAPaN B14 | 0.0 | 126449 - 317 adult glioma | 0.0 |
| CAPaN B15 | 0.0 | 126450 - 212 glioma | 0.2 |
| CAPaN B16 | 0.0 | 126451 - 456 glioma | 0.2 |
| CAPaN B17 | 0.0 | | |

**Table 12. Panel 1.2**

| Column A - Rel. Exp.(%) Ag821, Run 118348335 | | | |
|---|---|---|---|
| **Tissue Name** | **A** | **Tissue Name** | **A** |
| Endothelial cells | 0.0 | Renal ca. 786-0 | 65.5 |
| Heart (Fetal) | 0.0 | Renal ca. A498 | 24.7 |
| Pancreas | 0.9 | Renal ca. RXF 393 | 9.5 |
| Pancreatic ca. CAPAN 2 | 0.0 | Renal ca. ACHN | **100.0** |
| Adrenal Gland | 0.2 | Renal ca. UO-31 | 29.7 |
| Thyroid | 0.7 | Renal ca. TK-10 | 23.2 |
| Salivary gland | 0.4 | Liver | 0.2 |
| Pituitary gland | 0.5 | Liver (fetal) | 0.1 |
| Brain (fetal) | 0.3 | Liver ca. (hepatoblast) HepG2 | 2.4 |
| Brain (whole) | 0.3 | Lung | 0.3 |
| Brain (amygdala) | 0.2 | Lung (fetal) | 0.3 |
| Brain (cerebellum) | 0.3 | Lung ca. (small cell) LX-1 | 0.4 |
| Brain (hippocampus) | 0.3 | Lung ca. (small cell) NCI-H69 | 0.4 |
| Brain (thalamus) | 0.1 | Lung ca. (s.cell var.) SHP-77 | 0.1 |
| Cerebral Cortex | 0.4 | Lung ca. (large cell)NCI-H460 | 0.0 |
| Spinal cord | 0.2 | Lung ca. (non-sm. cell) A549 | 33.9 |
| glio/astro U87-MG | 0.0 | Lung ca. (non-s.cell) NCI-H23 | 0.1 |
| glio/astro U-118-MG | 0.0 | Lung ca. (non-s.cell) HOP-62 | 0.7 |
| astrocytoma SW1783 | 0.0 | Lung ca. (non-s.cl) NCI-H522 | 0.2 |
| neuro*; met SK-N-AS | 0.2 | Lung ca. (squam.) SW 900 | 0.2 |
| astrocytoma SF-539 | 0.1 | Lung ca. (squam.) NCI-H596 | 0.1 |
| astrocytoma SNB-75 | 0.0 | Mammary gland | 0.3 |
| glioma SNB-19 | 0.5 | Breast ca.* (pl.ef) MCF-7 | 0.2 |
| glioma U251 | 0.1 | Breast ca.* (pl.ef) MDA-MB-231 | 0.1 |
| glioma SF-295 | 0.0 | Breast ca.* (pl. ef) T47D | 0.3 |
| Heart | 0.6 | Breast ca. BT-549 | 0.1 |
| Skeletal Muscle | 0.2 | Breast ca. MDA-N | 0.0 |
| Bone marrow | 0.1 | Ovary | 0.1 |
| Thymus | 0.1 | Ovarian ca. OVCAR-3 | 0.1 |
| Spleen | 0.3 | Ovarian ca. OVCAR-4 | 0.2 |
| Lymph node | 0.2 | Ovarian ca. OVCAR-5 | 48.6 |
| Colorectal Tissue | 0.1 | Ovarian ca. OVCAR-8 | 0.1 |
| Stomach | 0.3 | Ovarian ca. IGROV-1 | 26.1 |
| Small intestine | 0.2 | Ovarian ca. (ascites) SK-OV-3 | 3.9 |
| Colon ca. SW480 | 0.0 | Uterus | 0.1 |
| Colon ca.* SW620 (SW480 met) | 0.2 | Placenta | 0.8 |
| Colon ca. HT29 | 0.5 | Prostate | 0.6 |

(continued)

| Column A - Rel. Exp.(%) Ag821, Run 118348335 | | | |
|---|---|---|---|
| Tissue Name | A | Tissue Name | A |
| Colon ca. HCT-116 | 0.0 | Prostate ca.* (bone met) PC-3 | 0.1 |
| Colon ca. CaCo-2 | 59.0 | Testis | 1.1 |
| Colon ca. Tissue (ODO3866) | 0.0 | Melanoma Hs688(A).T | 0.0 |
| Colon ca. HCC-2998 | 0.2 | Melanoma* (met) Hs688(B).T | 0.0 |
| Gastric ca.* (liver met) NCI-N87 | 0.2 | Melanoma UACC-62 | 0.0 |
| Bladder | 0.8 | Melanoma M14 | 0.0 |
| Trachea | 0.1 | Melanoma LOX IMVI | 0.0 |
| Kidney | 2.9 | Melanoma* (met) SK-MEL-5 | 0.0 |
| Kidney (fetal) | 0.0 | | |

**Table 13. Panel 3D**

| Column A - Rel. Exp.(%) Ag821, Run 164729956 | | | |
|---|---|---|---|
| Tissue Name | A | Tissue Name | A |
| Daoy- Medulloblastoma | 0.0 | Ca Ski- Cervical epidermoid carcinoma (metastasis) | 0.0 |
| TE671- Medulloblastoma | 0.0 | ES-2- Ovarian clear cell carcinoma | 0.0 |
| D283 Med- Medulloblastoma | 0.0 | Ramos- Stimulated with PMA/ionomycin 6h | 0.0 |
| PFSK-1- Primitive Neuroectodermal | 0.0 | Ramos- Stimulated with PMA/ionomycin 14h | 0.0 |
| XF-498- CNS | 0.0 | MEG-01- Chronic myelogenous leukemia (megokaryoblast) | 0.0 |
| SNB-78- Glioma | 0.0 | Raji- Burkitt's lymphoma | 0.0 |
| SF-268- Glioblastoma | 0.0 | Daudi- Burkitt's lymphoma | 0.1 |
| T98G- Glioblastoma | 0.0 | U266- B-cell plasmacytoma | 2.3 |
| SK-N-SH- Neuroblastoma (metastasis) | 0.0 | CA46- Burkitt's lymphoma | 0.0 |
| SF-295- Glioblastoma | 0.0 | RL- non-Hodgkin's B-cell lymphoma | 0.0 |
| Cerebellum | 0.1 | JM1-pre-B-cell lymphoma | 0.0 |
| Cerebellum | 0.0 | Jurkat- T cell leukemia | 0.0 |
| NCI-H292- Mucoepidermoid lung carcinoma | 0.0 | TF-1- Erythroleukemia | 0.0 |
| DMS-114- Small cell lung cancer | 0.0 | HUT 78- T-cell lymphoma | 0.0 |
| DMS-79- Small cell lung cancer | 0.3 | U937- Histiocytic lymphoma | 0.0 |
| NCI-H146- Small cell lung cancer | 0.0 | KU-812- Myelogenous leukemia | 0.0 |
| NCI-H526- Small cell lung cancer | 0.0 | 769-P- Clear cell renal carcinoma | 100.0 |
| NCI-N417- Small cell lung cancer | 0.0 | Caki-2- Clear cell renal carcinoma | 18.8 |
| NCI-H82- Small cell lung cancer | 1.6 | SW 839- Clear cell renal carcinoma | 37.4 |
| NCI-H157-Squamous cell lung NCI-H157-Squamous cancer (metastasis) | 0.0 | Rhabdoid kidney tumor | 0.0 |
| NCI-H1155- Large cell lung cancer | 0.0 | Hs766T- Pancreatic carcinoma (LN metastasis) | 0.1 |

(continued)

| Column A - Rel. Exp.(%) Ag821, Run 164729956 | | | |
|---|---|---|---|
| **Tissue Name** | **A** | **Tissue Name** | **A** |
| NCI-H1299- Large cell lung cancer | 0.0 | CAPAN-1- Pancreatic adenocarcinoma (liver metastasis) | 0.0 |
| NCI-H727- Lung carcinoid | 1.9 | SU86.86- Pancreatic carcinoma (liver metastasis) | 0.3 |
| NCI-UMC-11- Lung carcinoid | 14.4 | BxPC-3- Pancreatic adenocarcinoma | 0.0 |
| LX-1- Small cell lung cancer | 0.0 | HPAC- Pancreatic adenocarcinoma | 0.0 |
| Colo-205- Colon cancer | 0.1 | MIA PaCa-2- Pancreatic carcinoma | 0.0 |
| KM 12- Colon cancer | 0.0 | CFPAC-1- Pancreatic ductal adenocarcinoma | 4.4 |
| KM20L2- Colon cancer | 0.0 | PANC-1- Pancreatic epithelioid ductal carcinoma | 0.0 |
| NCI-H716- Colon cancer | 3.1 | T24- Bladder carcinma (transitional cell) | 0.0 |
| SW-48- Colon adenocarcinoma | 0.0 | 5637- Bladder carcinoma | 0.0 |
| SW1116- Colon adenocarcinoma | 0.0 | HT-1197- Bladder carcinoma | 5.6 |
| LS 174T- Colon adenocarcinoma | 0.0 | UM-UC-3- Bladder carcinma (transitional cell) | 0.0 |
| SW-948- Colon adenocarcinoma | 0.0 | A204- Rhabdomyosarcoma | 0.0 |
| SW-480- Colon adenocarcinoma | 0.0 | HT-1080- Fibrosarcoma | 0.0 |
| NCI-SNU-5- Gastric carcinoma | 0.0 | MG-63- Osteosarcoma | 0.0 |
| KATO III- Gastric carcinoma | 0.0 | SK-LMS-1- Leiomyosarcoma (vulva) | 0.0 |
| NCI-SNU-16- Gastric carcinoma | 0.0 | SJRH30- Rhabdomyosarcoma (met to bone marrow) | 0.0 |
| NCI-SNU-1- Gastric carcinoma | 0.8 | A431- Epidermoid carcinoma | 0.0 |
| RF-1- Gastric adenocarcinoma | 0.0 | WM266-4- Melanoma | 0.0 |
| RF-48- Gastric adenocarcinoma | 0.0 | DU 145- Prostate carcinoma (brain metastasis) | 0.0 |
| MKN-45- Gastric carcinoma | 11.3 | MDA-MB-468- Breast adenocarcinoma | 0.0 |
| NCI-N87- Gastric carcinoma | 0.0 | SCC-4- Squamous cell carcinoma of tongue | 0.0 |
| OVCAR-5- Ovarian carcinoma | 0.2 | SCC-9- Squamous cell carcinoma of tongue | 0.0 |
| RL95-2- Uterine carcinoma RL95-2-Uterine carcinoma | 0.0 | SCC-15- Squamous cell carcinoma of tongue | 0.0 |
| HelaS3- Cervical adenocarcinoma | 0.0 | CAL 27- Squamous cell carcinoma of tongue | 0.0 |

**Table 14. Panel 4D**

| Column A - Rel. Exp.(%) Ag821, Run 145386336<br>Column B - Rel. Exp.(%) Ag821, Run 145608640 | | | | | |
|---|---|---|---|---|---|
| **Tissue Name** | **A** | **B** | **Tissue Name** | **A** | **B** |
| Secondary Th1 act | 17.3 | 18.2 | HUVEC IL-1beta | 0.2 | 0.1 |
| Secondary Th2 act | 4.7 | 7.3 | HUVEC IFN gamma | 0.2 | 0.6 |
| Secondary Tr1 act | 12.7 | 7.2 | HUVEC TNF alpha + IFN gamma | 0.1 | 0.0 |

(continued)

| Tissue Name | A | B | Tissue Name | A | B |
|---|---|---|---|---|---|
| Secondary Th1 rest | 12.9 | 14.2 : | HUVEC TNF alpha + IL4 | 0.3 | 0.2 |
| Secondary Th2 rest | 2.7 | 2.7 | HUVEC IL-11 | 0.2 | 0.3 |
| Secondary Tr1 rest | 5.9 | 5.6 | Lung Microvascular EC none | 0.8 | 0.7 |
| Primary Th1 act | 4.5 | 6.5 | Lung Microvascular EC TNFalpha + IL-1beta | 0.3 | 0.1 |
| Primary Th2 act | 2.8 | 2.0 | Microvascular Dermal EC none | 1.2 | 0.9 |
| Primary Tr1 act Primary Tr1 act | 5.7 | 7.1 | Microsvasular Dermal EC TNFalpha + IL-1beta | 0.4 | 0.4 |
| Primary Th1 rest | 69.7 | 54.0 | Bronchial epithelium TNFalpha + ILlbeta | 0.7 | 0.4 |
| Primary Th2 rest | 23.7 | 29.9 | Small airway epithelium none | 0.1 | 0.1 |
| Primary Tr1 rest Primary Tr1 rest | 5.4 | 3.6 | Small airway epithelium TNFalpha + IL-1beta | 2.7 | 2.1 |
| CD45RA CD4 lymphocyte act | 0.7 | 0.5 | Coronery artery SMC rest | 0.2 | 0.1 |
| CD45RO CD4 lymphocyte act | 16.2 | 10.9 | Coronery artery SMC TNFalpha + IL-1 beta | 0.2 | 0.3 |
| CD8 lymphocyte act | 0.5 | 1.0 | Astrocytes rest | 1.0 | 0.3 |
| Secondary CD8 lymphocyte rest | 0.9 | 0.7 | Astrocytes TNFalpha + IL-1beta | 1.1 | 0.8 |
| Secondary CD8 lymphocyte act | 6.1 | 6.8 | KU-812 (Basophil) rest | 0.3 | 0.4 |
| CD4 lymphocyte none | 0.3 | 0.5 | KU-812 (Basophil) PMA/ionomycin | 0.9 | 0.9 |
| 2ry Th1/Th2/Tr1_anti-CD95 CH11 | 8.4 | 7.9 | CCD1106 (Keratinocytes) none | 0.0 | 0.1 |
| LAK cells rest | 5.2 | 3.0 | CCD1106 (Keratinocytes) TNFalpha + IL-1beta | 1.3 | 0.1 |
| LAK cells IL-2 | 9.1 | 11.2 | Liver cirrhosis | 2.5 | 2.0 |
| LAK cells IL-2+IL-12 | 14.8 | 13.0 | Lupus kidney | 100.0 | 80.7 |
| LAK cells IL-2+IFN gamma | 12.7 | 11.3 | NCI-H292 none | 0.0 | 0.2 |
| LAK cells IL-2+IL-18 | 8.9 | 7.5 | NCI-H292 IL-4 | 0.6 | 0.5 |
| LAK cells PMA/ionomycin | 6.6 | 5.7 | NCI-H292 IL-9 | 0.1 | 0.1 |
| NK Cells IL-2 rest | 11.1 | 6.9 | NCI-H292 IL-13 | 0.0 | 0.0 |
| Two Way MLR 3 day | 11.0 | 7.0 | NCI-H292 IFN gamma | 0.1 | 0.0 |
| Two Way MLR 5 day | 1.5 | 1.9 | HPAEC none | 0.5 | 0.2 |
| Two Way MLR 7 day | 20.7 | 3.4 | HPAEC TNF alpha + IL-1 beta | 0.4 | 0.2 |
| PBMC rest | 1.5 | 0.5 | Lung fibroblast none | 0.6 | 0.3 |
| PBMC PWM | 22.8 | 20.0 | Lung fibroblast TNF alpha + IL-1 beta | 0.1 | 0.0 |
| PBMC PHA-L | 9.6 | 8.7 | Lung fibroblast IL-4 | 0.4 | 0.3 |
| Ramos (B cell) none | 0.2 | 0.5 | Lung fibroblast IL-9 | 0.1 | 0.3 |
| Ramos (B cell) ionomycin | 0.9 | 0.5 | Lung fibroblast IL-13 | 0.7 | 0.9 |
| B lymphocytes PWM | 3.5 | 2.3 | Lung fibroblast IFN gamma | 0.2 | 0.4 |
| B lymphocytes CD40L and IL-4 | 0.8 | 0.5 | Dermal fibroblast CCD1070 rest | 0.2 | 0.4 |
| EOL-1 dbcAMP | 0.1 | 0.2 | Dermal fibroblast CCD1070 TNF alpha | 12.1 | 8.5 |
| EOL-1 dbcAMP PMA/ionomycin | 0.4 | 0.2 | Dermal fibroblast CCD1070 IL-1 beta | 0.1 | 0.4 |
| Dendritic cells none | 6.0 | 6.6 | Dermal fibroblast IFN gamma | 0.2 | 0.2 |

Column A - Rel. Exp.(%) Ag821, Run 145386336
Column B - Rel. Exp.(%) Ag821, Run 145608640

(continued)

| Column A - Rel. Exp.(%) Ag821, Run 145386336<br>Column B - Rel. Exp.(%) Ag821, Run 145608640 | | | | | |
|---|---|---|---|---|---|
| **Tissue Name** | **A** | **B** | **Tissue Name** | **A** | **B** |
| Dendritic cells LPS | 6.3 | 4.3 | Dermal fibroblast IL-4 | 0.1 | 0.2 |
| Dendritic cells anti-CD40 | 13.1 | 6.1 | IBD Colitis 2 | 0.5 | 0.1 |
| Monocytes rest | 0.8 | 0.6 | IBD Crohn's | 0.1 | 0.1 |
| Monocytes LPS | 7.5. | 3.9 | Colon | 0.2 | 0.6 |
| Macrophages rest | 15.7 | 15.2 | Lung | 0.7 | 0.6 |
| Macrophages LPS | 7.6 | 17.5 | Thymus | 97.9 | 100.0 |
| HUVEC none | 0.1 | 0.2 | Kidney | 4.7 | 1.8 |
| HUVEC starved | 0.7 | 1.4 | | | |

**Table 15. Panel 5 Islet**

| Column A - Rel. Exp.(%) Ag821, Run 268362824 | | | |
|---|---|---|---|
| **Tissue Name** | **A** | **Tissue Name** | **A** |
| 97457_Patient-02go_adipose | 0.0 | 94709_Donor 2 AM - A_adipose | 0.0 |
| 97476_Patient-07sk_skeletal muscle | 0.0 | 94710_Donor 2 AM - B_adipose | 0.0 |
| 97477_Patient-07ut_uterus | 0.1 | 94711_Donor 2 AM - C_adipose | 0.0 |
| 97478_Patient-07pl_placenta | 0.0 | 94712_Donor 2 AD - A_adipose | 0.1 |
| 99167_Bayer Patient 1 | 0.0 | 94713_Donor 2 AD - B_adipose | 0.2 |
| 97482_Patient-08ut_uterus | 0.1 | 94714_Donor 2 AD - C_adipose | 0.1 |
| 97483_Patient-08pl_placenta | 0.0 | 94742_Donor 3 U - A_Mesenchymal Stem Cells | 0.0 |
| 97486_Patient-09sk_skeletal muscle | 0.0 | 94743_Donor 3 U - B_Mesenchymal | 0.0 |
| | | Stem Cells | |
| 97487_Patient-09ut_uterus | 0.0 | 94730_Donor 3 AM - A_adipose | 0.0 |
| 97488_Patient-09pl_placenta | 0.0 | 94731_Donor 3 AM - B_adipose | 0.0 |
| 97492 Patient-10ut_uterus | 0.1 | 94732_Donor 3 AM - C_adipose | 0.0 |
| 97493 Patient-10pl_placenta | 0.0 | 94733_Donor 3 AD - A-adipose | 0.2 |
| 97495_Patient-11go_adipose | 0.0 | 94734_Donor 3 AD - B_adipose | 0.1 |
| 97496_Patient-11sk_skeletal muscle | 0.0 | 94735_Donor 3 AD - C_adipose | 0.1 |
| 97497_Patient-11ut_uterus | 0.1 | 77138_Liver_HepG2untreated | 9.5 |
| 97498_Patient-11pl_placenta | 0.0 | 73556_Heart_Cardiac stromal cells (primary) | 0.0 |
| 97500_Patient-12go_adipose | 0.0 | 81735_Small Intestine | 0.5 |
| 97501_Patient-12sk_skeletal muscle | 0.0 | 72409_Kidney_Proximal Convoluted Tubule | 82.9 |
| 97502_Patient-12ut_uterus | 0.1 | 82685_Small intestine_Duodenum | 0.0 |
| 97503_Patient-12pl_polacenta | 0.0 | 90650_Adrenal_Adrenocortical adenoma | 0.0 |
| 94721_Donor 2 U - A_Mesenchymal Stem Cells | 0.0 | 72410_Kidney_HRCE | **100.0** |
| 94722_Donor 2 U - B_Mesenchymal Stem Cells | 0.0 | 72411_Kidney_HRE | 70.7 |
| 94723_Donor 2 U - C_Mesenchymal Stem Cells | 0.0 | 73139_Uterus_Uterine smooth muscle cells | 0.0 |

**Table 16. general oncology screening panel_v_2.4**

| Column A - Rel. Exp.(%) Ag821, Run 258052111<br>Column B - Rel. Exp.(%) Ag821, Run 258680990<br>Column C - Rel. Exp.(%) Ag821, Run 259733172 | | | | | | | |
|---|---|---|---|---|---|---|---|
| **Tissue Name** | **A** | **B** | **C** | **Tissue Name** | **A** | **B** | **C** |
| Colon cancer 1 | 0.1 | 0.1 | 0.1 | Bladder NAT 2 | 0.0 | 0.0 | 0.0 |
| Colon NAT 1 | 0.0 | 0.0 | 0.0 | Bladder NAT 3 | 0.0 | 0.0 | 0.0 |
| Colon cancer 2 | 0.0 | 0.0 | 0.0 | Bladder NAT 4 | 0.0 | 0.0 | 0.0 |
| Colon NAT 2 | 0.1 | 0.1 | 0.1 | Prostate adenocarcinoma 1 | 0.1 | 0.2 | 0.2 |
| Colon cancer 3 | 0.2 | 0.2 | 0.2 | Prostate adenocarcinoma 2 | 0.0 | 0.0 | 0.0 |
| Colon NAT 3 | 0.6 | 0.7 | 0.7 | Prostate adenocarcinoma 3 | 0.0 | 0.0 | 0.0 |
| Colon malignant cancer 4 | 0.1 | 0.0 | 0.0 | Prostate adenocarcinoma 4 | 0.1 | 0.0 | 0.6 |
| Colon NAT 4 | 0.2 | 0.2 | 0.3 | Prostate NAT 5 | 0.0 | 0.0 | 0.0 |
| Lung cancer 1 | 0.0 | 0.1 | 0.1 | Prostate adenocarcinoma 6 | 0.0 | 0.0 | 0.0 |
| Lung NAT 1 | 0.0 | 0.0 | 0.0 | Prostate adenocarcinoma 7 | 0.0 | 0.0 | 0.0 |
| Lung cancer 2 | 3.7 | 1.9 | 2.7 | Prostate adenocarcinoma 8 | 0.0 | 0.0 | 0.0 |
| Lung NAT 2 | 0.0 | 0.0 | 0.0 | Prostate adenocarcinoma 9 | 0.2 | 0.0 | 0.1 |
| Squamous cell carcinoma 3 | 0.0 | 0.0 | 0.1 | Prostate NAT 10 | 0.0 | 0.0 | 0.0 |
| Lung NAT 3 | 0.0 | 0.0 | 0.0 | Kidney cancer 1 | 6.9 | 2.6 | 4.5 |
| Metastatic melanoma 1 | 1.1 | 0.1 | 0.1 | Kidney NAT 1 | 2.8 | 2.1 | 2.5 |
| Melanoma 2 | 0.0 | 0.0 | 0.0 | Kidney cancer 2 | **100.0** | **100.0** | **100.0** |
| Melanoma 3 | 0.0 | 0.0 | 0.0 | Kidney NAT 2 | 1.8 | 0.9 | 1.3 57.4 |
| Metastatic melanoma 4 | 0.2 | 0.1 | 0.2 | Kidney cancer 3 | 66.9 | 48.0 | |
| Metastatic melanoma 5 | 0.4 | 0.2 | 0.3 | Kidney NAT 3 | 0.5 | 0.2 | 0.3 |
| Bladder cancer 1 | 0.0 | 0.0 | 0.0 | Kidney cancer 4 | 23.0 | 11.7 | 22.7 |
| Bladder NAT 1 | 0.0 | 0.0 | 0.0 | Kidney NAT 4 | 1.2 | 0.9 | 1.1 |
| Bladder cancer 2 | 0.0 | 0.0 | 0.0 | | | | |

**Table 17. Ardais Kidney 1.0**

| Column A - Rel. Exp.(%) Ag821, Run 343519949 | | | |
|---|---|---|---|
| **Tissue Name** | **A** | **Tissue Name** | **A** |
| Kidney NAT(10DA) | 2.6 | Kidney cancer(10CO) | 13.2 |
| Kidney NAT(10B1) | 6.2 | Kidney cancer(10D0) | 94.0 |
| Kidney NAT(10DE) | 0.6 | Kidney cancer(10C8) | 0.1 |
| Kidney NAT(10DD) | 12.0 | Kidney cancer(10B4) | 0.5 |
| Kidney NAT(10DC) | 3.8 | Kidney NAT(10C5) | 3.6 |
| Kidney NAT(10DB) | 9.6 | Kidney cancer(10C4) | 45.7 |
| Kidney NAT(10D9) | 4.9 | Kidney NAT(10C3) | 4.4 |
| Kidney cancer(10D3) | 1.4 | Kidney cancer(10C2) | 45.4 |

(continued)

| Column A - Rel. Exp.(%) Ag821, Run 343519949 | | | |
|---|---|---|---|
| Tissue Name | A | Tissue Name | A |
| Kidney cancer(10CC) | 17.2 | Kidney NAT(10BF) | 4.8 |
| Kidney cancer(10D8) | 11.3 | Kidney cancer(10BE) | 0.1 |
| Kidney cancer(10CF) | 0.2 | Kidney NAT(10BD) | 4.8 |
| Kidney cancer(10D6) | 0.2 | Kidney cancer(10BC) | 39.8 |
| Kidney cancer(10CE) | 8.7 | Kidney NAT(10B9) | 0.8 |
| Kidney cancer(10D5) | 0.0 | Kidney cancer(10B8) | 100.0 |
| Kidney cancer(10CD) | 8.5 | Kidney NAT(10B7) | 3.0 |
| Kidney cancer(10D4) | 1.2 | Kidney cancer(10B6) | 0.5 |
| Kidney cancer(10CB) | 20.4 | Kidney NAT(10AD) | 0.2 |
| Kidney cancer(10D2) | 21.9 | Kidney cancer(10AC) | 0.7 |
| Kidney cancer(10CA) | 19.3 | Kidney NAT(10AB) | 1.0 |
| Kidney cancer(10D1) | 33.4 | Kidney cancer(10AA) | 10.0 |
| Kidney cancer(10C9) | 24.3 | Kidney NAT(10A9) | 1.1 |
| Kidney cancer(10C6) | 0.1 | Kidney cancer(10A8) | 0.1 |

**AI_comprehensive panel_v1.0 Summary:** The expression of the transcript is widespread at moderate to low levels with higher expression detected in lung tissue from patients with emphysema (CT=31) as compared to expression in normal lung (CTs=34-37).

**Ardais Panel 1.1 Summary:** Ag821 Highest expression of this gene is seen in a lung cancer sample (CT=27.4). This expression is consistent with the expression seen in Ardais Panel v1.0. Please see that panel for futher discussion of this gene in lung cancer.

**Ardais Panel v.1.0 Summary:** Ag821 Highest expression of this gene is seen in a lung cancer sample (CT=29.2). In addition, this gene is consistently over-expressed in lung cancer when compared to matched normal tissue samples. Thus, expression of this gene could be used to differentiate between the lung cancer samples and other samples on this panel, and as a marker of lung cancer. Furthermore, therapeutic modulation of the expression or function of this gene or gene product may be useful in the treatment of lung cancer.

**CNS_neurodegeneration_v1.0 Summary:** Ag821 This experiment confirms the expression of the CG93088-01 gene at low levels in the brain in an independent group of individuals. This gene appears to be up-regulated in the temporal cortex of Alzheimer's disease patients when compared with non-demented controls. Thus, therapeutic modulation of the expression or function of this gene or gene product can slow or stop the progression of Alzheimer's disease.

**General_screening_panel_v1.6 Summary:** Ag821 This gene is highly and specifically expressed by kidney cancer cell lines, suggesting that this gene product can be an effective kidney cancer marker. High levels of expression are also seen in a single colon cancer cell line, ovarian cancer cell lines and a lung cancer cell line. Thus, modulation of the expression or function of this gene can be useful in the treatment of kidney cancer.

**HASS Panel v1.0 Summary:** Ag821 Detectable levels of expression are limited to a kidney derived sample, consistent with expression in other panels.

**Panel 1.2 Summary:** Ag821 Expression in this panel is in agreement with Panel 1.6. Please see that panel for discussion of this gene.

**Panel 3D Summary:** Ag821 Expression of this gene is limited to cell lines from renal, gastric, lung, pancreatic, bladder, myeloma, and colon cancers.

**Panel 4D Summary:** Ag821 Two experiments with the same probe and primer set produce results that are in excellent agreement. The transcript is highly expressed in thymus and lupus kidney (CTs=27-28) but not in control kidney tissues or in normal colon. It is also induced in resting primary Th1 and Th2 cells, but is expressed in lower levels in naive T cells and in chronically activated T cells. The resting primary T cell RNA comes from cultures of T cells, purified from cord blood, stimulated with CD3 and CD28 monoclonal antibodies in the presence of either IL-12 (Th1 cultures) or IL-4 (Th2 cultures). These cultures contain many T cells that have not yet committed to the Th1 or Th2 pathway (Th0 cells). Thus, humanized monoclonal antibodies blocking Th2 effector T cell function would decrease inflammation associated with asthma, lupus and emphysema.

**Panel 5 Islet Summary:** Ag821 Detectable levels of expression are limited to kidney derived samples (CTs=26), consistent with other panels.

**general oncology screening panel_v_2.4 Summary:** Ag821 Very high levels of expression of this gene are seen in kidney cancer (CTs=24-25). Furthermore, this gene is more highly expressed in kidney cancer than in the corresponding normal adjacent tissue. Thus, expression of this gene could be used as a marker of this cancer. Furthermore, therapeutic modulation of the expression or function of this gene product can be useful in the treatment of kidney cancer.

**Ardais Kidney 1.0** Ag821 Highest expression of this gene is seen in a kidney cancer sample. Furthermore, this gene is consistently expressed at higher levels in kidney cancer than in the corresponding normal adjacent tissue, in agreement with previous panels. Thus, expression of this gene could be used as a marker of this cancer. Furthermore, therapeutic modulation of the expression or function of this gene product can be useful in the treatment of kidney cancer.

## B. CG51373-01: Nephrin

Expression of gene CG51373-01 was assessed using the primer-probe sets Ag271b, described in Table 18. Results of the RTQ-PCR runs are shown in Table 19 and Table 20.

**Table 18. Probe Name Ag271b**

| Primers | Sequences | Length | Start Position | SEQ ID No |
|---|---|---|---|---|
| Forward | 5'-caccgtgagccaactgcttat-3' | 21 | 792 | 36 |
| Probe | TET-5'-agacacgccctatgtccaggtccg-3'-TAMRA | 24 | 821 | 37 |
| Reverse | 5'-ttcgttcatgcttcggcaa-3' | 19 | 849 | 38 |

**Table 19. Panel 2.2**

| Column A - Rel. Exp.(%) Ag271b, Run 175148876 | | | |
|---|---|---|---|
| **Tissue Name** | **A** | **Tissue Name** | **A** |
| Normal Colon | 13.7 | Kidney Margin (OD04348) | **100.0** |
| Colon cancer (OD06064) | | 45.4 Kidney malignant cancer (OD06204B) | 6.1 |
| Colon Margin (OD06064) | 6.7 | Kidney normal adjacent tissue (OD06204E) | 12.3 |
| Colon cancer (OD06159) | 1.7 | Kidney Cancer (OD04450-01) | 68.8 |
| Colon Margin (OD06159) | 7.0 | Kidney Margin (OD04450-03) | 24.0 |
| Colon cancer (OD06297-04) | 5.3 | Kidney Cancer 8120613 | 0.0 |
| Colon Margin (OD06297-05) | 7.4 | Kidney Margin 8120614 | 11.7 |
| CC Gr.2 ascend colon (OD03921) | 10.5 | Kidney Cancer 9010320 | 15.5 |
| CC Margin (ODO3921) | 13.3 | Kidney Margin 9010321 | 16.6 |
| Colon cancer metastasis (OD06104) | 1.8 | Kidney Cancer 8120607 | 70.7 |
| Lung Margin (OD06104) | 3.0 | Kidney Margin 8120608 | 11.4 |
| Colon mets to lung (OD04451-01) | 21.0 | Normal Uterus | 36.6 |
| Lung Margin (OD04451-02) | 10.0 | Uterine Cancer 064011 | 11.7 |
| Normal Prostate | 2.0 | Normal Thyroid | 1.6 |
| Prostate Cancer (OD04410) | 3.9 | Thyroid Cancer 064010 | 10.2 |
| Prostate Margin (OD04410) | 10.3 | Thyroid Cancer A302152 | 9.3 |
| Normal Ovary | 78.5 | Thyroid Margin A302153 | 4.9 |

(continued)

| Column A - Rel. Exp.(%) Ag271b, Run 175148876 | | | |
|---|---|---|---|
| Tissue Name | A | Tissue Name | A |
| Ovarian cancer (OD06283-03) | 14.3 | Normal Breast | 31.4 |
| Ovarian Margin (OD06283-07) | 16.3 | Breast Cancer (OD04566) | 3.1 |
| Ovarian Cancer 064008 | 41.8 | Breast Cancer 1024 | 0.0 |
| Ovarian cancer (OD06145) | 36.6 | Cancer (OD04590-01) | 10.2 |
| Ovarian Margin (OD06145) | 33.7 | Breast Cancer Mets (OD04590-03) | 19.1 |
| Ovarian cancer (OD06455-03) | 6.0 | Breast Cancer Metastasis (OD04655-05) | 6.0 |
| Ovarian Margin (OD06455-07) | 18.7 | Breast Cancer 064006 | 14.2 |
| Normal Lung | 20.7 | Breast Cancer 9100266 | 12.5 |
| Invasive poor diff. lung adeno (OD04945-01 | 3.1 | Breast Margin 9100265 | 11.4 |
| Lung Margin (ODO4945-03) | 10.6 | Breast Cancer A209073 | 15.7 |
| Lung Malignant Cancer (OD03126) | 5.8 | Breast Margin A2090734 | 48.6 |
| Lung Margin (OD03126) | 10.4 | Breast cancer (OD06083) | 14.3 |
| Lung Cancer (OD05014A) | 6.2 | Breast cancer node metastasis (OD06083) | 15.6 |
| Lung Margin (OD05014B) | 11.5 | Normal Liver | 4.3 |
| Lung cancer (OD06081) | 5.3 | Liver Cancer 1026 12.3 | 12.3 |
| Lung Margin (OD06081) | 4.1 | Liver Cancer 1025 | 12.5 |
| Lung Cancer (OD04237-01) | 3.2 | Liver Cancer 6004-T | 12.4 |
| Lung Margin (OD04237-02) | 37.9 | Liver Tissue 6004-N | 3.0 |
| Ocular Melanoma Metastasis | 18.8 | Liver Cancer 6005-T | 21.0 |
| Ocular Melanoma Margin (Liver) | 8.3 | Liver Tissue 6005-N | 26.2 |
| Melanoma Metastasis | 40.1 | Liver Cancer 064003 | 2.5 |
| Melanoma Margin (Lung) | 13.5 | Normal Bladder | 9.7 |
| Normal Kidney | 16.0 | Bladder Cancer 1023 | 8.4 |
| Kidney Ca, Nuclear grade 2 (OD04338) | 57.0 | Bladder Cancer A302173 | 11.0 |
| Kidney Margin (OD04338) | 12.9 | Normal Stomach | 22.8 |
| Kidney Ca Nuclear grade 1/2 (OD04339) | 73.2 | Gastric Cancer 9060397 | 9.9 |
| Kidney Margin (OD04339) | 14.2 | Stomach Margin 9060396 | 6.6 |
| Kidney Ca, Clear cell type (OD04340) | 20.0 | Gastric Cancer 9060395 | 11.8 |
| Kidney Margin (OD04340) | 35.1 | Stomach Margin 9060394 | 23.7 |
| Kidney Ca, Nuclear grade 3 (OD04348) | 25.0 | Gastric Cancer 064005 | 6.1 |

**Table 20. Panel 4.1D**

| Column A - Rel. Exp.(%) Ag271b, Run 174261189 | | | |
|---|---|---|---|
| Tissue Name | A | Tissue Name | A |
| Secondary Th1 act | 0.0 | HUVEC IL-1beta | 21.0 |
| Secondary Th2 act | 0.4 | HUVEC IFN gamma | 19.2 |

(continued)

| Column A - Rel. Exp.(%) Ag271b, Run 174261189 | | | |
|---|---|---|---|
| Tissue Name | A | Tissue Name | A |
| Secondary Tr1 act | 0.3 | HUVEC TNF alpha + IFN gamma | 26.6 |
| Secondary Th1 rest | 0.0 | HUVEC TNF alpha + IL4 | 32.5 |
| Secondary Th2 rest | 0.0 | HUVEC IL-11 | 11.8 |
| Secondary Tr1 rest | 0.0 | Lung Microvascular EC none | 43.2 |
| Primary Th1 act | 0.0 | Lung Microvascular EC TNFalpha + IL-1beta | 34.4 |
| Primary Th2 act | 0.0 | Microvascular Dermal EC none | 35.8 |
| Primary Tr1 act | 0.6 | Microsvasular Dermal EC TNFalpha + IL-1beta | 26.4 |
| Primary Th1 rest | 0.3 | Bronchial epithelium TNFalpha + ILlbeta | 32.3 |
| Primary Th2 rest | 0.0 | Small airway epithelium none | 16.8 |
| Primary Tr1 rest | 0.0 | Small airway epithelium TNFalpha + IL-1beta | 33.2 |
| CD45RA CD4 lymphocyte act | 33.9 | Coronery artery SMC rest | 47.3 |
| CD45RO CD4 lymphocyte act | 0.0 | Coronery artery SMC TNFalpha + IL-1beta | 51.8 |
| CD8 lymphocyte act | 0.0 | Astrocytes rest | 59.0 |
| Secondary CD8 lymphocyte rest | 0.0 | Astrocytes TNFalpha + IL-1beta | 38.7 |
| Secondary CD8 lymphocyte act | 0.0 | KU-812 (Basophil) rest | 0.6 |
| CD4 lymphocyte none | 0.0 | KU-812 (Basophil) PMA/ionomycin | 1.0 |
| 2ry Th1/Th2/Tr1_anti-CD9 CH11 | 0.0 | CCD1106 (Keratinocytes) none | 36.1 |
| LAK cells rest | 0.1 | CCD1106 (Keratinocytes) TNFalpha + IL-1beta | 30.6 |
| LAK cells IL-2 | 0.0 | Liver cirrhosis | 4.4 |
| LAK cells IL-2+IL-12 | 0.0 | NCI-H292 none | 12.7 17.3 |
| LAK cells IL-2+IFN gamma | 0.0 | NCI-H292 IL-4 | |
| LAK cells IL-2+IL-18 | 0.0 | NCI-H292 IL-9 | 17.3 |
| LAK cells PMA/ionomycin | 0.1 | NCI-H292 IL-13 | 17.9 |
| NK Cells IL-2 rest | 0.0 | NCI-H292 IFN gamma | 12.8 |
| Two Way MLR 3 day | 0.0 | HPAEC none | 17.0 |
| Two Way MLR 5 day | 0.1 | HPAEC TNF alpha + IL-1 beta | 39.8 |
| Two Way MLR 7 day | 0.3 | Lung fibroblast none | 71.7 |
| PBMC rest | 0.0 | Lung fibroblast alpha + IL-1 beta | 55.5 |
| PBMC PWM | 0.0 | Lung fibroblast IL-4 | 66.4 |
| PBMC PHA-L | 0.0 | Lung fibroblast IL-9 | **100.0** |
| Ramos (B cell) none | 0.0 | Lung fibroblast IL-13 | 52.1 |
| Ramos (B cell) ionomycin | 0.0 | Lung fibroblast IFN gamma | 170.7 |
| B lymphocytes PWM | 0.0 | Dermal fibroblast CCD1070 rest | 66.0 |
| B lymphocytes CD40L and IL-4 | 0.3 | Dermal fibroblast CCD1070 TNF alpha | 61.1 |
| EOL-1 dbcAMP | 0.3 | Dermal fibroblast CCD1070 IL-1 beta | 61.6 |
| EOL-1 dbcAMP PMA/ionomycin | 0.0 | Dermal fibroblast IFN gamma | 43.2 |
| Dendritic cells none | 0.0 | Dermal fibroblast IL-4 | 59.0 |

(continued)

| Column A - Rel. Exp.(%) Ag271b, Run 174261189 | | | |
|---|---|---|---|
| Tissue Name | A | Tissue Name | A |
| Dendritic cells LPS | 0.6 | Dermal Fibroblasts rest | 36.6 |
| Dendritic cells anti-CD40 | 0.0 | Neutrophils TNFa+LPS | 2.0 |
| Monocytes rest | 0.0 | Neutrophils rest | 6.7 |
| Monocytes LPS | 0.0 | [Colon | 4.4 |
| Macrophages rest | 0.0 | Lung | 20.4 |
| Macrophages LPS | 0.0 | Thymus | 11.6 |
| HUVEC none | 18.2 | Kidney | 26.6 |
| HUVEC starved | 27.0 | | |

**Panel 2.2 Summary:** Ag271b This gene is significantly over-expressed in kidney cancer, melanoma and colon cancer when compared to normal adjacent tissue. Thus, expression of this gene could be used to differentiate between these samples and other samples on this panel and as a marker for these cancers. Furthermore, therapeutic targeting of CG51373-01 is anticipated to limit or block the extent of tumor cell migration and invasion and tumor metastasis, in these tumors.

**Panel 4.1D Summary:** Ag271b The CG51373-01 gene, which encodes the extracellular domain of an immunoglobin domain containing membrane protein, is expressed in panel 4.1D in the following resting and cytokine-activated cells and tissues: HUVEC, lung microvascular endothelial cells, small airway epithelium, coronary artery smooth muscle cells, astrocytes, lung fibroblasts, and dermal fibroblasts. The CG51373-01 gene product can be useful as a target for therapeutic antibodies which antagonize the function of the Ig domain-containing protein. Such antibodies can reduce or eliminate the symptoms in patients with inflammatory diseases and autoimmune diseases, such as multiple sclerosis, chronic obstructive pulmonary disease, asthma, emphysema, rheumatoid arthritis, or psoriasis.

## Example 8. Relevant pathways

**PathCalling™ Technology:** The sequence of Acc. No CG57008-02 was derived by laboratory screening of cDNA library by the two-hybrid approach. cDNA fragments covering either the full length of the DNA sequence, or part of the sequence, or both, were sequenced. *In silico* prediction was based on sequences available in CuraGen Corporation's proprietary sequence databases or in the public human sequence databases, and provided either the full-length DNA sequence, or some portion thereof.

cDNA libraries were derived from various human samples representing multiple tissue types, normal and diseased states, physiological states, and developmental states from different donors. Samples were obtained as whole tissue, primary cells or tissue cultured primary cells or cell lines. Cells and cell lines may have been treated with biological or chemical agents that regulate gene expression, for example, growth factors, chemokines or steroids. The cDNA thus derived was then directionally cloned into the appropriate two-hybrid vector (Gal4-activation domain (Gal4-AD) fusion). Such cDNA libraries (as well as commercially available cDNA libraries from Clontech (Palo Alto, CA)) were then transferred from *E.coli* into a CuraGen Corporation proprietary yeast strain (disclosed in U. S. Patents 6,057,101 and 6,083,693, incorporated herein by reference in their entireties).

Gal4-binding domain (Gal4-BD) fusions of a CuraGen Corportion proprietary library of human sequences was used to screen multiple Gal4-AD fusion cDNA libraries resulting in the selection of yeast hybrid diploids in each of which the Gal4-AD fusion contains an individual cDNA. Each sample was amplified using the polymerase chain reaction (PCR) using non-specific primers at the cDNA insert boundaries. Such PCR product was sequenced; sequence traces were evaluated manually and edited for corrections if appropriate. cDNA sequences from all samples were assembled together, sometimes including public human sequences, using bioinformatic programs to produce a consensus sequence for each assembly. Each assembly is included in CuraGen Corporation's database. Sequences were included as components for assembly when the extent of identity with another component was at least 95% over 50 bp. Each assembly represents a gene or portion thereof and includes information on variants, such as splice forms single nucleotide polymorphisms (SNPs), insertions, deletions and other sequence variations.

**Physical clone:** the cDNA fragment derived by the screening procedure, covering the entire open reading frame is, as a recombinant DNA, cloned into pACT2 plasmid (Clontech) used to make the cDNA library. The recombinant plasmid is inserted into the host and selected by the yeast hybrid diploid generated during the screening procedure by the mating of both CuraGen Corporation proprietary yeast strains N106' and YULH (U. S. Patents 6,057,101 and 6,083,693).

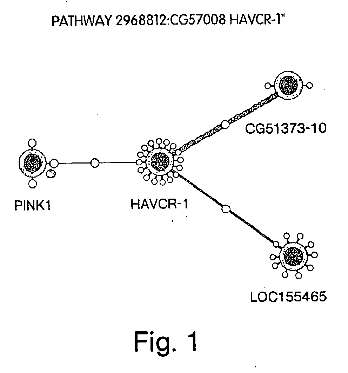

Interacting protein pairs are added to CuraGen's PathCalling™ Protein Interaction Database. This database allows for the discovery of novel pharmaceutical drug targets by virtue of their interactions and/or presence in pathologically related signaling pathways. Protein interactions are subsequently analyzed using bioinformatic tools within GeneScape™, which provides a means of visualization of binary protein interactions, protein complex formation, as well as complete cellular signaling pathways. Specifically, as shown in Figure 1, the sequences, which encode proteins CG57008-02 (HAVcR-1) (NOV1c), CG51373 (NEPH1)(NOV2), LOC15546, and COL1A1 proteins were found to interact and can result in the formation of a protein complex, or may constitute a series of complexes, which form in order to propagate a cellular signal, which is physiologically relevant to a disease pathology. The specific interactions, which constitute the specific complexes, may also be useful for therapeutic intervention through the use of recombinant protein or antibody therapies, small molecule drugs, or gene therapy approaches.

Protein interactions, which are identified through the mining of the PathCalling™ database, can be screened *in vitro* and *in vivo* to provide expression, functional, biochemical, and phenotypic information. Assays may be used alone or in conjunction and include, but are not limited to the following technologies; RTQ-PCR, transfection of recombinant proteins, co-immunoprecipitation and mass spectrometry, FRET, affinity chromatography, immunohistochemisty or immunocytochemistry, gene CHIP hybridizations, antisense (*i.e.* knock-down, knock-up), GeneCalling experiments, and/or biochemical assays (phosphorylation, dephosphorylation, protease, etc.).

As shown in Figure 1, PathCalling data shows that the mucin domain of the protein encoded by CG57008-02 (HAVcr-1) interacts with CG51373, nephrin-like I (NEPH1), a structural-cell adhesion molecule. Table 21 summarizes the amino acid sequences of the bait and prey used in three independent experiments to detect this novel interaction. This interaction suggests that CG51373 acts as a membrane-bound attractant for the CG57008-02 protein and that dysregulated signaling events promote kidney tumorigenesis. Expression data from RTQ-PCR Panel 2 (see Tables 16 and 19) show that both genes are over-expressed in kidney cancer when compared to expression in normal adjacent tissue, providing further support for this novel interaction.

While mucins are highly O-glycosylated in mammalian cells, this interaction was discovered using the yeast two-hybrid system and therefore the mucin domain is not expected to be O-glycosylated. Thus, this discovery may reflect an interaction that only occurs in the disease state, as mucin is known to be abnormally glycosylated in tumors. Figure 1 also shows that CG57008-02 interacts with LOC155465, a secreted protein that may play a role as a chemoattractant in organ development and is upregulated in some tumors (PMID: 9790916), and COL1A1, an extracellular matrix protein may be relevant. These interactions provide greater support and context for the novel CG57008-02 and CG51372 interaction.

This novel interaction also suggests that disruption of the interaction between CG57008-02 and CG51373 can prevent the peripheral feedback immune response that leads to asthma. In addition, RTQ-PCR Panel 4.1D (see Table 20) shows high levels of expression of CG51373 in cytokine-activated lung fibroblasts.

The use of antibodies to disrupt this interaction can be useful in the treatment of kidney cancers and asthma.

**Table 21. Yeast Two-hybrid Interaction Information**

| Interaction Frame | HAVCR1 Interaction Domain (aa) | Nephrin1 Interaction Domain (aa) | Number of Yeast Colonies Observed |
|---|---|---|---|
| 1 (+) | Bait:86-270 | Prey:159-492 | 1 |
| 1 (+) | Bait:86-270 | Prey:159-492 | 5 |
| 1 (+) | Bait:86-270 | Prey:159-49] | 1 |

Domain analysis by PathCalling 1x1 Matrix Assay

**Table 22. Fragments of CG57008 and CG51373 tested in Matrix 1x1 assay**

| CG57008 | Fragment (AA boundary) | CG51373 | Fragment (AA boundary) |
|---|---|---|---|
| CG57008-03 | 21-60 | CG51373-07 | 1-429 |
| CG57008-03 | 21-110 | CG51373-07 | 22-58 |
| CG57008-03 | 21-130 | CG51373-07 | 22-58 |
| CG57008-03 | 21-170 | CG51373-07 | 22-112 |

(continued)

| CG57008 | Fragment (AA boundary) | CG51373 | Fragment (AA boundary) |
|---|---|---|---|
| CG57008-03 | 21-210 | CG51373-07 | 22-145 |
| CG57008-03 | 21-240 | CG51373-07 | 22-205 |
| CG57008-03 | 21-290 | CG51373-07 | 22-255 |
| CG57008-03 | 60-290 | CG51373-07 | 22-317 |
| CG57008-03 | 106-290 | CG51373-07 | 22-355 |
| CG57008-03 | 131-210 | CG51373-07 | 22-429 |
| CG57008-03 | 131-290 | CG51373-07 | 51-429 |
| CG57008-03 | 170-290 | CG51373-07 | 106-429 |
| CG57008-03 | 209-290 | CG51373-07 | 159-205 |
| CG57008-03 | 240-290 | CG51373-07 | 159-255 |
| | | CG51373-07 | 159-317 |
| | | CG51373-07 | 159-355 |
| | | CG51373-07 | 159-429 |
| | | CG51373-07 | 200-429 |
| | | CG51373-07 | 255-429 |
| | | CG51373-07 | 300-429 |
| | | CG51373-07 | 340-429 |
| | | CG51373-09 | 159-255 |
| | | CG51373-09 | 159-317 |

**Table 23. Interactions Detected in the matrix 1x1 assay**

| Binding Domain (BD) Fusion Protein | Amino Acids in the BD Fusion Protein | Domain Description for BD Fusion Protein | Activation Domain (AD) Fusion Protein | Amino Acids in the AD Fusion Protein | Domain Description for AD Fusion Protein |
|---|---|---|---|---|---|
| CG57008-03 (CR014) | 106 - 290 | Extracellular domain minus Ig domain, contains mucin domain | CG51373-07 (CR016) | 159 - 255* | PKD domain |
| CG57008-03 (CR014) | 106 - 290 | Extracellular domain minus Ig domain, contains mucin domain | CG51373-07 (CR016) | 159 - 205* | Partial PKD domain |
| CG57008-03 (CR014) | 106 - 290 | Extracellular domain minus Ig domain, contains mucin domain | CG51373-09 (CR016) | 159 - 255 | PKD domain |
| CG57008-03 (CR014) | 60 - 290 | Extracellular domain that includes mucin domain and partial Ig domain | CG51373-07 (CR016) | 159 - 255* | PKD domain |

(continued)

| Binding Domain (BD) Fusion Protein | Amino Acids in the BD Fusion Protein | Domain Description for BD Fusion Protein | Activation Domain (AD) Fusion Protein | Amino Acids in the AD Fusion Protein | Domain Description for AD Fusion Protein |
|---|---|---|---|---|---|
| CG57008-03 (CR014) | 60 - 290 | Extracellular domain that includes mucin domain and partial Ig domain | CG51373-07 (CR016) | 159 - 205* | Partial PKD domain |
| CG57008-03 (CR014) | 60 - 290 | Extracellular domain that includes mucin domain and partial Ig domain | CG51373-09 (CR016) | 159 - 255 | PKD domain |
| CG51373-07 (CR016) | 22 - 255* | First set of 3 Ig domains + PKD domain | CG57008-03 (CR014) | 106 - 290 | Extracellular domain minus Ig domain, contains mucin domain |
| CG51373-07 (CR016) | 159 - 317 | PKD domain plus 4th Ig domain | CG57008-03 (CR014) | 106 - 290 | Extracellular domain minus Ig domain, contains mucin domain |
| CG51373-09 (CR016) | 159 - 255 | PKD domain | CG57008-03 (CR014) | 106 - 290 | Extracellular domain minus Ig domain, contains mucin domain |
| CG51373-09 (CR016) | 159 - 255 | PKD domain | CG57008-03 (CR014) | 60 - 290 | Extracellular domain that includes mucin domain and partial Ig domain |

Table 22 shows all the combinations and variants of CG57008 and CG51373 that were tested for interactions. These interactions were tested in both orientations with respect to yeast two-hybrid fusion proteins. The amino acid numbering for CG51373-07 numbers the initial Methionine as amino acid #1. Table 23 shows all the interactions that were detected in the matrix 1x1 assay. The number of positive interactions detected and their detection in both orientations with respect to yeast two-hybrid fusion proteins confirms the discovery of a novel interaction between CG57008, HAVcr-1, variants, and CG51373, nephrinI variants, and specifically between the mucin domain of HAVcr-1 and the PKD domain of nephrinI.

**Example 9. Preparation of Antibodies that Bind CG57008**

Techniques for producing the antibodies are known in the art and are described, for example, in "Antibodies, a Laboratory Manual" Eds Harlow and Lane, Cold Spring Harbor publisher. Both rabbits and mice are suitable for the production of polyclonal antibodies, while mice are also suitable for the production of monoclonal antibodies. Mice in which the human immunoglobulin genes replace mouse immunoglobulin genes can be used to produce fully human monoclonal antibodies. These antibodies have better pharmaceutical characteristics, have little or no antibody-directed immune reactions that result in loss of therapeutic efficacy, and have been shown to eradicate tumors in animal model (Yang XD, et al., Cancer Res, 59(6):1236-43 (1999)).

**Generation of human monoclonal antibodies**

Fully human IgG2 and IgG4 monoclonal antibodies (mAb), directed against CG57008-02 were generated from human antibody-producing XenoMouse strains engineered to be deficient in mouse antibody production and to contain the majority of the human antibody gene repertoire on megabase-sized fragments from the human heavy and kappa light chain loci as previously described in Yang et al., Cancer Res, 59(6):1236-43 (1999). The specificity of the antibodies was determined by ELISA.

Generation of the antigen and antibodies: The extracellular domain of CG57008-02 was subcloned to the baculovirus expression vector, pMelV5His (CuraGen Corporation) and expression studies were performed using the pBlueBac baculovirus expression system (Invitrogen Corporation) and confirmed by Western blot analyses. The sequence encodes the following polypeptide:

```
SVKVGGEAGPSVTLPCHYSGAVTSMCWNRGSCSLFTCQNGIVWTNGTHVTYRKDTRYKLLGDLSRRDVSLTI

ENTAVSDSGVYCCRVEHRGWFNDMKITVSLEIVPPKVTTTPIVTTVPTVTTVRTSTTVPTTTTVPTTTVPTT

MSIPTTTTVPTTMTVSTTTSVPTTTSIPTTTSVPVTTTVSTFVPPMPLPRQNHEPVATSPSSPQPAETHPTT

LQGAIRREPTSSPLYSYTTDGNDTVTESSDGLWNNNQTQLFLEHSLL   (SEQ ID NO:39).
```

This polypeptide was used to generate antibodies.

**Epitope binning and BIAcore affinity determination**

**Materials**

- MxhIgG - conjugated beads (Limit Exposure to Light)
- Wash Buffer (PBS, Tween 20{0.05%})
- Blocking Buffer
- 96-well microtiter filter plate (Milipore #MADVN 6550)
- mxhIgG-PE

**Procedure**

1. Prepare beads for coupling to primary unknown antibody. (Protect from light). Use individual tubes for each unknown supernatant. Calculate the volume of supernatant needed by using the following formula: (n+10) x 50$\mu$l (where n = total number of samples on plate). If the concentration is known, use at 0.5ug/ml. Gently vortex bead stock. Dilute beads in supernatant to a concentration of 2500 of each bead per well or 0.5X10$^5$/ml.

2. Incubate on a shaker in the dark at room temperature overnight, or 2 hours if at a known concentration of 0.5ug/ml.

3. Pre-wet filter plate by adding 200$\mu$l wash buffer per well. Aspirate.

4. Add 50ul of each bead to each well of filter plate. Wash once by adding 100ul/well wash buffer and aspirating.

5. Add antigen and controls to filter plate 50$\mu$l/well. Cover and incubate in the dark for 1 hour on shaker.

6. Wash 3 times.

7. Add secondary unknown antibody at 50ul/well. Use the same dilution (or concentration if known) as used for the primary antibody. Incubate in the dark for 2 hours at room temperature on shaker.

8. Wash 3 times.

9. Add 50ul/well biotinylated mxhIgG diluted 1:500. Incubate in the dark for 1hour on shaker at room temperature.

10. Turn on both Luminex 100 and XYP base. Open the Luminex software and start the "warm up" operation. Make sure the sheath fluid container has enough volume and the waste container has enough space so it won't overfill.

11. In Luminex software, click the "new session" button. The "settings" window will pop up. All of the default settings are correct except for the "number of samples".

12. Enter the number of samples in this field.

13. Click on the "bead set" tab and enter the designation numbers of the beads used in the assay.

14. Wash 3 times.

15. Add 50ul/wellStreptavidin-PE at 1:1000. Incubate in the dark for 15min. on shaker at room temperature.

16. Run two wash cycles on the Luminex100.

17. Wash 3 times.

18. Resuspend each well in 80ul blocking buffer. Carefully pipette up and down several times to resuspend beads.

19. Place the plate in the Luminex base to be read. Make sure position A1 is highlighted on your screen and click start. After the last sample has been read, perform two wash cycles and one soak cycle. Close the Luminex software. Then, turn off both the top and bottom of the Luminex 100. Release pressure in sheath fluid reservoir by loosening the cap.

**BIAcore Determination**

BIAcore determinations were done using methods known in the art, for example, "Validation parameters for a novel biosensor assay which simultaneously measures serum concentrations of a humanized monoclonal antibody and detects induced antibodies", R. Wong, D. Mytych, S.Jacobs, R.Bordens, S.Swanson, Journal of Immunological Methods, 209(1997)1-15.

Table 24 shows that the monoclonal antibodies generated belong to eight distinct bins.

Table 24

| Bins | Antibody | (affinity nM by BIAcore) |
|------|----------|--------------------------|
| 1 | Mab2.59 | (0.38) |
|   | Mab12.9 | (3.64) |
| 2 | Mab2.16 | (0.79) |
| 3 | Mab2.17 | (2.42) |
| 4 | Mab1.37 | (2.78) |
|   | Mab2.79 | (0.57) |
|   | Mab2.61 | (1.0) |
| 5 | Mab2.24 | (2.42) |
|   | Mab2.56 | (1.11) |
| 6 | Mab2.70 | (2.71) |
| 7 | Mab2.54 | (3.35) |
| 8 | Mab2.45 | (1.15) |

**Example 10. Specificity of the monoclonal antibodies for CG57008-02**

**ELISA Protocol**

    **Solution Preparation:**_Coating Buffer (0.1M Carbonate, pH9.5), 8.4 g. NaHCO3, 3.56g. Na2CO3, pH to 9.5, and dilute to 1 L. with ddH20
    **Assay Diluent:** Pharmingen #26411E

**Protocol:**

1) Coat a 96-well high protein binding ELISA plate (Coming Costar #3590) with 50 ul. of the CG57008-02 protein at a concentration of 5ug/mL. diluted in coating buffer, incubate overnight at 4 °C.
2) Following day wash the wells 5X 200-300 ul of 0.5% Tween-20 in PBS.
3) Block plates with 200ul of assay diluent for at least 1 hour at room temperature.
4) Dilute CR014 antibodies in assay diluent with the final concentrations of 7, 15, 31.3, 62.5, 125, 250, 500 and 1000 ng/ml. An anti-V5-HRP antibody was used at 1:1000 to detect the V5 containing peptide as the positive control for the ELISA.
5) Wash plate as in step 2).
6) Add 50ul of each antibody dilution to the proper wells, incubate for at least 2 hours at room temp.

7) Wash plate as in step 2).

8) Add 50ul of secondary antibody (goat anti-human-HRP at 1:1000 and incubate for 1 hour at room temp.

9) Wash plate as in step 2).

10) Develop assay with 100ul of TMB substrate solution/well. (1:1 ratio of solution A+B) (Pharmingen #2642KK)

11) Stop reaction with 50ul. sulfuric acid. 12) Read plate at 450nm with a correction of 550nm.

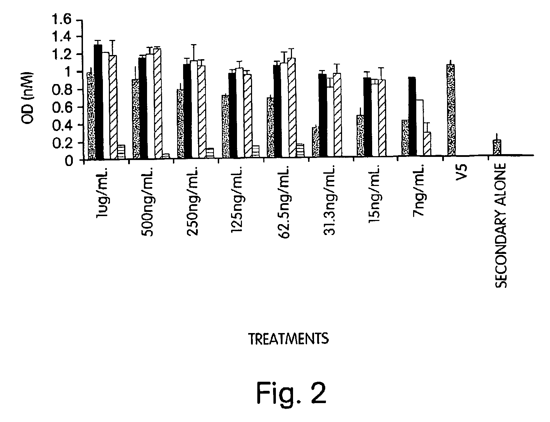

To demonstrate the specificity of the antibodies for CG57008-02, four of the antibodies, CR014.1.29, CR014.2.56.2, CR014.2.59.2, and CR014.2.45.1, as well as an isotype matched control mAb PK16.3, were tested by ELISA for reactivity against the CG57008-02 antigen. (Figure 2). The X axis depicts the antibody treatments in the order listed above and the Y axis is the optical density.

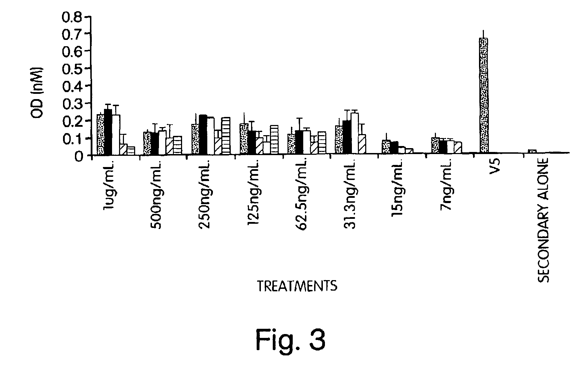

In addition, to eliminate the possibility that the observed immunoreactivity was directed against the V5-His tag added to the CG57008-02 construct, an unrelated protein containing the same V5-His tag was included in the assay as a control. (Figure 3). These results demonstrate that the four antibodies assayed specifically bind to the wild type CG57008-02 antigen.

**Example 11. Immunohistochemical (IHC) analysis of CG57008 expression in normal and tumor tissues**

Immunohistochemical (IHC) analysis of CG57008 expression in normal and tumor tissue specimens was performed using techniques known in the art.

**Immunohistochemistry staining Protocol**

**Sample Preparation**

1. Fix the tissue in 10% formalin at 4°C overnight.

2. Paraffin embed the fixed tissue.

3. Mount tissue sections on slides.

4. Rinse the slides twice for 2 minutes in 100% alcohols (18:1:1 100% ethanol: 100% methanol: 100% isopropanol) and twice for 2 minutes in a 95% solution of the 100% alcohols.

5. Place slides in an 80% solution of the 100% alcohols for 2 minutes and several times with fresh deionized water.

**SDS Antigen Retrieval**

1. Place slides face-up in incubation tray and cover each section with 1% SDS in TBS (100mM Tris pH 7.4,138mM NaCl, 27mM KCl).

2. Incubate for five minutes at room temperature, followed by three five minute washes with TBS.

**Blocking**

1. Immerse slides in a dish containing blocking buffer (serum from host species of secondary antibody to be used, diluted 1:10 in TBS).

2. Incubate at 37°C for one hour.

**Incubation with Primary Antibodies**

1. Cover the tissue sections with primary CG57008 (CR014) antibodies diluted in blocking buffer.

2. Incubate for 2 hours at 37°C.

3. Rinse twice in TBS for five minutes each wash.

**Incubation with Secondary Antibodies**

1. Cover the tissue sections with conjugated secondary antibody diluted in blocking buffer according to manufacturer's instructions.
2. Incubate at 37°C for one hour.
3. Rinse twice in TBS for five minutes each wash.
4. Counterstaining and visualization.

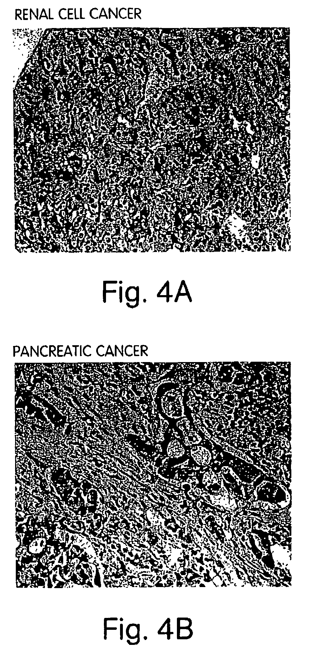

Table 26 summarizes the tissues that highly express CG57008-02 as detected using CG57008 2.59.2 mAb. The specimens are graded on a scale of 0-3, with a score of 1+ indicating that the staining is above that observed in control tissues stained with an isotype control irrelevant antibody. The corresponding histological specimens from one renal tumor and the pancreatic tumor are shown in Figures 4A and 4B. Table 25 shows all the tissues tested and the number of each type of tissue tested and results. In addition to these the renal and pancreatic tumors, specimens from head and neck cancer, ovarian cancer, gastric cancer, melanoma, lymphoma, prostate cancer, liver cancer, breast cancer, lung cancer, bladder cancer, colon cancer, esophageal cancer, and brain cancer, as well the corresponding normal tissues were stained with 2.59.2 mAb.

Overall, approximately 18% of the renal cancer tissue samples and 6% of the pancreatic cancer tissue samples examined were highly positive when stained with the 2.59.2 mAb recognizing the CG57008 antigen. No staining in normal tissues was seen. These results indicate that CG57008-02 is a marker of cancer in these tissues and that the 2.59.2 antibody can be used to differentiate cancers from normal tissues.

### Table 25.

| Array # | Position | Tissue | Staining Intensity |
|---------|----------|--------|-------------------|
| ITTA03354B | A3 | HEAD & NECK CA | 0 |
| ITTA03354B | A7 | HEAD & NECK CA | 0 |
| ITTA03354B | A8 | HEAD & NECK CA | 0 |
| ITTA03354B | A9 | HEAD & NECK CA | 0 |

| | | | |
|---|---|---|---|
| ITTA03354B | A10 | HEAD & NECK CA | 0 |
| ITTA03354B | B3 | HEAD & NECK CA | 0 |
| ITTA03354B | B4 | HEAD & NECK CA | 0 |
| ITTA03354B | B5 | HEAD & NECK CA | 0 |
| ITTA03354B | B6 | HEAD & NECK CA | 0 |
| ITTA03354B | B8 | HEAD & NECK CA | 0 |
| ITTA03354B | B9 | HEAD & NECK CA | 0 |
| ITTA03354B | C1 | HEAD & NECK CA | 0 |
| ITTA03354B | C2 | HEAD & NECK CA | 0 |
| ITTA03354B | C3 | HEAD & NECK CA | 0 |
| ITTA03354B | C5 | HEAD & NECK CA | 0 |
| ITTA03354B | C6 | HEAD & NECK CA | 0 |
| ITTA03354B | C7 | HEAD & NECK CA | 0 |
| ITTA03354B | C8 | HEAD & NECK CA | 0 |
| ITTA03354B | C9 | OVARIAN CA | 0 |
| ITTA03354B | C10 | OVARIAN CA | 0 |
| ITTA03354B | D1 | OVARIAN CA | 0 |
| ITTA03354B | D2 | OVARIAN CA | 0 |
| ITTA03354B | D3 | OVARIAN CA | 0 |
| ITTA03354B | D4 | OVARIAN CA | 0 |
| ITTA03354B | D5 | OVARIAN CA | 0 |
| ITTA03354B | D6 | OVARIAN CA | 0 |
| ITTA03354B | D7 | OVARIAN CA | 0 |
| ITTA03354B | D8 | OVARIAN CA | 0 |
| ITTA03354B | D9 | OVARIAN CA | 0 |
| ITTA03354B | E3 | OVARIAN CA | 0 |
| ITTA03354B | E4 | OVARIAN CA | 0 |
| ITTA03354B | E5 | OVARIAN CA | 0 |
| ITTA03354B | E6 | OVARIAN CA | 0 |
| ITTA03354B | E7 | OVARIAN CA | 0 |
| ITTA03354B | E9 | OVARIAN CA | 0 |
| ITTA03354B | E10 | OVARIAN CA | 0 |
| ITTA03354B | F1 | OVARIAN CA | 0 |
| ITTA03354B | F2 | OVARIAN CA | 0 |
| ITTA03354B | F3 | OVARIAN CA | 0 |
| ITTA03354B | F4 | PANCREATIC CA | 0 |
| ITTA03354B | F5 | PANCREATIC CA | 0 |
| ITTA03354B | F6 | PANCREATIC CA | 0 |
| ITTA03354B | F7 | PANCREATIC CA | 0 |
| ITTA03354B | F9 | PANCREATIC CA | 0 |
| ITTA03354B | F10 | PANCREATIC CA | 0 |
| ITTA03354B | G1 | PANCREATIC CA | 0 |
| ITTA03354B | G2 | PANCREATIC CA | 0 |

| | | | | | |
|---|---|---|---|---|---|
| ITTA03354B | G3 | PANCREATIC CA | 0 | | |
| ITTA03354B | H4 | RENAL TUMOR | 0 | | |
| ITTA03354B | H5 | RENAL TUMOR | | 1+ | |
| ITTA03354B | H6 | RENAL TUMOR | 0 | | |
| ITTA03354B | H7 | RENAL TUMOR | 0 | | |
| ITTA03354B | H8 | RENAL TUMOR | 0 | | |
| ITTA03354B | H9 | RENAL TUMOR | 0 | | |
| ITTA03354B | H10 | RENAL TUMOR | | | |
| ITTA03354B | I1 | RENAL TUMOR | 0 | | |
| ITTA03354B | I2 | RENAL TUMOR | 0 | | |
| ITTA03354B | I3 | RENAL TUMOR | 0 | | |
| ITTA03354B | I4 | RENAL TUMOR | 0 | | |
| ITTA03354B | I5 | RENAL TUMOR | 0 | | |
| ITTA03354B | I7 | RENAL TUMOR | 0 | | |
| ITTA03354B | I8 | RENAL TUMOR | | 1+ | |
| ITTA03354B | I9 | RENAL TUMOR | | 1+ | |
| ITTA03354B | I10 | RENAL TUMOR | | | |
| ITTA03354B | J1 | RENAL TUMOR | 0 | | |
| ITTA03354B | J2 | RENAL TUMOR | 0 | | |
| ITTA03354B | J3 | RENAL TUMOR | 0 | | |
| ITTA03354B | J4 | RENAL TUMOR | 0 | | |
| ITTA03354B | J6 | RENAL TUMOR | | 1+ | |
| ITTA03354B | J7 | RENAL TUMOR | 0 | | |
| ITTA03356C | C2 | GASTRIC CA | 0 | | |
| ITTA03356C | C4 | GASTRIC CA | 0 | | |
| ITTA03356C | C6 | GASTRIC CA | 0 | | |
| ITTA03356C | D3 | GASTRIC CA | 0 | | |
| ITTA03356C | D7 | GASTRIC CA | 0 | | |
| ITTA03356C | D8 | GASTRIC CA | 0 | | |
| ITTA03356C | D10 | GASTRIC CA | 0 | | |
| ITTA03356C | E6 | MELANOMA | 0 | | |
| ITTA03693A | A5 | LYMPHOMA | 0 | | |
| ITTA03693A | A6 | LYMPHOMA | 0 | | |
| ITTA03693A | C6 | PROSTATE CA | 0 | | |
| ITTA03693A | D5 | LIVER CA | 0 | | |
| ITTA03693A | E4 | LIVER CA | 0 | | |
| ITTA03693A | E5 | LIVER CA | 0 | | |
| ITTA03693A | F6 | LIVER CA | 0 | | |
| ITTA04331A | A4 | BREAST CA | 0 | | |
| ITTA04331A | A5 | BREAST CA | 0 | | |
| ITTA04331A | A6 | BREAST CA | 0 | | |
| ITTA04331A | B3 | BREAST CA | 0 | | |
| ITTA04331A | B4 | BREAST CA | 0 | | |

| | | | | |
|---|---|---|---|---|
| ITTA04331A | B5 | BREAST CA | 0 | |
| ITTA04331A | B7 | BREAST CA | 0 | |
| ITTA04331A | B9 | BREAST CA | 0 | |
| ITTA04331A | B10 | BREAST CA | 0 | |
| ITTA04331A | C1 | BREAST CA | 0 | |
| ITTA04331A | C3 | BREAST CA | 0 | |
| ITTA04331A | C5 | BREAST CA | 0 | |
| ITTA04331A | C6 | BREAST CA | 0 | |
| ITTA04331A | C7 | BREAST CA | 0 | |
| ITTA04331A | C8 | BREAST CA | 0 | |
| ITTA04331A | D1 | BREAST CA | 0 | |
| ITTA04331A | D3 | LUNG CA | 0 | |
| ITTA04331A | D5 | LUNG CA | 0 | |
| ITTA04331A | D6 | LUNG CA | 0 | |
| ITTA04331A | D8 | LUNG CA | 0 | |
| ITTA04331A | D9 | LUNG CA | 0 | |
| ITTA04331A | D10 | LUNG CA | 0 | |
| ITTA04331A | E2 | LUNG CA | 0 | |
| ITTA04331A | E6 | LUNG CA | 0 | |
| ITTA04331A | E7 | LUNG CA | | 1+ |
| ITTA04331A | E10 | LUNG CA | 0 | |
| ITTA04331A | F4 | BLADDER CA | 0 | |
| ITTA04331A | F7 | BLADDER CA | 0 | |
| ITTA04331A | F8 | BLADDER CA | | 1+ |
| ITTA04331A | F9 | BLADDER CA | | 1+ |
| ITTA04331A | F10 | BLADDER CA | 0 | |
| ITTA04331A | G3 | BLADDER CA | 0 | |
| ITTA04331A | G4 | BLADDER CA | 0 | |
| ITTA04331A | G10 | BLADDER CA | | 1+ |
| ITTA04331A | H3 | COLON CA | 0 | |
| ITTA04331A | H4 | COLON CA | 0 | |
| ITTA04331A | H5 | COLON CA | | 1+ |
| ITTA04331A | H7 | COLON CA | | 1+ |
| ITTA04331A | I6 | COLON CA | | 1+ |
| ITTA04331A | I8 | COLON CA | 0 | |
| ITTA04331A | I10 | COLON CA | 0 | |
| ITTA04331A | J3 | COLON CA | 0 | |
| ITTA04331A | J5 | COLON CA | | 1+ |
| ITTA04332A | A3 | HEAD & NECK CA | 0 | |
| ITTA04332A | A5 | HEAD & NECK CA | 0 | |
| ITTA04332A | A6 | HEAD & NECK CA | 0 | |
| ITTA04332A | A7 | HEAD & NECK CA | 0 | |
| ITTA04332A | A10 | HEAD & NECK CA | 0 | |

| | | | | |
|---|---|---|---|---|
| ITTA04332A | B4 | HEAD & NECK CA | 0 | |
| ITTA04332A | B5 | HEAD & NECK CA | 0 | |
| ITTA04332A | B6 | HEAD & NECK CA | 0 | |
| ITTA04332A | B7 | HEAD & NECK CA | 0 | |
| ITTA04332A | B10 | LYMPHOMA | 0 | |
| ITTA04332A | C1 | LYMPHOMA | 0 | |
| ITTA04332A | C3 | LYMPHOMA | 0 | |
| ITTA04332A | C7 | GASTRIC CA | 0 | |
| ITTA04332A | C8 | GASTRIC CA | 0 | |
| ITTA04332A | C10 | GASTRIC CA | 0 | |
| ITTA04332A | D5 | GASTRIC CA | 0 | |
| ITTA04332A | D6 | GASTRIC CA | 0 | |
| ITTA04332A | D9 | OVARIAN CA | 0 | |
| ITTA04332A | D10 | OVARIAN CA | | 1+ |
| ITTA04332A | E1 | OVARIAN CA | 0 | |
| ITTA04332A | E4 | OVARIAN CA | 0 | |
| ITTA04332A | E5 | OVARIAN CA | 0 | |
| ITTA04332A | E7 | OVARIAN CA | 0 | |
| ITTA04332A | E8 | RENAL CA | | 1+ |
| ITTA04332A | E10 | RENAL CA | 0 | |
| ITTA04332A | F3 | RENAL CA | | 1+ |
| ITTA04332A | F4 | RENAL CA | | 1+ |
| ITTA04332A | G1 | LIVER CA | 0 | |
| ITTA04332A | G7 | LIVER CA | 0 | |
| ITTA04332A | H1 | ESOPHAGUS CA | 0 | |
| ITTA04332A | H2 | ESOPHAGUS CA | 0 | |
| ITTA04332A | H5 | MELANOMA | 0 | |
| ITTA04332A | I4 | PROSTATE CA | 0 | |
| ITTA04332A | I5 | PROSTATE CA | 0 | |
| ITTA04332A | I7 | PROSTATE CA | 0 | |
| ITTA04332A | I9 | PROSTATE CA | 0 | |
| ITTA04332A | I10 | PROSTATE CA | | 1+ |
| ITTA04332A | J1 | PROSTATE CA | 0 | |
| ITTA04332A | J5 | PROSTATE CA | 0 | |
| ITTA04404A | A3 | BREAST CA | 0 | |
| ITTA04404A | A8 | BREAST CA | 0 | |
| ITTA04404A | B1 | BREAST CA | 0 | |
| ITTA04404A | B2 | BREAST CA | 0 | |
| ITTA04404A | B3 | COLON CA | 0 | |
| ITTA04404A | B9 | COLON CA | 0 | |
| ITTA04404A | C3 | COLON CA | 0 | |
| ITTA04404A | C6 | LYMPHOMA | 0 | |
| ITTA04404A | C9 | LYMPHOMA | 0 | |

| | | | | | |
|---|---|---|---|---|---|
| ITTA04404A | D1 | LYMPHOMA | 0 | | |
| ITTA04404A | D3 | LYMPHOMA | 0 | | |
| ITTA04404A | D4 | OVARIAN CA | 0 | | |
| ITTA04404A | E10 | LUNG CA | 0 | | |
| ITTA04404A | F1 | LUNG CA | 0 | | |
| ITTA04404A | F2 | LUNG CA | 0 | | |
| ITTA04404A | G1 | PROSTATE CA | 0 | | |
| ITTA04404A | G3 | PROSTATE CA | 0 | | |
| ITTA04404A | H1 | HEAD & NECK CA | 0 | | |
| ITTA04404A | H4 | MELANOMA | 0 | | |
| ITTA04404A | H7 | MELANOMA | 0 | | |
| ITTA04404A | H9 | MELANOMA | 0 | | |
| ITTA04404A | I1 | MELANOMA | 0 | | |
| ITTA04404A | I2 | MELANOMA | 0 | | |
| ITTA04404A | I3 | MELANOMA | 0 | | |
| ITTA04404A | I4 | RENAL CA | 0 | | |
| ITTA04404A | I10 | RENAL CA | 0 | | |
| ITTA04404A | J2 | RENAL CA | 0 | | |
| ITTA04404A | J5 | GASTRIC CA | 0 | | |
| ITTA04404A | J6 | GASTRIC CA | 0 | | |
| ITTA04404A | K2 | GASTRIC CA | 0 | | |
| ITTA04404A | K3 | GASTRIC CA | 0 | | |
| ITTA04404A | K4 | GASTRIC CA | 0 | | |
| INTA03355B | A2 | NORMAL BREAST | 0 | | |
| INTA03355B | A4 | NORMAL BREAST | 0 | | |
| INTA03355B | B2 | NORMAL BREAST | 0 | | |
| INTA03355B | B5 | NORMAL COLON | 0 | | |
| INTA03355B | B6 | NORMAL COLON | 0 | | |
| INTA03355B | C1 | NORMAL COLON | 0 | | |
| INTA03355B | C6 | NORMAL COLON | 0 | | |
| INTA03355B | D3 | NORMAL OVARY | 0 | | |
| INTA03355B | D5 | NORMAL OVARY | 0 | | |
| INTA03355B | E1 | NORMAL OVARY | 0 | | |
| INTA03355B | E4 | NORMAL OVARY | 0 | | |
| INTA03355B | E5 | NORMAL PANCREAS | 0 | | |
| INTA03355B | F1 | NORMAL PANCREAS | 0 | | |
| INTA03355B | F2 | NORMAL PANCREAS | 0 | | |
| INTA03355B | F4 | NORMAL PANCREAS | 0 | | |
| INTA03355B | F5 | NORMAL PANCREAS | 0 | | |
| INTA03355B | F6 | NORMAL PANCREAS | 0 | | |
| INTA03357A | A2 | NORMAL SKIN | 0 | | |
| INTA03357A | A3 | NORMAL SKIN | 0 | | |

| | | | | |
|---|---|---|---|---|
| INTA03357A | A4 | NORMAL SKIN | 0 | |
| INTA03357A | A5 | NORMAL SKIN | 0 | |
| INTA03357A | A6 | NORMAL SKIN | 0 | |
| INTA03357A | B3 | NORMAL SKIN | 0 | |
| INTA03357A | B4 | NORMAL SKIN | 0 | |
| INTA03357A | B5 | NORMAL SKIN | 0 | |
| INTA03357A | B6 | NORMAL KIDNEY | 0 | |
| INTA03357A | C1 | NORMAL KIDNEY | 0 | |
| INTA03357A | C2 | NORMAL KIDNEY | 0 | |
| INTA03357A | C3 | NORMAL KIDNEY | 0 | |
| INTA03357A | C4 | NORMAL KIDNEY | 0 | |
| INTA03357A | C6 | NORMAL KIDNEY | 0 | |
| INTA03357A | D1 | NORMAL KIDNEY | 0 | |
| INTA03357A | D2 | N. ESOPHAGUS | 0 | |
| INTA03357A | D4 | N. ESOPHAGUS | 0 | |
| INTA03357A | D5 | N. ESOPHAGUS | 0 | |
| INTA03357A | D6 | N. ESOPHAGUS | 0 | |
| INTA03357A | E1 | N. ESOPHAGUS | 0 | |
| INTA03357A | E2 | N. ESOPHAGUS | 0 | |
| INTA03357A | E4 | N. ESOPHAGUS | 0 | |
| INTA03357A | E5 | N. ESOPHAGUS | 0 | |
| INTA03357A | E6 | NORMAL STOMACH | 0 | |
| INTA03357A | F1 | NORMAL STOMACH | 0 | |
| INTA03357A | F2 | NORMAL STOMACH | 0 | |
| INTA03357A | F3 | NORMAL STOMACH | 0 | |
| INTA03357A | F5 | NORMAL STOMACH | 0 | |
| INTA03357A | F6 | NORMAL STOMACH | 0 | |
| INTA03689A | A3 | NORMAL LYMPH NODE | 0 | |
| INTA03689A | A5 | NORMAL LYMPH NODE | 0 | |
| INTA03689A | A6 | NORMAL LYMPH NODE | 0 | |
| INTA03689A | A7 | NORMAL LYMPH NODE | 0 | |
| INTA03689A | B6 | NORMAL LUNG | 0 | |
| INTA03689A | D3 | NORMAL PROSTATE | 0 | |
| INTA03689A | D5 | NORMAL PROSTATE | 0 | |
| INTA03689A | D6 | NORMAL PROSTATE | 0 | |
| INTA03689A | E3 | NORMAL PROSTATE | 0 | |
| INTA03689A | G2 | NORMAL TONSIL | 0 | |
| INTA03689A | G4 | NORMAL TONSIL | 0 | |
| INTA03689A | G5 | NORMAL TONSIL | 0 | |
| INTA03691A | A5 | NORMAL BLADDER | | 0 |

| | | | | | |
|---|---|---|---|---|---|
| INTA03691A | C1 | NORMAL HEAD & NECK | 0 | | |
| INTA03691A | C2 | NORMAL HEAD & NECK | 0 | | |
| INTA03691A | C3 | NORMAL HEAD & NECK | 0 | | |
| INTA03691A | C6 | NORMAL HEAD & NECK | 0 | | |
| INTA03691A | D1 | NORMAL HEAD & NECK | 0 | | |
| INTA03691A | D2 | NORMAL HEAD & NECK | 0 | | |
| INTA03691A | D3 | NORMAL HEAD & NECK | 0 | | |
| INTA03691A | D4 | NORMAL HEAD & NECK | 0 | | |
| INTA03691A | D6 | NORMAL LIVER | 0 | | |
| INTA03691A | F1 | NORMAL LIVER | 0 | | |
| INTA03691A | F2 | NORMAL LIVER | 0 | | |
| ITLI03490A | | LIVER CA | 0 | | |
| ITLI03618A | | LIVER CA | 0 | | |
| ITLI02881A | | LIVER CA | 0 | | |
| ITLI03624A | | LIVER CA | 0 | | |
| ITLI03373A | | LIVER CA | 0 | | |
| ITLI02774A | | LIVER CA | 0 | | |
| ITEC00610A | | ESOPHAGUS CA | 0 | | |
| ITEC01300A | | ESOPHAGUS CA | 0 | | |
| ITEC01420A | | ESOPHAGUS CA | 0 | | |
| ITEC01434A | | ESOPHAGUS CA | 0 | | |
| ITEC01648A | | ESOPHAGUS CA | 0 | | |
| ITEC02557A | | ESOPHAGUS CA | 0 | | |
| ITEC02560A | | ESOPHAGUS CA | 0 | | |
| ITEC02571A | | ESOPHAGUS CA | 0 | | |
| ITEC01649A | | ESOPHAGUS CA | 0 | | |
| ITBL03376A | | BLADDER CA | 0 | | |
| ITBL03378A | | BLADDER CA | 0 | | |
| ITBL03379A | | BLADDER CA | 0 | | |
| ITBL03622A | | BLADDER CA | 0 | | |
| ITLY02874A | | LYMPHOMA | | 1+ | C |
| ITLY03418A | | LYMPHOMA | 0 | | |
| ITLY010146A | | LYMPHOMA | 0 | | |
| ITPS01321A | | PANCREATIC CA | | 2+ | C, M |
| ITPS01413A | | PANCREATIC CA | | 1+ | C |
| ITPS01417A | | PANCREATIC CA | 0 | | |
| ITPS02460A | | PANCREATIC CA | 0 | | |
| ITPS02902A | | PANCREATIC CA | 0 | | |
| ITPS04646A | | PANCREATIC CA | 0 | | |
| ITPS04648A | | PANCREATIC CA | 0 | | |
| ITME0007-192-00943-8 | | MELANOMA | 0 | | |
| ITME0008-192-00552-9 | | MELANOMA | 0 | | |

| | | | | |
|---|---|---|---|---|
| ITME0008-192-00569-13 | MELANOMA | 0 | | |
| ITME0008-192-00570-14 | MELANOMA | | 1+ | C |
| ITBA03360A | BRAIN CA | 0 | | |
| ITBA03361A | BRAIN CA | 0 | | |
| ITBA0105-292-03728 | BRAIN CA | 0 | | |
| INLU01337A | NORMAL LUNG | 0 | | |
| INLU01506A | NORMAL LUNG | 0 | | |
| INLU01508A | NORMAL LUNG | 0 | | |
| INLU01510A | NORMAL LUNG | 0 | | |
| INCO03182A | NORMAL COLON | 0 | | |
| INCO03183A | NORMAL COLON | 0 | | |
| INCO03209A | NORMAL COLON | 0 | | |
| INBL03523A | NORMAL BLADDER | 0 | | |
| INBL0105-304-00594-1 | NORMAL BLADDER | 0 | | |
| INBL0105-303-01148-2 | NORMAL BLADDER | 0 | | |
| INBL0104-292-00189-4 | NORMAL BLADDER | 0 | | |
| INBA02029D | NORMAL BRAIN | 0 | | |
| INBX01635A | NORMAL BRAIN | 0 | | |
| INBX03359A | NORMAL BRAIN | | 1+ | C |

**Table 26.** Summay of expression of CG57008 protein detected by 2.59.2 mAb in renal and pancreatic tumors

| Tissue source | Test Article | Score[M=membrane, C=cytosolic] |
|---|---|---|
| RENAL CA | ABX-14 (2.59.2) | 1 + M |
| RENAL CA | ABX-14 (2.59.2) | 2+ M,C |
| RENAL CA | ABX-14 (2.59.2) | 1+ M,C |
| RENAL CA | ABX-14 (2.59.2) | 2+ M,C |
| PANCREATIC CA | ABX-14 (2.59.2) | 2+ M,C |

**Example 12. Quantification of membrane bound CG57008 protein by Flow Cytometry**

FACS analysis was performed to demonstrate the specificity of the anti-CG57008 antibodies for cell membrane-bound CG57008 and to identify preferred antibodies for use as a therapeutic or diagnostic agent. The analysis was performed on four renal cancer cell lines, ACHN (ATCC#:CRL-1611), CAKI-1 (ATCC#:HTB-46), CAKI-2 (ATCC#:HTB-47) and 786-0 (ATCC#:CRL-1932). A breast cancer cell line, BT549, that does not express CG57008 was used as a control. Table 27 shows that both antibodies, 2.59.2 and 2.70.2, specifically bind to CG57008 expressed on ACHN, CAKI-1, CAKI-2 and 786-O cells, but not BT549 cells. Based on the Geo Mean Ratios normalized to the pK16 isotype control, irrelevant antibody, ACHN cells have a higher cell surface expression of CG57008 protein.

**Table 27**

| Geo Mean Ratio (relative to pK16) | | | | | |
|---|---|---|---|---|---|
| Antibody | ACHN | CAKI-1 | 786-O | CAKI-2 | BT549 |
| 2.59.2 | 27.8 | 14.8 | 22.8 | 13.8 | 1.4 |
| 2.70.2 | 29.7 | 15.8 | 23.4 | 13.8 | 1.8 |

**Example 13. Antibody mediated toxin killing**

Kohls and Lappi, Biotechniques, 28 (1):162-5 (2000) have described a clonogenic assay to determine if a primary antibody can induce cancer cell death when used in combination with a saporin toxin conjugated secondary antibody reagent.

**A. Clonogenic Assay Protocol: Anti-CG57008 mAb-mediated toxin killing**

ACHN and BT549 cells were plated onto flat bottom tissue culture plates at a density of 3000 cells per well. On day 2 or once cells reach ~25% confluency, 100 ng/well secondary mAb-toxin (goat anti-human IgG-saporin; Advanced Targeting Systems; HUM-ZAP; cat. # IT-22) was added. EGFR, CR014.2.7.2, CR014.2.59.2, or isotype control mAb was then added to each well at the desired concentration (typically 1 to 500 ng/ml). On day 5, the cells were trypsinized, transferred to a 150 mm tissue culture dish, and incubated at 37 °C. Plates were examined daily. On days 10-12, all plates were Giemsa stained and colonies on the plates were counted. Plating efficiency was determined by counting the cells prior to transfer to 150 mm plates and compared to the number of colonies that eventually formed.

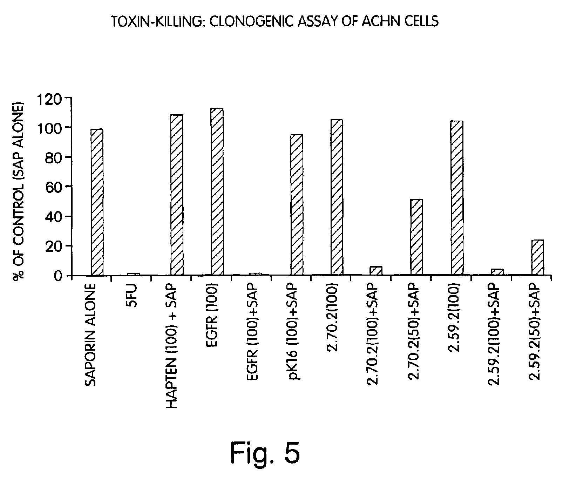

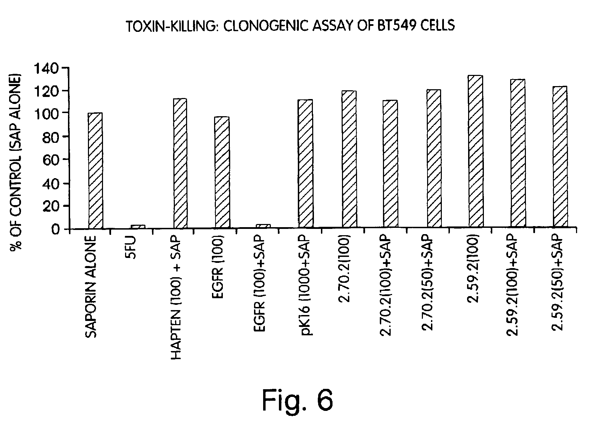

The percent viability in antigen positive ACHN and antigen negative BT549 cell lines are presented in Figure 5 and Figure 6, respectively. In this study, the cytotoxic chemotherapy reagent 5-FU was used as the positive control and induced almost complete killing, whereas addition of the saporin conjugated-goat anti-human secondary antibody alone had no effect. A monoclonal antibody (NeoMarkers MS-269-PABX) generated against the EGF receptor, which is expressed by both cell lines, was used to demonstrate primary antibody and secondary antibody-saporin conjugate specific killing. The results indicate that both cell lines were susceptible to EGFR mAb mediated toxin killing at 100 ng/ml. At the same dose, both the 2.59.2mAb and the 2.70.2 mAb induced over 90% ACHN cell death as compared to 0% BT549 cell death.

**B. Clonogenic Assay Protocol: Auristatin E conjugated antibody mediated toxin killing**

CAKI-1 and BT549 cells were plated onto flat bottom tissue culture plates at a density of 3000 cells per well. On day 2 or cells reach ~25% confluency, various concentrations (typically 1 to 1000 ng/ml) of unconjugated and Auristatin E-conjugated mAb, which included EGFR, CR014.2.7.2, CR014.2.59.2 or isotype control mAb (CR011.2.6.2), were added to cells. On day 5, the cells were trypsinized, transferred to a 150 mm tissue culture dish, and incubated at 37 °C. Plates were examined daily. On days 10-12, all plates were Giemsa stained and colonies on the plates were counted. Plating efficiency was determined by counting the cells prior to transfer to 150 mm plates and compared to the number of colonies that eventually formed.

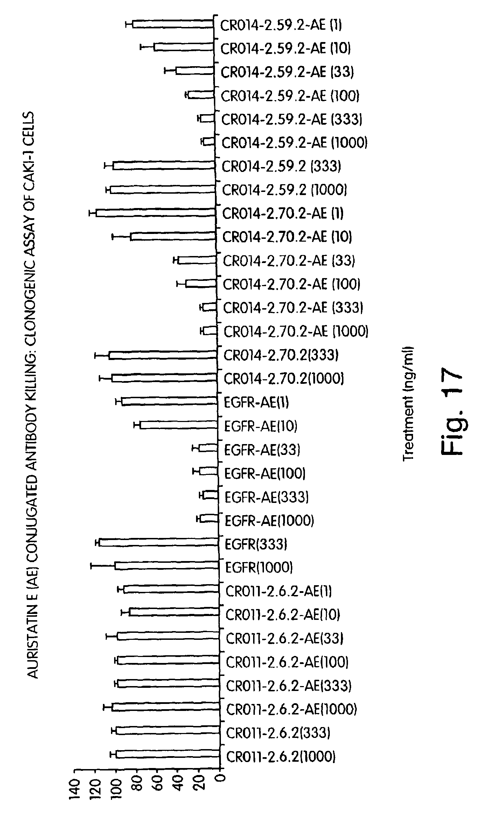

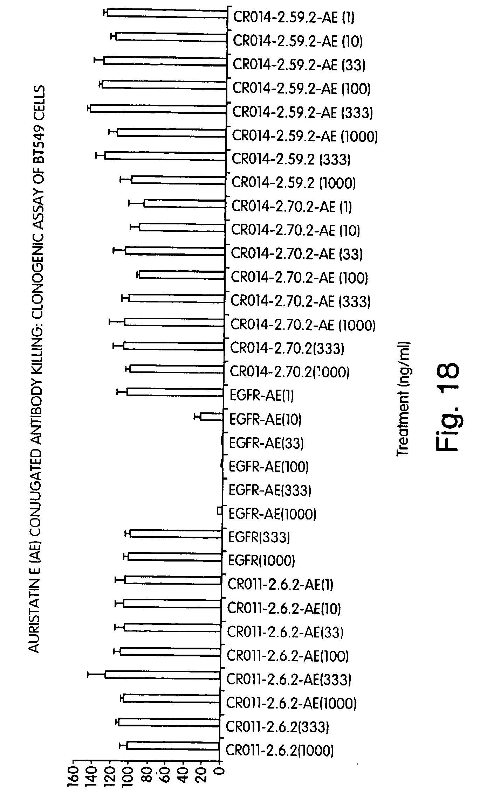

The percent viability in antigen positive CAKI-1 and antigen negative BT549 cell lines are presented in (Figure 17 and Figure 18), respectively. CR011.2.6.2 was used as the isotype control. A monoclonal antibody (NeoMarkers MS-269-PABX) generated against the EGF receptor, which is expressed by both cell lines, was used to demonstrate specific killing mediated by AE-conjugated antibody.

The results indicate that unconjugated and AE-conjugated CR011.2.6.2 mAb had no effect on growth of both CAKI-1 and BT549 cells. However, both cell lines were susceptible to AE-EGFR mAb mediated toxin killing in a dose-dependent fashion. At the maximum dose, both CG57008 mAbs (CR014.2.59.2 and 2.70.2) induced over 90 % CAKI-1 cell death when compared to their unconjugated counterparts. The response was dose dependent. At the same dose range, both CR014.2.59.2 and 2.70.2 mAbs did not affect the survival of BT549 cells.

**Example 14. Preparation and testing of chemotherapy and radio-immunoconjugated antibodies**

Cytotoxic chemotherapy or radiotherapy of cancer is limited by serious, sometimes life-threatening, side effects that arise from toxicities to sensitive normal cells because the therapies are not selective for malignant cells. Therefore, there is a need to improve the selectivity. One strategy is to couple therapeutics to antibodies that recognize tumor-associated antigens. This increases the exposure of the malignant cells to the ligand-targeted therapeutics but reduces the exposure of normal cells to the same agent. (reviewed in Allen, Nat Rev Cancer, 2(10):750-63 (2002)).

CG57008 is one of these tumor-associated antigens, as shown by its specific expression on cellular membranes of tumor cells by FACS and IHC. Therefore one embodiment of the invention uses monoclonal antibodies directed against CG57008 coupled to cytotoxic chemotherapic agents or radiotherapic agents as anti-tumor therapeutics.

Depending on the intended use of the antibody, *i.e.*, as a diagnostic or therapeutic reagent, radiolabels are known in the art and have been used for similar purposes. For instance, radionuclides which have been used in clinical diagnosis include $I^{131}$, $I^{125}$, $I^{123}$, $Tc^{99}$, $Ga^{67}$, as well as $In^{111}$. Antibodies have also been labeled with a variety of radionuclides for potential use in targeted immunotherapy (Peirersz et al. (1987). The use of monoclonal antibody conjugates for the diagnosis and treatment of cancer. Immunol. Cell Biol65: 111-125). These radionuclides include $Re^{188}$

and Re[186] as well as Y[90], and to a lesser extent Au[199] and Cu[67]. I[131] has also been used for therapeutic purposes. U.S. Pat. No. 5,460,785 provides a listing of such radioisotopes.

Radiotherapeutic chelators and chelator conjugates are known in the art. For instance, U.S. Pat. No. 4,831,175 is directed to polysubstituted diethylenetriaminepentaacetic acid chelates and protein conjugates containing the same, and methods for their preparation. U.S. Pat. Nos. 5,099,069; 5,246,692; 5,286,850; and 5,124,471 also relate to polysubstituted DTPA chelates.

Cytotoxic chemotherapies are known in the art and have been used for similar purposes. For instance, U.S. Pat. No 6,441,163 describes processes for the production of cytotoxic conjugates of maytansinoids and antibodies. The anti-tumor activity of a new tubulin polymerization inhibitor, auristatin PE, is also know in the art (Mohammad et al., Int J Oncol, 15(2):367-72 (1999).

Once these chemotherapy or radiolabel and antibody conjugates are made, they can be tested for their cytotoxic activity on CG57008-02 expressing cells. Such experiments use methods known in the art, such as MTS, cell counting and clonogenic assays.

**Example 15. FACS analysis of expression of CG57008-02 on CD4+ T cells**

**Method**

Mononuclear cells were isolated from human blood diluted 1:1 in PBS, by spinning over Ficoll for 20 min. The mononuclear cells were washed twice at 1000 rpm with PBS -Mg and Ca and re-suspended in Miltenyi buffer; PBS, 0.5% BSA, 5 mM EDTA at approximately $10^8$ cells/ml. 20 $\mu$L of CD4 Miltenyi beads were added per $10^7$ cells and incubated for 15 min on ice. Cells were washed with a 10-fold excess volume of Miltenyi buffer. VS positive selection column was washed with 3 mL of Miltenyi buffer. The pelleted cells were re-suspended at $10^8$ cells per mL of Miltenyi buffer and applied to the washed VS column. The column was then washed 3 times with 3 mL of Miltenyi buffer. Following this, the VS column was removed from the magnetic field and CD4+ cells were eluted from the column with 5 mL of Miltenyi buffer. Isolated CD4+ lymphocytes were pelleted and re-suspended in DMEM 5% FCS plus additives (non essential amino acids, sodium pyruvate, mercaptoethanol, glutamine, penicillin, and streptomycin) at $10^6$ cells/mL. $1 \times 10^6$ freshly islolated resting CD4+ T cells were transferred into flow cytometry tubes and washed with 2 ml/tube FACS staining buffer (FSB) containing PBS, 1% BSA and 0.05% $NaN_3$. Cells were spun down and supernatant removed. Cells were blocked with 20% goat serum in FSB for 30 minutes on ice. Cells were washed as above and incubated with 10 $\mu$g/ml of primary human anti CG57008-02 mAb or control PK16.3 mAb in FSB (200 $\mu$l) for 45 min on ice followed by washing. Secondary goat anti-human PE conjugated antibody was added at 1:50 dilution for 45 minutes on ice in the dark, washed, resuspended in 500 $\mu$l of PBS containing 1% formaldehyde and kept at 4°C until flow cytometry analysis was performed.

FACS analysis was performed to determine the expression of CG57008-2 protein as detected with five anti-CG57008-02 antibodies. (2.59.2, 1.29, 2.70.2, 2.56.2, 2.45.1) on human and mouse resting CD4+ T cells, as well as human activated and human polarized CD4+ T cells. These analyses demonstrated that freshly isolated resting human CD4+ T cells do not express CG57008-02, while a major fraction of polarized human Th2 and Th1 cells do express CG57008-02. (See Table 28). Table 29 demonstrates that over the course of 5 days, continual stimulation of T cells results in an increase in CG57008 expression, as measured by 2.70.2 antibody, as compared to the control PK16.3 antibody. Furthermore, addition of matrix metalloproteinase inhibitor (MMPI) did not measurably increase CG57008 expression, demonstrating that the receptor is not shed from T cells under these experimental conditions. Thus, expression of the CG57008 protein and specific antibody binding is specific to activated Th1 and Th2 cells, which in turn, are characteristic of inflammatory response, specifically asthma.

**Table 28.** FACS Analysis of the Expression of the CG57008-02 protein on human CD4+ Th2 Cells using five anti-cG57008-02 antibodies. The experiment is described in the left hand column and the labeled antibody is specified along the top row. Data is reported as the geometric mean of the fluorescence intensity.

| Experiment | Control PK16.3 | CR014 1.29 | CR014 2.45.1 | CR014 2.56.2 | CR014 2.59.2 | CR014 2.70.2 |
|---|---|---|---|---|---|---|
| Resting Human CD4+ T cells | 4.6 | 4.7 | 5.1 | 6 | 4.9 | N/A |
| Polarized Human CD4+ Th2 Cells | 8.4 | 22.3 | 42.4 | 564.1 | 22 | 27.8 |

**Table 29.** Percent of activated T cells that express CG57008

|  |  | Day 0 | Day 1 | Day 2 | Day 4 | Day 5 |
|---|---|---|---|---|---|---|
| *Control PK16.3* | - MMPI | 1 | 3 | 3 | 1 | 1 |
|  | +MMPI | 1 | 2 | 6 | 2 | 2 |
| *CR014 2.70.2* | - MMPI | 1 | 8 | 10 | 5 | 13 |
|  | + MMPI | 1 | 10 | 14 | 10 | 19 |

**Example 16. Cytokine assays**

IL-4, IL-5, IL-10, IL-13, and IFNγ production levels by activated Th1 and Th2 cell were measured in culture supernatants treated with anti-CG57008-01 antibodies using standard ELISA protocols. Cytokine production by Th1 or Th2 cells treated with anti-CG57008-02 antibodies was compared to Th1 or Th2 cells treated with the control PK16.3 antibody. In addition, the following samples were run in parallel as internal controls: *i*) anti-CD3 treated Th1 or Th2 cells, where no cytokine production is expected because of the absence of co-stimulation, *ii*) anti-CD3/anti-CD28 stimulated Th1 or Th2 cells, expected to show detectable cytokine production, and *iii*) untreated Th1 or Th2 cells.

CD4+ T cells were isolated as described in Example 15. Isolated CD4+ lymphocytes were then spun down and re-suspended in DMEM 5% FCS plus additives (non essential amino acids, sodium pyruvate, mercaptoethanol, glutamine, penicillin, and streptomycin) at $10^6$ cells/mL. Falcon 6-well non-tissue culture treated plates were precoated overnight with anti-CD3 (2 μg/ml) and anti-CD28 (10 μg/ml) (600ul total in Dulbecco's PBS) overnight at 4°C. The plates were washed with PBS and CD4+ lymphocytes were suspended at 500,000 cells/ml in Th2 medium: DMEM+ 10% FCS plus supplements and IL-2 5ng/ml, IL-4 5 ng/ml, anti-IFN gamma 5μg/ml and cells were stimulated 4-6 days 37 °C temp and 5 % CO2 in the presence of 5 μg/ml of Mab recognizing CG57008 or isotype matched negative control mAb PK16.3.

In another set of experiments, CD4+ lymphocytes were suspended at 500,000 cells/ml in Th1 medium: DMEM+ 10% FCS plus supplements and IL-2 5ng/ml, IL12 5 ng/ml, anti-IL-4 5μg/ml and stimulated 4-6 days 37 °C temp and 5 % CO2 in the presence of 5 μg/ml CR014 or isotype matched control mAb PK16.3. Cells were washed 2x in DMEM and resuspended in DMEM, 10% FCS plus supplements and 2 ng/ml IL-2 (500,000 cells/ml) in the presence of 5 μg/ml CR014 or control PK16.3 mAb and cultured (rested) for 4-6 days 37 °C temp and 5 % CO2. The process of activation and resting was repeated at least once more as described above with the addition of anti-CD95L to prevent apoptosis of cells through FAS. Falcon 96-well non-tissue culture treated plates precoated overnight with anti-CD3 mAb at 500 ng/mL and costimulatory molecule CG55790 (B7H2, 5μg/ml) were washed and 100 μl of CR014 treated Th1 or Th2 (200,000 cells) added per well. After 3 days of culture, the supernatants were removed and IL-4, IL-5, IL-10, IL-13, and IFNγ levels were determined by ELISA (Pharmingen, San Diego, CA or R&D Systems, Minneapolis, MN).

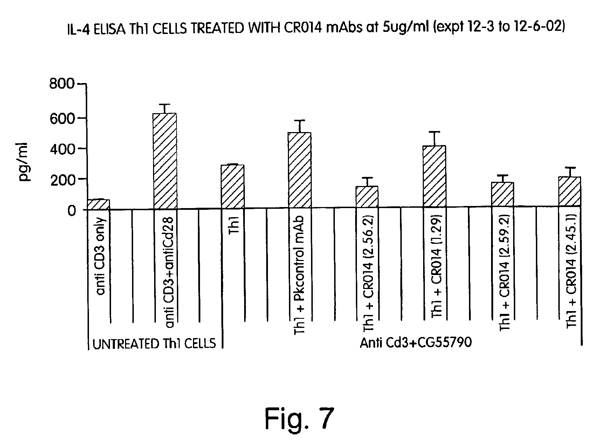

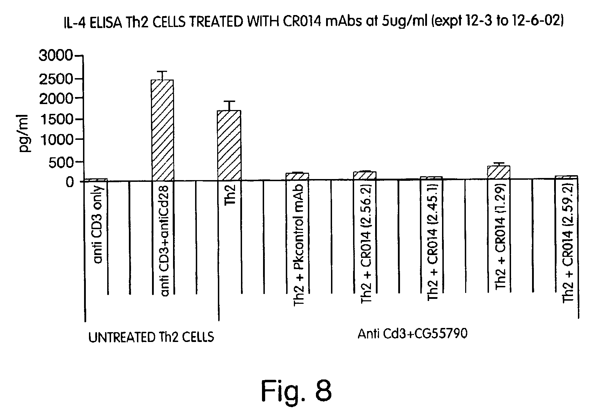

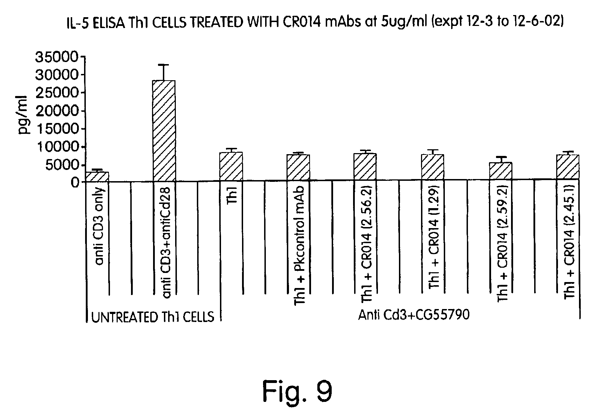

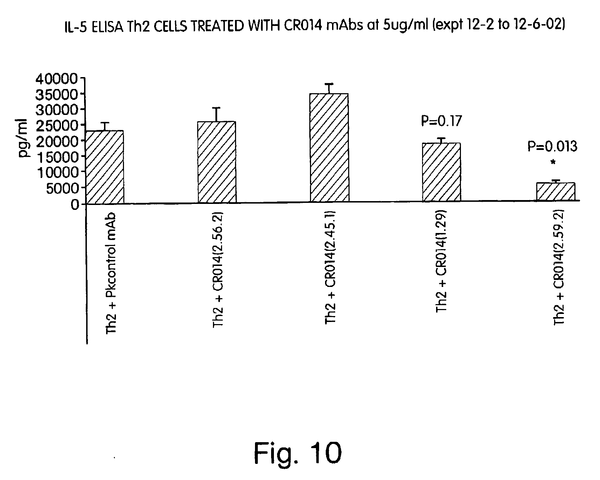

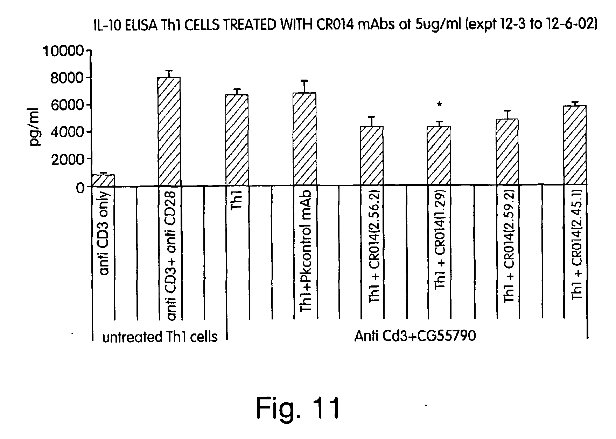

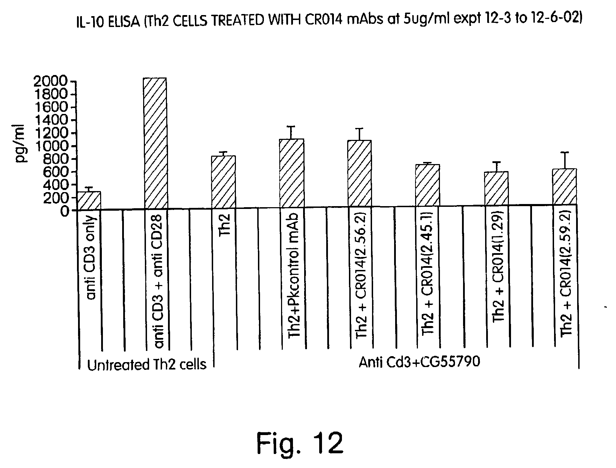

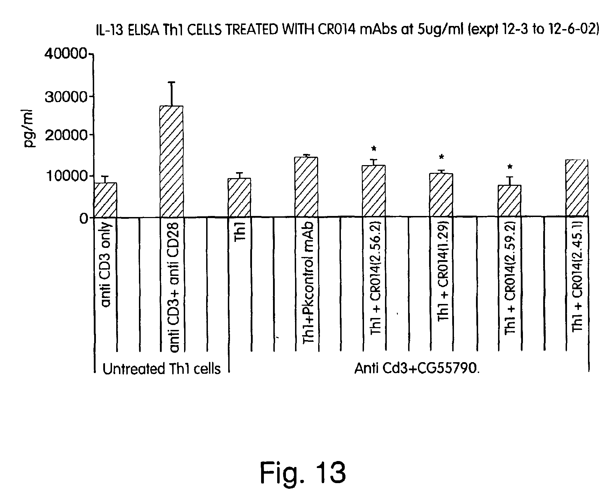

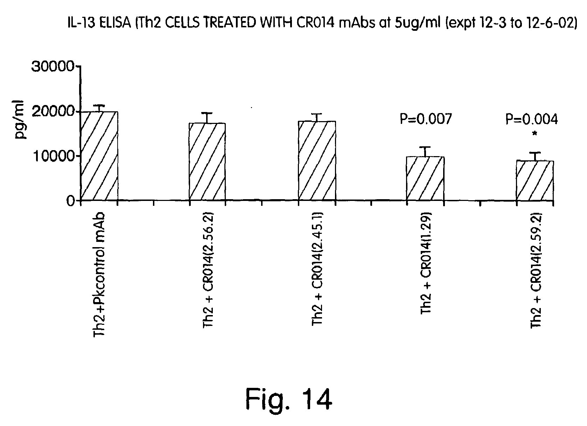

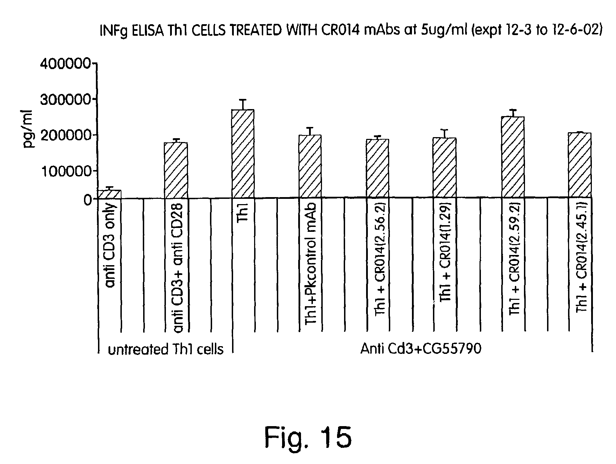

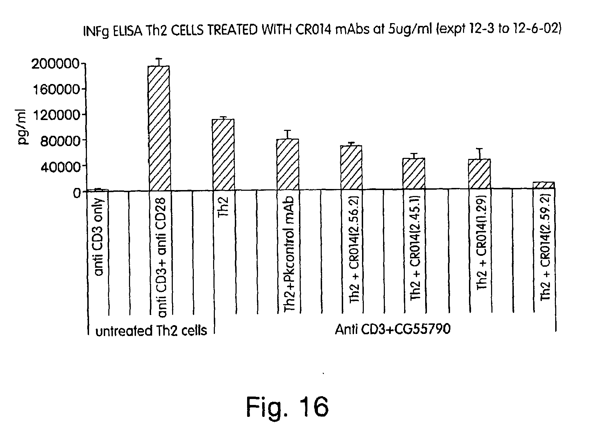

As demonstrated below, anti-CG57008-02 significantly inhibited release of the tested cytokines by Th1 and Th2 cells (see Figures 7 - 16). Results where inhibition of cytokine production is significant (p=.02-.008), are marked on the bar chart with an asterisk.

Table 30 and Table 31 summarize the bar-graphs shown in Figure 7 through Figure 16. A summary of Th2 cytokine inhibition data obtained from multiple experiments with different donors is provided in Table 32. Each experiment used purified CD4+ cells isolated from whole blood samples from two independent donors. Cytokine production is reported as the percent of cytokine production detected using the control PK16.3 mAb. The mAb used in each experiment is specified along the bottom row. Results that report significant cytokine inhibition are underlined in Table 32 below, a "ND" indicates that the experiment was not performed. These results do reflect donor dependent variability but show that mAbs 2.59.2 and 1.29 reproducibly block one or more of the Th2 cytokines.

**Table 30.** Cytokine Inhibition in CD4+ Th1 cells using anti-CG57008 antibodies in two independent human donors. Experiments that demonstrate significant inhibition of cytokine production are marked with an asterisk: *P= 0.01 to 0.05; **P=0.005 to 0.009; ***P=0.001 to 0.004

| Donor 12+17 | | PERCENTAGE OF CONTROL ANTIBODY | | | | |
|---|---|---|---|---|---|---|
| | Cytokines / CR014 mAbs | IL-5 | IL-4 | IL-10 | IL-13 | INF γ |
| TH1 | 2.56.2 | 100.17 | 28.49 * | 63.76 * | 86.45 | 93.69 |
| | 2.45.1 | 90.23 | 39.78 * | 83.98 | 96.25 | 100.6 |
| | 1.29 | 94.63 | 81.05 | 60.77 ** | 73.95 *** | 93.51 |
| | 2.59.2 | 66.62 * | 31.40 * | 68.99 * | 54.5 *** | 128.12 |

**Table 31.** Cytokine Inhibition in CD4+ Th2 cells using anti-CG57008 antibodies in two independent human donors. Experiments that demonstrate significant inhibition of cytokine production are marked with an asterisk: *P= 0.01 to 0.05; **P=0.005 to 0.009; ***P=0.001 to 0.004

| Donor 12+17 | | PERCENTAGE OF CONTROL ANTIBODY | | | | |
|---|---|---|---|---|---|---|
| | Cytokines / CR014 mAbs | IL-5 | IL-4 | IL-10 | IL-13 | INF γ |
| TH2 | 2.56.2 | 112.07 | 103.46 | 93.97 | 86.45 | 88.30 |
| | 2.45.1 | 148.7 | 25.66 *** | 55.97 * | 86.81 | 25.66 * |
| | 1.29 | 80.26 | 112.54 | 44.45 * | 48.91 ** | 112.54 |
| | 2.59.2 | 23.62 * | 19.17 ** | 43.86 * | 43.71 *** | 19.18 * |

**Table 32.** Summary of Cytokine Inhibition using mAbs 2.59.2 and 1.29 in 5 independent human donor groups. Results of experiments that report inhibition greater than 50% of that seen using the control PK16.3 antibody are underlined.

| Donor ID \ Cytokine | 12+17 | 12+14 | 13+14 | 14 | 12 |
|---|---|---|---|---|---|
| IL-4 | <u>19</u> | 626 | 130 | ND | ND |
| IL-5 | <u>24</u> | <u>5</u> | 122 | 67 | <u>2</u> |
| IL-10 | <u>44</u> | 83 | <u>19</u> | <u>45</u> | 109 |
| IL-13 | <u>44</u> | ND | <u>17</u> | 100 | 91 |

|  2.59.2 mAb  |  1.29 mAb  |
|---|---|

### Example 17. CR014 Animal Experiments

**For Inflammation:**

**FITC Contact Sensitivity: Th2 model using fluorescein isothyocyanate (FITC) as a contact sensitizer**

Animals (*e.g.* mice, rats, monkeys, humans) can develop what is called a "contact sensitivity" (CS) response to topical exposure with an antigen toward which they have prior immunity. There are two basic types of CS, one mediated by CD8 T cells (TNCB, DNFB contact sensitivity, poison ivy exposure), and another that triggers IL-4-secreting CD4 T cells (Th2) to recruit eosinphils to the site of antigen exposure. The response requires a T cell/antigen presenting cell interaction, production of "Th2" cytokines such as IL-4, and recruitment of eosinophils.

**Mouse strains:** Any mouse strain may be used, but this assay works best in BALB/c mice, males or females, from 6-8 weeks of age (between 20 and 35 grams body weight).

**Skin sensitization:** For skin sensitization, the abdomens of the mice are shaved to expose the skin and then painted with 10 ul of contact sensitizers (FITC in acetone & dibutyl pthalate).

**Ear measurement:** Five to six days later, mice will be anesthetized with an intraperitoneal injection of pentabarbitol sodium in water (60 mg/kg body weight). A relatively long-lasting anesthetic is required for the time needed to measure ear-thickness and "paint" the ears with the contact sensitizer although the experienced person can usually work with isoflurane. The "pre-challenge" ear thickness measurements are made using a dial thickness gauge (*e.g.* an engineer's micrometer) measuring in units of $10^{-4}$ inches. Three quick readings are made per ear (taking care to move stray hairs out of the way), and an average is taken.

**Antigen challenge:** While mice remain anesthetized, the dorsal surfaces of the ears of immunized and control mice are painted with FITC (0.5%) in the appropriate diluents. After 24 hours, the "post-challenge" ear thickness measurements are made. The 24 h change in ear thickness is proportional to the antigen-specific inflammatory response. Immediately following the ear measurements, mice are sacrificed and analysis performed as described above.

**Th2/eosinophilia measurement:** 24 hours post-challenge, the mice are anesthetized with isoflurane and "post-challenge" ear thickness measurements are made as above. The 24 h change in ear thickness is proportional to the magnitude of the antigen-specific inflammatory response. Immediately following the ear measurements, mice are sacrificed by CO2 inhalation and their ears collected for histologic analysis.

**Mouse ovalbumin (OVA) asthma: Th2 model using ovalbumin to induce eosinophil influx in the lungs:** Animals (*e.g.* mice, rats, monkeys, humans) can develop asthma in response to repeated antigen exposure to the lungs.

Ovalbumin has commonly been used to induce eosinophil influx and airway hyper-reactivity in mice. The response requires a T cell/antigen presenting cell interaction, production of "Th2" cytokines such as IL-4, IgE production, recruitment of eosinophils, and mast cell degranulation.

**Mouse strains:** Several mouse strains have been used. Some strains show better airway responses, others show better IgE, and others show clearer cytokine responses in the broncho-alveolar lavage (BAL) or serum.

**Asthma induction:** Mice are immunized on day 0 and day 5 with OVA i.p. (8 ug) in alum (2 mg). Animals receive two, 1h aerosol challenges with OVA (0.5% in PBS) on day 12, using an aerosol chamber. Control animals receive PBS aerosol. Half of the animals are tested on day 14 for effects on pulmonary function (airway hyperreactivity), and the other half are examined for cell types and numbers in their BAL on day 15.

**Pulmonary function assessment:** Pulmonary conductance ($GL$) and pulmonary dynamic compliance ($Cdyn$) changes are measured in response to methacholine, administered via a jugular venous catheter to anesthetized and ventilated mice in a whole-body plethysmograph. The output signals from 8-10 consecutive breaths are analyzed using a computerized cross-correlation method. The $GL$ and $Cdyn$ peak responses to each dose are expressed as a percentage of the baseline values before that dose.

**BAL assessment:** Animals are injected intraperitoneally with a tribromoethanol solution for deep anaesthesia and sacrificed. After thoracotomy, the trachea of each animal is cannulated and bronchoalveolar lavage (BAL) are performed twice with 0.5 ml PBS buffer 12. The collected volume is centrifuged and the supernatant is frozen at -70°C. The cellular content is resuspended in PBS and the cells are counted by quantification of an aliquot using a haemocytometer. The leukocyte differential is determined from Diff-Quik (Baxter)-stained cytocentrifuged BAL fluid, and the absolute eosinophil count derived as the product of the leukocyte count and the eosinophil fraction.

**Other assessments:** Other parameters that can be examined in this model are 1) total serum or BAL IgE, 2) OVA-specific serum or BAL IgE, and 3) serum or BAL cytokines.

**Mouse ear-swelling DTH : Th1 models with antigens such as hen egg lysozyme (HEL), ovalbumin (OVA), or chicken gammaglobulin**

**Th1 "tuberculin-type" DTH:** Animals (*e.g.* mice, rats, monkeys, humans) will develop what is called "delayed type hypersensitivity" (DTH) responses in response to intradermal or subcutaneous exposure to an antigen toward which they have prior immunity. There are two basic types of DTH, one being mediated by CD8 T cells (contact sensitivity, poison ivy exposure), and the other driven by IFN-gamma-secreting CD4 T cells (Th1) that recruit macrophages to the site of antigen exposure. The latter has been termed a tuberculin-type DTH, and shows a characteristic delayed inflammation occurring at 24-48 hours. The response requires a T cell/antigen presenting cell interaction, production of "Th1 cytokines such as IFN-gamma, and chemokines responsible for recruiting macrophages.

**Mouse strains:** Any mouse strain may be used, but the most work has been done using BALB/c and C57BL/6 mice, males or females, from 6-8 weeks of age (between 20 and 35 grams body weight).

**Antigen Immunization:** Mice are immunized with HEL, OVA, or chicken gammaglobulin (100 ug/mouse) in 100 ul CFA administered subcutaneously at the base of the tail.

**Ear measurement and challenge:** Seven to ten days later, the mice are anesthetized with an intraperitoneal injection of pentabarbitol sodium in water (60 mg/kg body weight). A relatively long-lasting anesthetic is required for the time needed to measure ear-thickness and inject the ears, although the experienced person can usually work with isoflurane. The "pre-challenge" ear thickness measurements are made using a dial thickness gauge (*e.g.* engineer's micrometer) measuring in units of $10^{-4}$ inches. Three quick readings are made per ear (taking care to move stray hairs out of the way), and an average is taken. While mice remain anesthetized, the dorsal surfaces of the ears of immunized and control mice are injected subcutaneously with antigen (40 ug of HEL, or 10 ug OVA, or 10 ug chicken gammaglobulin in 10 ul of PBS), using a tuberculin syringe and a 30g needle.

**DTH measurement:** 24 hours post-challenge, the mice are anesthetized with isoflurane and "post-challenge" ear thickness measurements are made as above. The 24 h change in ear thickness is proportional to the magnitude of the antigen-specific inflammatory response. Immediately following the ear measurements, mice are sacrificed by CO2 inhalation and their ears collected for histologic analysis.

**For Kidney Cancer: Efficacy Evaluation of CR014 Auristatin E- Conjugated Antibody Against the 786-0 Human Renal Cell Carcinoma Xenograft in Nude Mice**

The objective of this study is to evaluate the antitumor efficacy of CR014 against a human renal cell carcinoma (786-0) grown as a xenograft in athymic (nude) mice.

### Test System

| | |
|---|---|
| Species/strain: | Mouse/ CD-1 nu/nu athymic |
| Physiological state: | Normal |
| Age/weight range at start of study: | Animals aged 5 to 6 weeks with body weight of approximately 20 g |
| Animal supplier: | Charles River |
| Number/sex of animals: | TBD/Female |
| Identification: | Individually tattooed tails. |
| Randomization: | Animals will be randomized after tumor implantation, but before treatment commences. |
| Justification: | Human xenograft models represent a well-characterized system for testing of anti-tumor agents. |
| Replacement: | Animals will not be replaced during the course of the study. |

### Animal Housing and Environment

| | |
|---|---|
| Housing: | Static microisolators. |
| Acclimation: | 1 week. |
| Environmental conditions: | 12-hour light cycle at 21 - 22° C (70 - 72 °F) and 40% - 60% humidity. |
| Food/water and contaminants: | Irradiated standard rodent diet (NIH31 Modified and Irradiated) consisting of: 18% protein; 5% fat; and 5% fiber; water (reverse osmosis, 1 ppm Cl), ad libitum |

### Test Article

| | |
|---|---|
| Identity and lot number: | CR14, Batch # TBD |
| Physical description: | Clear Liquid |
| Source: | CuraGen Corporation, Branford, CT |
| Characterization/certification: | Purity of Batch # TBD |
| Storage conditions: | Tubes of CR14 should be stored at-70°C until ready for use. |
| Stability/expiration date: | TBD |
| Hazards/precautions: | No special handling requirements or precautions are needed. |
| Retention of reserve samples: | Reserve samples will be retained for confirmation. |
| Disposition of unused test article: | Unused CR14 should be stored at 4°C before being returned to CuraGen Corporation. |

### Vehicle

| | |
|---|---|
| Identity and lot number: | TBD |
| Source: | CuraGen Corporation, Branford CT, 06405 |
| Storage conditions: | TBD |
| Stability/expiration date: | TBD |

### Test Article/Vehicle Mixture

| | |
|---|---|
| Dosage form: | CR014 in the appropriate vehicle |
| Dosage preparation/storage: | TBD |
| Frequency of preparation: | TBD |
| Stability/expiration date: | TBD |
| Storage and analysis of dosing mixtures for concentration: | TBD |
| Disposition of unused dosing mixture: | Unused CR14 should be stored at 4°C before being returned to CuraGen Corporation. |

**Administration of Test Article**

| | |
|---|---|
| Route and method of administration: | Intravenous (i.v.) injection. |
| Justification for route of administration: | This is an intended clinical route of administration. |
| Frequency and duration of dosing: | Every 4 days for a total of 4 doses. |
| Administered doses: | In mg/kg, see Table |
| Administered volume(s): | Adjust per animal weight. |
| Justification for dose levels: | To determine the anti-tumor effects of an immunoconjugate of CR014 against the 786-0 human renal carcinoma xenograft. |

**Experimental Design**

After an acclimation period, mice are implanted subcutaneously with a total of 5 x $10^6$ 786-0 renal cell carcinoma cells in a volume of 0.20 mL of serum-free medium. Animals are randomized and individually identified. After tumors reach a volume of 150mm$^3$, treatment with CR014 will begin. CR014 is administered intravenously (i.v.) at the doses and schedule in Table 33. Mice will be observed daily for morbidity and mortality, and the tumors and body weight will be recorded twice weekly throughout the study period. The study design is shown in Table 33, and the study schedule is shown in Table 34.

**Table 33.** Study Design

| Group Number | Number of Animals | Treatment | Treatment Schedule* | Volume (mL) |
|---|---|---|---|---|
| 1 | 10 females | Vehicle Control, i.v. | q4d X 4 | Based on weight |
| 2 | 10 females | Isotype Control, i.v. | q4d X 4 | Based on weight |
| 3 | 10 females | Paclitaxel 20 mg/kg, i.v.* | qd X 5 | Based on weight |
| 4 | 10 females | 10.0 mg/kg CR014#1, i.v. | q4d X 4 | Based on weight |
| 5 | 10 females | 3.3 mg/kg CR014#1, i.v. | q4d X 4 | Based on weight |
| 6 | 10 females | 1.0 mg/kg CR014#1, i.v. | q4d X 4 | Based on weight |
| 7 | 10 females | 10.0 mg/kg CR014#2, i.v. | q4d X 4 | Based on weight |
| 8 | 10 females | 3.3 mg/kg CR014#2, i.v. | q4d X 4 | Based on weight |
| 9 | 10 females | 1.0 mg/kg CR014#2, i.v. | q4d X 4 | Based on weight |

*At present, the only FDA-approved agents for renal cell carcinoma are Interferon-alpha and Interleukin-2, both of which produce only a 15 % objective response with an extremely low (<5 %) rate of durable responses. (NCI Physicians Data Query). Consequently, there is no effective positive control for renal cell carcinoma in the xenograft model. The drug Paclitaxel has replaced the positive control in the study design as a reference agent.

| Table 34: Study Schedule | | | | | | | | | |
|---|---|---|---|---|---|---|---|---|---|
| **Event** | **Day -7** | **Day 0** | **Day 7** | | | | | | |
| **Receipt of animals [a]** | X | | | | | | | | |
| **Tumor Implantation** | | X | | | | | | | |
| **Body weights** | | X | | | | | | | |
| **Scheduled termination** | | | | | | | | | |
| | | | | | | | | | |

**Tumor implantation:** Tumor cells are harvested from sub-confluent exponentially growing cell cultures. Cells are counted and evaluated for viability using trypan blue before being suspended in serum-free medium. A total of 5 x $10^6$ cells in a volume of 0.20 mL are implanted subcutaneously in the flanks of mice. For this study, matrigel is not used to enhance tumor take rates.

**Tumor Measurement and Volume Determination:** Tumor growth is measured and recorded 3 times a week using a Vernier caliper. Length and width are measured for each tumor. Tumor weight is determined using the following formula:

$$\text{Tumor Weight (mg)} = \frac{w^2 \times l}{2}$$

**Clinical Observations/Signs:** Animals are observed daily for significant clinical signs, moribidity and mortality.

**Animals Found Dead or Moribund:** Percentage of animal mortality and time to death will be recorded for every group in the study. Mice may be defined as moribund and sacrificed if one or more of the following criteria are met:

1) Loss of body weight of 20% or greater in a 2-week period.
2) Tumors that inhibit normal physiological function such as eating, drinking, mobility and ability to urinate and or defecate.
3) Tumors that exceed a maximum dimension of 2000 mg as measured by calipers.
4) Ulcerated tumors, or tumors which bleed or produce exudates.
5) Prolonged diarrhea leading to weight loss.
6) Persistent wheezing and respiratory distress.

Animals can also be considered moribund if there is prolonged or excessive pain or distress as defined by clinical observations such as: prostration, hunched posture, paralysis/paresis, distended abdomen, ulcerations, abscesses, seizures and/or hemorrhages.

**Statistics (TGI):** The One-Way Analysis of Variance (ANOVA) and Mann-Whitney U test (analyzing means and medians, respectively) were used to determine the statistical significance of any difference between the mean group tumor weights on the day of %TGI calculations. Dunnett's test was applied to groups with unequal sample size and Welch's correction was applied when populations of unequal variance were compared. All statistical analyses were conducted at an $\alpha$ level of 0.05 (two-tailed). Prism (GraphPad) version 3.0 was used for all statistical analyses and graphic presentations.

**Statistics (TGD):** The Logrank test was used to compare differences in overall survival experience between treated and control groups. Statistical analyses were conducted at an $\alpha$ level of 0.05 (two-tailed). Prism (GraphPad) version 3 was used for all statistical analyses and graphic presentation.

1. Tumor volumes, doubling rate and tumor growth delay.
2. Tumor growth delay at 500, 1000, 1500 and 2000 $mm^3$.
3. Animal weight charts with percent weight change for treatment groups.

**Final Report:** The final report includes a summary of the methods and the raw data as well as results on tumor size, tumor weight, rate of tumor growth/cell line/sex, tumor growth delay, animal weights, percentage mortality, time to death and clinical observations.

## OTHER EMBODIMENTS

Although particular embodiments have been disclosed herein in detail, this has been done by way of example for purposes of illustration only, and is not intended to be limiting with respect to the scope of the appended claims, which follow. In particular, it is contemplated by the inventors that various substitutions, alterations, and modifications may be made to the invention without departing from the scope of the invention as defined by the claims. Other aspects, advantages, and modifications considered to be within the scope of the following claims. The claims presented are representative of the inventions disclosed herein. Other, unclaimed inventions are also contemplated. Applicants reserve the right to pursue such inventions in later claims.

## Claims

**1.** An isolated human or humanised antibody or an immunologically active fragment thereof that immunospecifically binds to a human Hepatitis A Virus cellular receptor I (HAVcr-1) polypeptide comprising a sequence selected from SEQID NO: 2, 4, 6, 8 and 39 for use in treating cancer in a subject selected from lung cancer and ovarian cancer, wherein said antibody or fragment binds the HAVcr-1 polypeptide with an equilibrium dissociation constant that is less than or equal to 100 nM and wherein the antibody or immunologically active fragment thereof is conjugated to a cytotoxic agent selected from the group consisting of a chemotherapeutic agent, a toxin, and a radioactive isotope.

2. The isolated antibody or immunologically active fragment for the use of claim 1, wherein the antibody is a monoclonal antibody or an immunologically active fragment thereof.

3. The isolated antibody or immunologically active fragment for the use of claim 1, wherein the cytotoxic agent is auristatin E or a derivative thereof.

4. The isolated antibody or immunologically active fragment for the use of any one of claims 1 to 3, wherein the immunologically active fragment is selected from Fab, Fab', F(ab')$_2$, Fv and single chain fragments.

5. The isolated antibody or immunologically active fragment for the use of any one of claims 1 to 3, wherein the treatment is of a human patient.

6. The isolated antibody or immunologically active fragment for the use of any one of claims 1 to 2 wherein the cancer is ovarian cancer.


**Patentansprüche**

1. Isolierter humaner oder humanisierter Antikörper oder ein immunologisch aktives Fragment davon, das immunspezifisch an ein humanes Hepatitis-A-Virus-zellulärer-Rezeptor-I-Polypeptid (HAVcr-1) bindet, umfassend eine Sequenz, ausgewählt aus SEQ ID NO: 2, 4, 6, 8 und 39 zur Verwendung bei der Behandlung von Krebs in einem Subjekt, ausgewählt aus Lungenkrebs und Eierstockkrebs, wobei der Antikörper oder das Fragment, das HAVcr-1-Polypeptid mit einer Gleichgewichtsdissoziationskonstante bindet, die kleiner oder gleich 100 nM ist und wobei der Antikörper oder das immunologisch aktive Fragment davon an ein zytotoxisches Mittel konjugiert ist, ausgewählt aus der Gruppe bestehend aus einem chemotherapeutischen Mittel, einem Toxin und einem radioaktiven Isotop.

2. Isolierter Antikörper oder immunologisch aktives Fragment zur Verwendung nach Anspruch 1, wobei der Antikörper ein monoklonaler Antikörper oder ein immunologisch aktives Fragment davon ist.

3. Isolierter Antikörper oder immunologisch aktives Fragment zur Verwendung nach Anspruch 1, wobei das zytotoxische Mittel Auristatin E oder ein Derivat davon ist.

4. Isolierter Antikörper oder immunologisch aktives Fragment zur Verwendung nach einem der Ansprüche 1 bis 3, wobei das immunologisch aktive Fragment ausgewählt ist aus Fab, Fab', F(ab')$_2$, Fv und Einzelkettenfragmenten.

5. Isolierter Antikörper oder immunologisch aktives Fragment zur Verwendung nach einem der Ansprüche 1 bis 3, wobei die Behandlung die Behandlung eines menschlichen Patienten ist.

6. Isolierter Antikörper oder immunologisch aktives Fragment zur Verwendung nach einem der Ansprüche 1 bis 2, wobei der Krebs Eierstockkrebs ist.


**Revendications**

1. Anticorps humain ou humanisé isole ou un fragment immunologiquement actif de celui-ci qui se lie de manière immunospécifique à un polypeptide I récepteur cellulaire du virus humain de l'hépatite A (HAVcr-1) comprenant une séquence choisie parmi SEQ ID NO : 2, 4, 6, 8 et 39, destiné à être utilisé dans le traitement du cancer chez un sujet choisi parmi le cancer du poumon et le cancer de l'ovaire, dans lequel ledit anticorps ou fragment se lie au polypeptide HAVcr-1 avec une constante de dissociation à l'équilibre qui est inférieure ou égale à 100 nM, et dans lequel l'anticorps ou le fragment immunologiquement actif de celui-ci est conjugué à un agent cytotoxique choisi dans le groupe constitué par un agent chimiothérapeutique, une toxine et un isotope radioactif.

**2.** Anticorps isolé ou fragment immunologiquement actif destiné à une utilisation selon la revendication 1, dans lequel l'anticorps est un anticorps monoclonal ou un fragment immunologiquement actif de celui-ci.

**3.** Anticorps isolé ou fragment immunologiquement actif destiné à une utilisation selon la revendication 1, dans lequel l'agent cytotoxique est l'auristatine E ou un dérivé de celle-ci.

**4.** Anticorps isolé ou fragment immunologiquement actif destiné à une utilisation selon l'une quelconque des revendications 1 à 3, dans lequel le fragment immunologiquement actif est choisi parmi Fab, Fab', F(ab')$_2$, Fv et des fragments à chaîne unique.

**5.** Anticorps isolé ou fragment immunologiquement actif destiné à une utilisation selon l'une quelconque des revendications 1 à 3, dans lequel le traitement est pour un patient humain.

**6.** Anticorps isolé ou fragment immunologiquement actif destiné à une utilisation selon l'une quelconque des revendications 1 et 2, dans lequel le cancer est le cancer de l'ovaire.

PATHWAY 2968812:CG57008 HAVCR-1"

## Fig. 1

TREATMENTS

Fig. 2

Fig. 3

RENAL CELL CANCER

# Fig. 4A

PANCREATIC CANCER

# Fig. 4B

TOXIN-KILLING: CLONOGENIC ASSAY OF ACHN CELLS

Fig. 5

TOXIN-KILLING: CLONOGENIC ASSAY OF BT549 CELLS

Fig. 6

IL-4 ELISA Th1 CELLS TREATED WITH CR014 mAbs at 5ug/ml (expt 12-3 to 12-6-02)

Fig. 7

IL-4 ELISA Th2 CELLS TREATED WITH CR014 mAbs at 5ug/ml (expt 12-3 to 12-6-02)

Fig. 8

IL-5 ELISA Th1 CELLS TREATED WITH CR014 mAbs at 5ug/ml (expt 12-3 to 12-6-02)

Fig. 9

IL-5 ELISA Th2 CELLS TREATED WITH CR014 mAbs at 5ug/ml (expt 12-2 to 12-6-02)

Fig. 10

IL-10 ELISA Th1 CELLS TREATED WITH CR014 mAbs at 5ug/ml (expt 12-3 to 12-6-02)

Fig. 11

IL-10 ELISA (Th2 CELLS TREATED WITH CR014 mAbs at 5ug/ml expt 12-3 to 12-6-02)

Fig. 12

IL-13 ELISA Th1 CELLS TREATED WITH CR014 mAbs at 5ug/ml (expt 12-3 to 12-6-02)

Fig. 13

IL-13 ELISA (Th2 CELLS TREATED WITH CR014 mAbs at 5ug/ml (expt 12-3 to 12-6-02)

Fig. 14

INFg ELISA Th1 CELLS TREATED WITH CR014 mAbs at 5ug/ml (expt 12-3 to 12-6-02)

Fig. 15

INFg ELISA Th2 CELLS TREATED WITH CR014 mAbs at 5ug/ml (expt 12-3 to 12-6-02)

Fig. 16

AURISTATIN E (AE) CONJUGATED ANTIBODY KILLING: CLONOGENIC ASSAY OF CAKI-1 CELLS

Treatment (ng/ml)

## Fig. 17

AURISTATIN E (AE) CONJUGATED ANTIBODY KILLING: CLONOGENIC ASSAY OF BT549 CELLS

CR014-2.59.2-AE (1)
CR014-2.59.2-AE (10)
CR014-2.59.2-AE (33)
CR014-2.59.2-AE (100)
CR014-2.59.2-AE (333)
CR014-2.59.2-AE (1000)
CR014-2.59.2 (333)
CR014-2.59.2 (1000)
CR014-2.70.2-AE (1)
CR014-2.70.2-AE (10)
CR014-2.70.2-AE (33)
CR014-2.70.2-AE (100)
CR014-2.70.2-AE (333)
CR014-2.70.2-AE (1000)
CR014-2.70.2(333)
CR014-2.70.2(1000)
EGFR-AE(1)
EGFR-AE(10)
EGFR-AE(33)
EGFR-AE(100)
EGFR-AE(333)
EGFR-AE(1000)
EGFR(333)
EGFR(1000)
CR011-2.6.2-AE(1)
CR011-2.6.2-AE(10)
CR011-2.6.2-AE(33)
CR011-2.6.2-AE(100)
CR011-2.6.2-AE(333)
CR011-2.6.2-AE(1000)
CR011-2.6.2(333)
CR011-2.6.2(1000)

160 140 120 100 80 60 40 20 0

Treatment (ng/ml)

Fig. 18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0200691 A
- WO 9744460 A1
- WO 9604376 A1
- WO 200153312 A1
- WO 9938881 A1
- WO 9744460 A
- WO 200200691 A2
- US 2002090672 A1
- WO 200154474 A2
- WO 200177137 A1
- WO 200154708 A1
- WO 0044895 A
- WO 9932619 A
- WO 0175164 A
- WO 019251 A
- WO 0129058 A
- WO 0189304 A
- WO 0216620 A
- WO 0229858 A
- US 4987071 A, Cech
- US 5116742 A, Cech
- WO 8809810 A
- WO 8910134 A
- US 4816567 A
- US 5225539 A
- US 5545807 A
- US 5545806 A
- US 5569825 A
- US 5625126 A
- US 5633425 A
- US 5661016 A
- WO 9402602 A
- WO 9633735 A
- WO 9634096 A
- US 5939598 A
- US 5916771 A
- WO 9953049 A
- US 4946778 A
- WO 9308829 A
- WO 9627011 A

- US 4676980 A
- WO 9100360 A
- WO 92200373 A
- EP 03089 A
- WO 9411026 A
- US 4485045 A
- US 4544545 A
- US 5013556 A
- US 3773919 A
- US 4873316 A
- EP 264166 A
- US 4736866 A
- US 4870009 A
- US 4873191 A
- WO 9011354 A
- WO 9101140 A
- WO 920968 A
- WO 9304169 A
- US 4522811 A
- US 5328470 A
- US 5223409 A, Ladner
- US 5233409 A, Ladner
- US 5283317 A
- WO 9410300 A, Brent
- US 5272057 A
- US 4683195 A
- US 4683202 A
- US 5493531 A
- WO 9416101 A
- US 5459039 A
- US 6057101 A
- US 6083693 A
- US 5460785 A
- US 4831175 A
- US 5099069 A
- US 5246692 A
- US 5286850 A
- US 5124471 A
- US 6441163 B

### Non-patent literature cited in the description

- *J Biol Chem.,* 13 February 1998, vol. 273 (7), 4135-42
- *J Virol.,* August 1998, vol. 72 (8), 6621-8
- **KUEHN EW.** *Am J Physiol Renal Physiol,* December 2002, vol. 283 (6), F1326-36

- **BAILLY V.** *J Biol Chem,* 18 October 2002, vol. 277 (42), 39739-48
- **PREST SJ.** *FASEB J,* 12 February 2002
- **CHEN YH.** *Biochem Biophys Res Commun,* 11 August 2000, vol. 274 (3), 576-82

- **MCINTIRE JJ.** *Nat Immunol.,* December 2001, vol. 2 (12), 1 109-16
- **WILLS-KARP M.** *Nature Immunology,* vol. 2, 1095-1096
- **MCINTIRE JJ.** *Nature Immunology,* vol. 2, 1109-1116
- MOLECULAR CLONING: A LABORATORY MANUAL. Cold Spring Harbor Laboratory Press, 1989
- CURRENT PROTOCOLS IN MOLECULAR BIOLOGY. John Wiley & Sons, 1993
- **AUSUBEL et al.** CURRENT PROTOCOLS IN MOLECULAR BIOLOGY. John Wiley & Sons, 1993
- CURRENT PROTOCOLS IN MOLECULAR BIOLOGY. John Wiley & Sons, 1989, 6.3.1-6.3.6
- **KRIEGER.** GENE TRANSFER AND EXPRESSION, A LABORATORY MANUAL. Stockton Press, 1990
- **KRIEGLER.** GENE TRANSFER AND EXPRESSION, A LABORATORY MANUAL. Stockton Press, 1990
- **SHILO ; WEINBERG.** *Proc Natl Acad Sci USA,* 1981, vol. 78, 6789-6792
- **TUSCHL ; ZAMORE ; LEHMANN ; BARTEL ; SHARP.** *Genes & Dev.,* 1999, vol. 13, 3191-3197
- **ELBASHIR et al.** *EMBO J.,* 2001, vol. 20 (23), 6877-88
- **ELBASHIR ; LENDECKEL ; TUSCHL.** *Genes & Dev.,* 2001, vol. 15, 188-200
- **HARBORTH et al.** *J. Cell Science,* 2001, vol. 114, 4557-4565
- **SAMBROOK et al.** Molecular Cloning. Cold Spring Harbor Laboratory Press, 1989
- **TUSCHL et al.** *Genes Dev.,* 1999, vol. 13, 3191-3197
- **TUSCHL et al.** *Biochemistry,* 1993, vol. 32, 11658-11668
- **GAULTIER et al.** *Nucl. Acids Res.,* 1987, vol. 15, 6625-6641
- **INOUE et al.** *Nucl. Acids Res.,* 1987, vol. 15, 6131-6148
- **INOUE et al.** *FEBS Lett.,* 1987, vol. 215, 327-330
- **HASELHOFF ; GERLACH.** *Nature,* 1988, vol. 334, 585-591
- **BARTEL et al.** *Science,* 1993, vol. 261, 1411-1418
- **HELENE.** *Anticancer Drug Des,* 1991, vol. 6, 569-84
- **HELENE et al.** *Ann. N.Y. Acad. Sci,* 1992, vol. 660, 27-36
- **MAHER.** *Bioassays,* 1992, vol. 14, 807-15
- **HYRUP et al.** *Bioorg Med Chem,* 1996, vol. 4, 5-23
- **PERRY-O'KEEFE et al.** *Proc. Natl. Acad. Sci. USA,* 1996, vol. 93, 14670-14675
- **FINN et al.** *Nucl Acids Res,* 1996, vol. 24, 3357-3363
- **MAG et al.** *Nucl Acid Res,* 1989, vol. 17, 5973-5988
- **PETERSEN et al.** *Bioorg. Med. Chem. Lett.,* 1975, vol. 5, 1119-11124
- **LETSINGER et al.** *Proc. Natl. Acad. Sci. U.S.A.,* 1989, vol. 86, 6553-6556
- **LEMAITRE et al.** *Proc. Natl. Acad. Sci.,* 1987, vol. 84, 648-652
- **KROL et al.** *BioTechniques,* 1988, vol. 6, 958-976
- **ZON.** *Pharm. Res,* 1988, vol. 5, 539-549
- **NEEDLEMAN ; WUNSCH.** *J Mol Biol,* 1970, vol. 48, 443-453
- CURRENT PROTOCOLS IN MOLECULAR BIOLOGY. John Wiley & Sons, 1992
- **NARANG.** *Tetrahedron,* 1983, vol. 39, 3
- **ITAKURA et al.** *Annu. Rev. Biochem.,* 1984, vol. 53, 323
- **ITAKURA et al.** *Science,* 1984, vol. 198, 1056
- **IKE et al.** *Nucl. Acids Res.,* 1983, vol. 11, 477
- **ARKIN ; YOURVAN.** *Proc. Natl. Acad. Sci. USA,* 1992, vol. 89, 7811-7815
- **DELGRAVE et al.** *Protein Engineering,* 1993, vol. 6, 327-331
- **HOPP ; WOODS.** *Proc. Nat. Acad. Sci. USA,* 1981, vol. 78, 3824-3828
- **KYTE ; DOOLITTLE.** *J. Mol. Biol.,* 1982, vol. 157, 105-142
- **HARLOW E ; LANE D.** Antibodies: A Laboratory Manual. Cold Spring Harbor Laboratory Press, 1988
- **D. WILKINSON.** The Scientist. The Scientist, Inc, 17 April 2000, vol. 14, 25-28
- **KOHLER ; MILSTEIN.** *Nature,* 1975, vol. 256, 495
- **GODING.** Monoclonal Antibodies: Principles and Practice. Academic Press, 1986, 59-103
- **KOZBOR.** *J. Immunol.,* 1984, vol. 133, 3001
- **BRODEUR et al.** Monoclonal Antibody Production Techniques and Applications. Marcel Dekker, Inc, 1987, 51-63
- **MUNSON ; POLLARD.** *Anal. Biochem.,* 1980, vol. 107, 220
- **MORRISON.** *Nature,* 1994, vol. 368, 812-13
- **JONES et al.** *Nature,* 1986, vol. 321, 522-525
- **RIECHMANN et al.** *Nature,* 1988, vol. 332, 323-327
- **VERHOEYEN et al.** *Science,* 1988, vol. 239, 1534-1536
- **PRESTA.** *Curr. Op. Struct. Biol.,* 1992, vol. 2, 593-596
- **KOZBOR et al.** *Immunol Today,* 1983, vol. 4, 72

- **COLE et al.** MONOCLONAL ANTIBODIES AND CANCER THERAPY. Alan R. Liss, Inc, 1985, 77-96
- **COTE et al.** *Proc Natl Acad Sci USA,* 1983, vol. 80, 2026-2030
- **COLE et al.** MONOCLONAL ANTIBODIES AND CANCER THERAPY. Alan R. Liss, Inc, 1985, 77-96
- **HOOGENBOOM ; WINTER.** *J. Mol. Biol.,* 1991, vol. 227, 381
- **MARKS et al.** *J. Mol. Biol.,* 1991, vol. 222, 581
- **MARKS et al.** *Bio/Technology,* 1992, vol. 10, 779-783
- **LONBERG et al.** *Nature,* 1994, vol. 368, 856-859
- **FISHWILD et al.** *Nature Biotechnology,* 1996, vol. 14, 845-51
- **NEUBERGER.** *Nature Biotechnology,* 1996, vol. 14, 826
- **LONBERG ; HUSZAR.** *Intern. Rev. Immunol.,* 1995, vol. 13, 65-93
- **HUSE et al.** *Science,* 1989, vol. 246, 1275-1281
- **MILSTEIN ; CUELLO.** *Nature,* 1983, vol. 305, 537-539
- **TRAUNECKER et al.** *EMBO J.,* 1991, vol. 10, 3655-3659
- **SURESH et al.** *Methods in Enzymology,* 1986, vol. 121, 210
- **BRENNAN et al.** *Science,* 1985, vol. 229, 81
- **SHALABY et al.** *J. Exp. Med.,* 1992, vol. 175, 217-225
- **KOSTELNY et al.** *J. Immunol.,* 1992, vol. 148 (5), 1547-1553
- **HOLLINGER et al.** *Proc. Natl. Acad. Sci. USA,* 1993, vol. 90, 6444-6448
- **GRUBER et al.** *J. Immunol.,* 1994, vol. 152, 5368
- **TUTT et al.** *J. Immunol.,* 1991, vol. 147, 60
- **CARON et al.** *J. Exp Med.,* 1992, vol. 176, 1191-1195
- **SHOPES.** *J. Immunol.,* 1992, vol. 148, 2918-2922
- **WOLFF et al.** *Cancer Research,* 1993, vol. 53, 2560-2565
- **STEVENSON et al.** *Anti-Cancer Drug Design,* 1989, vol. 3, 219-230
- **VITETTA et al.** *Science,* 1987, vol. 238, 1098
- **EPSTEIN et al.** *Proc. Natl. Acad. Sci. USA,* 1985, vol. 82, 3688
- **HWANG et al.** *Proc Natl Acad. Sci. USA,* 1980, vol. 77, 4030
- **MARTIN et al.** *J. Biol. Chem.,* 1982, vol. 257, 286-288
- **GABIZON et al.** *J. National Cancer Inst.,* 1989, vol. 81 (19), 1484
- Remington : The Science And Practice Of Pharmacy. Mack Pub. Co, 1995
- Drug Absorption Enhancement: Concepts, Possibilities, Limitations, And Trends. Harwood Academic Publishers, 1994
- Peptide And Protein Drug Delivery. M. Dekker, 1991, vol. 4
- **MARASCO et al.** *Proc. Natl. Acad. Sci. USA,* 1993, vol. 90, 7889-7893
- ELISA: Theory and Practice: Methods in Molecular Biology. Human Press, 1995, vol. 42
- **E. DIAMANDIS ; T. CHRISTOPOULUS.** Immunoassay. Academic Press, Inc, 1996
- **P. TIJSSEN.** Practice and Theory of Enzyme Immunoassays. Elsevier Science Publishers, 1985
- **GOEDDEL.** GENE EXPRESSION TECHNOLOGY: METHODS IN ENZYMOLOGY. Academic Press, 1990, vol. 185
- **SMITH ; JOHNSON.** *Gene,* 1988, vol. 67, 31-40
- **AMRANN et al.** *Gene,* 1988, vol. 69, 301-315
- **STUDIER et al.** GENE EXPRESSION TECHNOLOGY: METHODS IN ENZYMOLOGY. Academic Press, 1990, vol. 185, 60-89
- **GOTTESMAN.** GENE EXPRESSION TECHNOLOGY: METHODS IN ENZYMOLOGY. Academic Press, 1990, vol. 185, 119-128
- **WADA et al.** *Nucl. Acids Res.,* 1992, vol. 20, 2111-2118
- **BALDARI et al.** *EMBO J.,* 1987, vol. 6, 229-234
- **KURJAN ; HERSKOWITZ.** *Cell,* 1982, vol. 30, 933-943
- **SCHULTZ et al.** *Gene,* 1987, vol. 54, 113-123
- **SMITH et al.** *Mol. Cell. Biol.,* 1983, vol. 3, 2156-2165
- **LUCKLOW ; SUMMERS.** *Virology,* 1989, vol. 170, 31-39
- **SEED.** *Nature,* 1987, vol. 329, 840
- **KAUFMAN et al.** *EMBO J.,* 1987, vol. 6, 187-195
- **SAMBROOK et al.** MOLECULAR CLONING: A LABORATORY MANUAL. Cold Spring Harbor Laboratory Press, 1989
- **PINKERT et al.** *Genes Dev.,* 1987, vol. 1, 268-277
- **CALAME ; EATON.** *Adv. Immunol,* 1988, vol. 43, 235-275
- **WINOTO ; BALTIMORE.** *EMBO J.,* 1989, vol. 8, 729-733
- **BANERJI et al.** *Cell,* 1983, vol. 33, 729-740
- **QUEEN ; BALTIMORE.** *Cell,* 1983, vol. 33, 741-748
- **BYRNE ; RUDDLE.** *Proc. Natl. Acad. Sci. USA,* 1989, vol. 86, 5473-5477
- **EDLUND et al.** *Science,* 1985, vol. 230, 912-916
- **KESSEL ; GRUSS.** *Science,* 1990, vol. 249, 374-379

- **CAMPES ; TILGHMAN.** *Genes Dev.,* 1989, vol. 3, 537-546
- **WEINTRAUB et al.** Antisense RNA as a molecular tool for genetic analysis. *Reviews-Trends in Genetics,* 1986, vol. 1 (1
- **HOGAN.** MANIPULATING THE MOUSE EMBRYO. Cold Spring Harbor Laboratory Press, 1986
- **THOMAS et al.** *Cell,* 1987, vol. 51, 503
- **LI et al.** *Cell,* 1992, vol. 69, 915
- **BRADLEY.** TERATOCARCINOMAS AND EMBRYONIC STEM CELLS: A PRACTICAL APPROACH. IRL, 1987, 113-152
- **BRADLEY.** *Curr. Opin. Biotechnol.,* 1991, vol. 2, 823-829
- **LAKSO et al.** *Proc. Natl. Acad. Sci. USA,* 1992, vol. 89, 6232-6236
- **O'GORMAN et al.** *Science,* 1991, vol. 251, 1351-1355
- **WILMUT et al.** *Nature,* 1997, vol. 385, 810-813
- Remington's Pharmaceutical Sciences
- **CHEN et al.** *Proc. Natl. Acad. Sci. USA,* 1994, vol. 91, 3054-3057
- **LAM.** *Anticancer Drug Design,* 1997, vol. 12, 145
- **DEWITT et al.** *Proc. Natl. Acad. Sci. U.S.A.,* 1993, vol. 90, 6909
- **ERB et al.** *Proc. Natl. Acad. Sci. U.S.A.,* 1994, vol. 91, 11422
- **ZUCKERMANN et al.** *J. Med. Chem.,* 1994, vol. 37, 2678
- **CHO et al.** *Science,* 1993, vol. 261, 1303
- **CARRELL et al.** *Angew. Chem. Int. Ed. Engl.,* 1994, vol. 33, 2059
- **CARELL et al.** *Angew. Chem. Int. Ed. Engl.,* 1994, vol. 33, 2061
- **GALLOP et al.** *J. Med. Chem.,* 1994, vol. 37, 1233
- **HOUGHTEN.** *Biotechniques,* 1992, vol. 13, 412-421
- **LAM.** *Nature,* 1991, vol. 354, 82-84
- **FODOR.** *Nature,* 1993, vol. 364, 555-556
- **CULL et al.** *Proc. Natl. Acad. Sci. USA,* 1992, vol. 89, 1865-1869
- **SCOTT ; SMITH.** *Science,* 1990, vol. 249, 386-390
- **DEVLIN.** *Science,* 1990, vol. 249, 404-406
- **CWIRLA et al.** *Proc. Natl. Acad. Sci. U.S.A.,* 1990, vol. 87, 6378-6382
- **FELICI.** *J. Mol. Biol.,* 1991, vol. 222, 301-310
- **ZERVOS et al.** *Cell,* 1993, vol. 72, 223-232
- **MADURA et al.** *J. Biol. Chem.,* 1993, vol. 268, 12046-12054
- **BARTEL et al.** *Biotechniques,* 1993, vol. 14, 920-924
- **IWABUCHI et al.** *Oncogene,* 1993, vol. 8, 1693-1696
- **D'EUSTACHIO et al.** *Science,* 1983, vol. 220, 919-924
- **VERMA et al.** HUMAN CHROMOSOMES: A MANUAL OF BASIC TECHNIQUES. Pergamon Press, 1988
- **EGELAND et al.** *Nature,* 1987, vol. 325, 783-787
- **LANDEGRAN et al.** *Science,* 1988, vol. 241, 1077-1080
- **NAKAZAWA et al.** *Proc. Natl. Acad. Sci. USA,* 1994, vol. 91, 360-364
- **ABRAVAYA et al.** *Nucl. Acids Res,* 1995, vol. 23, 675-682
- **GUATELLI et al.** *Proc. Natl. Acad. Sci. USA,* 1990, vol. 87, 1874-1878
- **KWOH et al.** *Proc. Natl. Acad. Sci. USA,* 1989, vol. 86, 1173-1177
- **LIZARDI et al.** *BioTechnology,* 1988, vol. 6, 1197
- **CRONIN et al.** *Human Mutation,* 1996, vol. 7, 244-255
- **KOZAL et al.** *Nat. Med,* 1996, vol. 2, 753-759
- **MAXIM ; GILBERT.** *Proc. Natl. Acad. Sci. USA,* 1977, vol. 74, 560
- **SANGER.** *Proc. Natl. Acad. Sci. USA,* 1977, vol. 74, 5463
- **NAEVE, et al.** *Biotechniques,* 1995, vol. 19, 448
- **COHEN et al.** *Adv. Chromatography,* 1996, vol. 36, 127-162
- **GRIFFIN et al.** *Appl. Biochem. Biotechnol,* 1993, vol. 38, 147-159
- **MYERS et al.** *Science,* 1985, vol. 230, 1242
- **COTTON et al.** *Proc. Natl. Acad. Sci. USA,* 1988, vol. 85, 4397
- **SALEEBA et al.** *Methods Enzymol.,* 1992, vol. 217, 286-295
- **HSU et al.** *Carcinogenesis,* 1994, vol. 15, 1657-1662
- **ORITA et al.** *Proc. Natl. Acad. Sci. USA,* 1989, vol. 86, 2766
- **COTTON.** *Mutat. Res,* 1993, vol. 285, 125-144
- **HAYASHI.** *Genet. Anal. Tech. Appl.,* 1992, vol. 9, 73-79
- **KEEN et al.** *Trends Genet.,* 1991, vol. 7, 5
- **MYERS et al.** *Nature,* 1985, vol. 313, 495
- **ROSENBAUM ; REISSNER.** *Biophys. Chem.,* 1987, vol. 265, 12753
- **SAIKI et al.** *Nature,* 1986, vol. 324, 163
- **SAIKI et al.** *Proc. Natl. Acad. Sci. USA,* 1989, vol. 86, 6230
- **GIBBS et al.** *Nucl. Acids Res,* 1989, vol. 17, 2437-2448
- **PROSSNER.** *Tibtech,* 1993, vol. 11, 238
- **GASPARINI et al.** *Mol. Cell Probes,* 1992, vol. 6, 1

- **BARANY.** *Proc. Natl. Acad. Sci. USA,* 1991, vol. 88, 189
- **EICHELBAUM.** *Clin. Exp. Pharmacol. Physiol.,* 1996, vol. 23, 983-985
- **LINDER.** *Clin. Chem.,* 1997, vol. 43, 254-266
- **CAPECCHI.** *Science,* 1989, vol. 244, 1288-1292
- **ALDERBORN et al.** Determination of Single Nucleotide Polymorphisms by Real-time Pyrophosphate DNA Sequencing. *Genome Research,* 2000, vol. 10 (8), 1249-1265
- **MARK F. PITTENGER et al.** Multilineage Potential of Adult Human Mesenchymal Stem Cells. *Science,* 02 April 1999, 143-147
- Antibodies, a Laboratory Manual. Cold Spring Harbor publisher
- **YANG XD et al.** *Cancer Res,* 1999, vol. 59 (6), 1236-43
- **YANG et al.** *Cancer Res,* 1999, vol. 59 (6), 1236-43
- **R. WONG ; D. MYTYCH ; S.JACOBS ; R.BORDENS ; S.SWANSON.** Validation parameters for a novel biosensor assay which simultaneously measures serum concentrations of a humanized monoclonal antibody and detects induced antibodies. *Journal of Immunological Methods,* 1997, vol. 209, 1-15
- **KOHLS ; LAPPI.** *Biotechniques,* 2000, vol. 28 (1), 162-5
- **ALLEN.** *Nat Rev Cancer,* 2002, vol. 2 (10), 750-63
- **PEIRERSZ et al.** The use of monoclonal antibody conjugates for the diagnosis and treatment of cancer. *Immunol. Cell Biol,* 1987, vol. 65, 111-125
- **MOHAMMAD et al.** *Int J Oncol,* 1999, vol. 15 (2), 367-72